# EUROPEAN PATENT APPLICATION

(11) **EP 4 559 933 A1**
(43) Date of publication of application: **28.05.2025**
(21) Application number: 23842433.7
(22) Date of filing: 21.07.2023
(51) Int. Cl.: C07K 16/18, C07K 16/28, A61K 9/00, A61K 39/44, C12N 5/10, C12N 1/21, C12N 15/13, A61K 39/395, A61P 35/00

(54) **MUTANT PROMOTING HOMOLOGOUS PAIRING OF HEAVY AND LIGHT CHAINS OF MULTISPECIFIC ANTIBODY**

(30) Priority: 22.07.2022 CN 202210915171; 17.03.2023 CN 202310261507
(71) Applicant: Innovent Biologics (Suzhou) Co., Ltd., Suzhou, Jiangsu 215123 (CN)
(72) Inventor: YANG, Xiao, Suzhou, Jiangsu 215123 (CN); ISSAFRAS, Hassan, Suzhou, Jiangsu 215123 (CN); GAO, Changshou, Suzhou, Jiangsu 215123 (CN); WANG, Yi, Suzhou, Jiangsu 215123 (CN)
(74) Representative: Sagittarius IP
(86) International application number: PCT/CN2023/108651
(87) International publication number: WO 2024/017371

(57) **Abstract**

The present disclosure relates to a multispecific antibody, such as a bispecific antibody, comprising a mutated amino acid pair capable of promoting homologous pairing of heavy and light chains of the multispecific antibody.

## Description

The present disclosure relates to a multispecific antibody, such as a bispecific antibody, comprising a mutated amino acid pair capable of promoting homologous pairing of heavy and light chains of the multispecific antibody.

### Background of the Invention:

Antibody molecules occupy a dominant position in the current biological therapy market. Bispecific antibodies (bsAbs) are single molecules that simultaneously bind to two different antigens or two different epitopes of the same antigen; they are different to naturally occurring immunoglobulin G (IgG) monospecific antibodies (mAbs). Due to its additional targeting capabilities, bsAb often provide improved clinical benefits in the treatment of complex diseases (such as cancer and immune disorders) and the diseases relate to various cell surface receptors or ligands. Many efforts have been made to engineer mAb into bsAb, resulting in over 60 forms of bsAb. Many bsAbs can be engineered by linking antibody fragments, such as single-chain variable fragments (scFv), antigen-binding fragments (Fab), and heavy chain (VH) and light chain (VL) variable domains. However, these new forms that differ from the conventional IgG structure typically have poorer physiochemical properties, such as low solubility, susceptibility of aggregation, difficulty in large-scale production, poor thermostability, poor pharmacokinetics, and potential immunogenicity. The significant production challenges of bispecific antibodies in terms of quantity, quality, and stability have hindered them from more widespread clinical application and acceptance.

Heterodimeric Fc technology has been developed to assist in the production of bispecific antibodies. For example, Carter et al. employed a "Kih" (Knobs-into-holes) model in modifying some amino acids of antibody heavy chains, and relatively successfully achieved bispecific antibodies (Ridgway, Presta et al. 1996: Carter 2001). They mutated a small amino acid with a side chain on the CH3 region of the first heavy chain of Fc into a large amino acid with a side chain, creating a "knob" (such as T366Y), and mutated particular amino acids on CH3 of the second heavy chain into small amino acids with a side chain, creating a "hole" (Y407T and the like), that is, used spatial conformation complementarity to achieve the formation of a heterodimer (WO9627011). Thereafter, the proportion of the heterodimer (WO1998050431) was increased through methods such as random mutation-phage display. At the same time, a disulfide bond was introduced into the CH3 domain to increase the [proportion of the] heterodimer, but the capability to hinder homodimer formation was still inadequate. Hence, the proportion of the entire heterodimer was approximately 70 - 80%.

Alternatively, strand-exchange engineered domain (SEED) heterogeneity represents another spatial mutation-based design strategy, which employs interchangeable amino acid sequences in IgG and IgA CH3 domains (AG SEED CH3 and GA SEED CH3) to form complementary structures. IgG and IgA CH3 derivatives produce complementary sequences. Hence, two complementary heavy chains may be assembled to produce a heterodimer, thereby eliminating the possibility of producing homodimers due to the lack of complementarity (Muda, M, et al., Therapeutic assessment of SEED: A new engineered antibody platform designed to generate mono- and bispecific antibodies. Protein Eng. Des. Sel. (PEDS), 2011, 24, 447 - 454).

In addition to the spatial mutations proposed earlier, electrostatic interaction has also been widely used to promote heterodimer formation in the H. This method mutates individual amino acids in the CH3 domain of one heavy chain into Lys carrying a positive charge, and replaces individual amino acids in the CH3 of the other heavy chain with Asp or Glu carrying a negative charge. The charged amino acids then readily form heterodimers by electrostatic attraction. Gunasekaran et al. first introduced charged amino acids to form Fc-heterodimeric bispecific antibodies (Gunasekaran,K. et al., Enhancing antibody Fc heterodimer formation through electrostatic steering effects: applications to bispecific molecules and monovalent IgG. J. Biol. Chem., 2010, 285, 19637 - 19646.).

CN104011221B provides a bispecific antibody with a modified polypeptide chain comprising a disulfide bond reshaping mutation in the variable region and constant region. However, this mutation is still inadequate to achieve over 90% complete correct pairing of bispecific antibody expressed by a single cell line. The amino acid pair selected for the disulfide bond reshaping in this patent was not adequately optimized and a part of which is a molecule that cannot form a disulfide bond.

CN109475627A provides a method for preparing an antibody mixture, but it relates to the preparation of a mixture of two types of monospecific antibody, and the charge mutation was mainly introduced into the constant region, which requires a large amount of mutation to be introduced to achieve optimal pairing effects.

Therefore, a new bispecific antibody platform is still required for this field, where the platform requires the introduction of only a few mutations to ensure minimal mismatches during expression on the resulting bispecific antibody, while maintaining affinity and expression level. At the same time, due to the resulting bispecific antibody having fewer mutations, it has lower immunogenicity, and the expression and recombination of antibodies on the bispecific antibody platform is capable of being carried out in a cell line.

### Summary of the Invention

The present disclosure provides a new multispecific antibody (such as a new bispecific antibody) platform, which obtains a multispecific antibody by introducing a charge mutation in the variable region of the bispecific antibody while introducing a disulfide bond reshaping mutation into the CH1/CL region.
(1) Few mismatches, higher proportion of correctly paired molecules;
(2) immunogenicity risk is comparable to natural IgG;
(3) has a similar or the same affinity as the antibody before the introduction of mutation;
(4) has a relatively high expression level, for example, a comparable or higher expression level than the antibody before the introduction of mutation or the monospecific antibody;
(5) is capable of being expressed in a cell line, avoiding expression and in vitro recombination in different cell lines.

At the same time, the position of the mutation and amino acid introduced in the multispecific antibody platform of the present disclosure are relatively conservative. Hence, it is applicable to various multispecific antibodies (bispecific antibodies).

### Description of Drawings:

Figure 1 shows an exemplary bispecific antibody structure (monovalent 1+1 configuration) and a mutation that it may comprise.
Figure 2 shows the proportion of correctly assembled molecules of the mutation combination shown in Table 7, as determined by LC-MS analysis, wherein A: antibody 1 heavy chain; a: antibody 1 light chain; B: antibody 2 heavy chain; b: antibody 2 light chain; AaBb: correctly-paired bsAb;
Figure 2A: LS-MS results of combination 1 in Table 7;
Figure 2B: LC-MS results of combination 2 in Table 7;
Figure 2C: LC-MS results of combination 3 in Table 7.
Figure 3 shows the proportion of correctly assembled molecules after the introduction of disulfide bond reshaping and charge mutation into different antibodies as shown in Table 8, as determined by LC-MS analysis, wherein A: antibody 1 heavy chain; a: antibody 1 light chain; B: antibody 2 heavy chain; b: antibody 2 light chain; AaBb: correctly paired bsAb.
Figure 3A: LC-MS results of Her2/PD-1 combination in Table 8.
Figure 3B: LC-MS results of Her2/CD47 combination in Table 8.
Figure 3C: LC-MS results of Her2/cMET combination in Table 8.
Figure 4 shows the proportion of correctly assembled molecules after the introduction of different charge mutations and the same disulfide bond reshaping mutation into different antibodies as shown in Table 9, as determined by LC-MS analysis, wherein A: antibody 1 heavy chain; a: antibody 1 light chain; B: antibody 2 heavy chain; b: antibody 2 light chain; AaBb: correctly paired bsAb.
Figure 4A: LC-MS results of combination 1 in Table 9.
Figure 4B: LC-MS results of combination 2 in Table 9.
Figure 4C: LC-MS results of combination 3 in Table 9.
Figure 4D: LC-MS results of combination 4 in Table 9.
Figure 4E: LC-MS results of combination 5 in Table 9.
Figure 5 shows the configuration of three types of bispecific antibodies, wherein Figure 5A shows a bispecific antibody with a 2+1N-terminal configuration; Figure 5B shows a bispecific antibody with a 2+1C-terminal configuration; Figure 5C shows a bispecific antibody with a 2+2C-terminal configuration.
Figure 6 shows the proportion of correctly assembled molecules applying the multivalent configuration molecule constructed by the present disclosure shown in Table 11, as determined by LC-MS analysis, wherein HC1: antibody 1 heavy chain; LC1: antibody 1 light chain; HC2: antibody 2 heavy chain; LC2: antibody 2 light chain; Intact: correctly paired multivalent bsAb.
Figure 6A: LC-MS results of GPRC5D/CD3-1 in Table 11.
Figure 6B: LC-MS results of GPRC5D/CD3-2 in Table 11.
Figure 6C: LC-MS results of GPRC5D/CD3-3 in Table 11.
Figure 6D: LC-MS results of GPRC5D/CD3-4 in Table 11.
Figure 6E: LC-MS results of GPRC5D/CD3-5 in Table 11.
Figure 6F: LC-MS results of GPRC5D/CD3-6 in Table 11.
Figure 7A and Figure 7B show examples of charge and disulfide bond reshaping mutation introduced into a BCMA/GPRC5D/CD3 trispecific antibody.
Figure 8 shows the killing effect of PBMC on H929 cells as mediated by an example antibody, wherein Figure 8A shows the killing effect of BCMA-positive H929 cells by PBMC as mediated by an example antibody, and Figure 8B shows the killing effect of GPRC5D-positive H929 cells by PBMC as mediated by an example antibody.
Figures 9A - C show non-reduced polyacrylamide gel electrophoresis results after purification of different mutant mAb; Figures 9D - M show denaturing non-reduced capillary electrophoresis (nrCE-SDS) results after the formation of a disulfide bond antibody.

### Definition:

The present disclosure should be understood as not being limited to the particular methodologies, solutions, and reagents described herein, because these may be changed. The terms used herein should be understood to describe the specific embodiments only and are not intended to limit the scope of the present disclosure; they are only limited by the attached Claims. Unless otherwise defined, all technical and scientific terms used herein have the same meaning as those typically understood by those of ordinary skill in the art of the present disclosure.

The following definitions will be used to explain the Specification. As long as appropriate, terms used in singular form may further include the plural form, vice versa. It should be understood that the terms used herein are to describe the specific embodiments, and are not intended to be limitative.

The term "approximately" when used in combination with a numerical value mean to cover numerical values within the range of the lower limit 5% smaller than the specified numerical value and the upper limit 5% greater than the specified numerical value.

As used herein, the term "and/or' mean any option among the options or two or more or all of the options.

When "first" and "second" are mentioned herein, they are for the purpose of differentiating two domains or chains and not to indicate the position of the two domains in any way.

As used herein, the terms "comprise" and "include" mean to include all elements, integers, or steps, but do not exclude any other elements, integers, or steps. When the terms "comprise" and "include" are used herein, unless otherwise specified, they also cover a combination of the elements, integers, or steps. For example, when "comprising" the antibody variable region of a particular specific sequence is mentioned, it also covers the variable region of the antibody formed by the specific sequence.

As used herein, all the amino acid positions in the variable region of the heavy and light chains are numbered according to the Kabat numbering scheme described by Kabat et al., Sequences of Proteins of Immunological Interest, Version 5. Public Health Service, National Institutes of Health, Bethesda, MD (1991) and referred to as "Kabat number" herein.

As used herein, amino acid positions in domains outside of the antibody variable region mentioned (for example, constant region, for example, Fc region) are numbered according to the EU numbering scheme described in Edelman, G.M. et al., Proc. Natl. Acad. USA, 63, 78-85 (1969) and referred to as "EU number" herein. When the position number and/or amino acid residue is assigned to a particular antibody isotype, it is applicable to the corresponding position and/or amino acid residue of any other antibody isotype, and this is known to skilled artisans in the art.

General information of the nucleotide sequence of light and heavy chains of immunoglobulin are provided in Kabat, E.A. et al., Sequences of Proteins of Immunological Interest, Version 5, Public Health Service, National Institutes of Health, Bethesda, MD (1991).

The term "amino acid substitution" or "amino acid mutation" refers to replacing at least one amino acid residue in a pre-determined parental amino acid sequence with a different "substituent" amino acid residue. This substituent residue or plurality of residues may be "naturally occurring amino acid residues" (that is, coded by genetic code) and selected from: alanine (A1a); arginine (Arg); asparagine (Asn); aspartate (Asp); cysteine (Cys); glutamine (Gln); glutamic acid (Glu); glycine (Gly); histidine (His); isoleucine (Ile); leucine (Leu); lysine (Lys); methionine (Met); phenylalanine (Phe); proline (Pro); serine (Ser); threonine (Thr); tryptophan (Trp); tyrosine (Tyr); and valine (Val). The definition of amino acid substitution herein also covers replacement with one or a plurality of non-naturally occurring amino acid residues. "Non-naturally occurring amino acid residues" refer to residues that, aside from those naturally occurring amino acid residues mentioned above, are capable of covalently bonding to adjacent amino acid residues in the polypeptide chain. Examples of non-naturally occurring amino acid residues include norleucine, ornithine, norvaline, homoserine, Aib and other amino acid residue analogs.

The term "CH1 region" refers to the part extending from EU position 118 to EU position 220 (EU numbering scheme) in the antibody heavy chain polypeptide. In one embodiment, the CH1 domain comprises the amino acid sequence ASTKGPSVFPLAPSSKSTSGGTAALGCLVKDYFPEPVTVSWNSGALTSGVHTFPAVLQSSGLYSLSSVVTVP SSSLGTQTYICNVNHKPSNTKVDKKVEPKSC (SEQ ID NO:116). In one embodiment, a internal disulfide bond is removed by mutating Cys in CH1 into non-Cys or a new disulfide bond is reshaped by mutating non-Cys into Cys.

The term "CH2 region" refers to the part extending from EU position 231 to EU position 340 (EU numbering scheme) in the antibody heavy chain polypeptide. In one embodiment, the CH2 domain comprisesthe amino acid sequence

APELLGGPSVFLFPPKPKDTLMISRTPEVTCVVVDVSHEDPEVKFNWYVDGVEVHNAKTKPREEQYNSTY

RVVSVLTVLHQDWLNGKEYKCKVSNKALPAPIEKTISKAK (SEQ ID NO:117).

The term "CH3 region" refers to the part extending from EU position 341 to EU position 447 in the antibody heavy chain polypeptide. In one embodiment, the CH3 domain comprises the amino acid sequence

GQPREPQVYTLPPSRDELTKNQVSLTCLVKGFYPSDIAVEWESNGQPENNYKTTPPVLDSDGSFFLYSKLTV
DKSRWQQGNVFSCSVMHEALHNHYTQKSLSLSPGK (SEQ ID NO:118).

The term "hinge region" refers to the heavy chain polypeptide part of the antibody, and this part links the CH1 region and the CH2 region in the heavy chain of the wild-type antibody, for example, the IgG1 hinge region, for example, the sequence of D221 to P230 according to EU numbering. The hinge region of other IgG subtypes may be identified by aligning the Cys residue in the hinge region of the IgG1 subtype sequence.

The hinge region is typically a dimer molecule formed by two polypeptides with the same amino acid sequence. In one embodiment, the hinge region has the amino acid sequence DKTHTCPXCP (SEQ ID NO:119), wherein X is S or P. In one embodiment, the hinge region comprises the amino acid sequence HTCPXCP (SEQ ID NO:120), wherein X is S or P. In one embodiment, the hinge region comprises the amino acid sequence CPXCP (SEQ ID NO:121), wherein X is S or P. In one embodiment, the disulfide bond in the hinge region is removed by mutating Cys in the hinge region into non-Cys.

The multispecific antibody in the present disclosure may comprise a linker. The term "linker" used herein refers to any molecule that is capable of directly linking different parts of the multispecific antibody. Examples of linkers that establish covalent links between different parts of the multispecific antibody include peptide linkers and non-protein polymers, including but are not limited to polyethylene glycol (PEG), polypropylene glycol, or copolymers of polyoxyethylene or polyethylene glycol, or polypropylene glycol. In some embodiments, the term "peptide linker" according to the present disclosure refers to an amino acid sequence, wherein the sequence links amino acid sequences of various parts of the multispecific antibody together. Preferably, the peptide linker has a length adequate to link two entities in a way that maintains their conformation relative to each other, to prevent hindering the desired activity. The peptide linker may predominantly include or not predominantly include the following amino acid residues: Gly, Ser, Ala or Thr. Useful linkers include Gly-Ser polymers, including for example, (GS)n, (GSGGS) n, (GGGGS) n, (GGGS)n and (GGGGS)nG, wherein n is an integer of at least 1 (and preferably, 2, 3, 4, 5, 6, 7, 8, 9, 10). Useful linkers also include Gly-Ala polymers, Ala-Ser polymers and other flexible linkers.

The term "Fc domain" or "Fc region" is used herein to define the C-terminal region of at least one part of the constant region contained in the immunoglobulin H. This term includes natural sequence Fc region and variant Fc region. The natural immunoglobulin "Fc domain" comprises two or three constant domains, that is, the CH2 domain, CH3 domain, and optionally the CH4 domain. For example, in natural antibodies, the immunoglobulin Fc domain comprises the second and third constant domains (CH2 domain and CH3 domain) of two heavy chains derived from IgG, IgA, and IgD antibodies; or comprises the second, third, and fourth constant domains (CH2 domain, CH3 domain, and CH4 domain) of two heavy chains derived from IgM and IgE antibodies. Unless otherwise specified herein, the amino acid residue numbers in the Fc region or heavy chain constant region are numbered according to the EU numbering scheme (also referred to as EU index) in Kabat et al., Sequences of Proteins of Immunological Interest, Version 5, Public Health Service, National Institutes of Health, Bethesda, MD, 1991. However, the C-terminal Lys (Lys447) in the Fc region may or may not exist. The two Fc regions may undergo dimerization to constitute a dimeric Fc and two different Fc [regions] may undergo heterodimerization to form a heterodimer Fc. The term "Fc region", "Fc part" and "dimeric Fc (for example, heterodimeric Fc)" herein does not include the heavy chain variable region VH and light chain variable region VL, as well as the heavy chain constant region CH1 and light chain constant region CL of the immunoglobulin, but may include the N-terminal hinge region of the heavy chain constant region in some cases. In one embodiment, a human IgG heavy chain Fc region extends from Asp221, Cys226, or Asp231 to the C-terminus of the H.

In one embodiment, a human IgG1 Fc region polypeptide (comprising the hinge region) comprises the following amino acid sequence:

DKTHTCPPCPAPELLGGPSVFLFPPKPKDTLMISRTPEVTCVVVDVSHEDPEVKFNWYVDGVEVHNAKTK
PREEQYNSTYRVVSVLTVLHQDWLNGKEYKCKVSNKALPAPIEKTISKAKGQPREPQVYTLPPSRDELTKN

QVSLTCLVKGFYPSDIAVEWESNGQPENNYKTTPPVLDSDGSFFLYSKLTVDKSRWQQGNVFSCSVMHEA
LHNHYTQKSLSLSPGK (SEQ ID NO:122).

The human IgG4 Fc region polypeptide (comprising the hinge region) comprises the following amino acid sequence:

PPCPSCPAPEFLGGPSVFLFPPKPKDTLMISRTPEVTCVVVDVSQEDPEVQFNWYVDGVEVHNAKTKPRE
EQFNSTYRVVSVLTVLHQDWLNGKEYKCKVSNKGLPSSIEKTISKAKGQPREPQVYTLPPSQEEMTKNQV
SLTCLVKGFYPSDIAVEWESNGQPENNYKTTPPVLDSDGSFFLYSRLTVDKSRWQEGNVFSCSVMHEALH
NHYTQKSLSLSLGK (SEQ ID NO:123).

The antibody produced in the method reported herein comprises a Fc region and in one embodiment, the Fc region is derived from a human-derived Fc region. In one embodiment, the Fc region comprises all the parts of the human constant region. The antibody Fc region is directly involved in complement activation, C1q binding, C3 activation and Fc receptor binding. In one embodiment, the Fc region is a human Fc region. In one embodiment, the Fc region is a human IgG4 subtype. In one embodiment, the Fc region is a human IgG1 subtype.

"Heterodimeric Fc scaffold" herein refers to a scaffold comprising two different Fc regions or two different Fc regions formed after dimerization. It may be linked to an antigen-binding domain at the N-terminus or C-terminus thereof (for example, to the heavy chain and/or light chain variable region of the antibody that is capable of binding to the target molecule, the antigen-binding fragment of the antibody, or the soluble part of the ligand or receptor that is capable of binding to the target molecule) and is used to construct a multispecific antibody, for example, bispecific antibody.

Amino acid mutation is expressed using (original amino acid, amino acid position, mutated amino acid). For example, when the mutation site is located in the V region, "A43D" refers to the substitution of Ala amino acid located at Kabat position 43 with Asp (D); when the mutation site is located in the C region, "Q124C" refers to the substitution of Gln located at EU position 124 with Cys (C). When mentioning the mutation combination, the mutations in the combination are connected using a plus sign (+). "A43D+Q124C" represents that the combination simultaneously comprises mutations A43D and Q124C. Where there are a plurality of mutation possibilities at a particular position, it is expressed by the symbol "/" herein. For example, "K370T/S mutation" represents that the residue K at position 370 may be substituted with a T or S residue.

Where the amino acid position is mentioned in the present disclosure, unless otherwise specified, it refers to the amino acid position according to the IgG1 heavy chain or kappa light chain number, that is, it covers the amino acid position based on the IgG1 heavy chain or Kappa light chain number and the amino acid position of the amino acid position corresponding to other heavy or light chains. For example, when C220 is mentioned, it covers the amino acid of an IgG1 heavy chain at position 220 under EU numbering, and the corresponding amino acid in other heavy chains. For example, the amino acid at position 131 in IgG2, IgG3, or IgG4.

It should be noted that when describing mutations, the original amino acid at a specific position may be the described amino acid or may be another amino acid at the corresponding position. For example, it should be noted that when describing mutations, it may include situations where Ala at position 43 of the variable region is mutated to Asp, and where Ala does not correspond to position 43, the corresponding amino acid at position 43 is mutated to Asp, as long as the original amino acid corresponds to position 43 of the variable region. The terms "host cell", "host cell line" and "host cell culture product" are used interchangeably and refer to cells introduced into foreign nucleic acid, including the offspring of such cells. Host cells include "transformants" and "transformed cells", which include primary transformed cells and offspring derived therein, regardless of the passage number. Offspring may not be identical to the parental cells in terms of nucleic acid content, and may even contain mutations. Such mutated offspring are included herein and have similar functions or biological activity screened or selected in the initial transformed cells.

The term "vector" used herein refers to a nucleic acid molecule capable of proliferating another nucleic acid that is linked thereto. This term includes vectors with a self-replicating nucleic acid structure and vectors bound to the genome of the host cell introduced therein. Some vectors are capable of guiding the expression of the nucleic acid they are operably linked to. Such vectors are referred to as "expression vectors" herein.

The term "binding molecule" refers to any molecule that is capable of specifically binding to a target, for example, an antibody or the antigen-binding fragment or fusion protein thereof.

The term "target" refers to the object to be bound by the binding molecule. The target may be an antigen, and may also be a ligand or receptor. The term "antigen" refers to a molecule that induces an immune response. Such immune response may relate to antibody production or specific immune cell activation, or both. Skilled artisans should understand that any macromolecules, including basically all proteins or peptides, may be used as antigens. In addition, antigens may be derived from recombinant or genomic DNA. As used herein, the term "epitope" refers to the part in the antigen with specific interaction with the antibody molecule. In some embodiments, the antigen is a tumor-associated antigen (that is, the antigen related to tumor onset and progression) or a T cell engager. When the binding molecule in the present disclosure relates to a target-binding region derived from the antibody, "target" and "antigen" may be used interchangeably.

The term "target-binding region" used herein refers to a binding molecule, for example, the part of a multispecific binding molecule or bispecific binding molecule bound to a particular target or antigen. The target-binding region may be, for example, an antibody, immunoglobulin or antibody fragment. Such target-binding region may or may not have a tertiary structure independent to the remaining part of the binding molecule, and may be used as an independent entity to bind or not to bind to the target thereof. The target-binding region may also be a receptor or ligand, or a domain of a receptor that is capable of binding to a ligand. The "target-binding region" of a multispecific antibody or bispecific antibody may also be referred to as an "antigen-binding region".

The term "antigen-binding region" used herein refers to an antibody or the antigen-binding fragment thereof, for example, any part of a multispecific antibody or bispecific antibody bound to a particular target or antigen. The antigen-binding region may be, for example, an antibody, immunoglobulin or antibody fragment. Such antigen-binding region may or may not have a tertiary structure independent to the remaining part of a multispecific antibody or bispecific antibody, and may be used as an independent entity to bind or not to bind to the antigen/epitope thereof. Where the binding molecule in the present disclosure relates to a target-binding region derived from an antibody, "target-binding region" and "antigen-binding region" may be used interchangeably.

The term "multispecific binding molecule" refers to a multispecific binding molecule that is at least bispecific, for example, a bispecific binding molecule, that is, the molecule comprises at least a first target-binding region and a second target-binding region, wherein the first target-binding region binds to a type of target or antigen and the second target-binding region binds to another antigen or target. The multispecific binding molecule according to the present disclosure also covers a multispecific molecule comprising a plurality of target-binding regions/binding sites, such as a trispecific binding molecule. In some embodiments, the multispecific binding molecule of the present disclosure is a multispecific antibody. In some embodiments, the bispecific binding molecule of the present disclosure is a bispecific antibody. In some embodiments, the multispecific binding molecule of the present disclosure is a fusion protein, which may comprise, for example, a target-binding region from an antibody and a target-binding region from a receptor or ligand.

As used herein, the term "monospecific" refers to a polypeptide/protein molecule having one or a plurality of target binding sites, and each site among the sites binds to the same site or structure of the same target, or the same epitope of the same antigen.

As used herein, the term "multispecific" binding molecule, for example, an antigen, refers to there being at least two target binding sites, and each target binding site among the at least two target binding sites binds to a different site/structure of the same target or a different target. The multispecific binding molecule is a binding molecule with binding specificity to at least two different targets or sites/structures. In one embodiment, such a bispecific binding molecule is provided herein, and it has binding specificity to a first target and a second target.

As used herein, the term "multispecific" antibody refers to an antibody having at least two antigen binding sites, and each antigen binding site among the at least two antigen binding sites binds to different epitopes of the same antigen or different epitopes of different antigens. The multispecific antibody is an antibody with binding specificity to at least two different antigens/epitopes. In one embodiment, such bispecific antibody is provided herein, and it has binding specificity to a first antigen and a second antigen.

Where the "first antigen-binding region" is mentioned in terms of multispecific antibody or bispecific antibody, it refers to the binding region for binding with the first antigen, and does not intend to limit the number of the antigen-binding regions contained in the antibody. For example, the multispecific antibody or bispecific antibody may comprise one or more than one first antigen-binding regions. For example, the bispecific antibody comprises the first antigen-binding region and the second antigen-binding region, but may comprise one or more than one first antigen-binding regions and one or more than one second antigen-binding regions.

Where the "first target-binding region" is mentioned in terms of binding molecule, it refers to the binding region for binding with the first target, and does not intend to limit the number of the target-binding regions contained in the binding molecule. For example, the multispecific binding molecule or bispecific antibody may comprise one or more than one first target-binding regions. For example, the bispecific antibody comprises the first target-binding region and the second target-binding region, but may comprise one or more than one first target-binding regions and one or more than one second target-binding regions.

Where the "first antigen-binding region" is mentioned in terms of antibody, it refers to the binding region for binding with the first antigen, and does not intend to limit the number of the antigen-binding regions contained in the antibody. For example, the multispecific antibody or bispecific antibody may comprise one or more than one first antigen-binding regions. For example, the bispecific antibody comprises the first antigen-binding region and the second antigen-binding region, but may comprise one or more than one first antigen-binding regions and one or more than one second antigen-binding regions.

Where "target or antigen-binding region is derived from an antibody" is mentioned, it refers to the binding domain constituting the target/antigen-binding region being from or derived from a specific antigen-binding domain of the antibody. For example, a specific Fab of the antigen-binding region being from or derived from the corresponding fragment of the antibody, such as Fab, or a heavy chain variable region and/or light chain variable region of the antigen-binding region being from or derived from the heavy chain variable region and/or light chain variable region of the antibody, or one, two, three, four, five, or six CDRs of the antigen-binding region being the CDRs of the antibody.

The term "derived from" refers to the fragment in the antigen-binding region being basically the same as the antibody fragment from which it is derived, but with a mutation, for example, substitution, deletion, or addition, at one or a plurality of sites. In one specific embodiment, the mutation is not in the CDR of the antibody.

The term "whole antibody" or "full-length antibody" may be used interchangeably herein and refers to an antibody molecule with a natural immunoglobulin molecular structure. A full-length conventional four-chain IgG antibody comprises two heavy chains (H) and two light chains (L) linked to each other by a disulfide bond. A full-length heavy chain antibody that only has heavy chain and lacks light chain comprises two heavy chains linked to each other by a disulfide bond. The heavy chain of a full-length conventional four-chain IgG antibody typically composes of a heavy chain variable region (abbreviated as VH herein) and heavy chain constant region, wherein the heavy chain constant region comprises at least three domains, namely CH1, CH2, and CH3. The light chain of a full-length antibody is composed of a light chain variable region (abbreviated as VL herein) and CL, wherein the CL is composed of a domain CL. Each heavy chain variable region VH and each light chain variable region VL is composed of three CDRs and four FRs, arranged in the following order from the N-terminus to the C-terminus: FR1, CDR1, FR2, CDR2, FR3, CDR3, FR4. The term "antibody fragment" includes a part of a complete antibody. In a preferred embodiment, the antibody fragment is an antigen-binding fragment.

The term "antigen-binding fragment" of the antibody is a molecule different from the full-length antibody, comprising a part of the full-length antibody but is an antigen that is capable of binding to the full-length antibody or an antigen that competitively binds to the full-length antibody (that is, the full-length antibody from which the antigen-binding fragment is derived). The antigen-binding fragment of the complete antibody may be prepared through recombinant DNA technology or through enzymatic or chemical cleavage. The antigen-binding fragment includes but is not limited to Fab, Fab', F(ab')2, Fv, single-strand Fv, diabody, single-domain antibody (sdAb), and nanobody. For example, a Fab fragment may be obtained from papain digestion of the full-length antibody. In addition, F(ab')2 is produced from pepsin digestion of the whole antibody below the disulfide bond in the hinge region. F(ab')2 is a dimer of Fab' and is a divalent antibody fragment. F(ab')2 may be reduced under neutral conditions by destroying the disulfide bond in the hinge region, transforming the F(ab')2 dimer into a Fab' monomer. The Fab' monomer is basically a Fab fragment with a hinge region. The Fv fragment is composed of one-armed VL and VH domains of the antibody. The two domains of the Fv fragment, namely VL and VH, may be independently genetically coded, but may also be recombined and these two domains may be linked using a synthetic linking peptide into a single protein chain, with the VL region and VH region in the single protein chain paired to form a single-chain Fv (scFv).

"Fab fragment" or "Fab" may be used interchangeably herein and is used to refer to an immunoglobulin fragment composed of two polypeptide chains, comprising an immunoglobulin heavy chain variable domain VH, heavy chain constant domain CH1, light chain variable domain VL, and light chain constant domain CL, wherein one polypeptide chain comprises VH and one constant region selected from CH1 and CL from the N-terminus to the C-terminus, and the other polypeptide chain comprises VL and another constant region selected from CL and CH1 from the N-terminus to the C-terminus, wherein the VH domain and VL domain are paired to form an antigen binding site. The Fab polypeptide chain comprising the heavy chain constant region CH1 herein is also referred to as "Fab heavy chain"; correspondingly, the Fab polypeptide chain comprising the light chain constant region CL is also referred to as "Fab light chain".

The "complementarity-determining region" or "CDR" are regions within the variable domain of an antibody that are highly variable in sequence and form structurally defined loops ("hypervariable loops") and/or contain antigen-contacting residues ("antigen contact points"). The CDR is mainly responsible for binding with antigens/epitopes. The CDRs of Heavy chain and light chain are typically referred to as CDR1, CDR2, and CDR3, and numbered starting from the N-terminus. CDRs located in the antibody heavy chain variable domain are referred to as HCDR1, HCDR2, and HCDR3, while CDRs located in the antibody light chain variable domain are referred to as LCDR1, LCDR2, and LCDR3. In a given light chain variable region or heavy chain variable region amino acid sequence, the precise amino acid sequence boundaries of each CDR may be determined using any or a combination of various known antibody CDR assignment schemes, which include, for example, CDR defined by Chothia (Chothia et al. (1989) Nature 342: 877-883, Al-Lazikani et al., Standard conformations for the canonical structures of immunoglobulins", Journal of Molecular Biology, 273, 927-948 (1997)) based on antibody three-dimensional structure and CDR loop topology, Kabat (Kabat et al., Sequences of Proteins of Immunological Interest, Version 4, U.S. Department of Health and Human Services, National Institutes of Health (1987)), AbM (University of Bath), Contact (University College London), international ImMunoGeneTics database (IMGT) (imgt.cines.fr/ on the World Wide Web) based on antibody sequence variability, and North based on affinity propagation clustering using a large number of crystal structures. Unless otherwise specified, the term "CDR" or "CDR sequence" in the present disclosure covers CDR sequences identified by any of the above methods. CDR may also be identified based on reference CDR sequences with the same Kabat number position (such as any exemplary CDR of the present disclosure).

Sequence identity between sequences is calculated as follows.

In order to determine the identity percentage of two amino acid sequences or two nucleic acid sequences, the sequences are aligned for optimal comparison purposes (for example, an empty space may be introduced into one or both of the first and second amino acid sequences or nucleic acid sequences for optimal comparison or non-homologous sequences may be discarded for comparison purposes). In a preferred embodiment, for comparison purposes, the length of the compared reference sequence is at least 30%, preferably at least 40%, more preferably at least 50%, 60%, and even more preferably at least 70%, 80%, 90%, or 100% of the reference sequence length. Thereafter, the amino acid residue or nucleotide at the corresponding amino acid position or nucleotide position is compared. When the position in the first sequence is occupied by the same amino acid residue or nucleotide at the corresponding position in the second sequence, the molecule at this position is the same.

### Detailed Description of the Invention

### I. Binding molecule

The present disclosure relates to a type of binding molecule comprising a first target-binding region and a second target-binding region, and optionally one or a plurality of other target-binding regions, wherein one or two or more of the target-binding regions comprise one or a plurality of mutations favorable for preventing mismatch and/or promoting heterodimerization. In some embodiments, the binding molecule also comprises one or a plurality of mutations for promoting heterodimer purification. In some embodiments, the mutation that the binding molecule of the present disclosure comprises is selected from disulfide bond reshaping mutation, charge mutation and/or one or a plurality of other mutations favorable for preventing mismatch and/or promoting heterodimerization. In some embodiments, the first target-binding region and the second target-binding region bind to the same target or site/structure on the target. In some embodiments, the first target-binding region and the second target-binding region bind to different targets or sites/structures on the target.

In some embodiments, the binding molecule of the present disclosure is a fusion protein, which may comprise, for example, a target-binding region from an antibody and a target-binding region from a receptor or ligand. In some embodiments, exemplary examples of the ligand or receptor include granule-associated cytokines or receptors thereof, such as tumor necrosis factor (TNF), vascular endothelial growth factor (VEGF) or transforming growth factor (TGF, for example, TGF-β) or receptors thereof, as well as fragments thereof.

In some embodiments, the binding molecule is an antibody. In some embodiments, the present disclosure relates to an antibody comprising a mutation, for example, a monospecific antibody or multispecific antibody, for example, a bispecific antibody, comprising a first antigen-binding region and a second antigen-binding region, and optionally one or a plurality of other antigen-binding regions, wherein one or two or more of the antigen-binding regions comprise one or a plurality of mutations favorable for preventing mismatch and/or promoting heterodimerization. In some embodiments, the antibody also comprises one or a plurality of mutations for promoting heterodimer purification. In some embodiments, the mutation that the binding molecule of the present disclosure comprises is selected from disulfide bond reshaping mutation, charge mutation and/or one or a plurality of other mutations favorable for preventing mismatch and/or promoting heterodimerization. In some embodiments, the first antigen-binding region and the second antigen-binding region bind to the same antigen or epitope. In some embodiments, the first antigen-binding region and the second antigen-binding region bind to different antigens or epitopes.

In some embodiments, the target-binding region or antigen-binding region comprises a heavy chain variable region and a light chain variable region. In some embodiments, the target-binding region or antigen-binding region comprises a light chain constant region CL. In some embodiments, the target-binding region or antigen-binding region comprises a heavy chain constant region CH1.

In some embodiments, the CH1 region is a human IgG CH1, for example, human IgG1 CH1, human IgG2 CH1, human IgG3 CH1, or human IgG4 CH1. In one embodiment, the CH1 region comprises the amino acid sequence SEQ ID NO:116, or has at least 90% identity with it, for example, an amino acid sequence with 95%, 96%, 97%, 99%, or 100% identity or is composed thereof.

In some embodiments, the CL region is a human Kappa light chain CL region or human Lambda light chain CL region. In some embodiments, the CL region comprises the amino acid sequence SEQ ID NO:54 or SEQ ID NO:55, or has at least 90% identity with it, for example, an amino acid sequence with 95%, 96%, 97%, 99%, or 100% identity or is composed thereof.

In the present disclosure, the limitations of the antigen-binding region are similarly applicable to the target-binding region.

### I-1 Disulfide bond reshaping mutation

Disulfide bond reshaping mutation is an effective strategy to force homologous light chain and heavy chain pairing, thereby preventing mismatch between light chain and heavy chain of different target-binding regions of the binding molecule or multispecific antibody. The disulfide bond linking the light chain and heavy chain or one or a plurality of target-binding regions is reshaped and the position of the disulfide bond is changed such that mismatched light chain and heavy chain cannot effectively form a disulfide bond.

Therefore, in one embodiment, in the binding molecule of the present disclosure, for example, an antibody, at least one target-binding region or antigen-binding region comprises CH1-CL, and the disulfide bond at the CH1-CL interface is reshaped, for example, a disulfide bond reshaping mutation is present on the CH1-CL interface. In one embodiment, in the binding molecule of the present disclosure, for example, an antibody, two or more target-binding regions comprise a disulfide bond reshaping mutation at the CH1-CL1 interface.

The "disulfide bond reshaping mutation" herein refers to a mutation at the position of an amino acid pair at the interface, wherein non-Cys at the position of the amino acid pair is substituted with Cys. For example, two non-Cys at the position of the amino acid pair is substituted with Cys, allowing for the formation of an interchain disulfide bond.

In some embodiments, the disulfide bond reshaping mutation of the present disclosure is a mutation at the position of an amino acid pair at the CH1-CL interface of an antigen-binding region (for example, the antigen-binding region comprising Fab), wherein non-Cys at the position of the amino acid pair is substituted with Cys. In some embodiments, two non-Cys at the position of the amino acid pair is substituted with Cys, allowing for the formation of an interchain disulfide bond.

In some embodiments, the mutation refers to amino acids in a non-Cys amino acid pair, where the distance between α carbon atoms at the CH1-CL interface is less than 8Å, and more preferably less than 6Å, being mutated to Cys. In some embodiments, the amino acid side chains in the amino acid pair point towards each other. In some embodiments, amino acids in a non-Cys amino acid pair, where the distance between α carbon atoms at the CH1-CL interface is less than 8Å, and more preferably less than 6Å, are mutated to Cys, and the amino acid side chains in the non-Cys amino acid pair point towards each other.

In some embodiments, a non-Cys amino acid in the heavy chain CH1 in the antigen-binding region (for example, IgG1, IgG2, IgG3, or IgG4) is substituted with a Cys amino acid at positions 126, 128, 134, 136, 139, 168, 170, 171, or 173 (EU number). In some embodiments, a non-Cys amino acid in the light chain CL in the antigen-binding region is substituted with a Cys amino acid at positions 114, 116, 118, 124, 160, 162, or 164 (EU number).

In some embodiments, a non-Cys amino acid in the heavy chain CH1 at the CH1-CL [interface] of the antigen-binding region (for example, IgG1, IgG2, IgG3, or IgG4) is substituted with a Cys amino acid at position 126 (EU number), and a non-Cys amino acid in the light chain CL is substituted with a Cys amino acid at position 124 (EU number).

In some embodiments, a non-Cys amino acid in the heavy chain CH1 at the CH1-CL [interface] of the antigen-binding region (for example, IgG1, IgG2, IgG3, or IgG4) is substituted with a Cys amino acid at position 168 (EU number), and a non-Cys amino acid in the light chain CL is substituted with a Cys amino acid at position 164 (EU number).

In some embodiments, a non-Cys amino acid in the heavy chain CH1 at the CH1-CL [interface] of the antigen-binding region (for example, IgG1, IgG2, IgG3, or IgG4) is substituted with a Cys amino acid at position 170 (EU number), and a non-Cys amino acid in the light chain CL is substituted with a Cys amino acid at position 162 or 164 (EU number).

In some embodiments, a non-Cys amino acid in the heavy chain CH1 at the CH1-CL [interface] of the antigen-binding region (for example, IgG1, IgG2, IgG3, or IgG4) is substituted with a Cys amino acid at position 173 (EU number), and a non-Cys amino acid in the light chain CL is substituted with a Cys amino acid at position 160 or 162 (EU number).

In some embodiments, a non-Cys amino acid in the heavy chain CH1 at the CH1-CL [interface] of the antigen-binding region (for example, IgG1, IgG2, IgG3, or IgG4) is substituted with a Cys amino acid at position 128 (EU number), and a non-Cys amino acid in the light chain CL is substituted with a Cys amino acid at position 118 (EU number).

In some embodiments, a non-Cys amino acid in the heavy chain CH1 at the CH1-CL [interface] of the antigen-binding region (for example, IgG1, IgG2, IgG3, or IgG4) is substituted with a Cys amino acid at position 134 (EU number), and a non-Cys amino acid in the light chain CL is substituted with a Cys amino acid at position 116 (EU number).

In some embodiments, a non-Cys amino acid in the chain CH1 at the CH1-CL [interface] of the antigen-binding region (for example, IgG1, IgG2, IgG3, or IgG4) is substituted with a Cys amino acid at position 136 (EU number), and a non-Cys amino acid in the chain CL is substituted with a Cys amino acid at position 114 (EU number).

In some embodiments, a non-Cys amino acid in the heavy chain CH1 at the CH1-CL [interface] of the antigen-binding region (for example, IgG1, IgG2, IgG3, or IgG4) is substituted with a Cys amino acid at position 171 (EU number), and a non-Cys amino acid in the light chain CL is substituted with a Cys amino acid at position 162 (EU number).

In some embodiments, a non-Cys amino acid in the heavy chain CH1 at the CH1-CL [interface] of the antigen-binding region (for example, IgG1, IgG2, IgG3, or IgG4) is substituted with a Cys amino acid at position 139 (EU number), and a non-Cys amino acid in the light chain CL is substituted with a Cys amino acid at position 116 (EU number).

Non-Cys amino acids, in some aspects, include naturally occurring and/or non-classical amino acids. Naturally occurring non-Cys amino acids include Gly, Ala, Val, Leu, Ile, Pro, Ser, Thr, Met, His, Lys, Arg, Glu, Asp, Gln, Asp, Phe, Tyr, and Trp. Non-classical amino acids may sometimes be incorporated through cell expression systems (for example, prokaryotic cell and/or eukaryotic cell expression systems). Examples of non-classical amino acids include ornithine, diaminobutyric acid, norleucine, pyrylalanine, thienylalanine, naphthylalanine and phenylglycine.

In some embodiments, the disulfide bond reshaping mutation comprises F126C in CH1 of the antigen-binding region and comprises Q124C (EU number) in CL.

In some embodiments, the disulfide bond reshaping mutation comprises H168C in CH1 of the antigen-binding region and comprises T164C (EU number) in CL.

In some embodiments, the disulfide bond reshaping mutation comprises HF170C in CH1 of the antigen-binding region and comprises T164C (EU number) in CL.

In some embodiments, the disulfide bond reshaping mutation comprises HF170C in CH1 of the antigen-binding region and comprises S162C (EU number) in CL.

In some embodiments, the disulfide bond reshaping mutation comprises V173C in CH1 of the antigen-binding region and comprises S162C (EU number) in CL.

In some embodiments, the disulfide bond reshaping mutation comprises V173C in CH1 of the antigen-binding region and comprises Q160C (EU number) in CL.

In some embodiments, the disulfide bond reshaping mutation comprises L128C in CH1 of the antigen-binding region and comprises F118C (EU number) in CL.

In some embodiments, the disulfide bond reshaping mutation comprises S134C in CH1 of the antigen-binding region and comprises F116C (EU number) in CL.

In some embodiments, the disulfide bond reshaping mutation comprises S136C in CH1 of the antigen-binding region and comprises S114C (EU number) in CL.

In some embodiments, the disulfide bond reshaping mutation comprises P171C in CH1 of the antigen-binding region and comprises S162C (EU number) in CL.

In some embodiments, the disulfide bond reshaping mutation comprises T139C in CH1 of the antigen-binding region and comprises F116C (EU number) in CL.

Therefore, in one embodiment of the present disclosure, the present disclosure relates to a type of binding molecule, for example, a multispecific antibody, comprising a first antigen-binding region and a second antigen-binding region, and optionally other antigen-binding regions, wherein the first antigen-binding region comprises a first CH1 and a first CL,
(i) the first CH1 comprises F126C mutation, and the first CL comprises Q124C mutation (EU number);
(ii) the first CH1 comprises H168C mutation, and the first CL comprises T164C mutation (EU number);
(iii) the first CH1 comprises F170C mutation, and the first CL comprises T164C mutation (EU number);
(iv) the first CH1 comprises F170C mutation, and the first CL comprises S162C mutation (EU number);
(v) the first CH1 comprises V173C mutation, and the first CL comprises S162C mutation (EU number);
(vi) the first CH1 comprises V173C mutation, and the first CL comprises Q160C mutation (EU number);
(vii) the first CH1 comprises L128C mutation, and the first CL comprises F118C mutation (EU number);
(viii) the first CH1 comprises S134C mutation, and the first CL comprises F116C mutation (EU number);
(ix) the first CH1 comprises S136C mutation, and the first CL comprises S114C mutation (EU number);
(x) the first CH1 comprises P171C mutation, and the first CL comprises S162C mutation (EU number);
(xi) the first CH1 comprises T139C mutation, and the first CL comprises F116C mutation (EU number);

In some embodiments, the CH1 is from IgG1, IgG2, IgG3, or IgG4, preferably from IgG1 or IgG4. In some embodiments, the CL is from Kappa light chain or λ light chain.

In some embodiments, the disulfide bond reshaping mutation also comprises substituting natural Cys with non-Cys. In some embodiments, interchain Cys at the heavy chain constant region-light chain constant region interface of one or two or a plurality of antigen-binding regions is substituted with non-Cys. In some embodiments, a Cys amino acid of the CH1 in the antigen-binding region is substituted with a non-Cys amino acid at position 220 (EU number). In some embodiments, a Cys amino acid in the IgG heavy chain CH1 in the antigen-binding region is substituted with a non-Cys amino acid at position 220 (EU number), or a Cys in the IgG2, IgG3, or IgG4 heavy chain CH1 in the antigen-binding region is substituted with a non-Cys amino acid at position 131 (EU number). In some embodiments, a Cys amino acid in the light chain CL in the antigen-binding region is substituted with a non-Cys amino acid at position 214 (EU number). In some embodiments, a Cys amino acid in the IgG1 heavy chain CH1 in the antigen-binding region is substituted with a non-Cys amino acid at position 220 (EU number), and a Cys amino acid in the light chain CL is substituted with a non-Cys amino acid at position 214, or a Cys amino acid in the IgG2, IgG3, or IgG4 heavy chain CH1 region is substituted with a non-Cys amino acid at position 131 (EU number), and a Cys amino acid in the light chain CL is substituted with a non-Cys amino acid at position 214 (EU number). Exemplary mutations of Cys to non-Cys are described in, for example, CN104011221B. In some embodiments, the Cys is substituted with Ser, Ala or Val, for example, Ser.

In some embodiments, the disulfide bond reshaping mutation comprises C220S/A/V (IgG1 subtype, or comprises C131S/A/V at the corresponding position of IgG2, IgG3, or IgG4) in the CH1 of the antigen-binding region, and comprises C214S/A/V (EU number) in the CL.

Therefore, in one embodiment of the present disclosure, the present disclosure relates to a bispecific antibody comprising a first antigen-binding region and a second antigen-binding region, wherein the first antigen-binding region comprises CH1 and CL,
(i) the first CH1 comprises F126C mutation, and the first CL comprises Q124C mutation (EU number);
(ii) the first CH1 comprises H168C mutation, and the first CL comprises T164C mutation (EU number);
(iii) the first CH1 comprises F170C mutation, and the first CL comprises T164C mutation (EU number);
(iv) the first CH1 comprises F170C mutation, and the first CL comprises S162C mutation (EU number);
(v) the first CH1 comprises V173C mutation, and the first CL comprises S162C mutation (EU number);
(vi) the first CH1 comprises V173C mutation, and the first CL comprises Q160C mutation (EU number);
(vii) the first CH1 comprises L128C mutation, and the first CL comprises F118C mutation (EU number);
(viii) the first CH1 comprises S134C mutation, and the first CL comprises F116C mutation (EU number);
(ix) the first CH1 comprises S136C mutation, and the first CL comprises S114C mutation (EU number);
(x) the first CH1 comprises P171C mutation, and the first CL comprises S162C mutation (EU number);
(xi) the first CH1 comprises T139C mutation, and the first CL comprises F116C mutation (EU number);

the CH1 also comprises C220S/A/V mutation (or corresponding C131S/A/V), and the CL also comprises C214S/A/V mutation (EU number);
preferably, the CH1 also comprises C220S (EU number), and the CL also comprises C214S (EU number).

In some embodiments, the first antigen-binding region comprises CH1 and CL, with the CH1 comprising T139C and C220S mutations (or corresponding C131S), and the CL comprising F116C and C214S mutations (EU number).

In some embodiments, the first antigen-binding region comprises CH1 and CL, with the CH1 comprising F126C and C220S mutations (or corresponding C131S), and the CL comprising Q124C and C214S mutations (EU number).

In some embodiments, the first antigen-binding region comprises CH1 and CL, with the CH1 comprising H168C and C220S mutations (or corresponding C131S), and the CL comprising T164C and C214S mutations (EU number).

In some embodiments, the first antigen-binding region comprises CH1 and CL, with the CH1 comprising F170C and C220S mutations (or corresponding C131S), and the CL comprising T164C and C214S mutations (EU number).

In some embodiments, the first antigen-binding region comprises CH1 and CL, with the CH1 comprising F170C and C220S mutations (or corresponding C131S), and the CL comprising S162C and C214S mutations (EU number).

In some embodiments, the first antigen-binding region comprises CH1 and CL, with the CH1 comprising V173C and C220S mutations (or corresponding C131S), and the CL comprising S162C and C214S mutations (EU number).

In some embodiments, the first antigen-binding region comprises CH1 and CL, with the CH1 comprising V173C and C220S mutations (or corresponding C131S), and the CL comprising Q160C and C214S mutations (EU number).

In some embodiments, the first antigen-binding region comprises CH1 and CL, with the CH1 comprising L128C and C220S mutations (or corresponding C131S), and the CL comprising F118C and C214S mutations (EU number).

In some embodiments, the first antigen-binding region comprises CH1 and CL, with the CH1 comprising S134C and C220S mutations (or corresponding C131S), and the CL comprising F116C and C214S mutations (EU number).

In some embodiments, the first antigen-binding region comprises CH1 and CL, with the CH1 comprising S136C and C220S mutations (or corresponding C131S), and the CL comprising S114C and C214S mutations (EU number).

In some embodiments, the first antigen-binding region comprises CH1 and CL, with the CH1 comprising P171C and C220S mutations (or corresponding C131S), and the CL comprising S162C and C214S mutations (EU number).

In some embodiments, CH1 comprises an amino acid sequence with at least 90%, 91%, 92%, 93%, 94%, 95%, 96%, 97%, 98%, or 99% identity with the amino acid sequence shown in SEQ ID NO:1, and comprises F126C. In some embodiments, CH1 comprises or is composed of the amino acid sequence shown in SEQ ID NO:1. In some embodiments, CH1 comprises an amino acid sequence with at least 90%, 91%, 92%, 93%, 94%, 95%, 96%, 97%, 98%, or 99% identity with the amino acid sequence shown in SEQ ID NO:129, and comprises F126C and C220S. In some embodiments, CH1 comprises or is composed of the amino acid sequence shown in SEQ ID NO:129.

In some embodiments, CH1 comprises an amino acid sequence with at least 90%, 91%, 92%, 93%, 94%, 95%, 96%, 97%, 98%, or 99% identity with the amino acid sequence shown in SEQ ID NO:132, and comprises H168C. In some embodiments, CH1 comprises or is composed of the amino acid sequence shown in SEQ ID NO:132. In some embodiments, CH1 comprises an amino acid sequence with at least 90%, 91%, 92%, 93%, 94%, 95%, 96%, 97%, 98%, or 99% identity with the amino acid sequence shown in SEQ ID NO:133, and comprises H168C and C220S. In some embodiments, CH1 comprises or is composed of the amino acid sequence shown in SEQ ID NO:133.

In some embodiments, CH1 comprises an amino acid sequence with at least 90%, 91%, 92%, 93%, 94%, 95%, 96%, 97%, 98%, or 99% identity with the amino acid sequence shown in SEQ ID NO:134, and comprises F170C. In some embodiments, CH1 comprises or is composed of the amino acid sequence shown in SEQ ID NO:134. In some embodiments, CH1 comprises an amino acid sequence with at least 90%, 91%, 92%, 93%, 94%, 95%, 96%, 97%, 98%, or 99% identity with the amino acid sequence shown in SEQ ID NO:135, and comprises F170C and C220S. In some embodiments, CH1 comprises or is composed of the amino acid sequence shown in SEQ ID NO:135.

In some embodiments, CH1 comprises an amino acid sequence with at least 90%, 91%, 92%, 93%, 94%, 95%, 96%, 97%, 98%, or 99% identity with the amino acid sequence shown in SEQ ID NO:136, and comprises V173C. In some embodiments, CH1 comprises or is composed of the amino acid sequence shown in SEQ ID NO:136. In some embodiments, CH1 comprises an amino acid sequence with at least 90%, 91%, 92%, 93%, 94%, 95%, 96%, 97%, 98%, or 99% identity with the amino acid sequence shown in SEQ ID NO:137, and comprises V173C and C220S. In some embodiments, CH1 comprises or is composed of the amino acid sequence shown in SEQ ID NO:137.

In some embodiments, CH1 comprises an amino acid sequence with at least 90%, 91%, 92%, 93%, 94%, 95%, 96%, 97%, 98%, or 99% identity with the amino acid sequence shown in SEQ ID NO:138, and comprises L128C. In some embodiments, CH1 comprises or is composed of the amino acid sequence shown in SEQ ID NO:138. In some embodiments, CH1 comprises an amino acid sequence with at least 90%, 91%, 92%, 93%, 94%, 95%, 96%, 97%, 98%, or 99% identity with the amino acid sequence shown in SEQ ID NO:139, and comprises L128C and C220S. In some embodiments, CH1 comprises or is composed of the amino acid sequence shown in SEQ ID NO:139.

In some embodiments, CH1 comprises an amino acid sequence with at least 90%, 91%, 92%, 93%, 94%, 95%, 96%, 97%, 98%, or 99% identity with the amino acid sequence shown in SEQ ID NO:140, and comprises S134C. In some embodiments, CH1 comprises or is composed of the amino acid sequence shown in SEQ ID NO:140. In some embodiments, CH1 comprises an amino acid sequence with at least 90%, 91%, 92%, 93%, 94%, 95%, 96%, 97%, 98%, or 99% identity with the amino acid sequence shown in SEQ ID NO:141, and comprises S134C and C220S. In some embodiments, CH1 comprises or is composed of the amino acid sequence shown in SEQ ID NO:141.

In some embodiments, CH1 comprises an amino acid sequence with at least 90%, 91%, 92%, 93%, 94%, 95%, 96%, 97%, 98%, or 99% identity with the amino acid sequence shown in SEQ ID NO:142, and comprises S136C. In some embodiments, CH1 comprises or is composed of the amino acid sequence shown in SEQ ID NO:142. In some embodiments, CH1 comprises an amino acid sequence with at least 90%, 91%, 92%, 93%, 94%, 95%, 96%, 97%, 98%, or 99% identity with the amino acid sequence shown in SEQ ID NO:143, and comprises S136C and C220S. In some embodiments, CH1 comprises or is composed of the amino acid sequence shown in SEQ ID NO:143.

In some embodiments, CH1 comprises an amino acid sequence with at least 90%, 91%, 92%, 93%, 94%, 95%, 96%, 97%, 98%, or 99% identity with the amino acid sequence shown in SEQ ID NO:144, and comprises P171C. In some embodiments, CH1 comprises or is composed of the amino acid sequence shown in SEQ ID NO:144. In some embodiments, CH1 comprises an amino acid sequence with at least 90%, 91%, 92%, 93%, 94%, 95%, 96%, 97%, 98%, or 99% identity with the amino acid sequence shown in SEQ ID NO:145, and comprises P171C and C220S. In some embodiments, CH1 comprises or is composed of the amino acid sequence shown in SEQ ID NO:145.

In some embodiments, CH1 comprises an amino acid sequence with at least 90%, 91%, 92%, 93%, 94%, 95%, 96%, 97%, 98%, or 99% identity with the amino acid sequence shown in SEQ ID NO:146, and comprises T139C. In some embodiments, CH1 comprises or is composed of the amino acid sequence shown in SEQ ID NO:146. In some embodiments, CH1 comprises an amino acid sequence with at least 90%, 91%, 92%, 93%, 94%, 95%, 96%, 97%, 98%, or 99% identity with the amino acid sequence shown in SEQ ID NO:147, and comprises T139C and C220S. In some embodiments, CH1 comprises or is composed of the amino acid sequence shown in SEQ ID NO:147.

In some embodiments, CL comprises an amino acid sequence with at least 90%, 91%, 92%, 93%, 94%, 95%, 96%, 97%, 98%, or 99% identity with the amino acid sequence shown in SEQ ID NO:2, and comprises Q124C. In some embodiments, CL comprises or is composed of the amino acid sequence shown in SEQ ID NO:2. In some embodiments, CL comprises an amino acid sequence with at least 90%, 91%, 92%, 93%, 94%, 95%, 96%, 97%, 98%, or 99% identity with the amino acid sequence shown in SEQ ID NO:128, and comprises Q124C and C124S. In some embodiments, CL comprises or is composed of the amino acid sequence shown in SEQ ID NO:128.

In some embodiments, CL comprises an amino acid sequence with at least 90%, 91%, 92%, 93%, 94%, 95%, 96%, 97%, 98%, or 99% identity with the amino acid sequence shown in SEQ ID NO:148, and comprises T164C. In some embodiments, CL comprises or is composed of the amino acid sequence shown in SEQ ID NO:148. In some embodiments, CL comprises an amino acid sequence with at least 90%, 91%, 92%, 93%, 94%, 95%, 96%, 97%, 98%, or 99% identity with the amino acid sequence shown in SEQ ID NO:149, and comprises T164C and C124S. In some embodiments, CL comprises or is composed of the amino acid sequence shown in SEQ ID NO:149.

In some embodiments, CL comprises an amino acid sequence with at least 90%, 91%, 92%, 93%, 94%, 95%, 96%, 97%, 98%, or 99% identity with the amino acid sequence shown in SEQ ID NO:150, and comprises S162C. In some embodiments, CL comprises or is composed of the amino acid sequence shown in SEQ ID NO:150. In some embodiments, CL comprises an amino acid sequence with at least 90%, 91%, 92%, 93%, 94%, 95%, 96%, 97%, 98%, or 99% identity with the amino acid sequence shown in SEQ ID NO:151, and comprises S162C and C124S. In some embodiments, CL comprises or is composed of the amino acid sequence shown in SEQ ID NO:151.

In some embodiments, CL comprises an amino acid sequence with at least 90%, 91%, 92%, 93%, 94%, 95%, 96%, 97%, 98%, or 99% identity with the amino acid sequence shown in SEQ ID NO:152, and comprises Q160C. In some embodiments, CL comprises or is composed of the amino acid sequence shown in SEQ ID NO:152. In some embodiments, CL comprises an amino acid sequence with at least 90%, 91%, 92%, 93%, 94%, 95%, 96%, 97%, 98%, or 99% identity with the amino acid sequence shown in SEQ ID NO:35, and comprises Q160C and C124S. In some embodiments, CL comprises or is composed of the amino acid sequence shown in SEQ ID NO:35.

In some embodiments, CL comprises an amino acid sequence with at least 90%, 91%, 92%, 93%, 94%, 95%, 96%, 97%, 98%, or 99% identity with the amino acid sequence shown in SEQ ID NO:36, and comprises F118C. In some embodiments, CL comprises or is composed of the amino acid sequence shown in SEQ ID NO:36. In some embodiments, CL comprises an amino acid sequence with at least 90%, 91%, 92%, 93%, 94%, 95%, 96%, 97%, 98%, or 99% identity with the amino acid sequence shown in SEQ ID NO:37, and comprises F118C and C124S. In some embodiments, CL comprises or is composed of the amino acid sequence shown in SEQ ID NO:37.

In some embodiments, CL comprises an amino acid sequence with at least 90%, 91%, 92%, 93%, 94%, 95%, 96%, 97%, 98%, or 99% identity with the amino acid sequence shown in SEQ ID NO:38, and comprises F116C. In some embodiments, CL comprises or is composed of the amino acid sequence shown in SEQ ID NO:38. In some embodiments, CL comprises an amino acid sequence with at least 90%, 91%, 92%, 93%, 94%, 95%, 96%, 97%, 98%, or 99% identity with the amino acid sequence shown in SEQ ID NO:98, and comprises F116C and C124S. In some embodiments, CL comprises or is composed of the amino acid sequence shown in SEQ ID NO:98.

In some embodiments, CL comprises an amino acid sequence with at least 90%, 91%, 92%, 93%, 94%, 95%, 96%, 97%, 98%, or 99% identity with the amino acid sequence shown in SEQ ID NO:99, and comprises S114C. In some embodiments, CL comprises or is composed of the amino acid sequence shown in SEQ ID NO:99. In some embodiments, CL comprises an amino acid sequence with at least 90%, 91%, 92%, 93%, 94%, 95%, 96%, 97%, 98%, or 99% identity with the amino acid sequence shown in SEQ ID NO:100, and comprises S114C and C124S. In some embodiments, CL comprises or is composed of the amino acid sequence shown in SEQ ID NO:100.

### I-2 Charge mutation

Charge mutation is an effective strategy to force homologous light chain and heavy chain pairing, thereby preventing mismatch between light chain and heavy chain of two target-binding regions, wherein an amino acid pair is found on the contact surface of the heavy chain and light chain and mutated into amino acids carrying opposite charges, promoting homologous pairing based on the principle of opposite charges attract.

In one embodiment, the binding molecule of the present disclosure, for example, an antibody (for example, a multispecific antibody, for example, a bispecific antibody) comprises a charge mutation. In some embodiments, the charge mutation is a mutation of an amino acid pair on the contact surface of heavy chain and light chain of at least one antigen-binding region, wherein the two amino acids in the amino acid pair are mutated to opposite charges. In some embodiments, the amino acid pair has one or a plurality of the following features:
a. the amino acid pair is a conservative amino acid pair located at the heavy chain variable region and light chain variable region interface of the antigen-binding region;
b. the distance between α carbon atoms of the amino acid pair is less than 12Å; and/or
c. the side chains of the amino acid pair face each other.

In some embodiments, the charge mutation includes mutating the two amino acids on the amino acid pair selected from the following positions into amino acids carrying opposite charges; position 39 of the heavy chain variable region and position 38 of the light chain variable region, position 45 of the heavy chain variable region and position 87 of the light chain variable region, position 45 of the heavy chain variable region and position 44 of the light chain variable region, position 91 of the heavy chain variable region and position 43 of the light chain variable region, or position 91 of the heavy chain variable region and position 44 of the light chain variable region (Kabat number).

In some embodiments, the charge mutation includes mutating the two amino acids on the amino acid pair selected from the following positions into amino acids carrying opposite charges; Q39 of the heavy chain variable region and Q38 of the light chain variable region, L45 of the heavy chain variable region and Y87 of the light chain variable region, L45 of the heavy chain variable region and P44 of the light chain variable region, Y91 of the heavy chain variable region and A43 of the light chain variable region, or Y91 of the heavy chain variable region and P44 of the light chain variable region (Kabat number).

In some embodiments, the position of one or a plurality of amino acid pairs, respectively, in the antigen-binding region for binding to different antigens comprise a charge mutation. In some embodiments, the amino acid position comprising a charge mutation in the antigen-binding region for binding to different antigens may be the same or different. Where the amino acid position comprising the charge mutation is the same, the mutated amino acids of the amino acid pair in the antigen-binding region for binding with different antigens are different. For example, in some embodiments, the same position of one or a plurality of amino acid pairs of the first antigen-binding region and the second antigen-binding region comprise a charge mutation, but the charge mutation on the amino acid pair in one antigen-binding region is different from the charge mutation on the amino acid pair in another antigen-binding region. For example, at the same position, the charge carried by the mutated amino acid in the first antigen-binding region is the opposite of the charge carried by the mutated amino acid in the second antigen-binding region. For example, in the first antigen-binding region, the heavy chain amino acid at position X1 is mutated into an amino acid carrying a positive charge while the light chain amino acid at position X2 is mutated into an amino acid carrying a negative charge. At the same time, in the second antigen-binding region, the heavy chain amino acid at position X1 is mutated into an amino acid carrying a negative charge while the light chain amino acid at position X2 is mutated into an amino acid carrying a positive charge, wherein the amino acid carrying a positive charge in the first antigen-binding region may be the same or different from the amino acid carrying a positive charge in the second antigen-binding region, for example, they are the same; and the amino acid carrying a negative charge in the first antigen-binding region may be the same or different from the amino acid carrying a negative charge in the second antigen-binding region, for example, they are the same.

In some embodiments, an amino acid in the heavy chain variable region in the amino acid pair is substituted with an amino acid carrying a positive charge, and an amino acid in the light chain variable region is substituted with an amino acid carrying a negative charge. In some embodiments, an amino acid in the heavy chain variable region in the amino acid pair is substituted with an amino acid carrying a negative charge, and an amino acid in the light chain variable region is substituted with an amino acid carrying a positive charge.

In some embodiments, in the first antigen-binding region, the amino acid at position 39 of the heavy chain variable region is substituted with an amino acid carrying a positive charge, and the amino acid at position 38 of the light chain variable region is substituted with an amino acid carrying a negative charge; and/or in the second antigen-binding region, the amino acid at position 39 of the heavy chain variable region is substituted with an amino acid carrying a negative charge, and the amino acid at position 38 of the light chain variable region is substituted with an amino acid carrying a positive charge.

In some embodiments, in the first antigen-binding region, the amino acid at position 45 of the heavy chain variable region is substituted with an amino acid carrying a positive charge, and the amino acid at position 87 of the light chain variable region is substituted with an amino acid carrying a negative charge; and/or in the second antigen-binding region, the amino acid at position 45 of the heavy chain variable region is substituted with an amino acid carrying a negative charge, and the amino acid at position 87 of the light chain variable region is substituted with an amino acid carrying a positive charge.

In some embodiments, in the first antigen-binding region, the amino acid at position 45 of the heavy chain variable region is substituted with an amino acid carrying a positive charge, and the amino acid at position 44 of the light chain variable region is substituted with an amino acid carrying a negative charge; and/or in the second antigen-binding region, the amino acid at position 45 of the heavy chain variable region is substituted with an amino acid carrying a negative charge, and the amino acid at position 44 of the light chain variable region is substituted with an amino acid carrying a positive charge.

In some embodiments, in the first antigen-binding region, the amino acid at position 91 of the heavy chain variable region is substituted with an amino acid carrying a positive charge, and the amino acid at position 43 of the light chain variable region is substituted with an amino acid carrying a negative charge; and/or in the second antigen-binding region, the amino acid at position 91 of the heavy chain variable region is substituted with an amino acid carrying a negative charge, and the amino acid at position 43 of the light chain variable region is substituted with an amino acid carrying a positive charge.

In some embodiments, in the first antigen-binding region, the amino acid at position 91 of the heavy chain variable region is substituted with an amino acid carrying a positive charge, and the amino acid at position 44 of the light chain variable region is substituted with an amino acid carrying a negative charge; and/or in the second antigen-binding region, the amino acid at position 91 of the heavy chain variable region is substituted with an amino acid carrying a negative charge, and the amino acid at position 44 of the light chain variable region is substituted with an amino acid carrying a positive charge.

In some embodiments, the amino acid carrying a positive charge is selected from K, R, and H, and/or the amino acid carrying a negative charge is selected from E or D. In some embodiments, the amino acid carrying a positive charge is selected from K and R, and/or the amino acid carrying a negative charge is selected from E or D. In some embodiments, the amino acid carrying a positive charge is K, and/or the amino acid carrying a negative charge is D.

In some embodiments, in an antigen-binding region, the amino acid at position 39 of the heavy chain variable region is substituted with K, and the amino acid at position 38 of the light chain variable region is substituted with D. In some embodiments, in an antigen-binding region, the amino acid at position 39 of the heavy chain variable region is substituted with D, and the amino acid at position 38 of the light chain variable region is substituted with K.

In some embodiments, in an antigen-binding region, the amino acid at position 45 of the heavy chain variable region is substituted with K, and the amino acid at position 87 of the light chain variable region is substituted with D. In some embodiments, in an antigen-binding region, the amino acid at position 45 of the heavy chain variable region is substituted with D, and the amino acid at position 87 of the light chain variable region is substituted with K.

In some embodiments, in an antigen-binding region, the amino acid at position 45 of the heavy chain variable region is substituted with K, and the amino acid at position 44 of the light chain variable region is substituted with D. In some embodiments, in an antigen-binding region, the amino acid at position 45 of the heavy chain variable region is substituted with D, and the amino acid at position 44 of the light chain variable region is substituted with K.

In some embodiments, in an antigen-binding region, the amino acid at position 91 of the heavy chain variable region is substituted with K, and the amino acid at position 43 of the light chain variable region is substituted with D. In some embodiments, in an antigen-binding region, the amino acid at position 91 of the heavy chain variable region is substituted with D, and the amino acid at position 43 of the light chain variable region is substituted with K.

In some embodiments, in an antigen-binding region, the amino acid at position 91 of the heavy chain variable region is substituted with K, and the amino acid at position 44 of the light chain variable region is substituted with D. In some embodiments, in an antigen-binding region, the amino acid at position 91 of the heavy chain variable region is substituted with D, and the amino acid at position 44 of the light chain variable region is substituted with K.

In some embodiments, the binding molecule of the present disclosure, for example, an antibody, such as a multispecific antibody, for example, a bispecific antibody, comprises a first antigen-binding region and a second antigen-binding region comprising substitutions selected from the following:
(i). in the first antigen-binding region, the amino acid at position 39 of the heavy chain variable region is substituted with K, and the amino acid at position 38 of the light chain variable region is substituted with D, while in the second antigen-binding region, the amino acid at position 39 of the heavy chain variable region is substituted with D, and the amino acid at position 38 of the light chain variable region is substituted with K;
(ii). in the first antigen-binding region, the amino acid at position 45 of the heavy chain variable region is substituted with K, and the amino acid at position 87 of the light chain variable region is substituted with D, while in the second antigen-binding region, the amino acid at position 45 of the heavy chain variable region is substituted with D, and the amino acid at position 87 of the light chain variable region is substituted with K;
(iii). in the first antigen-binding region, the amino acid at position 45 of the heavy chain variable region is substituted with K, and the amino acid at position 44 of the light chain variable region is substituted with D, while in the second antigen-binding region, the amino acid at position 45 of the heavy chain variable region is substituted with D, and the amino acid at position 44 of the light chain variable region is substituted with K;
(iv). in the first antigen-binding region, the amino acid at position 91 of the heavy chain variable region is substituted with K, and the amino acid at position 43 of the light chain variable region is substituted with D, while in the second antigen-binding region, the amino acid at position 91 of the heavy chain variable region is substituted with D, and the amino acid at position 43 of the light chain variable region is substituted with K;
(v). in the first antigen-binding region, the amino acid at position 91 of the heavy chain variable region is substituted with K, and the amino acid at position 44 of the light chain variable region is substituted with D, while in the second antigen-binding region, the amino acid at position 91 of the heavy chain variable region is substituted with D, and the amino acid at position 44 of the light chain variable region is substituted with K.

### I-3 Combinatorial mutation

In some embodiments, the binding molecule of the present disclosure, for example, an antibody, for example, a multispecific antibody, for example, a bispecific antibody, comprises a first target-binding region (antigen-binding region) and a second target-binding region (antigen-binding region) comprising a disulfide bond reshaping mutation and a charge mutation.

In some embodiments, the present disclosure relates to a type of antibody, for example, a multispecific antibody, for example, a bispecific antibody, comprising a first antigen-binding region and a second antigen-binding region, wherein the first antigen-binding region comprises a heavy chain variable region, a heavy chain constant region CH1, a light chain variable region and a light chain constant region CL, and the second antigen-binding region comprises a heavy chain variable region VH and a light chain variable region VL.

In some embodiments, the first antigen-binding region comprises a disulfide bond reshaping mutation at the CH1-CL interface, and comprises a charge mutation in VH and VL.

In some embodiments, the first antigen-binding region comprises a disulfide bond reshaping mutation at the CH1-CL interface, and comprises a charge mutation in VH and VL; the second antigen-binding region comprises a charge mutation in VH and VL.

In some embodiments, the first antigen-binding region comprises mutation combinations selected from the following:

| | | | | |
|---|---|---|---|---|
| | Heavy Chain VH Mutation | Heavy Chain CH1 Mutation | Light Chain VL Mutation | Light Chain CL Mutation |
| Combinati on 1 | Q39K | F126C, C220S | Q38D | Q124C, C214S |
| | Q39K | H168C, C220S | Q38D | T164C, C214S |
| | Q39K | F170C, C220S | Q38D | T164C, C214S |
| | Q39K | F170C, C220S | Q38D | S162C, C214S |
| | Q39K | V173C, C220S | Q38D | S162C, C214S |
| | Q39K | V173C, C220S | Q38D | Q160C, C214S |
| | Q39K | L128C, C220S | Q38D | F118C, C214S |
| | Q39K | S134C, C220S | Q38D | F116C, C214S |
| | Q39K | S136C, C220S | Q38D | S114C, C214S |
| | Q39K | P171C, C220S | Q38D | S162C, C214S |
| | Q39K | T139C, C220S | Q38D | F116C, C214S |
| | | | | |
| Combinati on 2 | L45K | F126C, C220S | Y87D | Q124C, C214S |
| | L45K | H168C, C220S | Y87D | T164C, C214S |
| | L45K | F170C, C220S | Y87D | T164C, C214S |
| | L45K | F170C, C220S | Y87D | S162C, C214S |
| | L45K | V173C, C220S | Y87D | S162C, C214S |
| | L45K | V173C, C220S | Y87D | Q160C, C214S |
| | L45K | L128C, C220S | Y87D | F118C, C214S |
| | L45K | S134C, C220S | Y87D | F116C, C214S |
| | L45K | S136C, C220S | Y87D | S114C, C214S |
| | L45K | P171C, C220S | Y87D | S162C, C214S |
| | L45K | T139C, C220S | Y87D | F116C, C214S |
| | | | | |
| Combinati on 3 | L45K | F126C, C220S | P44D | Q124C, C214S |
| | L45K | H168C, C220S | P44D | T164C, C214S |
| | L45K | F170C, C220S | P44D | T164C, C214S |
| | L45K | F170C, C220S | P44D | S162C, C214S |
| | L45K | V173C, C220S | P44D | S162C, C214S |
| | L45K | V173C, C220S | P44D | Q160C, C214S |
| | L45K | L128C, C220S | P44D | F118C, C214S |
| | L45K | S134C, C220S | P44D | F116C, C214S |
| | L45K | S136C, C220S | P44D | S114C, C214S |
| | L45K | P171C, C220S | P44D | S162C, C214S |
| | L45K | T139C, C220S | P44D | F116C, C214S |
| | | | | |
| Combinati on 4 | Y91K | F126C, C220S | A43D | Q124C, C214S |
| | Y91K | H168C, C220S | A43D | T164C, C214S |
| | Y91K | F170C, C220S | A43D | T164C, C214S |
| | Y91K | F170C, C220S | A43D | S162C, C214S |
| | Y91K | V173C, C220S | A43D | S162C, C214S |
| | Y91K | V173C, C220S | A43D | Q160C, C214S |
| | Y91K | L128C, C220S | A43D | F118C, C214S |
| | Y91K | S134C, C220S | A43D | F116C, C214S |
| | Y91K | S136C, C220S | A43D | S114C, C214S |
| | Y91K | P171C, C220S | A43D | S162C, C214S |
| | Y91K | T139C, C220S | A43D | F116C, C214S |
| | | | | |
| Combinati on 5 | Y91K | F126C, C220S | P44D | Q124C, C214S |
| | Y91K | H168C, C220S | P44D | T164C, C214S |
| | Y91K | F170C, C220S | P44D | T164C, C214S |
| | Y91K | F170C, C220S | P44D | S162C, C214S |
| | Y91K | V173C, C220S | P44D | S162C, C214S |
| | Y91K | V173C, C220S | P44D | Q160C, C214S |
| | Y91K | L128C, C220S | P44D | F118C, C214S |
| | Y91K | S134C, C220S | P44D | F116C, C214S |
| | Y91K | S136C, C220S | P44D | S114C, C214S |
| | Y91K | P171C, C220S | P44D | S162C, C214S |
| | Y91K | T139C, C220S | P44D | F116C, C214S |

In some embodiments, the second antigen-binding region comprises mutation combinations selected from the following:

| | Heavy Chain VH Mutation | Light Chain VL Mutation |
|---|---|---|
| Combination 1 | Q39D | Q38K |
| Combination 2 | L45D | Y87K |
| Combination 3 | L45D | P44K |
| Combination 4 | Y91D | A43K |
| Combination 5 | Y91D | P44K |

In some embodiments, the present disclosure relates to a type of antibody, for example, a multispecific antibody, for example, a bispecific antibody, comprising a first antigen-binding region and a second antigen-binding region, wherein the first antigen-binding region comprises a heavy chain variable region VH, a heavy chain constant region CH1, a light chain variable region VL and a light chain constant region CL, and the second antigen-binding region comprises a heavy chain variable region VH and a light chain variable region VL, wherein the antibody comprises the following mutation combinations:

| | First Antigen-Binding Region | | | | Second Antigen-Binding Region | |
|---|---|---|---|---|---|---|
| | Heavy Chain VH Mutation | Heavy Chain CH1 Mutation | Light Chain VL Mutation | Light Chain CL Mutation | Heavy Chain VH Mutation | Light Chain VL Mutation |
| Comb inatio n 1 | Q39K | F126C, C220S | Q38D | Q124C, C2145 | Q39D | Q38K |
| | Q39K | H168C, C220S | Q38D | T164C, C214S | Q39D | Q38K |
| | Q39K | F170C, C220S | Q38D | T164C, C214S | Q39D | Q38K |
| | Q39K | F170C, C220S | Q38D | S162C, C214S | Q39D | Q38K |
| | Q39K | V173C, C220S | Q38D | S162C, C214S | Q39D | Q38K |
| | Q39K | V173C, C220S | Q38D | Q160C, C214S | Q39D | Q38K |
| | Q39K | L128C, C220S | Q38D | F118C, C214S | Q39D | Q38K |
| | Q39K | S134C, C220S | Q38D | F116C, C214S | Q39D | Q38K |
| | Q39K | S136C, C220S | Q38D | S114C, C214S | Q39D | Q38K |
| | Q39K | P171C, C220S | Q38D | S162C, C214S | Q39D | Q38K |
| | Q39K | T139C, C220S | Q38D | F116C, C214S | Q39D | Q38K |
| | | | | | | |
| Comb inatio n 2 | L45K | F126C, C220S | Y87D | Q124C, C2145 | L45D | Y87K |
| | L45K | H168C, C220S | Y87D | T164C, C214S | L45D | Y87K |
| | L45K | F170C, C220S | Y87D | T164C, C214S | L45D | Y87K |
| | L45K | F170C, C220S | Y87D | S162C, C214S | L45D | Y87K |
| | L45K | V173C, C220S | Y87D | S162C, C214S | L45D | Y87K |
| | L45K | V173C, C220S | Y87D | Q160C, C214S | L45D | Y87K |
| | L45K | L128C, C220S | Y87D | F118C, C214S | L45D | Y87K |
| | L45K | S134C, C220S | Y87D | F116C, C214S | L45D | Y87K |
| | L45K | S136C, C220S | Y87D | S114C, C214S | L45D | Y87K |
| | L45K | P171C, C220S | Y87D | S162C, C214S | L45D | Y87K |
| | L45K | T139C, C220S | Y87D | F116C, C214S | L45D | Y87K |
| | | | | | | |
| Comb inatio n 3 | L45K | F126C, C220S | P44D | Q124C, C2145 | L45D | P44K |
| | L45K | H168C, | P44D | T164C, | L45D | P44K |
| | | C220S | | C214S | | |
| | L45K | F170C, C220S | P44D | T164C, C214S | L45D | P44K |
| | L45K | F170C, C220S | P44D | S162C, C214S | L45D | P44K |
| | L45K | V173C, C220S | P44D | S162C, C214S | L45D | P44K |
| | L45K | V173C, C220S | P44D | Q160C, C214S | L45D | P44K |
| | L45K | L128C, C220S | P44D | F118C, C214S | L45D | P44K |
| | L45K | S134C, C220S | P44D | F116C, C214S | L45D | P44K |
| | L45K | S136C, C220S | P44D | S114C, C214S | L45D | P44K |
| | L45K | P171C, C220S | P44D | S162C, C214S | L45D | P44K |
| | L45K | T139C, C220S | P44D | F116C, C214S | L45D | P44K |
| | | | | | | |
| Comb inatio n 4 | Y91K | F126C, C220S | A43D | Q124C, C214S | Y91D | A43K |
| | Y91K | H168C, C220S | A43D | T164C, C214S | Y91D | A43K |
| | Y91K | F170C, C220S | A43D | T164C, C214S | Y91D | A43K |
| | Y91K | F170C, C220S | A43D | S162C, C214S | Y91D | A43K |
| | Y91K | V173C, C220S | A43D | S162C, C214S | Y91D | A43K |
| | Y91K | V173C, C220S | A43D | Q160C, C214S | Y91D | A43K |
| | Y91K | L128C, C220S | A43D | F118C, C214S | Y91D | A43K |
| | Y91K | S134C, C220S | A43D | F116C, C214S | Y91D | A43K |
| | Y91K | S136C, C220S | A43D | S114C, C214S | Y91D | A43K |
| | Y91K | P171C, C220S | A43D | S162C, C214S | Y91D | A43K |
| | Y91K | T139C, C220S | A43D | F116C, C214S | Y91D | A43K |
| | | | | | | |
| Comb inatio n 5 | Y91K | F126C, C220S | P44D | Q124C, C214S | Y91D | P44K |
| | Y91K | H168C, C220S | P44D | T164C, C214S | Y91D | P44K |
| | Y91K | F170C, C220S | P44D | T164C, C214S | Y91D | P44K |
| | Y91K | F170C, C220S | P44D | S162C, C214S | Y91D | P44K |
| | Y91K | V173C, C220S | P44D | S162C, C214S | Y91D | P44K |
| | Y91K | V173C, C220S | P44D | Q160C, C214S | Y91D | P44K |
| | Y91K | L128C, | P44D | F118C, | Y91D | P44K |
| | | C220S | | C214S | | |
| | Y91K | S134C, C220S | P44D | F116C, C214S | Y91D | P44K |
| | Y91K | S136C, C220S | P44D | S114C, C214S | Y91D | P44K |
| | Y91K | P171C, C220S | P44D | S162C, C214S | Y91D | P44K |
| | Y91K | T139C, C220S | P44D | F116C, C214S | Y91D | P44K |

In some embodiments, in the antibody, for example, a multispecific antibody (for example, a bispecific antibody), one or a plurality or all the antigen-binding regions that bind to the same antigen comprise the same mutation.

In some embodiments, in the antibody, for example, a multispecific antibody (for example, a bispecific antibody), one or a plurality or all the antigen-binding regions that bind to the same antigen comprise different mutations.

### I-4 Light chain constant region substitution

In some embodiments, the binding molecule of the present disclosure, for example, an antibody, such as a multispecific antibody, comprises a first target-binding region (antigen-binding region) and a second target-binding region (antigen-binding region), wherein the light chain constant region that one or a plurality or all the first target-binding regions (antigen-binding regions) comprise is a Kappa light chain constant region, and the light chain constant region that one or a plurality or all the second target-binding regions comprise is a Lambda light chain constant regions, or the light chain constant region that one or a plurality or all the first target-binding regions (antigen-binding regions) comprise is a Lambda light chain constant regions, and the light chain constant region that one of a plurality or all the second target-binding regions (antigen-binding regions) comprise is a Kappa light chain constant region.

### I-5 Fc region mutation

In some embodiments, one or a plurality of target-binding regions (antigen-binding regions) of the binding molecule of the present disclosure, for example, an antibody, such as a multispecific antibody (for example, a bispecific antibody), comprises a Fc region, wherein the comprising Fc region may be the same or different.

In some embodiments, a first Fc region and a second Fc region are different and may be heterodimerized to form a heterodimeric Fc scaffold.

Fc region herein refers to a C-terminal region that comprises at least a part of the immunoglobulin heavy chain of the constant region and may comprise a natural sequence Fc region and a variant Fc region. The natural sequence Fc region covers various naturally occurring immunoglobulin Fc sequences, for example, the Fc region of various IgG subtypes and the allotypes thereof (Gestur Vidarsson et al., IgG subclasses and allotypes: from structure to effector functions, 20 October 2014, doi: 10.3389/fimmu.2014.00520). In some embodiments, the Fc region of the present disclosure comprises antibody CH2 and CH3. In some embodiments, the antibody Fc region may also carry an IgG hinge region or part of an IgG hinge region at the N-terminus, for example, an IgG1 hinge region or part of an IgG1 hinge region, for example, the sequence of D221 to P230 according to EU numbering. The hinge region may contain mutations.

Unless otherwise specified herein, the amino acid residue number in the Fc region is based on the EU numbering scheme, also known as the EU index, such as that mentioned in Kabat, E.A. et al., Sequences of Proteins of Immunological Interest, Version 5, Public Health Service, National Institutes of Health, Bethesda, MD (1991), NIH Publication 91 - 3,242.

In some embodiments, the Fc region is a human IgG Fc, for example, human IgG1 Fc, human IgG2 Fc, human IgG3 Fc, or human IgG4 Fc. In one embodiment, the Fc region comprises or is composed of the amino acid sequence SEQ ID NO:122 or 123, or has at least 90% identity with it, for example, an amino acid sequence with 95%, 96%, 97%, 99%, or higher identity.

As understood by skilled artisans in the art, in order to promote the formation of the multispecific antibody of the present disclosure as a heterodimer, the Fc region that the multispecific antibody of the present disclosure comprises may comprise mutations favorable for heterodimerization of the first Fc region and the second Fc region. In one embodiment, a mutation is introduced into the CH3 region of the two Fc regions.

A known method in the art to promote Fc region heterodimerization. For example, the CH3 region of the first Fc region and the CH3 region of the second Fc region are engineered in a complementary manner, such that each CH3 region (or the heavy chain comprising it) can no longer homodimerize with itself but is forced to heterodimerize with the other CH3 region that has been complementarily engineered (such that the CH3 region of the first and second Fc regions heterodimerize and a homodimer does not form between two first CH3 regions or two second CH3 regions).

Preferably, based on the Knob-into-hole technology, a corresponding Knob mutation and Hole mutation are introduced into the first Fc region and the second Fc region. Refer to for example, US 5, 731, 168; US 7, 695, 936; Ridgway et al., Prot Eng 9, 617-621 (1996) and Carter, J Immunol Meth 248, 7 - 15(2001) for this technology.

In a particular embodiment, in the CH3 region of one Fc region, Thr residue at position 366 is substituted with Trp residue (T366W) (Knob mutation); while in the CH3 region of another Fc region, Tyr residue at position 407 is substituted with Val residue (Y407V) (Hole mutation), optionally Thr residue at position 366 is substituted with Ser residue (T366S) and Tyr residue at position 407 is substituted with Val residue (Y407V) (numbered based on the EU index).

In another embodiment, in the CH3 region of one Fc region, Thr residue at position 366 is substituted with Trp residue (T366W) and Ser residue at position 354 is substituted with Cys residue (S354C) or Glu residue at position 356 is substituted with Cys residue (E356C) (particularly, Ser residue at position 354 is substituted with Cys residue); while in the CH3 region of another Fc region, Tyr residue at position 407 is substituted with Val residue (Y407V) (Hole mutation), optionally Thr residue at position 366 is substituted with Ser residue (T366S) and Leu residue at position 368 is substituted with Ala residue (L368A) (numbered based on the EU index), optionally Tyr residue at position 349 is substituted with Cys residue (Y349C) (numbered based on the EU index).

In a specific embodiment, one Fc region comprises the amino acid substitution T366W, and another Fc region comprises the amino acid substitutions T366S, L368A, and Y407V (numbered based on the EU index).

In a specific embodiment, one Fc region comprises the amino acid substitutions S354C and T366W, and another Fc region comprises the amino acid substitutions Y349C, T366S, L368A, and Y407V (numbered based on the EU index).

The corresponding mutation may be introduced into the first Fc region and the second Fc region based on Innobody technology. Refers to, for example, PCT/CN2021/143141 for this technology.

In a particular embodiment,
the first CH3 region comprises S364R/K mutation (preferably S364R), and optionally one or a plurality of other mutations. In some embodiments, the second CH3 region comprises K370S/T/A/V mutation (preferably K370S), and optionally one or a plurality of other mutations. In some embodiments, the first CH3 region comprises S364R/K mutation, and the second CH3 region comprises K370S/T/A/V mutation. In some embodiments, the first CH3 region comprises S364R mutation, and the second CH3 region comprises K370S mutation.

In some embodiments, the first CH3 region comprises S364R/K (preferably S364R) and D399K/R (preferably D399K) mutation. In some embodiments, the second CH3 region comprises K370S/T/A/V mutation (preferably K370S), and K409D/E (preferably K409D) mutation. In some embodiments, the first CH3 region comprises S364R/K+D399K/R, and the second CH3 region comprises K370S/T/A/V+Y349T/S/A/V. In some embodiments, the first CH3 region comprises S364R+D399K, and the second CH3 region comprises K370S+Y349T. In some embodiments, the first CH3 region also comprises E375N/Q (preferably E375N), and/or T350V/A (preferably T350V). In some embodiments, the second CH3 region also comprises K409D/E (preferably K409D), Q347D/E (preferably Q347D) and/or T350V/A (preferably T350V).

In some embodiments, the first CH3 region comprises S364R+D399K, and the second CH3 region comprises K370S+Y349T+K409D. In some embodiments, the first CH3 region also comprises E357N. In some embodiments, the second CH3 region also comprises Q347D. In some embodiments, the first CH3 region also comprises E357N, and the second CH3 region also comprises Q347D. In some embodiments, the first CH3 region and the second CH3 region also comprise T350V, respectively, or both comprise T350V.

Therefore, in some embodiments, the first CH3 region comprises S364R+D399K, and the second CH3 region comprises K370S+Y349T+K409D+Q347D. In some embodiments, the first CH3 region comprises S364R+D399K+E357N, and the second CH3 region comprises K370S+Y349T+K409D+Q347D. In some embodiments, the first CH3 region comprises S364R+D399K+E357N+T350V, and the second CH3 region comprises K370S+Y349T+K409D+Q347D+T350V.

In some embodiments, the first CH3 region comprises K409E/D (preferably K409E). In some embodiments, the second CH3 region comprises D399K/R (preferably D399K) or K370T/S/A/V (preferably K370T). In some embodiments, the first CH3 region comprises K409E/D (preferably K409E), and the second CH3 region comprises D399K/R (preferably D399K). IN some embodiments, the first CH3 region also comprises T411R/K (preferably T411R). In some embodiments, the second CH3 region also comprises K370T/S/A/V (preferably K370T). In some embodiments, the [first] CH3 region comprises K409E/D+T411R/K, and the second CH3 region comprises D399K/R+K370T/S/A/V. In some embodiments, the [first] CH3 region comprises K409E+T411R, and the second CH3 region comprises D399K+K370T.

In some specific embodiments, the first and second CH3 regions have the following mutation combinations:

| First CH3 Region | Second CH3 Region |
|---|---|
| S364R, D399K, E357N, T350V | K370S, Y349T, Q347D, K409D, T350V |
| K409E, T411R | D399K, K370T |
| S364R, D399K, E357N | K370S, Y349T, Q347D, K409D |
| S364R, D399K | K370S, Y349T, Q347D, K409D |
| S364R, D399K | K370S, Y349T, K409D |

In one embodiment, the CH3 of one Fc region comprises S364R and D399K mutations, and CH3 mutations of another Fc region comprises Y349T, K370S, and K409D mutations.

Therefore, in a specific embodiment, the binding molecule of the present disclosure, for example, an antibody, such as a multispecific antibody, comprises heterodimerization of two Fc regions, wherein
a) a polypeptide of one Fc region comprises mutation T366W, while a polypeptide of another Fc region comprises T366S, L368A, and Y407V, or
b) a polypeptide of one Fc-region comprises mutations S354C and T366W, while a polypeptide of another Fc-region comprises mutations Y349C, T366S, L368A, and Y407V, or
c) a polypeptide of one Fc-region comprises mutations S364R and D399K, while a polypeptide of another Fc-region comprises mutations Y349T, K370S, and K409D.

Therefore, in a specific embodiment, the binding molecule of the present disclosure, for example, an antibody, such as a multispecific antibody, comprises heterodimerization of two Fc regions, wherein
a) a polypeptide of one Fc region comprises or is composed of the amino acid sequence shown in SEQ ID NO:20 or 131, while a polypeptide of another Fc region comprises or is composed of the amino acid sequence shown in SEQ ID NO:101 or 130;
b) a polypeptide of one Fc region comprises or is composed of the amino acid sequence shown in SEQ ID NO:125, while a polypeptide of another Fc region comprises or is composed of the amino acid sequence shown in SEQ ID NO:124; or
c) one Fc region comprises or is composed of the amino acid sequence shown in SEQ ID NO:126, while a polypeptide of another Fc region comprises or is composed of the amino acid sequence shown in SEQ ID NO:127.

Therefore, in a specific embodiment, the binding molecule of the present disclosure, for example, an antibody, such as a multispecific antibody, comprises heterodimerization of two Fc regions, wherein
a) a polypeptide of one Fc region comprises an amino acid sequence that has at least 85%, 90%, 95%, 96%, 97%, 98%, or 99% identity with the amino acid sequence shown in SEQ ID NO:20 or 131, while a polypeptide of another Fc region comprises an amino acid sequence that has at least 85%, 90%, 95%, 96%, 97%, 98%, or 99% identity with the amino acid sequence shown in SEQ ID NO:101 or 130.
b) a polypeptide of one Fc region comprises an amino acid sequence that has at least 85%, 90%, 95%, 96%, 97%, 98%, or 99% identity with the amino acid sequence shown in SEQ ID NO:125, while a polypeptide of another Fc region comprises an amino acid sequence that has at least 85%, 90%, 95%, 96%, 97%, 98%, or 99% identity with the amino acid sequence shown in SEQ ID NO:124; or
c) one Fc region comprises an amino acid sequence that has at least 85%, 90%, 95%, 96%, 97%, 98%, or 99% identity with the amino acid sequence shown in SEQ ID NO:126, while a polypeptide of another Fc region comprises an amino acid sequence that has at least 85%, 90%, 95%, 96%, 97%, 98%, or 99% identity with the amino acid sequence shown in SEQ ID NO:127.

Therefore, in a specific embodiment, the binding molecule of the present disclosure, for example, an antibody, such as a multispecific antibody, comprises heterodimerization of two Fc regions, wherein
a) a polypeptide of one Fc region comprises an amino acid sequence that has at least 85%, 90%, 95%, 96%, 97%, 98%, or 99% identity with the amino acid sequence shown in SEQ ID NO:20 or 131 and comprises mutation T366W, while a polypeptide of another Fc region comprises an amino acid sequence that has at least 85%, 90%, 95%, 96%, 97%, 98%, or 99% identity with the amino acid sequence shown in SEQ ID NO:101 or 130 and comprises T366S, L368A, and Y407V; or
b) a polypeptide of one Fc region comprises an amino acid sequence that has at least 85%, 90%, 95%, 96%, 97%, 98%, or 99% identity with the amino acid sequence shown in SEQ ID NO:125 and comprises mutations S354C and T366W, while a polypeptide of another Fc region comprises an amino acid sequence that has at least 85%, 90%, 95%, 96%, 97%, 98%, or 99% identity with the amino acid sequence shown in SEQ ID NO:124 and comprises mutations Y349C, T366S, L368A, and Y407V; or
c) one Fc region comprises an amino acid sequence that has at least 85%, 90%, 95%, 96%, 97%, 98%, or 99% identity with the amino acid sequence shown in SEQ ID NO:126 and comprises mutations S364R and D399K, while a polypeptide of another Fc region comprises an amino acid sequence that has at least 85%, 90%, 95%, 96%, 97%, 98%, or 99% identity with the amino acid sequence shown in SEQ ID NO:127 and comprises mutations Y349T, K370S, and K409D.

In some embodiments, the Fc region also comprises other mutations favorable for heterodimer purification.

The Fc region in the binding molecule of the present disclosure, for example, an antibody may also be mutated to obtain the desired properties. Fc region mutations are known in the art.

In one embodiment, the Fc region is modified to [alter] effector function properties therein (for example, the complement activation function in the Fc region) In one embodiment, the effector function is reduced or eliminated compared to Fc regions of wildtype isotypes. In one embodiment, the effector function is reduced or eliminated through methods selected from the following: by using a Fc isotype with a naturally reduced or eliminated effector function, and Fc region modification.

In one preferred embodiment, the Fc region has a reduced Fc region-mediated effector function, for example, a reduced or eliminated ADCC or ADCP or CDC effector function, for example, comprising a mutation to achieve the above function.

As understood by skilled artisans in the art, according to the expected use of the binding molecule of the present disclosure, for example, an antibody molecule, the binding molecule of the present disclosure, for example, an antibody molecule, may also comprise a modification in the Fc domain that changes the binding affinity of one or a plurality of Fc receptors. In one embodiment, the Fc receptor is a Fc γ receptor, particularly a human Fc γ receptor. In some embodiments, the Fc region comprises a mutation that reduces binding to the Fc γ receptor. For example, in some embodiments, the Fc region used for the present disclosure has L234A/L235A mutation that reduces binding to the Fc γ receptor. In another preferred embodiment, a Fc fragment may have a mutation that causes an increase in the serum half-life, for example, a mutation that improves binding of the Fc fragment to FcRn.

### I-6. Bispecific antibody or multispecific antibody

In some embodiments, the antibody of the present disclosure is a bispecific antibody.

The term "bispecific antibody" refers to an antibody comprising a first antigen-binding region and a second antigen-binding region, wherein the first antigen-binding region binds to a type of antigen or epitope and the second antigen-binding region binds to another antigen or another epitope. Therefore, the bispecific antibody according to the present disclosure comprises specificity to two different types of antigens, or two different epitopes of one type of antigen. Bispecific antibody forms include an IgG-like antibody (Fan et al. (2015) Journal of Hematology & Oncology. 8: 130). The most common type of IgG-like antibody comprises two Fab regions and two Fc regions, and the heavy chain and light chain of each Fab may be from independent monoclonal antibodies.

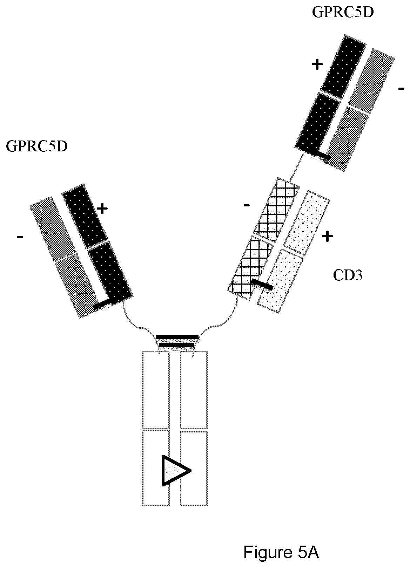

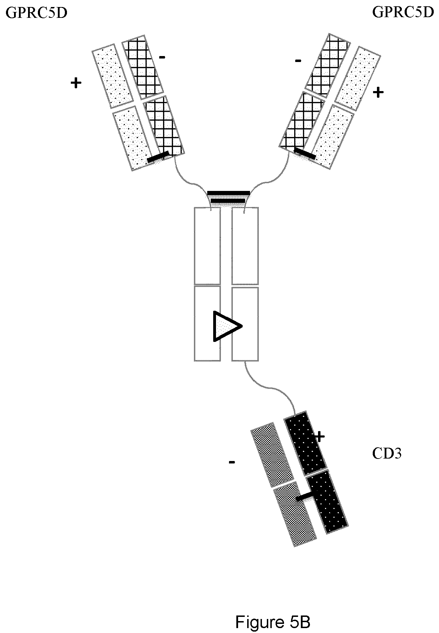

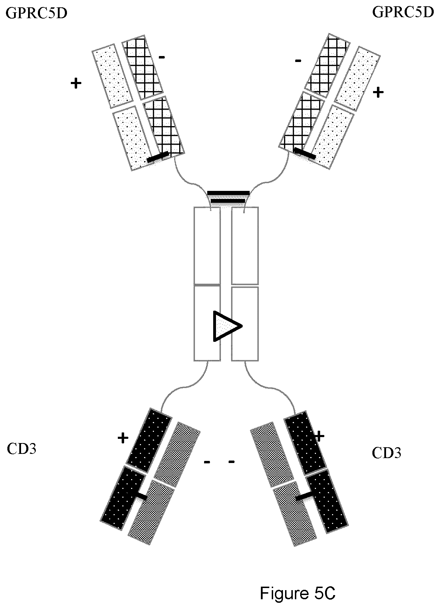

The bispecific antibody of the present disclosure may be prepared using bispecific antibody forms or technology that are known in the art. Refer to, for example, Labrijn, et al. Bispecific antibodies: a mechanistic review of the pipeline. Nature Reviews Drug Discovery, 2019, 18(8): 1- 24 for specific exemplary bispecific antibody forms used in the context of the present disclosure. In some embodiments, the bispecific antibody of the present disclosure has the following formats: bispecific antibody with a 2+1N-terminal configuration (Figure 5A), bispecific antibody with a 2+1C-terminal configuration (Figure 5B), bispecific antibody with a 2+2C-terminal configuration (Figure 5C). In some embodiments, the bispecific antibody of the present disclosure also comprises a 2+2N configuration or tandem Fab configuration.

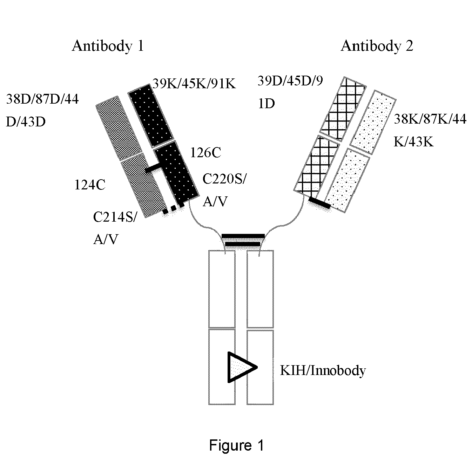

In some embodiments, the bispecific antibody of the present disclosure comprises a first antigen-binding region that specifically binds to a first antigen and a second antigen-binding region that specifically binds to a second antigen, wherein the first antigen-binding region comprises a First Fab, the second antigen-binding region comprises a second Fab, wherein the First Fab is linked to the N-terminus of the first Fc region at the C-terminus of the CH1 thereof (with or without a linker, for example, a hinge region), and the Second Fab is linked to the N-terminus of the second Fc region at the C-terminus of the CH1 thereof (with or without a linker, for example, a hinge region). In a specific embodiment, the bispecific antibody is an IgG-like antibody with the configuration shown in Figure 1. In a specific embodiment, the First Fab comprises a charge mutation and a disulfide bond reshaping mutation, the Second Fab comprises a charge mutation, and optionally, the first Fc region and the second Fc region comprise an Innobody mutation or Knob-into-hole mutation. Preferably, the charge mutation that the First Fab comprises and the charge mutation that the Second Fab comprises have the same position, but the charge carried by the mutated amino acid at the same position is the opposite. In one embodiment, the bispecific antibody comprises:
Heavy chain 1: comprises or is composed of the following from the N-terminus to the C-terminus: the First Fab heavy chain variable region-heavy chain constant region CH1-first Fc region, wherein the heavy chain constant region CH1 is linked to the N-terminus of the first Fc region at the C-terminus thereof with or without a linker (for example, a hinge region); Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-second Fc region, wherein the heavy chain constant region CH1 is linked to the N-terminus of the second Fc region at the C-terminus thereof with or without a linker (for example, a hinge region);
Light chain 2: comprises or is composed of the following from the N-terminus to the C-terminus: the Second Fab light chain variable region-light chain constant region.

In some embodiments, the bispecific antibody of the present disclosure comprises one first antigen-binding region that specifically binds to a first antigen and two second antigen-binding regions that specifically bind to a second antigen, wherein the first antigen-binding region comprises a First Fab, the second antigen-binding region comprises a Second Fab, wherein one second Fab is linked to the N-terminus of the first Fc region at the C-terminus of the CH1 thereof (with or without a linker, for example, a hinge region), one first Fab is linked to the N-terminus of the second Fc region at the C-terminus of the CH1 thereof (with or without a linker, for example, a hinge region), and the other second Fab is linked to the N-terminus of the First Fab heavy chain variable region at the C-terminus of the CH1 thereof (with or without a linker). In a specific embodiment, the bispecific antibody has a 2+1N-terminal configuration, and the configuration shown in Figure 5A. In a specific embodiment, the one or two Second Fabs comprise the charge mutation according to the present disclosure, the First Fab comprises the charge mutation and disulfide bond reshaping mutation according to the present disclosure, and optionally, mutations are introduced into the first Fc region and the second Fc region based on Innobody or Knob-into-hole. Preferably, the two Second Fabs comprise the same charge mutation. Preferably, the charge mutation that the Second Fab comprises has the same position as the charge mutation that the First Fab comprises, but the charge carried by the mutated amino acid at the same position is the opposite. In one embodiment, the bispecific antibody comprises:
Heavy chain 1: comprises or is composed of the following from the N-terminus to the C-terminus: the Second Fab heavy chain variable region-heavy chain constant region CH1-First Fab heavy chain variable region-heavy chain constant region CH1-second Fc region, wherein the First Fab heavy chain constant region CH1 is linked to the N-terminus of the second Fc region at the C-terminus thereof with or without a linker (for example, a hinge region), and the Second Fab is linked to the N-terminus of the First Fab heavy chain variable region of at the C-terminus of the CH1 thereof (with or without a linker);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-first Fc region, wherein the heavy chain constant region CH1 is linked to the N-terminus of the first Fc region at the C-terminus thereof with or without a linker (for example, a hinge region); Light chain 2: comprises or is composed of the following from the N-terminus to the C-terminus: the Second Fab light chain variable region-light chain constant region,
wherein the bispecific antibody comprises two light chains 2.

In some embodiments, the bispecific antibody of the present disclosure comprises one first antigen-binding region that specifically binds to a first antigen and two second antigen-binding regions that specifically bind to a second antigen, wherein the first antigen-binding region comprises a First Fab, the second antigen-binding region comprises a Second Fab, wherein the two Second Fabs are linked to the N-terminus of the first Fc region and the N-terminus of the second Fc region, respectively, at the C-terminus of the CH1 thereof (with or without a linker, for example, a hinge region), and the N-terminus of the First Fab heavy chain variable region is linked to the C-terminus of the second Fc region (with or without a linker). In a specific embodiment, the bispecific antibody has a 2+1C-terminal configuration and the configuration shown in Figure 5B. In a specific embodiment, one or two Second Fabs comprise the charge mutation according to the present disclosure, the First Fab comprises the charge mutation and disulfide bond reshaping mutation according to the present disclosure, and optionally, the first Fc region and the second Fc region comprise an Innobody mutation or Knob-into-hole mutation. Preferably, the two Second Fabs comprise the same charge mutation. Preferably, the charge mutation that the First Fab comprises and the charge mutation that the Second Fab comprises have the same position, but the charge carried by the mutated amino acid at the same position is the opposite. In one embodiment, the bispecific antibody comprises
Heavy chain 1: comprises or is composed of the following from the N-terminus to the C-terminus: the Second Fab heavy chain variable region-heavy chain constant region CH1-second Fc region-First Fab heavy chain variable region-heavy chain constant region CH1, wherein the Second Fab is linked to the N-terminus of the second Fc region at the C-terminus of the CH1 thereof (with or without a linker, for example, a hinge region), and the N-terminus of the First Fab heavy chain variable region is linked to the C-terminus of the second Fc region (with or without a linker);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-first Fc region, wherein the heavy chain constant region CH1 is linked to the N-terminus of the first Fc region at the C-terminus thereof with or without a linker (for example, a hinge region);
Light chain 2: comprises or is composed of the following from the N-terminus to the C-terminus: the Second Fab light chain variable region-light chain constant region,
wherein the bispecific antibody comprises two light chains 2.

In some embodiments, the bispecific antibody of the present disclosure comprises two first antigen-binding regions that specifically bind to a first antigen and two second antigen-binding regions that specifically bind to a second antigen, wherein the first antigen-binding region comprises a First Fab, the second antigen-binding region comprises a Second Fab, wherein the two Second Fabs are linked to the N-terminus of the two Fc regions at the C-terminus of the CH1 thereof (with or without a linker, for example, a hinge region) (the Fc region may be the same or different), and the N-terminus of the heavy chain variable region of the two First Fabs is linked to the C-terminus of the two Fc regions (with or without a linker). In a specific embodiment, the bispecific antibody has a 2+2C-terminal configuration, and the configuration shown in Figure 5C. In a specific embodiment, one or two Second Fabs comprise the charge mutation according to the present disclosure, and one or two First Fabs comprise the charge mutation and disulfide bond reshaping mutation according to the present disclosure. In a specific embodiment, two Second Fabs comprise the same charge mutation of the present disclosure and/or two First Fabs comprise the charge mutation and disulfide bond reshaping mutation according to the present disclosure. Preferably, the charge mutation that the First Fab comprises and the charge mutation that the Second Fab comprises have the same position, but the charge carried by the mutated amino acid at the same position is the opposite. In one embodiment, the bispecific antibody comprises
Heavy chain: comprises or is composed of the following from the N-terminus to the C-terminus: the Second Fab heavy chain variable region-heavy chain constant region CH1 -Fc region-First Fab heavy chain variable region- heavy chain constant region CH1, wherein the Second Fab is linked to the N-terminus of the Fc region at the C-terminus of the CH1 thereof (with or without a linker, for example, a hinge region), and the N-terminus of the First Fab heavy chain variable region is linked to the C-terminus of the Fc region (with or without a linker).
Light chain 1: comprises or is composed of the following from the N-terminus to the C-terminus: the First Fab light chain variable region-light chain constant region;
Light chain 2: comprises or is composed of the following from the N-terminus to the C-terminus: the Second Fab light chain variable region-light chain constant region,
wherein the bispecific antibody comprises two heavy chains.

In some embodiments, the antibody of the present disclosure is a multispecific antibody. In some embodiments, the multispecific antibody of the present disclosure is a trispecific antibody.

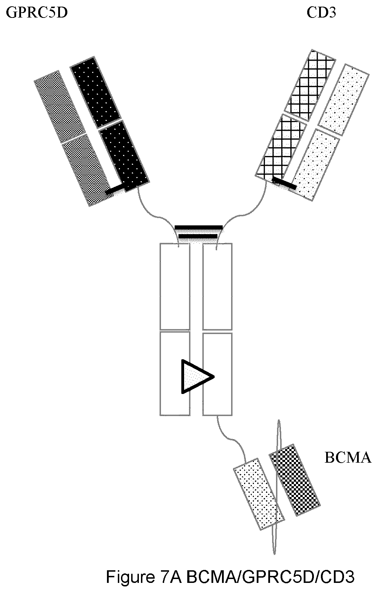

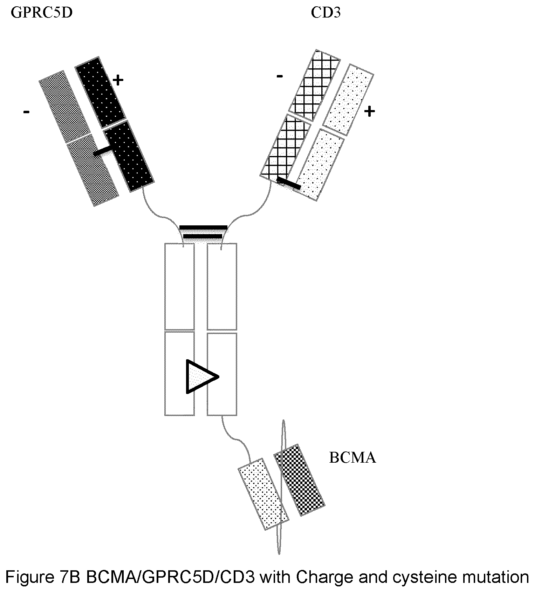

In some embodiments, the trispecific antibody of the present disclosure comprises a first antigen-binding region that specifically binds to a first antigen, a second antigen-binding region that specifically binds to a second antigen, and a third antigen-binding region that specifically binds to a third antigen, wherein the first antigen-binding region comprises a First Fab, the second antigen-binding region comprises a Second Fab, wherein the First Fab is linked to the N-terminus of the first Fc region at the C-terminus of the CH1 thereof (with or without a linker, for example, a hinge region), the Second Fab is linked to the N-terminus of the second Fc region at the C-terminus of the CH1 thereof (with or without a linker, for example, a hinge region), the third antigen-binding region comprises a scFv (for example, VH-VL, or VL-VH) that is linked to the C-terminus of the second Fc region at the N-terminus or C-terminus of the VH or VL thereof (for example, scFv is VL-VH, for example, the N-terminus of VL) with or without a linker. In a specific embodiment, the configuration of the trispecific antibody is a 2+1C-terminal configuration (Figure 7A or B). In a specific embodiment, the First Fab comprises a charge mutation and a disulfide bond reshaping mutation, the Second Fab comprises a charge mutation, and optionally, the first Fc region and the second Fc region comprise an Innobody mutation or Knob-into-hole mutation. Preferably, the charge mutation that the First Fab comprises and the charge mutation that the Second Fab comprises have the same position, but the charge carried by the mutated amino acid at the same position is the opposite. In one embodiment, the multispecific antibody comprises
Heavy chain 1: comprises or is composed of the following from the N-terminus to the C-terminus: the First Fab heavy chain variable region-heavy chain constant region CH1-first Fc region, wherein the First Fab is linked to the N-terminus of the first Fc region at the C-terminus of the CH1 thereof (with or without a linker, for example, a hinge region);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-second Fc region -scFv, wherein the heavy chain constant region CH1 is linked to the N-terminus of the second Fc region at the C-terminus of the heavy chain constant region CH1 with or without a linker (for example, a hinge region), and scFv is linked to the C-terminus of the second Fc region at the N-terminus or C-terminus of the VH or VL thereof (for example, scFv is VL-VH, for example, the N-terminus of VL) with or without a linker.
Light chain 2: comprises or is composed of the following from the N-terminus to the C-terminus: the Second Fab light chain variable region-light chain constant region.

### I-7. Antigen and antigen-binding region

The binding molecule of the present disclosure, such as an antibody, for example, a multispecific antibody, for example, a bispecific antibody, is capable of binding to any target, such as an antigen applicable to an antibody, or ligand, or receptor.

In some embodiments, the antigen is a tumor-associated antigen (TA). In some embodiments, the TA is an immune checkpoint molecule.

In some embodiments, the target or antigen is selected from FAP, CEA, p95 HER2, BCMA, EpCAM, MSLN, MCSP, HER-1, HER-2, HER-3, CD19, CD20, CD22, CD33, CD38, CD52Flt3, EpCAM, IGF-1R, FOLR1, Trop-2, CA-12-5, HLA-DR, MUC-1 (mucoprotein), GD2, A33-antigen, PSMA, PSCA, transferrin-receptor, TNC (tenascin), CA-IX, CD3, B7H3, EGFR, Hel, cMET, Axl, GPRC5D, PD-1, PD-L1, CD47, and immune activation molecules such as 4-1BB, CD40, and OX40.

In some embodiments, the target or antigen is selected from one or a plurality of the following targets/antigens: CD3, BCMA, CD20, Her2, Her3, B7H3, EGFR, Hel, cMET, Axl, GPRC5D, immune checkpoint molecules such as PD-1, PD-L1, CD47, 4-1BB, CD40, OX40, and different epitopes of the same antigen.

In some embodiments, the bispecific antibody of the present disclosure binds to two different epitopes of one antigen or binds to two antigens.

In some specific embodiments, the bispecific antibody of the present disclosure binds to two antigens selected from the following:
CD3/BCMA, CD3/CD20,CD3/Her2, Her2/Her3, B7H3/EGFR, PD-L1/CD47, PD-L1/CD40, PD-L1/4-1BB, EGFR/Her3, Her2/Hel, Her2/PD-1, Her2/PD-L1, Her2/CD47, Her2/cMet, and CD3/GPRC5D.

In some specific embodiments, the trispecific antibody of the present disclosure binds to BCMA, GPRC5D, and CD3.

The target-binding region or antigen-binding region of the binding molecule of the present disclosure, for example, an antibody, may be from an antibody bound to the above antigen.

### ➢ Antigen-binding region that specifically binds to Her2

In some embodiments, the antigen-binding region is from an antibody that specifically binds to Her2, for example, trastuzumab.

In some embodiments, the antigen-binding region comprises one, two, three, four, five, or six CDRs of a known antibody that specifically binds to Her2, such as trastuzumab.

In some embodiments, the antigen-binding region comprises one, two, and three heavy chain variable region CDRs, that is, HCDR1, HCDR2, and HCDR3, of a known antibody that specifically binds to Her2, such as trastuzumab.

In some embodiments, the antigen-binding region comprises one, two, and three light chain variable region CDRs, that is, LCDR1, LCDR2, and LCDR3, of a known antibody that specifically binds to Her2, such as trastuzumab.

In some embodiments, the antigen-binding region comprises three heavy chain variable region CDRs and three light chain variable region CDRs of a known antibody that specifically binds to Her2, such as trastuzumab.

In some embodiments, the antigen-binding region comprises a heavy chain variable region and a light chain variable region of a known antibody that specifically binds to Her2, such as trastuzumab., and the mutation according to the present disclosure.

In some embodiments, the antigen-binding region comprises a Fab of a known antibody that specifically binds to Her2, such as trastuzumab., and the mutation according to the present disclosure.

### ➢ Antigen-binding region that specifically binds to PD-1

In some embodiments, the antigen-binding region is from an antibody that specifically binds to PD-1, for example, the PD-1 antibody disclosed in WO2017025016, for example, sintilimab.

In some embodiments, the antigen-binding region comprises one, two, three, four, five, or six CDRs of a known antibody that specifically binds to PD-1, for example, the PD-1 antibody disclosed in WO2017025016, for example, sintilimab.

In some embodiments, the antigen-binding region comprises one, two, and three heavy chain variable region CDRs, that is, HCDR1, HCDR2, and HCDR3, of a known antibody that specifically binds to PD-1, for example, the PD-1 antibody disclosed in WO2017025016, for example, sintilimab.

In some embodiments, the antigen-binding region comprises one, two, and three light chain variable region CDRs, that is, LCDR1, LCDR2, and LCDR3, of a known antibody that specifically binds to PD-1, for example, the PD-1 antibody disclosed in WO2017025016, for example, sintilimab.

In some embodiments, the antigen-binding region comprises three heavy chain variable region CDRs and three light chain variable region CDRs of a known antibody that specifically binds to PD-1, for example, the PD-1 antibody disclosed in WO2017025016, for example, sintilimab.

In some embodiments, the antigen-binding region comprises a heavy chain variable region and a light chain variable region of a known antibody that specifically binds to PD-1, for example, the PD-1 antibody disclosed in WO2017025016, for example, sintilimab, and the mutation according to the present disclosure.

In some embodiments, the antigen-binding region comprises a Fab of a known antibody that specifically binds to PD-1, for example, the PD-1 antibody disclosed in WO2017025016, for example, sintilimab, and the mutation according to the present disclosure.

### ➢ Antigen-binding region that specifically binds to CD47

In some embodiments, the antigen-binding region is from an antibody that specifically binds to CD47, for example, the antibody disclosed in WO2019042285A1 that specifically binds to CD47, for example, ADI26630 disclosed therein.

In some embodiments, the antigen-binding region comprises one, two, three, four, five or six CDRs of a known antibody that specifically binds to CD47, for example, the antibody disclosed in WO2019042285A1 that specifically binds to CD47, for example, ADI26630.

In some embodiments, the antigen-binding region comprises one, two, and three heavy chain variable region CDRs, that is, HCDR1, HCDR2, and HCDR3 of a known antibody that specifically binds to CD47, for example, the antibody disclosed in WO2019042285A1 that specifically binds to CD47, for example, ADI26630.

In some embodiments, the antigen-binding region comprises one, two, and three light chain variable region CDRs, that is, LCDR1, LCDR2, and LCDR3 of a known antibody that specifically binds to CD47, for example, the antibody disclosed in WO2019042285A1 that specifically binds to CD47, for example, ADI26630.

In some embodiments, the antigen-binding region comprises three heavy chain variable region CDRs and three light chain variable region CDRs of a known antibody that specifically binds to CD47, for example, the antibody disclosed in WO2019042285A1 that specifically binds to CD47, for example, ADI26630.

In some embodiments, the antigen-binding region comprises a heavy chain variable region and a light chain variable region of a known antibody that specifically binds to CD47, for example, the antibody disclosed in WO2019042285A1 that specifically binds to CD47, for example, ADI26630, and the mutation according to the present disclosure.

In some embodiments, the antigen-binding region comprises a known antibody that specifically binds to CD47, for example, the antibody disclosed in WO2019042285A1 that specifically binds to CD47, for example, ADI26630 disclosed therein, and the mutation according to the present disclosure.

### ➢ Antigen-binding region that specifically binds to cMet

In some embodiments, the antigen-binding region is from an antibody that specifically binds to cMet, for example, onartuzumab.

In some embodiments, the antigen-binding region comprises one, two, three, four, five or six CDRs of a known antibody that specifically binds to cMet, for example, onartuzumab.

In some embodiments, the antigen-binding region comprises one, two, and three heavy chain variable region CDRs, that is, HCDR1, HCDR2, and HCDR3, of a known antibody that specifically binds to cMet, for example, onartuzumab.

In some embodiments, the antigen-binding region comprises one, two, and three light chain variable region CDRs, that is, LCDR1, LCDR2, and LCDR3, of a known antibody that specifically binds to cMet, for example, onartuzumab.

In some embodiments, the antigen-binding region comprises three heavy chain variable region CDRs and three light chain variable region CDRs of a known antibody that specifically binds to cMet, for example, onartuzumab.

In some embodiments, the antigen-binding region comprises a heavy chain variable region and a light chain variable region of a known antibody that specifically binds to cMet, for example, onartuzumab, and the mutation according to the present disclosure.

Some antigen-binding regions comprise a Fab of a known antibody that specifically binds to cMet, for example, onartuzumab, and the mutation according to the present disclosure.

### ➢ Antigen-binding region that specifically binds to GPRC5D

In some embodiments, the antigen-binding region is from an antibody that specifically binds to GPRC5D, for example, the antibody disclosed in PCT/CN2022/076832 that specifically binds to GPRC5D.

In some embodiments, the antibody that specifically binds to GPRC5D comprises three CDRs from the heavy chain variable region (HCDRs), namely HCDR1, HCDR2, and HCDR3, wherein the HCDR1, HCDR2, and HCDR3 are the HCDR1, HCDR2, and HCDR3 of the heavy chain variable region shown in SEQ ID NO:87.

In some embodiments, the antibody that specifically binds to GPRC5D comprises three CDRs from the light chain variable region (LCDRs), namely LCDR1, LCDR2, and LCDR3, wherein the LCDR1, LCDR2, and LCDR3 are the LCDR1, LCDR2, and LCDR3 of the light chain variable region shown in SEQ ID NO:83.

In some embodiments, the HCDR1 of the antibody that specifically binds to GPRC5D comprises or is composed of the amino acid sequence of SEQ ID NO:88; HCDR2 comprises or is composed of the amino acid sequence of SEQ ID NO:89, HCDR3 comprises or is composed of the amino acid sequence of SEQ ID NO:90, LCDR1 comprises or is composed of the amino acid sequence of SEQ ID NO:84, LCDR2 comprises or is composed of the amino acid sequence of SEQ ID NO:85, and/or LCDR3 comprises or is composed of the amino acid sequence of SEQ ID NO:86.

In some embodiments, the antibody that specifically binds to GPRC5D comprises or is composed of three CDRs from the heavy chain variable region (HCDRs), namely HCDR1, HCDR2, and HCDR3, and three CDRs from the light chain variable region (LCDRs), namely LCDR1, LCDR2, and LCDR3, wherein HCDR1 comprises or is composed of the amino acid sequence of SEQ ID NO:88; HCDR2 comprises or is composed of the amino acid sequence of SEQ ID NO:89, HCDR3 comprises or is composed of the amino acid sequence of SEQ ID NO:90, LCDR1 comprises or is composed of the amino acid sequence of SEQ ID NO:84, LCDR2 comprises or is composed of the amino acid sequence of SEQ ID NO:85, and/or LCDR3 comprises or is composed of the amino acid sequence of SEQ ID NO:86.

In some aspects, the antibody that specifically binds to GPRC5D comprises a heavy chain variable region (VH), wherein the VH
(i) comprises or is composed of an amino acid sequence with at least 90%, 91%, 92%, 93%, 94%, 95%, 96%, 97%, 98%, or 99% identity with the amino acid sequence shown in SEQ ID NO:87; or
(ii) comprises or is composed of the amino acid sequence of SEQ ID NO:87; or
(iii) comprises or is composed of the amino acid sequence with one or a plurality of (preferably no more than 10, more preferably no more than five, four, three, two, and one) amino acid changes (preferably amino acid substitutions, more preferably conservative amino acid substitutions) of SEQ ID NO:87, preferably, the amino acid changes do not occur in the CDR.

In some aspects, the antibody that specifically binds to GPRC5D comprises a light chain variable region (VL), wherein the VL
(i) comprises or is composed of an amino acid sequence with at least 90%, 91%, 92%, 93%, 94%, 95%, 96%, 97%, 98%, or 99% identity with the amino acid sequence shown in SEQ ID NO:83; or
(ii) comprises or is composed of the amino acid sequence of SEQ ID NO:83; or
(iii) comprises or is composed of the amino acid sequence with one or a plurality of (preferably no more than 10, more preferably no more than five, four, three, two, and one) amino acid changes (preferably amino acid substitutions, more preferably conservative amino acid substitutions) of SEQ ID NO:83, preferably, the amino acid changes do not occur in the CDR.

In some embodiments, the antibody that specifically binds to GPRC5D comprises VH and VL, wherein VH comprises or is composed of the sequence shown in SEQ ID NO:87; and/or VL comprises or is composed of the sequence shown in SEQ IQ NO:83.

In some embodiments, the antigen-binding region comprises one, two, three, four, five or six CDRs of a known or the above antibody that specifically binds to GPRC5D.

In some embodiments, the antigen-binding region comprises one, two, and three heavy chain variable region CDRs, that is, HCDR1, HCDR2, and HCDR3, of a known or the above antibody that specifically binds to GPRC5D.

In some embodiments, the antigen-binding region comprises one, two, and three light chain variable region CDRs, that is, LCDR1, LCDR2, and LCDR3, of a known or the above antibody that specifically binds to GPRC5D.

In some embodiments, the antigen-binding region comprises three heavy chain variable region CDRs and three light chain variable region CDRs of a known or the above antibody that specifically binds to GPRC5D.

In some embodiments, the antigen-binding region comprises a heavy chain variable region and light chain variable region of a known or the above antibody that specifically binds to GPRC5D, and the mutation according to the present disclosure.

Some antigen-binding regions comprise a Fab of a known or the above antibody that specifically binds to GPRC5D, and the mutation according to the present disclosure.

### ➢ Antigen-binding region that specifically binds to CD3

In some embodiments, the antigen-binding region is from an antibody that specifically binds to CD3, for example, the antibody disclosed in WO2022068809 that specifically binds to CD3, for example, sp34.24 or sp34.87 therein.

In some embodiments, the antigen-binding region comprises one, two, three, four, five or six CDRs of a known antibody that specifically binds to CD3, for example, the antibody disclosed in WO2022068809 that specifically binds to CD3, for example, sp34.24 or sp34.87.

In some embodiments, the antigen-binding region comprises one, two, and three heavy chain variable region CDRs, that is, HCDR1, HCDR2, and HCDR3 of a known antibody that specifically binds to CD3, for example, the antibody disclosed in WO2022068809 that specifically binds to CD3, for example, sp34.24 or sp34.87.

In some embodiments, the antigen-binding region comprises one, two, and three light chain variable region CDRs, that is, LCDR1, LCDR2, and LCDR3, of a known antibody that specifically binds to CD3, for example, the antibody disclosed in WO2022068809 that specifically binds to CD3, for example, sp34.24 or sp34.87.

In some embodiments, the antigen-binding region comprises three heavy chain variable region CDRs and three light chain variable region CDRs of a known antibody that specifically binds to CD3, for example, the antibody disclosed in WO2022068809 that specifically binds to CD3, for example, sp34.24 or sp34.87.

In some embodiments, the antigen-binding region comprises a heavy chain variable region and light chain variable region of a known antibody that specifically binds to CD3, for example, the antibody disclosed in WO2022068809 that specifically binds to CD3, for example, sp34.24 or sp34.87, and the mutation according to the present disclosure.

Some antigen-binding regions comprise a Fab of a known antibody that specifically binds to CD3, for example, the antibody disclosed in WO2022068809 that specifically binds to CD3, for example, sp34.24 or sp34.87, and the mutation according to the present disclosure.

### ➢ Antigen-binding region that specifically binds to BCMA

In some embodiments, the antigen-binding region is from an antibody that specifically binds to BCMA, for example, the antibody disclosed in PCT/CN2022/076832 that specifically binds to BCMA.

In some embodiments, the antibody that specifically binds to BCMA comprises three CDRs from the heavy chain variable region (HCDRs), namely HCDR1, HCDR2, and HCDR3, wherein the HCDR1, HCDR2, and HCDR3 are the HCDR1, HCDR2, and HCDR3 of the heavy chain variable region shown in SEQ ID NO:103.

In some embodiments, the antibody that specifically binds to BCMA comprises three CDRs from the light chain variable region (LCDRs), namely LCDR1, LCDR2, and LCDR3, wherein the LCDR1, LCDR2, and LCDR3 are the LCDR1, LCDR2, and LCDR3 of the light chain variable region shown in SEQ ID NO:107.

In some embodiments, the HCDR1 of the antibody that specifically binds to BCMA comprises or is composed of the amino acid sequence of SEQ ID NO:104; HCDR2 comprises or is composed of the amino acid sequence of SEQ ID NO:105, HCDR3 comprises or is composed of the amino acid sequence of SEQ ID NO:106, LCDR1 comprises or is composed of the amino acid sequence of SEQ ID NO:108, LCDR2 comprises or is composed of the amino acid sequence of SEQ ID NO:109, and/or LCDR3 comprises or is composed of the amino acid sequence of SEQ ID NO:110.

In some embodiments, the antibody that specifically binds to BCMA comprises three CDRs from the heavy chain variable region (HCDRs), namely HCDR1, HCDR2, and HCDR3, and three CDRs from the light chain variable region (LCDRs), namely LCDR1, LCDR2, and LCDR3, wherein HCDR1 comprises or is composed of the amino acid sequence of SEQ ID NO:104; HCDR2 comprises or is composed of the amino acid sequence of SEQ ID NO:105, HCDR3 comprises or is composed of the amino acid sequence of SEQ ID NO:106, LCDR1 comprises or is composed of the amino acid sequence of SEQ ID NO:108, LCDR2 comprises or is composed of the amino acid sequence of SEQ ID NO:109, and/or LCDR3 comprises or is composed of the amino acid sequence of SEQ ID NO:110.

In some aspects, the antibody that specifically binds to BCMA comprises a heavy chain variable region (VH), wherein the VH
(i) comprises or is composed of an amino acid sequence with at least 90%, 91%, 92%, 93%, 94%, 95%, 96%, 97%, 98%, or 99% identity with the amino acid sequence shown in SEQ ID NO:103; or
(ii) comprises or is composed of the amino acid sequence of SEQ ID NO:103; or
(iii) comprises or is composed of the amino acid sequence with one or a plurality of (preferably no more than 10, more preferably no more than five, four, three, two, and one) amino acid changes (preferably amino acid substitutions, more preferably conservative amino acid substitutions) of SEQ ID NO:103, preferably, the amino acid changes do not occur in the CDR.

In some aspects, the antibody that specifically binds to BCMA comprises a light chain variable region (VL), wherein the VL
(i) comprises or is composed of an amino acid sequence with at least 90%, 91%, 92%, 93%, 94%, 95%, 96%, 97%, 98%, or 99% identity with the amino acid sequence shown in SEQ ID NO:107; or
(ii) comprises or is composed of the amino acid sequence of SEQ ID NO:107; or
(iii) comprises or is composed of the amino acid sequence with one or a plurality of (preferably no more than 10, more preferably no more than five, four, three, two, and one) amino acid changes (preferably amino acid substitutions, more preferably conservative amino acid substitutions) of SEQ ID NO:107, preferably, the amino acid changes do not occur in the CDR.

In some embodiments, the antibody that specifically binds to BCMA comprises a VH and a VL, wherein the VH comprises or is composed of the sequence shown in SEQ ID NO:103; and/or the VL comprises or is composed of the sequence shown in SEQ IQ NO:107.

In some embodiments, the antigen-binding region comprises one, two, three, four, five, or six CDRs of a known or the above antibody that specifically binds to BCMA.

In some embodiments, the antigen-binding region comprises one, two, and three heavy chain variable region CDRs, that is, HCDR1, HCDR2, and HCDR3, of a known or the above antibody that specifically binds to BCMA.

In some embodiments, the antigen-binding region comprises one, two, and three light chain variable region CDRs, that is, LCDR1, LCDR2, and LCDR3, of a known or the above antibody that specifically binds to BCMA.

In some embodiments, the antigen-binding region comprises three heavy chain variable region CDRs and three light chain variable region CDRs of a known or the above antibody that specifically binds to BCMA.

In some embodiments, the antigen-binding region comprises a heavy chain variable region and light chain variable region of a known or the above antibody that specifically binds to BCMA, and the mutation according to the present disclosure.

Some antigen-binding regions comprise a Fab of a known or the above antibody that specifically binds to BCMA, and the mutation according to the present disclosure.

### 1.8. Binding fragment of binding molecule

The present disclosure also relates to a binding fragment herein, for example, an antibody fragment, particularly a fragment of a target molecule bound by a specific binding molecule, for example, an antigen-binding fragment.

In some embodiments, the antigen-binding fragment is Fab.

In some embodiments, the antigen-binding fragment comprises one or a plurality of Fabs, wherein one or a plurality of Fabs comprise the mutation or mutation combination according to the present disclosure.

### 1.9. Exemplary embodiments

1. An antibody or antigen-binding fragment thereof, comprising one or a plurality of first antigen-binding regions that specifically bind to a first antigen and one or a plurality of second antigen-binding regions that specifically bind to a second antigen, wherein at least one or a plurality of the antigen-binding regions comprise a heavy chain CH1 and light chain CL, and comprises a disulfide bond reshaping mutation at the CH1-CL interface, and/or at least one or a plurality of the antigen-binding regions comprise a heavy chain variable region and a light chain variable region, wherein an amino acid pair at the heavy chain variable region and light chain variable region interface comprises a charge mutation that mutates the two amino acids on the amino acid pair into amino acids with opposite charges, respectively; wherein the first antigen and second antigen may be the same or different;
preferably, only one antigen-binding region comprises the disulfide bond reshaping mutation at the CH1-CL interface.
2. The antibody or antigen-binding fragment thereof according to Embodiment 1, wherein the disulfide bond reshaping mutation comprises
(i) substituting a non-Cys amino acid at position 126 of the heavy chain CH1 to a Cys amino acid (EU number), and substituting a non-Cys amino acid at position 124 of the light chain CL to a Cys amino acid (EU number);
(ii) substituting a non-Cys amino acid at position 168 of the heavy chain CH1 to a Cys amino acid (EU number), and substituting a non-Cys amino acid at position 164 of the light chain CL to a Cys amino acid (EU number);
(iii) substituting a non-Cys amino acid at position 170 of the heavy chain CH1 to a Cys amino acid (EU number), and substituting a non-Cys amino acid at position 162 or 164 of the light chain CL to a Cys amino acid (EU number);
(iv) substituting a non-Cys amino acid at position 173 of the heavy chain CH1 to a Cys amino acid (EU number), and substituting a non-Cys amino acid at position 160 or 162 of the light chain CL to a Cys amino acid (EU number);
(v) substituting a non-Cys amino acid at position 128 of the heavy chain CH1 to a Cys amino acid (EU number), and substituting a non-Cys amino acid at position 118 of the light chain CL to a Cys amino acid (EU number);
(vi) substituting a non-Cys amino acid at position 134 of the heavy chain CH1 to a Cys amino acid (EU number), and substituting a non-Cys amino acid at position 116 of the light chain CL to a Cys amino acid (EU number);
(vii) substituting a non-Cys amino acid at position 136 of the heavy chain CH1 to a Cys amino acid (EU number), and substituting a non-Cys amino acid at position 114 of the light chain CL to a Cys amino acid (EU number);
(viii) substituting a non-Cys amino acid at position 171 of the heavy chain CH1 to a Cys amino acid (EU number), and substituting a non-Cys amino acid at position 162 of the light chain CL to a Cys amino acid (EU number);
(ix) substituting a non-Cys amino acid at position 139 of the heavy chain CH1 to a Cys amino acid (EU number), and substituting a non-Cys amino acid at position 116 of the light chain CL to a Cys amino acid (EU number).

3. The antibody or antigen-binding fragment thereof according to Embodiment 2, wherein the disulfide bond reshaping mutation comprises
(i) F126C in CH1 and Q124C in CL (EU number);
(ii) H168C in CH1 and T164C in CL (EU number);
(iii) F170C in CH1 and T164C in CL (EU number);
(iv) F170C in CH1 and S162C in CL (EU number);
(v) V173C in CH1 and S162C in CL (EU number);
(vi) V173C in CH1 and Q160C in CL (EU number);
(vii) L128C in CH1 and F118C in CL (EU number);
(viii) S134C in CH1 and F116C in CL (EU number);
(ix) S136C in CH1 and S114C in CL (EU number);
(x) P171C in CH1 and S162C in CL (EU number);
(xi) T139C in CH1 and F116C in CL (EU number).

4. The antibody or antigen-binding fragment thereof, comprising one or a plurality of first antigen-binding regions that specifically bind to a first antigen and one or a plurality of second antigen-binding regions that specifically bind to a second antigen, wherein at least one of the antigen-binding regions comprise a heavy chain CH1 and light chain CL, and comprises a disulfide bond reshaping mutation at the CH1-CL interface, wherein the disulfide bond reshaping mutation comprises T139C in CH1 and F116C in CL, wherein the first antigen-binding region and the second antigen-binding region may the same or different.
5. The antibody or antigen-binding fragment thereof according to any one of Embodiments 2 - 4, wherein the disulfide bond reshaping mutation also comprises substituting an interchain Cys at the heavy chain constant region-light chain constant region interface with a non-Cys.
6. The antibody or antigen-binding fragment thereof according to Embodiment 5, wherein substituting an interchain Cys with a non-Cys comprises substituting the Cys at position 220 in the CH1 with a non-Cys, and substituting the Cys at position 214 of the CL with a non-Cys.
7. The antibody or antigen-binding fragment thereof according to Embodiment 6, wherein substituting an interchain Cys with a non-Cys comprises C220S/A/V in CH1 and C214S/A/V in CL (EU number).
8. The antibody or antigen-binding fragment thereof according to Embodiment 6, wherein the disulfide bond reshaping mutation comprises
(i) F126C and C220S in CH1 and Q124C and C214S in CL (EU number);
(ii) H168C and C220S in CH1 and T164C and C214S in CL (EU number);
(iii) F170C and C220S in CH1 and T164C and C214S in CL (EU number);
(iv) F170C and C220S in CH1 and S162C and C214S in CL (EU number);
(v) V173C and C220S in CH1 and S162C and C214S in CL (EU number);
(vi) V173C and C220S in CH1 and Q160C and C214S in CL (EU number);
(vii) L128C and C220S in CH1 and F118C and C214S in CL (EU number);
(viii) S134C and C220S in CH1 and F116C and C214S in CL (EU number);
(ix) S136C and C220S in CH1 and S114C and C214S in CL (EU number);
(x) P171C and C220S in CH1 and S162C and C214S in CL (EU number);
(xi) T139C and C220S in CH1 and F116C and C214S in CL (EU number).

9. The antibody or antigen-binding fragment thereof according to any one of Embodiments 1 - 8, wherein the CH1 region is a human IgG1 CH1, for example, human IgG1 CH1, human IgG2 CH1, human IgG3 CH1, or human IgG4 CH1, for example, the CH1 region comprises the amino acid sequence shown in SEQ ID NO:116;
and/or the CL region is a human Kappa light chain CL region or a human Lambda light chain CL region, for example, the CL region comprises the amino acid sequence of SEQ ID NO:54 or SEQ ID NO:55.
10. The antibody or antigen-binding fragment thereof according to Embodiment 8 or 9, wherein CH1
(i) comprises an amino acid sequence with at least 90%, 91%, 92%, 93%, 94%, 95%, 96%, 97%, 98%, or 99% identity with the amino acid sequence shown in SEQ ID NO:129, and comprises F126C and C220S; or comprises or is composed of the amino acid sequence shown in SEQ ID NO:129;
(ii) comprises an amino acid sequence with at least 90%, 91%, 92%, 93%, 94%, 95%, 96%, 97%, 98%, or 99% identity with the amino acid sequence shown in SEQ ID NO:133, and comprises H168C and C220S; or comprises or is composed of the amino acid sequence shown in SEQ ID NO:133;
(iii) comprises an amino acid sequence with at least 90%, 91%, 92%, 93%, 94%, 95%, 96%, 97%, 98%, or 99% identity with the amino acid sequence shown in SEQ ID NO:135, and comprises F170C and C220S; or comprises or is composed of the amino acid sequence shown in SEQ ID NO:135;
(iv) comprises an amino acid sequence with at least 90%, 91%, 92%, 93%, 94%, 95%, 96%, 97%, 98%, or 99% identity with the amino acid sequence shown in SEQ ID NO:137, and comprises V173C and C220S; or comprises or is composed of the amino acid sequence shown in SEQ ID NO:137;
(v) comprises an amino acid sequence with at least 90%, 91%, 92%, 93%, 94%, 95%, 96%, 97%, 98%, or 99% identity with the amino acid sequence shown in SEQ ID NO:139, and comprises L128C and C220S; or comprises or is composed of the amino acid sequence shown in SEQ ID NO:139;
(vi) comprises an amino acid sequence with at least 90%, 91%, 92%, 93%, 94%, 95%, 96%, 97%, 98%, or 99% identity with the amino acid sequence shown in SEQ ID NO:141, and comprises S134C and C220S; or comprises or is composed of the amino acid sequence shown in SEQ ID NO:141;
(vii) comprises an amino acid sequence with at least 90%, 91%, 92%, 93%, 94%, 95%, 96%, 97%, 98%, or 99% identity with the amino acid sequence shown in SEQ ID NO:143, and comprises S136C and C220S; or comprises or is composed of the amino acid sequence shown in SEQ ID NO:143;
(viii) comprises an amino acid sequence with at least 90%, 91%, 92%, 93%, 94%, 95%, 96%, 97%, 98%, or 99% identity with the amino acid sequence shown in SEQ ID NO:145, and comprises P171C and C220S; or comprises or is composed of the amino acid sequence shown in SEQ ID NO:145;
(ix) comprises an amino acid sequence with at least 90%, 91%, 92%, 93%, 94%, 95%, 96%, 97%, 98%, or 99% identity with the amino acid sequence shown in SEQ ID NO:147, and comprises T139C and C220S; or comprises or is composed of the amino acid sequence shown in SEQ ID NO:147.

11. The antibody or antigen-binding fragment thereof according to any one of Embodiments 8 - 10, wherein CL
(i) comprises an amino acid sequence with at least 90%, 91%, 92%, 93%, 94%, 95%, 96%, 97%, 98%, or 99% identity with the amino acid sequence shown in SEQ ID NO:128, and comprises Q124C and C124S; or comprises or is composed of the amino acid sequence shown in SEQ ID NO:128;
(ii) comprises an amino acid sequence with at least 90%, 91%, 92%, 93%, 94%, 95%, 96%, 97%, 98%, or 99% identity with the amino acid sequence shown in SEQ ID NO:149, and comprises T164C and C124S; or comprises or is composed of the amino acid sequence shown in SEQ ID NO:149;
(iii) comprises an amino acid sequence with at least 90%, 91%, 92%, 93%, 94%, 95%, 96%, 97%, 98%, or 99% identity with the amino acid sequence shown in SEQ ID NO:151, and comprises S162C and C124S; or comprises or is composed of the amino acid sequence shown in SEQ ID NO:151;
(iv) comprises an amino acid sequence with at least 90%, 91%, 92%, 93%, 94%, 95%, 96%, 97%, 98%, or 99% identity with the amino acid sequence shown in SEQ ID NO:35, and comprises Q160C and C124S; or comprises or is composed of the amino acid sequence shown in SEQ ID NO:35;
(v) comprises an amino acid sequence with at least 90%, 91%, 92%, 93%, 94%, 95%, 96%, 97%, 98%, or 99% identity with the amino acid sequence shown in SEQ ID NO:37, and comprises F118C and C124S; or comprises or is composed of the amino acid sequence shown in SEQ ID NO:37;
(vii) comprises an amino acid sequence with at least 90%, 91%, 92%, 93%, 94%, 95%, 96%, 97%, 98%, or 99% identity with the amino acid sequence shown in SEQ ID NO:98, and comprises F116C and C124S; or comprises or is composed of the amino acid sequence shown in SEQ ID NO:98; or
(viii) comprises an amino acid sequence with at least 90%, 91%, 92%, 93%, 94%, 95%, 96%, 97%, 98%, or 99% identity with the amino acid sequence shown in SEQ ID NO:100, and comprises S114C and C124S; or comprises or is composed of the amino acid sequence shown in SEQ ID NO:100.

12. The antibody or antigen-binding fragment thereof according to any one of Embodiments 1-11, wherein the position of the amino acid pair at the heavy chain variable region and light chain variable region interface is selected from: position 39 of the heavy chain variable region and position 38 of the light chain variable region, position 45 of the heavy chain variable region and position 87 of the light chain variable region, position 45 of the heavy chain variable region and position 44 of the light chain variable region, position 91 of the heavy chain variable region and position 43 of the light chain variable region, or position 91 of the heavy chain variable region and position 44 of the light chain variable region (Kabat number).
13. The antibody or antigen-binding fragment thereof according to any one of Embodiments 1-12, wherein the position of the amino acid pair at the heavy chain variable region and light chain variable region interface in at least one first antigen-binding region is the same as the position in at least one second antigen-binding region.
14. The antibody or antigen-binding fragment thereof according to Embodiment 13, wherein the mutated amino acid at the position of the amino acid pair in the first antigen-binding region has the opposite charge to the mutated amino acid at the same position in the second antigen-binding region.
15. The antibody or antigen-binding fragment thereof according to Embodiment 14, wherein
(1) in the first antigen-binding region, the amino acid at position 39 of the heavy chain variable region is substituted with an amino acid carrying a positive charge, and the amino acid at position 38 of the light chain variable region is substituted with an amino acid carrying a negative charge; and in the second antigen-binding region, the amino acid at position 39 of the heavy chain variable region is substituted with an amino acid carrying a negative charge, and the amino acid at position 38 of the light chain variable region is substituted with an amino acid carrying a positive charge;
(2) in the first antigen-binding region, the amino acid at position 45 of the heavy chain variable region is substituted with an amino acid carrying a positive charge, and the amino acid at position 87 of the light chain variable region is substituted with an amino acid carrying a negative charge; and in the second antigen-binding region, the amino acid at position 45 of the heavy chain variable region is substituted with an amino acid carrying a negative charge, and the amino acid at position 87 of the light chain variable region is substituted with an amino acid carrying a positive charge;
(3) in the first antigen-binding region, the amino acid at position 45 of the heavy chain variable region is substituted with an amino acid carrying a positive charge, and the amino acid at position 44 of the light chain variable region is substituted with an amino acid carrying a negative charge; and in the second antigen-binding region, the amino acid at position 45 of the heavy chain variable region is substituted with an amino acid carrying a negative charge, and the amino acid at position 44 of the light chain variable region is substituted with an amino acid carrying a positive charge;
(4) in the first antigen-binding region, the amino acid at position 91 of the heavy chain variable region is substituted with an amino acid carrying a positive charge, and the amino acid at position 43 of the light chain variable region is substituted with an amino acid carrying a negative charge; and in the second antigen-binding region, the amino acid at position 91 of the heavy chain variable region is substituted with an amino acid carrying a negative charge, and the amino acid at position 43 of the light chain variable region is substituted with an amino acid carrying a positive charge; or
(5) in the first antigen-binding region, the amino acid at position 91 of the heavy chain variable region is substituted with an amino acid carrying a positive charge, and the amino acid at position 44 of the light chain variable region is substituted with an amino acid carrying a negative charge; and in the second antigen-binding region, the amino acid at position 91 of the heavy chain variable region is substituted with an amino acid carrying a negative charge, and the amino acid at position 44 of the light chain variable region is substituted with an amino acid carrying a positive charge.

16. The antibody or antigen-binding fragment thereof according to Embodiment 15, wherein the amino acid carrying a positive charge is selected from K, R, or H, and/or the amino acid carrying a negative charge is selected from E or D.
17. The antibody or antigen-binding fragment thereof according to Embodiment 16, wherein
(i). in the first antigen-binding region, the amino acid at position 39 of the heavy chain variable region is substituted with K, and the amino acid at position 38 of the light chain variable region is substituted with D, while in the second antigen-binding region, the amino acid at position 39 of the heavy chain variable region is substituted with D, and the amino acid at position 38 of the light chain variable region is substituted with K;
(ii). in the first antigen-binding region, the amino acid at position 45 of the heavy chain variable region is substituted with K, and the amino acid at position 87 of the light chain variable region is substituted with D, while in the second antigen-binding region, the amino acid at position 45 of the heavy chain variable region is substituted with D, and the amino acid at position 87 of the light chain variable region is substituted with K;
(iii). in the first antigen-binding region, the amino acid at position 45 of the heavy chain variable region is substituted with K, and the amino acid at position 44 of the light chain variable region is substituted with D, while in the second antigen-binding region, the amino acid at position 45 of the heavy chain variable region is substituted with D, and the amino acid at position 44 of the light chain variable region is substituted with K;
(iv). in the first antigen-binding region, the amino acid at position 91 of the heavy chain variable region is substituted with K, and the amino acid at position 43 of the light chain variable region is substituted with D, while in the second antigen-binding region, the amino acid at position 91 of the heavy chain variable region is substituted with D, and the amino acid at position 43 of the light chain variable region is substituted with K;
(v). in the first antigen-binding region, the amino acid at position 91 of the heavy chain variable region is substituted with K, and the amino acid at position 44 of the light chain variable region is substituted with D, while in the second antigen-binding region, the amino acid at position 91 of the heavy chain variable region is substituted with D, and the amino acid at position 44 of the light chain variable region is substituted with K.

18. The antibody or antigen-binding fragment thereof according to any one of Embodiments 1- 17, wherein the multispecific antibody comprises a charge mutation and a disulfide bond reshaping mutation.
19. The antibody or antigen-binding fragment thereof according to any one of Embodiments 1 - 18, wherein one or a plurality or all the antigen-binding regions that bind to the same antigen comprise the same mutation.
20. The antibody or antigen-binding fragment thereof according to any one of Embodiments 1 - 19, wherein the first antigen-binding region comprises a heavy chain variable region, a heavy chain constant region CH1, a light chain variable region and a light chain constant region CL, and the second antigen-binding region comprises a heavy chain variable region VH and a light chain variable region VL.
21. The antibody or antigen-binding fragment thereof according to Embodiment 20, wherein
1) the first antigen-binding region comprises the disulfide bond reshaping mutation defined in any one of Embodiments 1 - 17; or
2) the first antigen-binding region comprises the charge mutation defined in any one of Embodiments 1 - 17;
3) the first antigen-binding region comprises the disulfide bond reshaping mutation and charge mutation defined in any one of Embodiments 1 - 17; or
4) the first antigen-binding region comprises the disulfide bond reshaping mutation and charge mutation defined in any one of Embodiments 1 - 17; and the second antigen-binding region comprises the charge mutation defined in any one of Embodiments 1 - 17.

22. The antibody or antigen-binding fragment thereof according to Embodiment 21, wherein
1) the first antigen-binding region comprises the disulfide bond reshaping mutation defined in any one of Embodiments 2 - 8;
2) the first antigen-binding region comprises the charge mutation defined in any one of Embodiments 12 - 17;
3) the first antigen-binding region comprises the disulfide bond reshaping mutation defined in any one of Embodiments 2 - 8 and comprises the charge mutation defined in any one of Embodiments 12 - 17; or
4) the first antigen-binding region and the second antigen-binding region comprise the charge mutation defined in any one of Embodiments 12 - 17, and the first antigen-binding region also comprises the disulfide bond reshaping mutation defined in any one of Embodiments 2 - 8.

23. The antibody or antigen-binding fragment thereof according to Embodiment 22, wherein the first antigen-binding region of the multispecific antibody comprises mutation combinations selected from the following:

| | | | | |
|---|---|---|---|---|
| | Heavy Chain VH Mutation | Heavy Chain CH1 Mutation | Light Chain VL Mutation | Light Chain CL Mutation |
| Combination 1 | Q39K | F126C, C220S | Q38D | Q124C, C214S |
| | Q39K | H168C, C220S | Q38D | T164C, C214S |
| | Q39K | F170C, C220S | Q38D | T164C, C214S |
| | Q39K | F170C, C220S | Q38D | S162C, C214S |
| | Q39K | V173C, C220S | Q38D | S162C, C214S |
| | Q39K | V173C, C220S | Q38D | Q160C, C214S |
| | Q39K | L128C, C220S | Q38D | F118C, C214S |
| | Q39K | S134C, C220S | Q38D | F116C, C214S |
| | Q39K | S136C, C220S | Q38D | S114C, C214S |
| | Q39K | P171C, C220S | Q38D | S162C, C214S |
| | Q39K | T139C, C220S | Q38D | F116C, C214S |
| | | | | |
| Combination 2 | L45K | F126C, C220S | Y87D | Q124C, C214S |
| | L45K | H168C, C220S | Y87D | T164C, C214S |
| | L45K | F170C, C220S | Y87D | T164C, C214S |
| | L45K | F170C, C220S | Y87D | S162C, C214S |
| | L45K | V173C, C220S | Y87D | S162C, C214S |
| | L45K | V173C, C220S | Y87D | Q160C, C214S |
| | L45K | L128C, C220S | Y87D | F118C, C214S |
| | L45K | S134C, C220S | Y87D | F116C, C214S |
| | L45K | S136C, C220S | Y87D | S114C, C214S |
| | L45K | P171C, C220S | Y87D | S162C, C214S |
| | L45K | T139C, C220S | Y87D | F116C, C214S |
| | | | | |
| Combination 3 | L45K | F126C, C220S | P44D | Q124C, C214S |
| | L45K | H168C, C220S | P44D | T164C, C214S |
| | L45K | F170C, C220S | P44D | T164C, C214S |
| | L45K | F170C, C220S | P44D | S162C, C214S |
| | L45K | V173C, C220S | P44D | S162C, C214S |
| | L45K | V173C, C220S | P44D | Q160C, C214S |
| | L45K | L128C, C220S | P44D | F118C, C214S |
| | L45K | S134C, C2205 | P44D | F116C, C214S |
| | L45K | S136C, C220S | P44D | S114C, C214S |
| | L45K | P171C, C220S | P44D | S162C, C214S |
| | L45K | T139C, C220S | P44D | F116C, C214S |
| | | | | |
| Combination 4 | Y91K | F126C, C220S | A43D | Q124C, C214S |
| | Y91K | H168C, C220S | A43D | T164C, C214S |
| | Y91K | F170C, C220S | A43D | T164C, C214S |
| | Y91K | F170C, C220S | A43D | S162C, C214S |
| | Y91K | V173C, C220S | A43D | S162C, C214S |
| | Y91K | V173C, C220S | A43D | Q160C, C214S |
| | Y91K | L128C, C220S | A43D | F118C, C214S |
| | Y91K | S134C, C220S | A43D | F116C, C214S |
| | Y91K | S136C, C220S | A43D | S114C, C214S |
| | Y91K | P171C, C220S | A43D | S162C, C214S |
| | Y91K | T139C, C220S | A43D | F116C, C214S |
| | | | | |
| Combination 5 | Y91K | F126C, C220S | P44D | Q124C, C214S |
| | Y91K | H168C, C220S | P44D | T164C, C214S |
| | Y91K | F170C, C220S | P44D | T164C, C214S |
| | Y91K | F170C, C220S | P44D | S162C, C214S |
| | Y91K | V173C, C220S | P44D | S162C, C214S |
| | Y91K | V173C, C220S | P44D | Q160C, C214S |
| | Y91K | L128C, C2205 | P44D | F118C, C214S |
| | Y91K | S134C, C2205 | P44D | F116C, C214S |
| | Y91K | S136C, C220S | P44D | S114C, C214S |
| | Y91K | P171C, C220S | P44D | S162C, C214S |
| | Y91K | T139C, C220S | P44D | F116C, C214S |

optionally, the second antigen-binding region also comprises mutation combinations selected from the following:

| | Heavy Chain VH Mutation | Light Chain VL Mutation |
|---|---|---|
| Combination 1 | Q39D | Q38K |
| Combination 2 | L45D | Y87K |
| Combination 3 | L45D | P44K |
| Combination 4 | Y91D | A43K |
| Combination 5 | Y91D | P44K |

∘
24. The antibody or antigen-binding fragment thereof according to Embodiment 23, wherein the first antigen-binding region and the second antigen-binding region of the multispecific antibody comprise the following mutation combinations, respectively:

| | First Antigen-Binding Region | | | | Second Antigen-Binding Region | |
|---|---|---|---|---|---|---|
| | Heavy Chain VH Mutation | Heavy Chain CH1 Mutation | Light Chain VL Mutation | Light Chain CL Mutation | Heavy Chain VH Mutation | Light Chain VL Mutation |
| Comb inatio n 1 | Q39K | F126C, C220S | Q38D | Q124C, C214S | Q39D | Q38K |
| | Q39K | H168C, C220S | Q38D | T164C, C214S | Q39D | Q38K |
| | Q39K | F170C, C220S | Q38D | T164C, C214S | Q39D | Q38K |
| | Q39K | F170C, C220S | Q38D | S162C, C214S | Q39D | Q38K |
| | Q39K | V173C, C220S | Q38D | S162C, C214S | Q39D | Q38K |
| | Q39K | V173C, C220S | Q38D | Q160C, C214S | Q39D | Q38K |
| | Q39K | L128C, C220S | Q38D | F118C, C214S | Q39D | Q38K |
| | Q39K | S134C, C220S | Q38D | F116C, C214S | Q39D | Q38K |
| | Q39K | S136C, C220S | Q38D | S114C, C214S | Q39D | Q38K |
| | Q39K | P171C, C220S | Q38D | S162C, C214S | Q39D | Q38K |
| | Q39K | T139C, C220S | Q38D | F116C, C214S | Q39D | Q38K |
| | | | | | | |
| Comb inatio n 2 | L45K | F126C, C220S | Y87D | Q124C, C214S | L45D | Y87K |
| | L45K | H168C, | Y87D | T164C, | L45D | Y87K |
| | | C220S | | C214S | | |
| | L45K | F170C, C220S | Y87D | T164C, C214S | L45D | Y87K |
| | L45K | F170C, C220S | Y87D | S162C, C214S | L45D | Y87K |
| | L45K | V173C, C220S | Y87D | S162C, C214S | L45D | Y87K |
| | L45K | V173C, C220S | Y87D | Q160C, C214S | L45D | Y87K |
| | L45K | L128C, C220S | Y87D | F118C, C214S | L45D | Y87K |
| | L45K | S134C, C220S | Y87D | F116C, C214S | L45D | Y87K |
| | L45K | S136C, C220S | Y87D | S114C, C214S | L45D | Y87K |
| | L45K | P171C, C220S | Y87D | S162C, C214S | L45D | Y87K |
| | L45K | T139C, C220S | Y87D | F116C, C214S | L45D | Y87K |
| | | | | | | |
| Comb inatio n 3 | L45K | F126C, C220S | P44D | Q124C, C214S | L45D | P44K |
| | L45K | H168C, C220S | P44D | T164C, C214S | L45D | P44K |
| | L45K | F170C, C220S | P44D | T164C, C214S | L45D | P44K |
| | L45K | F170C, C220S | P44D | S162C, C214S | L45D | P44K |
| | L45K | V173C, C220S | P44D | S162C, C214S | L45D | P44K |
| | L45K | V173C, C220S | P44D | Q160C, C214S | L45D | P44K |
| | L45K | L128C, C220S | P44D | F118C, C214S | L45D | P44K |
| | L45K | S134C, C220S | P44D | F116C, C214S | L45D | P44K |
| | L45K | S136C, C220S | P44D | S114C, C214S | L45D | P44K |
| | L45K | P171C, C220S | P44D | S162C, C214S | L45D | P44K |
| | L45K | T139C, C220S | P44D | F116C, C214S | L45D | P44K |
| | | | | | | |
| Comb inatio n 4 | Y91K | F126C, C220S | A43D | Q124C, C214S | Y91D | A43K |
| | Y91K | H168C, C220S | A43D | T164C, C214S | Y91D | A43K |
| | Y91K | F170C, C220S | A43D | T164C, C214S | Y91D | A43K |
| | Y91K | F170C, C220S | A43D | S162C, C214S | Y91D | A43K |
| | Y91K | V173C, C220S | A43D | S162C, C214S | Y91D | A43K |
| | Y91K | V173C, C220S | A43D | Q160C, C214S | Y91D | A43K |
| | Y91K | L128C, | A43D | F118C, | Y91D | A43K |
| | | C220S | | C214S | | |
| | Y91K | S134C, C220S | A43D | F116C, C214S | Y91D | A43K |
| | Y91K | S136C, C220S | A43D | S114C, C214S | Y91D | A43K |
| | Y91K | P171C, C220S | A43D | S162C, C214S | Y91D | A43K |
| | Y91K | T139C, C220S | A43D | F116C, C214S | Y91D | A43K |
| | | | | | | |
| Comb inatio n 5 | Y91K | F126C, C220S | P44D | Q124C, C214S | Y91D | P44K |
| | Y91K | H168C, C220S | P44D | T164C, C214S | Y91D | P44K |
| | Y91K | F170C, C220S | P44D | T164C, C214S | Y91D | P44K |
| | Y91K | F170C, C220S | P44D | S162C, C214S | Y91D | P44K |
| | Y91K | V173C, C220S | P44D | S162C, C214S | Y91D | P44K |
| | Y91K | V173C, C220S | P44D | Q160C, C214S | Y91D | P44K |
| | Y91K | L128C, C220S | P44D | F118C, C214S | Y91D | P44K |
| | Y91K | S134C, C220S | P44D | F116C, C214S | Y91D | P44K |
| | Y91K | S136C, C220S | P44D | S114C, C214S | Y91D | P44K |
| | Y91K | P171C, C220S | P44D | S162C, C214S | Y91D | P44K |
| | Y91K | T139C, C220S | P44D | F116C, C214S | Y91D | P44K |

∘
25. The antibody or antigen-binding fragment thereof, comprising one or a plurality of first antigen-binding regions that specifically bind to a first antigen and one or a plurality of second antigen-binding regions that specifically bind to a second antigen, wherein at least one of the antigen-binding regions comprise a heavy chain variable region and light chain variable region, an amino acid pair at the heavy chain variable and light chain variable region comprise a charge mutation that mutates the two amino acids on the amino acid pair into amino acids with opposite charges, respectively, wherein the position of the amino acid pair at the heavy chain variable region and light chain variable region interface is selected from: position 45 of the heavy chain variable region and position 87 of the light chain variable region, position 45 of the heavy chain variable region and position 44 of the light chain variable region, position 91 of the heavy chain variable region and position 43 of the light chain variable region, or position 91 of the heavy chain variable region and position 44 of the light chain variable region (Kabat number).
26. The antibody or antigen-binding fragment thereof according to Embodiment 25, wherein the position of the amino acid pair at the heavy chain variable region and light chain variable region interface in the at least one first antigen-binding region is the same as the position in the at least one second antigen-binding region, wherein the first antigen and second antigen are the same or different.
27. The antibody or antigen-binding fragment thereof according to Embodiment 26, wherein the mutated amino acid at the position of the amino acid pair in the first antigen-binding region has the opposite charge to the mutated amino acid at the same position in the second antigen-binding region.
28. The antibody or antigen-binding fragment thereof according to Embodiment 27, wherein
(1) in the first antigen-binding region, the amino acid at position 45 of the heavy chain variable region is substituted with an amino acid carrying a positive charge, and the amino acid at position 87 of the light chain variable region is substituted with an amino acid carrying a negative charge; and in the second antigen-binding region, the amino acid at position 45 of the heavy chain variable region is substituted with an amino acid carrying a negative charge, and the amino acid at position 87 of the light chain variable region is substituted with an amino acid carrying a positive charge;
(2) in the first antigen-binding region, the amino acid at position 45 of the heavy chain variable region is substituted with an amino acid carrying a positive charge, and the amino acid at position 44 of the light chain variable region is substituted with an amino acid carrying a negative charge; and in the second antigen-binding region, the amino acid at position 45 of the heavy chain variable region is substituted with an amino acid carrying a negative charge, and the amino acid at position 44 of the light chain variable region is substituted with an amino acid carrying a positive charge;
(3) in the first antigen-binding region, the amino acid at position 91 of the heavy chain variable region is substituted with an amino acid carrying a positive charge, and the amino acid at position 43 of the light chain variable region is substituted with an amino acid carrying a negative charge; and in the second antigen-binding region, the amino acid at position 91 of the heavy chain variable region is substituted with an amino acid carrying a negative charge, and the amino acid at position 43 of the light chain variable region is substituted with an amino acid carrying a positive charge; or
(4) in the first antigen-binding region, the amino acid at position 91 of the heavy chain variable region is substituted with an amino acid carrying a positive charge, and the amino acid at position 44 of the light chain variable region is substituted with an amino acid carrying a negative charge; and in the second antigen-binding region, the amino acid at position 91 of the heavy chain variable region is substituted with an amino acid carrying a negative charge, and the amino acid at position 44 of the light chain variable region is substituted with an amino acid carrying a positive charge.

29. The antibody or antigen-binding fragment thereof according to Embodiment 28, wherein the amino acid carrying a positive charge is selected from K, R, or H, and/or the amino acid carrying a negative charge is selected from E or D.
30. The antibody or antigen-binding fragment thereof according to Embodiment 29, wherein
(i). in the first antigen-binding region, the amino acid at position 45 of the heavy chain variable region is substituted with K, and the amino acid at position 87 of the light chain variable region is substituted with D, while in the second antigen-binding region, the amino acid at position 45 of the heavy chain variable region is substituted with D, and the amino acid at position 87 of the light chain variable region is substituted with K;
(ii). in the first antigen-binding region, the amino acid at position 45 of the heavy chain variable region is substituted with K, and the amino acid at position 44 of the light chain variable region is substituted with D, while in the second antigen-binding region, the amino acid at position 45 of the heavy chain variable region is substituted with D, and the amino acid at position 44 of the light chain variable region is substituted with K;
(iii). in the first antigen-binding region, the amino acid at position 91 of the heavy chain variable region is substituted with K, and the amino acid at position 43 of the light chain variable region is substituted with D, while in the second antigen-binding region, the amino acid at position 91 of the heavy chain variable region is substituted with D, and the amino acid at position 43 of the light chain variable region is substituted with K; or
(iv). in the first antigen-binding region, the amino acid at position 91 of the heavy chain variable region is substituted with K, and the amino acid at position 44 of the light chain variable region is substituted with D, while in the second antigen-binding region, the amino acid at position 91 of the heavy chain variable region is substituted with D, and the amino acid at position 44 of the light chain variable region is substituted with K.

31. The antibody or antigen-binding fragment thereof according to any one of Embodiments 1 - 30, wherein the first antigen-binding region comprises the same mutation, and the second antigen-binding region comprises the same mutation.
32. The antibody or antigen-binding fragment thereof according to any one of Embodiments 1- 31, wherein the first antigen-binding region is a Fab fragment of the antibody, and/or the second antigen-binding region is the Fab fragment of the antibody.
33. The antibody or antigen-binding fragment thereof according to any one of Embodiments 1- 32, wherein the light chain constant region that one or a plurality or all the first antigen-binding regions of the antibody comprise is a Kappa light chain constant region, and the light chain constant region that one or a plurality or all the second antigen-binding regions comprise is a Lambda light chain constant region, or wherein the light chain constant region that one or a plurality or all the first antigen-binding regions comprise is a Lambda light chain constant region, and the light chain constant region that one or a plurality or all the second antigen-binding regions comprise is a Kappa light chain constant region.
34. The antibody or antigen-binding fragment thereof according to any one of Embodiments 1 - 33, wherein the antibody also comprises a first Fc region and the second Fc region, wherein the first Fc region and the second Fc region are the same or different.
35. The antibody or antigen-binding fragment thereof according to Embodiment 34, wherein the first Fc region and the second Fc region are human IgG Fc, respectively, for example, human IgG1 Fc, human IgG2 Fc, human IgG3 Fc, or human IgG4 Fc, for example, it comprises or is composed of the amino acid sequence SEQ ID NO:122 or 123, or has at least 90% identity with it, for example, an amino acid sequence with 95%, 96%, 97%, 99%, or higher identity.
36. The multispecific antibody according to Embodiment 34 or 35, wherein based on the Knob-into-hole technology, a corresponding Knob mutation and Hole mutation are introduced into the first Fc region and the second Fc region.
37. The antibody or antigen-binding fragment thereof according to Embodiment 36, wherein
a) a polypeptide of one Fc region comprises mutation T366W, while a polypeptide of another Fc region comprises T366S, L368A, and Y407V (numbered based on the EU index), or
b) one Fc region comprises the amino acid substitutions S354C and T366W, and another Fc region comprises the amino acid substitutions Y349C, T366S, L368A, and Y407V (numbered based on the EU index).

38. The multispecific antibody according to Embodiment 36, wherein
a) a polypeptide of one Fc region comprises or is composed of the amino acid sequence shown in SEQ ID NO:125, while a polypeptide of another Fc region comprises or is composed of the amino acid sequence shown in SEQ ID NO:124;
b) a polypeptide of one Fc region comprises an amino acid sequence that has at least 85%, 90%, 95%, 96%, 97%, 98%, or 99% identity with the amino acid sequence shown in SEQ ID NO:125, while a polypeptide of another Fc region comprises an amino acid sequence that has at least 85%, 90%, 95%, 96%, 97%, 98%, or 99% identity with the amino acid sequence shown in SEQ ID NO:124;
c) a polypeptide of one Fc region comprises an amino acid sequence that has at least 85%, 90%, 95%, 96%, 97%, 98%, or 99% identity with the amino acid sequence shown in SEQ ID NO:125 and comprises mutations S354C and T366W, while a polypeptide of another Fc region comprises an amino acid sequence that has at least 85%, 90%, 95%, 96%, 97%, 98%, or 99% identity with the amino acid sequence shown in SEQ ID NO:124 and comprises mutations Y349C, T366S, L368A, and Y407V;
d) a polypeptide of one Fc region comprises or is composed of the amino acid sequence shown in SEQ ID NO:20 or 131, while a polypeptide of another Fc region comprises or is composed of the amino acid sequence shown in SEQ ID NO:101 or 130;
e) a polypeptide of one Fc region comprises an amino acid sequence that has at least 85%, 90%, 95%, 96%, 97%, 98%, or 99% identity with the amino acid sequence shown in SEQ ID NO:20 or 131, while a polypeptide of another Fc region comprises an amino acid sequence that has at least 85%, 90%, 95%, 96%, 97%, 98%, or 99% identity with the amino acid sequence shown in SEQ ID NO:101 or 130; or
f) a polypeptide of one Fc region comprises an amino acid sequence that has at least 85%, 90%, 95%, 96%, 97%, 98%, or 99% identity with the amino acid sequence shown in SEQ ID NO:20 or 131 and comprises mutation T366W, while a polypeptide of another Fc region comprises an amino acid sequence that has at least 85%, 90%, 95%, 96%, 97%, 98%, or 99% identity with the amino acid sequence shown in SEQ ID NO:101 or 130 and comprises T366S, L368A, and Y407V.

39. The antibody or antigen-binding fragment thereof according to Embodiment 34 or 35, wherein a mutation is introduced into the first Fc region and the second Fc region based on Innobody technology to promote heterodimerization of the first Fc region and the second Fc region.
40. The antibody or antigen-binding fragment thereof according to Embodiment 39, wherein the CH3 of one Fc region comprises S364R and D399K mutations, and the CH3 mutations of another Fc region comprise Y349T, K370S, and K409D mutations.
41. The antibody or antigen-binding fragment thereof according to Embodiment 40, wherein
a) a polypeptide of one Fc region comprises or is composed of the amino acid sequence shown in SEQ ID NO:126, while a polypeptide of another Fc region comprises or is composed of the amino acid sequence shown in SEQ ID NO:127;
b) a polypeptide of one Fc region comprises an amino acid sequence that has at least 85%, 90%, 95%, 96%, 97%, 98%, or 99% identity with the amino acid sequence shown in SEQ ID NO:126, while a polypeptide of another Fc region comprises an amino acid sequence that has at least 85%, 90%, 95%, 96%, 97%, 98%, or 99% identity with the amino acid sequence shown in SEQ ID NO:127; or
c) one Fc region comprises an amino acid sequence that has at least 85%, 90%, 95%, 96%, 97%, 98%, or 99% identity with the amino acid sequence shown in SEQ ID NO:126 and comprises mutations S364R and D399K, while a polypeptide of another Fc region comprises an amino acid sequence that has at least 85%, 90%, 95%, 96%, 97%, 98%, or 99% identity with the amino acid sequence shown in SEQ ID NO:127 and comprises mutations Y349T, K370S, and K409D.

42. The antibody or antigen-binding fragment thereof according to any one of Embodiments 34 - 41, wherein the first and/or second Fc region comprises a L234A/L235A mutation.
43. The antibody or antigen-binding fragment thereof according to any one of Embodiments 1 - 42, wherein the antigen bound to the antibody or antigen-binding fragment thereof is selected from one or a plurality of the following: CD3, BCMA, CD20, Her2, Her3, B7H3, EGFR, Hel, cMET, Axl, GPRC5D, immune checkpoint molecules such as PD-1, PD-L1, CD47, immune activation molecules such as 4-1BB, CD40, and OX40.
44. The antibody or antigen-binding fragment thereof according to any one of Embodiments 1 - 43, wherein the first antigen and second antigen are different and the antibody is a bispecific antibody.
45. The antibody or antigen-binding fragment thereof according to Embodiment 44, wherein the bispecific antibody binds to two antigens selected from the following: CD3/BCMA, CD3/CD20, CD3/Her2, Her2/Her3, B7H3/EGFR, PD-L1/CD47, PD-L1/CD40, PD-L1/4-1BB, EGFR/Her3, Her2/Hel, Her2/PD-1, Her2/PD-L1, Her2/CD47, Her2/cMet, or CD3/GPRC5D.
46. The antibody or antigen-binding fragment thereof according to Embodiment 44 or 45, wherein the bispecific antibody of the present disclosure comprises a first antigen-binding region that specifically binds to a first antigen and a second antigen-binding region that specifically binds to a second antigen, wherein the first antigen-binding region comprises a First Fab, the second antigen-binding region comprises a Second Fab, wherein the First Fab is linked to the N-terminus of the first Fc region at the C-terminus of the CH1 thereof (with or without a linker, for example, a hinge region), and the Second Fab is linked to the N-terminus of the second Fc region at the C-terminus of the CH1 thereof (with or without a linker, for example, a hinge region).
47. The antibody or antigen-binding fragment thereof according to Embodiment 46, wherein the bispecific antibody is an IgG-like antibody with the configuration shown in Figure 1.
48. The antibody or antigen-binding fragment thereof according to Embodiment 46 or 47, comprising
Heavy chain 1: comprises or is composed of the following from the N-terminus to the C-terminus: the First Fab heavy chain variable region-heavy chain constant region CH1-first Fc region, wherein the heavy chain constant region CH1 is linked to the N-terminus of the first Fc region at the C-terminus thereof with or without a linker (for example, a hinge region); Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-second Fc region, wherein the heavy chain constant region CH1 is linked to the N-terminus of the second Fc region at the C-terminus thereof with or without a linker (for example, a hinge region);
Light chain 2: comprises or is composed of the following from the N-terminus to the C-terminus: the Second Fab light chain variable region-light chain constant region.

49. The antibody or antigen-binding fragment thereof according to Embodiment 48; wherein
(1) the first antigen-binding region specifically binds to Her2, wherein heavy chain 1 and light chain 1 comprise the amino acid sequences shown in the following SEQ ID NO:, respectively,or comprise amino acid sequences that have at least 85%, 90%, 95%, 96%, 97%, 98%, or 99% identity with the amino acid sequences shown, or are composed of the sequences shown in the following SEQ ID NO:
   i) SEQ ID NO:43 and SEQ ID NO:45;
   ii) SEQ ID NO:48 and SEQ ID NO:49;
   iii) SEQ ID NO:21 and SEQ ID NO:22;
   iv) SEQ ID NO:25 and SEQ ID NO:26;
   v) SEQ ID NO:25 and SEQ ID NO:29;
   vi) SEQ ID NO:31 and SEQ ID NO:32; or
   vii) SEQ ID NO:31 and SEQ ID NO:29;
   and/or
(2) the second antigen-binding region specifically binds to Hel, and heavy chain 2 and light chain 2 comprise the following SEQ ID NO:, respectively:or comprise amino acid sequences that have at least 85%, 90%, 95%, 96%, 97%, 98%, or 99% identity with the amino acid sequences shown, or are composed of the sequences shown in the following SEQ ID NO:
   i) SEQ ID NO:41 and SEQ ID NO:42;
   ii) SEQ ID NO:23 and SEQ ID NO:24;
   iii) SEQ ID NO:27 and SEQ ID NO:28;
   iv) SEQ ID NO:27 and SEQ ID NO:30;
   v) SEQ ID NO:33 and SEQ ID NO:34; or
   vi) SEQ ID NO:33 and SEQ ID NO:30;
(2)-1 The second antigen-binding region specifically binds to PD-1, and heavy chain 2 and light chain 2 comprise the following SEQ ID NO:, respectively; or comprise amino acid sequences that have at least 85%, 90%, 95%, 96%, 97%, 98%, or 99% identity with the amino acid sequences shown, or are composed of the sequences shown in the following SEQ ID NO: SEQ ID NO:46 and SEQ ID NO:47;
(2)-2 The second antigen-binding region specifically binds to CD47, and heavy chain 2 and light chain 2 comprise the following SEQ ID NO:, respectively; or comprise amino acid sequences that have at least 85%, 90%, 95%, 96%, 97%, 98%, or 99% identity with the amino acid sequences shown, or are composed of the sequences shown in the following SEQ ID NO: SEQ ID NO:50 and SEQ ID NO:51;
(3)-2 The second antigen-binding region specifically binds to cMet, and heavy chain 2 and light chain 2 comprise the following SEQ ID NO:, respectively; or comprise amino acid sequences that have at least 85%, 90%, 95%, 96%, 97%, 98%, or 99% identity with the amino acid sequences shown, or are composed of the sequences shown in the following SEQ ID NO: SEQ ID NO:52 and SEQ ID NO:53.

50. The multispecific antibody according to Embodiment 44 or 45, wherein the bispecific antibody comprises a first antigen-binding region that specifically binds to a first antigen and two second antigen-binding regions that specifically bind to a second antigen, wherein the first antigen-binding region comprises a First Fab, the second antigen-binding region comprises a Second Fab, wherein one second Fab is linked to the N-terminus of the first Fc region at the C-terminus of the CH1 thereof (with or without a linker, for example, a hinge region), one first Fab is linked to the N-terminus of the second Fc region at the C-terminus of the CH1 thereof (with or without a linker, for example, a hinge region), and the other second Fab is linked to the N-terminus of the First Fab heavy chain variable region at the C-terminus of the CH1 thereof (with or without a linker).
51. The antibody or antigen-binding fragment thereof according to Embodiment 50, wherein the bispecific antibody has a 2+1N-terminal configuration with the configuration shown in Figure 5A.
52. The antibody or antigen-binding fragment thereof according to Embodiment 51, wherein the bispecific antibody comprises
Heavy chain 1: comprises or is composed of the following from the N-terminus to the C-terminus: the Second Fab heavy chain variable region-heavy chain constant region CH1-First Fab heavy chain variable region-heavy chain constant region CH1-second Fc region, wherein the First Fab heavy chain constant region CH1 is linked to the N-terminus of the second Fc region at the C-terminus thereof with or without a linker (for example, a hinge region), and the Second Fab is linked to the N-terminus of the First Fab heavy chain variable region of at the C-terminus of the CH1 thereof (with or without a linker);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-first Fc region, wherein the heavy chain constant region CH1 is linked to the N-terminus of the first Fc region at the C-terminus thereof with or without a linker (for example, a hinge region);
Light chain 2: comprises or is composed of the following from the N-terminus to the C-terminus: the Second Fab light chain variable region-light chain constant region,
wherein the bispecific antibody comprises two light chains 2.

53. The antibody or antigen-binding fragment thereof according to Embodiment 52, wherein the first antigen-binding region binds to CD3, and the second antigen-binding region binds to GPRC5D, for example, wherein
1) heavy chain 1 comprises the amino acid sequence shown in SEQ ID NO:58, or comprises or is composed of an amino acid sequence with at least 85%, 90%, 95%, 96%, 97%, 98%, or 99% identity with the amino acid sequence shown in SEQ ID NO:58;
   light chain 1 comprises the amino acid sequence shown in SEQ ID NO:59, or comprises or is composed of an amino acid sequence with at least 85%, 90%, 95%, 96%, 97%, 98%, or 99% identity with the amino acid sequence shown in SEQ ID NO:59;
   heavy chain 2 comprises the amino acid sequence shown in SEQ ID NO:57, or comprises or is composed of an amino acid sequence with at least 85%, 90%, 95%, 96%, 97%, 98%, or 99% identity with the amino acid sequence shown in SEQ ID NO:57; and/or
   light chain 2 comprises the amino acid sequence shown in SEQ ID NO:56, or comprises or is composed of an amino acid sequence with at least 85%, 90%, 95%, 96%, 97%, 98%, or 99% identity with the amino acid sequence shown in SEQ ID NO:56; and/or
2) heavy chain 1 comprises the amino acid sequence shown in SEQ ID NO:62, or comprises or is composed of an amino acid sequence with at least 85%, 90%, 95%, 96%, 97%, 98%, or 99% identity with the amino acid sequence shown in SEQ ID NO:62;
   light chain 1 comprises the amino acid sequence shown in SEQ ID NO:63, or comprises or is composed of an amino acid sequence with at least 85%, 90%, 95%, 96%, 97%, 98%, or 99% identity with the amino acid sequence shown in SEQ ID NO:63;
   heavy chain 2 comprises the amino acid sequence shown in SEQ ID NO:61, or comprises or is composed of an amino acid sequence with at least 85%, 90%, 95%, 96%, 97%, 98%, or 99% identity with the amino acid sequence shown in SEQ ID NO:61; and/or
   light chain 2 comprises the amino acid sequence shown in SEQ ID NO:60, or comprises or is composed of an amino acid sequence with at least 85%, 90%, 95%, 96%, 97%, 98%, or 99% identity with the amino acid sequence shown in SEQ ID NO:60.

54. The antibody or antigen-binding fragment thereof according to Embodiment 44 or 45, wherein the bispecific antibody of the present disclosure comprises one first antigen-binding region that specifically binds to a first antigen and two second antigen-binding regions that specifically bind to a second antigen, wherein the first antigen-binding region comprises a First Fab, the second antigen-binding region comprises a Second Fab, wherein the two Second Fabs are linked to the N-terminus of the first Fc region and the N-terminus of the second Fc region, respectively, at the C-terminus of the CH1 thereof (with or without a linker, for example, a hinge region), and the N-terminus of the First Fab heavy chain variable region is linked to the C-terminus of the second Fc region (with or without a linker).
55. The antibody or antigen-binding fragment thereof according to Embodiment 54, wherein the bispecific antibody has a 2+1C-terminal configuration with the configuration shown in Figure 5B.
56. The antibody or antigen-binding fragment thereof according to Embodiment 55, wherein the bispecific antibody comprises
Heavy chain 1: comprises or is composed of the following from the N-terminus to the C-terminus: the Second Fab heavy chain variable region-heavy chain constant region CH1-second Fc region-First Fab heavy chain variable region-heavy chain constant region CH1, wherein the Second Fab is linked to the N-terminus of the second Fc region at the C-terminus of the CH1 thereof (with or without a linker, for example, a hinge region), and the N-terminus of the First Fab heavy chain variable region is linked to the C-terminus of the second Fc region (with or without a linker);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-first Fc region, wherein the heavy chain constant region CH1 is linked to the N-terminus of the first Fc region at the C-terminus thereof with or without a linker (for example, a hinge region);
Light chain 2: comprises or is composed of the following from the N-terminus to the C-terminus: the Second Fab light chain variable region-light chain constant region,
wherein the bispecific antibody comprises two light chains 2.

57. The antibody or antigen-binding fragment thereof according to Embodiment 56, wherein the first antigen-binding region binds to CD3, and the second antigen-binding region binds to GPRC5D, for example, wherein
1) heavy chain 1 comprises the amino acid sequence shown in SEQ ID NO:64, or comprises or is composed of an amino acid sequence with at least 85%, 90%, 95%, 96%, 97%, 98%, or 99% identity with the amino acid sequence shown in SEQ ID NO:64;
   light chain 1 comprises the amino acid sequence shown in SEQ ID NO:59, or comprises or is composed of an amino acid sequence with at least 85%, 90%, 95%, 96%, 97%, 98%, or 99% identity with the amino acid sequence shown in SEQ ID NO:59;
   heavy chain 2 comprises the amino acid sequence shown in SEQ ID NO:57, or comprises or is composed of an amino acid sequence with at least 85%, 90%, 95%, 96%, 97%, 98%, or 99% identity with the amino acid sequence shown in SEQ ID NO:57; and/or
   light chain 2 comprises the amino acid sequence shown in SEQ ID NO:56, or comprises or is composed of an amino acid sequence with at least 85%, 90%, 95%, 96%, 97%, 98%, or 99% identity with the amino acid sequence shown in SEQ ID NO:56; and/or
2) heavy chain 1 comprises the amino acid sequence shown in SEQ ID NO:65, or comprises or is composed of an amino acid sequence with at least 85%, 90%, 95%, 96%, 97%, 98%, or 99% identity with the amino acid sequence shown in SEQ ID NO:65;
   light chain 1 comprises the amino acid sequence shown in SEQ ID NO:63, or comprises or is composed of an amino acid sequence with at least 85%, 90%, 95%, 96%, 97%, 98%, or 99% identity with the amino acid sequence shown in SEQ ID NO:63;
   heavy chain 2 comprises the amino acid sequence shown in SEQ ID NO:61, or comprises or is composed of an amino acid sequence with at least 85%, 90%, 95%, 96%, 97%, 98%, or 99% identity with the amino acid sequence shown in SEQ ID NO:61; and/or
   light chain 2 comprises the amino acid sequence shown in SEQ ID NO:60, or comprises or is composed of an amino acid sequence with at least 85%, 90%, 95%, 96%, 97%, 98%, or 99% identity with the amino acid sequence shown in SEQ ID NO:60.

58. The antibody or antigen-binding fragment thereof according to Embodiment 44 or 45, wherein the bispecific antibody of the present disclosure comprises two first antigen-binding regions that specifically bind to a first antigen and two second antigen-binding regions that specifically bind to a second antigen, wherein the first antigen-binding region comprises a First Fab, the second antigen-binding region comprises a Second Fab, wherein the two Second Fabs are linked to the N-terminus of the two Fc regions at the C-terminus of the CH1 thereof (with or without a linker, for example, a hinge region) (the Fc region may be the same or different), and the N-terminus of the heavy chain variable region of the two First Fabs is linked to the C-terminus of the two Fc regions (with or without a linker).
59. The antibody or antigen-binding fragment thereof according to Embodiment 58, wherein the bispecific antibody has a 2+2C-terminal configuration with the configuration shown in Figure 5C.
60. The antibody or antigen-binding fragment thereof according to Embodiment 59, wherein the bispecific antibody comprises
Heavy chain: comprises or is composed of the following from the N-terminus to the C-terminus: the Second Fab heavy chain variable region-heavy chain constant region CH1 -Fc region-First Fab heavy chain variable region- heavy chain constant region CH1, wherein the Second Fab is linked to the N-terminus of the Fc region at the C-terminus of the CH1 thereof (with or without a linker, for example, a hinge region), and the N-terminus of the First Fab heavy chain variable region is linked to the C-terminus of the Fc region (with or without a linker).
Light chain 1: comprises or is composed of the following from the N-terminus to the C-terminus: the First Fab light chain variable region-light chain constant region;
Light chain 2: comprises or is composed of the following from the N-terminus to the C-terminus: the Second Fab light chain variable region-light chain constant region,
wherein the bispecific antibody comprises two heavy chains.

61. The antibody or antigen-binding fragment thereof according to Embodiment 60, wherein the first antigen-binding region binds to BD3, and the second antigen-binding region binds to GPRC5D, for example, wherein
1) the heavy chain comprises the amino acid sequence shown in SEQ ID NO:66, or comprises or is composed of an amino acid sequence with at least 85%, 90%, 95%, 96%, 97%, 98%, or 99% identity with the amino acid sequence shown in SEQ ID NO:66;
   light chain 1 comprises the amino acid sequence shown in SEQ ID NO:59, or comprises or is composed of an amino acid sequence with at least 85%, 90%, 95%, 96%, 97%, 98%, or 99% identity with the amino acid sequence shown in SEQ ID NO:59; and/or
   light chain 2 comprises the amino acid sequence shown in SEQ ID NO:56, or comprises or is composed of an amino acid sequence with at least 85%, 90%, 95%, 96%, 97%, 98%, or 99% identity with the amino acid sequence shown in SEQ ID NO:56; and/or
2) the heavy chain comprises the amino acid sequence shown in SEQ ID NO:67, or comprises or is composed of an amino acid sequence with at least 85%, 90%, 95%, 96%, 97%, 98%, or 99% identity with the amino acid sequence shown in SEQ ID NO:67;
   light chain 1 comprises the amino acid sequence shown in SEQ ID NO:63, or comprises or is composed of an amino acid sequence with at least 85%, 90%, 95%, 96%, 97%, 98%, or 99% identity with the amino acid sequence shown in SEQ ID NO:63; and/or
   light chain 2 comprises the amino acid sequence shown in SEQ ID NO:60, or comprises or is composed of an amino acid sequence with at least 85%, 90%, 95%, 96%, 97%, 98%, or 99% identity with the amino acid sequence shown in SEQ ID NO:60.

62. The antibody or antigen-binding fragment thereof according to any one of Embodiments 1 - 43, wherein the first antigen and second antigen are different, and the antibody also comprises an antigen-binding region that specifically binds to one or a plurality of other antigens.
63. The antibody or antigen-binding fragment thereof according to Embodiment 62, wherein the antibody is a trispecific antibody.
64. The antibody or antigen-binding fragment thereof according to Embodiment 63, wherein the trispecific antibody of the present disclosure comprises a first antigen-binding region that specifically binds to a first antigen, a second antigen-binding region that specifically binds to a second antigen, and a third antigen-binding region that specifically binds to a third antigen, wherein the first antigen-binding region comprises a First Fab, the second antigen-binding region comprises a Second Fab, wherein the First Fab is linked to the N-terminus of the first Fc region at the C-terminus of the CH1 thereof (with or without a linker, for example, a hinge region), the Second Fab is linked to the N-terminus of the second Fc region at the C-terminus of the CH1 thereof (with or without a linker, for example, a hinge region), the third antigen-binding region comprises a scFv (for example, VH-VL, or VL-VH) that is linked to the C-terminus of the second Fc region at the N-terminus of the VH or VL thereof (for example, scFv is VL-VH, for example, the N-terminus of VL) with or without a linker.
65. The antibody or antigen-binding fragment thereof according to Embodiment 64, wherein the configuration of the trispecific antibody is a 2+1C-terminal configuration, as shown in Figure 7A or B.
66. The antibody or antigen-binding fragment thereof according to Embodiment 65, wherein the multispecific antibody comprises
Heavy chain 1: comprises or is composed of the following from the N-terminus to the C-terminus: the First Fab heavy chain variable region-heavy chain constant region CH1-first Fc region, wherein the First Fab is linked to the N-terminus of the first Fc region at the C-terminus of the CH1 thereof (with or without a linker, for example, a hinge region);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-second Fc region -scFv, wherein the heavy chain constant region CH1 is linked to the N-terminus of the second Fc region at the C-terminus of the heavy chain constant region CH1 with or without a linker (for example, a hinge region), and scFv is linked to the C-terminus of the second Fc region at the N-terminus or C-terminus of the VH or VL thereof (for example, scFv is VL-VH, for example, the N-terminus of VL) with or without a linker.
Light chain 2: comprises or is composed of the following from the N-terminus to the C-terminus: the Second Fab light chain variable region-light chain constant region.

67. The antibody or antigen-binding fragment thereof according to Embodiment 66, wherein the first antigen-binding region specifically binds to GPRC5D, the second antigen-binding region specifically binds to CD3, and/or the third antigen-binding region specifically binds to BCMA, for example, wherein
heavy chain 1 comprises the amino acid sequence shown in SEQ ID NO:71, or comprises or is composed of an amino acid sequence with at least 85%, 90%, 95%, 96%, 97%, 98%, or 99% identity with the amino acid sequence shown in SEQ ID NO:71;
light chain 1 comprises the amino acid sequence shown in SEQ ID NO:72, or comprises or is composed of an amino acid sequence with at least 85%, 90%, 95%, 96%, 97%, 98%, or 99% identity with the amino acid sequence shown in SEQ ID NO:72;
heavy chain 2 comprises the amino acid sequence shown in SEQ ID NO:73, or comprises or is composed of an amino acid sequence with at least 85%, 90%, 95%, 96%, 97%, 98%, or 99% identity with the amino acid sequence shown in SEQ ID NO:73; and/or
light chain 2 comprises the amino acid sequence shown in SEQ ID NO:74, or comprises or is composed of an amino acid sequence with at least 85%, 90%, 95%, 96%, 97%, 98%, or 99% identity with the amino acid sequence shown in SEQ ID NO:74.

68. A nucleic acid, coding the antibody or antigen-binding fragment thereof according to any one of Embodiments 1 - 67, or any or a plurality of chains thereof.
69. An expression vector, comprising the nucleic acid according to Embodiment 68.
70. A host cell, comprising the nucleic acid according to Embodiment 68 or the expression vector according to Embodiment 69.
71. A method for preparing the antibody or antigen-binding fragment thereof according to any one of Embodiments 1 - 67, comprising
a) introducing the nucleic acid coding the various chains of the antibody or antigen-binding fragment thereof into the host cell;
b) expressing in the host cell and assembling the antibody or antigen-binding fragment thereof;
optionally, the antibody or antigen-binding fragment thereof is purified, for example, purified by Protein A.
72. An immunoconjugate, comprising the antibody or antigen-binding fragment thereof according to any one of Embodiments 1 - 67.
73. A fusion protein, comprising the antibody or antigen-binding fragment thereof according to any one of Embodiments 1 - 67.
74. A T cell receptor, comprising the antibody or antigen-binding fragment thereof according to any one of Embodiments 1 - 67.

### II. Preparation of binding molecule

The present disclosure provides an in vitro method for producing a binding molecule, for example, an antibody. In some embodiments, the binding molecule is an antibody or fusion protein. In some embodiments, the antibody is a multispecific antibody, for example, a bispecific antibody.

In one aspect, the method for preparing the binding molecule of the present disclosure, for example, an antibody, comprises:
a) introducing the nucleic acid coding various chains of the binding molecule, for example, an antibody, into the host cell;
b) expressing in the host cell and assembly the binding molecule, for example, an antibody;
optionally, the binding molecule is purified, for example, purified by Protein A.

In yet another embodiment, a compound, for example, L-arginine, is added into the culture medium during the production of the binding molecule, for example, an antibody. In one embodiment, the Arg is used to stabilize protein.

In some embodiments, the product of the present disclosure maintains the same expression level and affinity as the binding molecule, for example, an antibody, before the mutation.

In some embodiments, the method of the present disclosure obtains a stable binding molecule, for example, an antibody, such as a multispecific antibody (for example, a bispecific antibody) at a high yield.

In some embodiments, the method of the present disclosure is capable of expressing various chains of the binding molecule, for example, an antibody, such as a multispecific antibody (for example, a bispecific antibody) in a cell line and assembling them, preferably avoiding expression and in vitro recombination in two cell lines.

### III Nucleic acid and host cell

The present disclosure provides a nucleic acid that codes any chain or any monomer or domain of the binding molecule of the present disclosure, for example, an antibody, such as a multispecific antibody, for example, a bispecific antibody. Methods familiar in the art may be used to produce a polynucleotide sequence that codes various chains. In addition, the polynucleotide and nucleic acid of the present disclosure may comprise a segment that codes secretory signal peptide, and is operably linked to an antibody or protein that codes an antibody or the binding molecule of the present disclosure, for example, an antibody, such as a multispecific antibody, for example, a bispecific antibody, thereby guiding the secretory expression of the binding molecule of the present disclosure, for example, an antibody, such as a multispecific antibody, for example, a bispecific antibody, and various chains thereof.

The present disclosure also provides a vector comprising the nucleic acid of the present disclosure. In one embodiment, the vector is an expression vector, for example, an eukaryotic expression vector. The vector includes but is not limited to viruses, plasmids, cosmids, lambda phages and yeast artificial chromosomes (YACs). In a preferred embodiment, the expression vector of the present disclosure is a pCNDA vector, for example, pCNDA3.1 expression vector.

The present disclosure also provides a host cell comprising the nucleic acid or the vector. The host cell suitable for replication and supports the expression of the binding molecule of the present disclosure, for example, an antibody, such as a multispecific antibody, for example, a bispecific antibody, is known in the art. Such cells may be transfected or transduced using a particular expression vector, and are capable of growing a large amount of cells containing the vector for inoculation of a large-scale fermentation tank, thereby obtaining a sufficient amount of the multispecific antibody, for example, a bispecific antibody, for clinical application.

In one embodiment, the host cell is eukaryotic. In another embodiment, the host cell is selected from yeast cells, mammalian cells (for example, CHO cells or 293 cells, for example, Expi293 cells). Examples of usable mammalian host cell lines are SV40-transformed monkey kidney CV1 line (COS-7); human embryonic kidney line (293 or 293T cells), such as, for example, those recorded in Graham et al., JGenVirol36, 59 (1977), baby hamster kidney cells (BHK), mouse sertoli cells (TM4 cells, such as, for example, those recorded in Mather, BiolReprod23, 243 - 251 (1980)), monkey kidney cells (CV1), Chlorocebus kidney cells (VERO-76), human cervical cancer cells (HELA), canine kidney cells (MDCK), buffalo rat liver cells (BRL3A), human lung cells (W138), human liver cells (HepG2), mouse breast cancer cells (MMT060562), TRI cells (such as, for example, those recorded in Mather et al., AnnalsN.Y.AcadSci 383, 44 - 68(1982)), MRC5 cells and FS4 cells. Other usable mammalian host cell lines include Chinese hamster ovary (CHO) cells, including dhfr-CHO cells (Urlaub et al., ProcNatlAcadSciUSA77, 4,216 (1980)); and myeloma cell lines such as Y0, NS0, P3X63 and Sp2/0. In one embodiment, the host cell is an eukaryotic cell, preferably a mammalian cell such as a CHO cell, human embryonic kidney (HEK) cell or lymphocyte (for example, Y0, NS0, Sp20 cell).

### IV. Other molecules comprising the binding molecule of the present disclosure or a fragment thereof

In some embodiments, the first and/or second target-binding region of the binding molecule of the present disclosure is capable of conjugating with drugs, precursors, or toxins, or receptor groups containing drugs, precursors, or toxins. Such receptor groups may be, for example, non-natural amino acids.

Therefore, the present disclosure also relates to an immunoconjugate (ADC) of the binding molecule of the present disclosure, for example, an antibody, such as a multispecific antibody, for example, a bispecific antibody.

In some embodiments, the binding molecule of the present disclosure or the fragment thereof may also be constructed in a T cell receptor (TCR). In some embodiments, the variable region of the TCR comprises the mutation or mutation combination according to the present disclosure, for example, a disulfide bond reshaping mutation and/or charge mutation.

In some embodiments, the binding molecule of the present disclosure or the fragment thereof may fuse with other proteins to constitute a fusion protein. In some embodiments, examples of other proteins include, for example, ligands or receptors, for example, granule-associated cytokines or receptors thereof, such as tumor necrosis factor (TNF), vascular endothelial growth factor (VEGF), transforming growth factor (TGF, for example, TGF-β) or receptors thereof, as well as fragments thereof.

### V. Product and uses

In some embodiments, the present disclosure also relates to a composition (for example, a pharmaceutical composition) comprising the binding molecule, for example, an antibody, such as a multispecific antibody, for example, a bispecific antibody.

In one embodiment, the composition also comprises a pharmaceutical excipient. In one embodiment, the composition, for example, a pharmaceutical composition, comprises the binding molecule of the present disclosure, and a combination of one or a plurality of other types of therapeutic agents.

The composition of the present disclosure may also comprise a suitable pharmaceutical excipient, such as known pharmaceutical vectors and pharmaceutical vehicles in the art, including buffers. As used herein, "pharmaceutical vectors" include any or all physiologically compatible solvents, dispersing media, isotonic agents, absorption-delaying agents, and the like. Refer to "Handbook of Pharmaceutical Excipients", Version 8, R.C.Rowe, P.J.Seskey and S.C. Owen, Pharmaceutical Press, London, Chicago for the usage and uses of pharmaceutical excipients. The composition of the present disclosure may be in a plurality of forms. Such forms, for example, include liquid, semi-solid, and solid dosage forms, such as liquid solutions (for example, injectable solutions and infusible solutions), powders or suspensions, liposomal agents and suppositories. The preferred form depends on the expected mode of administration and therapeutic use. The binding molecule of the present disclosure with the desired purity may be mixed with one or a plurality of types of optional pharmaceutical excipients to prepare a drug comprising the binding molecule of the present disclosure, for example, a lyophilized formulation or an aqueous solution.

In some embodiments, the present disclosure relates to a method using the binding molecule, for example, an antibody, such as a multispecific antibody, for example, a bispecific antibody, to treat diseases, for example, tumors, for example, cancer, for therapeutic uses, or for preparing drugs for the treatment.

Depending on the therapeutic use thereof, the binding molecule of the present disclosure, for example, an antibody, such as a multispecific antibody, for example, a bispecific antibody, may be combined with other therapeutic agents. For example, when the binding molecule is used to treat tumors, the therapeutic agent is, for example, various therapeutic agents used to treat tumors, for example, chemotherapeutic agents, angiogenesis inhibitors, cytokines, cytotoxic agents, other antibodies, small-molecule drugs or immunomodulators (for example, immune checkpoint inhibitors or agonists).

In some embodiments, the present disclosure also provides a pharmaceutical combination or pharmaceutical combination product comprising the binding molecule of the present disclosure, and one or a plurality of other types of therapeutic agents, and the like.

Another objective of the present disclosure is to provide a kit of parts comprising the pharmaceutical combination of the present disclosure, preferably, the kit is a drug dosage form. As such, a dose unit may be provided based on the dosing regimen or dosing interval of the drug.

In one embodiment, the kit of parts of the present disclosure comprises, in the same package:
- a first container containing the pharmaceutical composition comprising the binding molecule of the present disclosure;
- a second container containing the pharmaceutical composition comprising other therapeutic agents.

### Embodiments

### Embodiment 1. Design of mutant antibody

### Disulfide bond reshaping

An effective strategy to force homologous light chain and heavy chain pairing and avoid mismatch between light chain and heavy chain of two antibodies is to reshape the disulfide bond linking the light chain and heavy chain of one of the antibodies and change the position of the disulfide bond such that mismatched light chain and heavy chain cannot effectively form a disulfide bond. We screened the amino acids at the contact surface of heavy chain and light chain and selected two amino acid pairs where the distance between α carbon atoms is less than 8Å, preferably less than 6Å, and the side chains of the amino acids point towards each other. The selected amino acid pairs are as shown in Table 1 (EU number):

**Table 1: Selection of Position for Disulfide Bond Reshaping**

| Heavy Chain Position | Example CH1 | Light Chain Position | Example CL |
|---|---|---|---|
| F126C | SEQ ID NO:1 | Q124C | SEQ ID NO:2 |
| H168C | SEQ ID NO:132 | T164C | SEQ ID NO:148 |
| F170C | SEQ ID NO:134 | T164C | SEQ ID NO:148 |
| F170C | SEQ ID NO:134 | S162C | SEQ ID NO:150 |
| V173C | SEQ ID NO:136 | S162C | SEQ ID NO:150 |
| V173C | SEQ ID NO:136 | Q160C | SEQ ID NO:152 |
| L128C | SEQ ID NO:138 | F118C | SEQ ID NO:36 |
| S134C | SEQ ID NO:140 | F116C | SEQ ID NO:38 |
| S136C | SEQ ID NO:142 | S114C | SEQ ID NO:99 |
| P171C | SEQ ID NO:144 | S162C | SEQ ID NO:150 |
| T139C | SEQ ID NO:146 | F116C | SEQ ID NO:38 |

### Charge mutation

Another effective strategy to force homologous light chain and heavy chain pairing and avoid mismatch between light chain and heavy chain of two antibodies is to find an amino acid pair on the contact surface of the heavy chain and light chain and mutate them into amino acids carrying opposite charges, promoting homologous pairing based on the principle of opposite charges attract. The selection criteria are conservative amino acid pair at the heavy chain variable region and light chain variable region interface, where the distance between α carbon atoms of the amino acid pair is less than 12Å, with the side chains facing each other. The selected amino acid pairs are shown in Table 2 (Kabat number).

**Table 2: Charge Mutation Combinations**

| | Antibody 1 Heavy Chain Mutation | Exa mpl e VH | Antibody 1 Light Chain Mutation | Exa mpl e VL | Antibody 2 Heavy Chain Mutation | Exa mpl e VH | Antibody 2 Light Chain Mutation | Exa mpl e VL |
|---|---|---|---|---|---|---|---|---|
| Combin ation 1 | Q39K | SEQ ID NO: 3 | Q38D | SEQ ID NO: 4 | Q39D | SEQ ID NO: 5 | Q38K | SEQ ID NO: 6 |
| Combin ation 2 | L45K | SEQ ID NO: 7 | Y87D | SEQ ID NO: 8 | L45D | SEQ ID NO: 9 | Y87K | SEQ ID NO: 10 |
| Combin ation 3 | L45K | SEQ ID | P44D | SEQ ID | L45D | SEQ ID | P44K | SEQ ID |
| | | NO: 7 | | NO: 11 | | NO: 9 | | NO: 12 |
| Combin ation 4 | Y91K | SEQ ID NO: 13 | A43D | SEQ ID NO: 14 | Y91D | SEQ ID NO: 15 | A43K | SEQ ID NO: 16 |
| Combin ation 5 | Y91K | SEQ ID NO: 13 | P44D | SEQ ID NO: 11 | Y91D | SEQ ID NO: 15 | P44K | SEQ ID NO: 12 |

The final mutation used for the bispecific antibody expressed in a cell line is a combination of disulfide bond reshaping and charge mutations, as shown in Table 3:

**Table 3: Combination of Charge Mutation and Disulfide Bond Reshaping Mutation in V Region**

| | Antibody 1 | | | | Antibody 2 | |
|---|---|---|---|---|---|---|
| | Heavy Chain VH Mutation | Heavy Chain CH1 Mutation | Light Chain VL Mutation | Light Chain CL Mutation | Heavy Chain VH Mutation | Light Chain VL Mutation |
| Combi nation 1 | Q39K | F126C, C220S | Q38D | Q124C, C214S | Q39D | Q38K |
| Combi nation 2 | L45K | F126C, C220S | Y87D | Q124C, C214S | L45D | Y87K |
| Combi nation 3 | L45K | F126C, C220S | P44D | Q124C, C214S | L45D | P44K |
| Combi nation 4 | Y91K | F126C, C220S | A43D | Q124C, C214S | Y91D | A43K |
| Combi nation 5 | Y91K | F126C, C220S | P44D | Q124C, C214S | Y91D | P44K |
| Combi nation 6 | Q39K | H168C, C220S | Q38D | T164C, C214S | Q39D | Q38K |
| Combi nation 7 | Q39K | F170C, C220S | Q38D | T164C, C214S | Q39D | Q38K |
| Combi nation 8 | Q39K | F170C, C220S | Q38D | S162C, C214S | Q39D | Q38K |
| Combi nation 9 | Q39K | V173C, C220S | Q38D | S162C, C214S | Q39D | Q38K |
| Combi nation 10 | Q39K | V173C, C220S | Q38D | Q160C, C214S | Q39D | Q38K |
| Combi nation 11 | Q39K | S134C, C220S | Q38D | F116C, C214S | Q39D | Q38K |
| Combi nation 12 | Q39K | S136C, C220S | Q38D | S114C, C214S | Q39D | Q38K |
| Combi nation 13 | Q39K | P171C, C220S | Q38D | S162C, C214S | Q39D | Q38K |
| Combi nation 14 | Q39K | T139C, C220S | Q38D | F116C, C214S | Q39D | Q38K |

Where antibody 1 is an anti-Her 2 mAb and antibody 2 is an anti-Hel mAb, see Combination 1 - Combination 5 in Table 3 in the sequence table for the sequence of the full-length chain of the exemplary bispecific antibody, that is the antibody of Combination 1 - Combination 5 in Table 9.

### Substitution in light chain constant region

For the ease of downstream purification and removal of trace mismatched antibodies, a light chain constant region of one of the antibodies may be selected for substitution from C Kappa to C Lambda (if the light chain constant region of one of the antibodies is a Lambda light chain, substitution is not required), such that one of the two antibody Fabs is Kappa and the other is Lambda, and downstream purification is performed on the Kappa and Lambda light chain with a selective affinity medium.

### Embodiment 2. Expression of mutant in HEK293 cells and purification of mutant

The various chains of the various antibodies of the present disclosure were sent to GENEWIZ for gene synthesis and constructed in a pCDNA3.1 template plasmid, and a certain amount of plasmids were prepared. See Table 4 for a summary of the sequence of the chains of the various antibodies used in the embodiment.

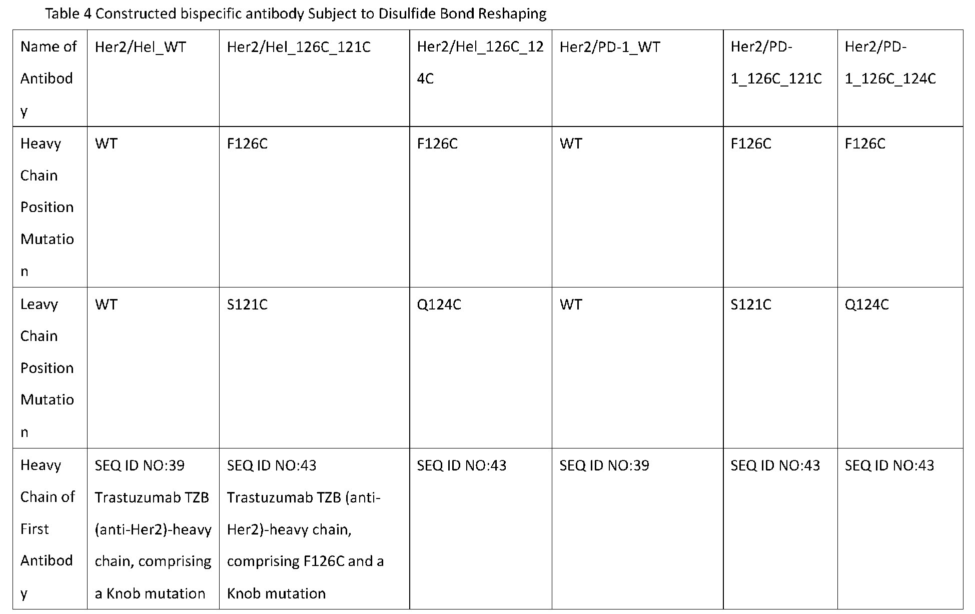

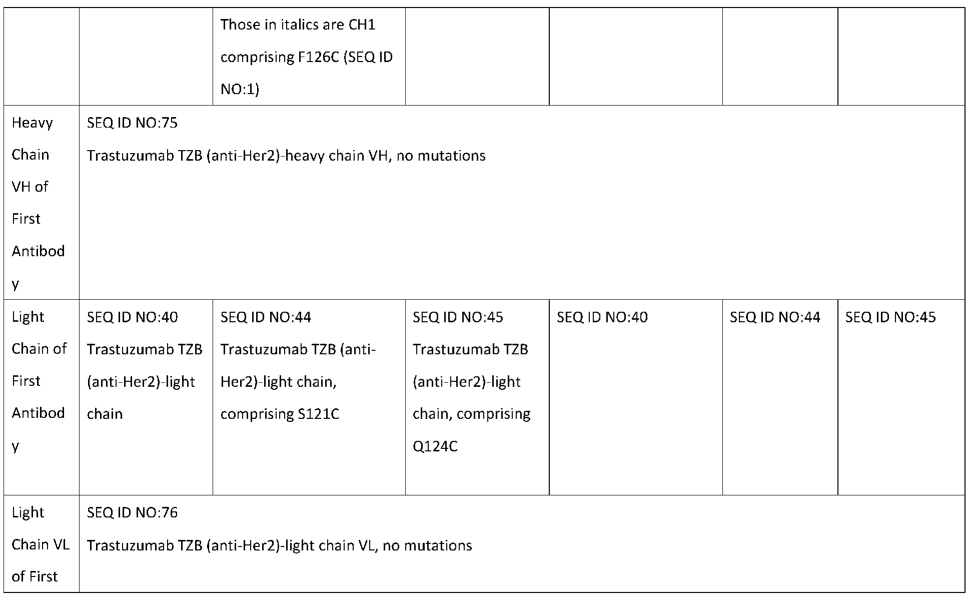

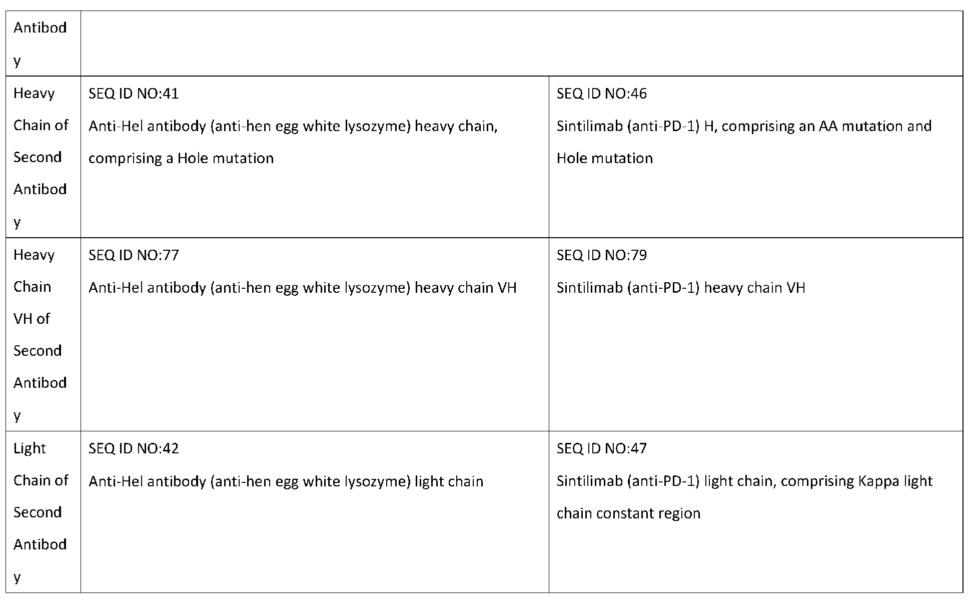

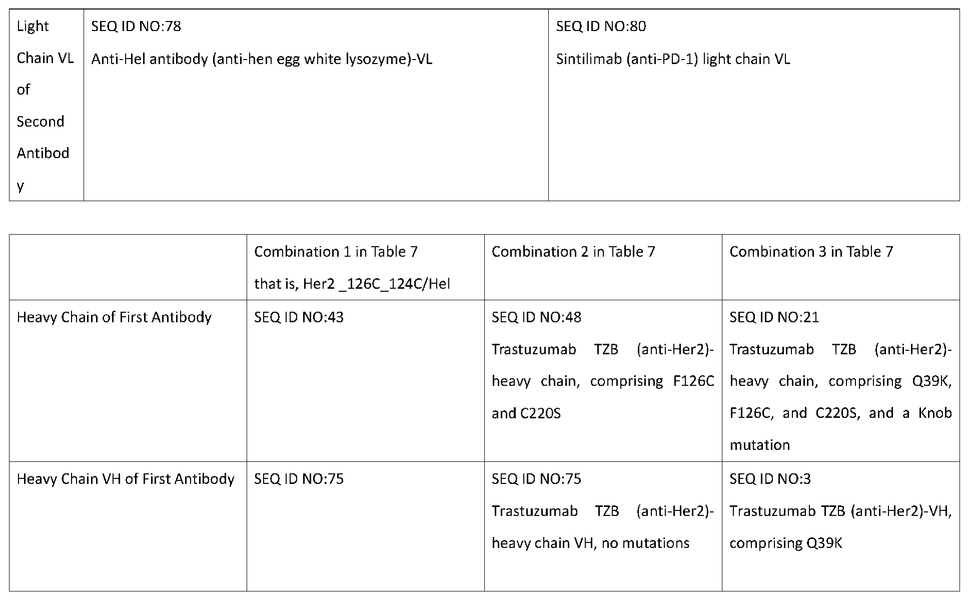

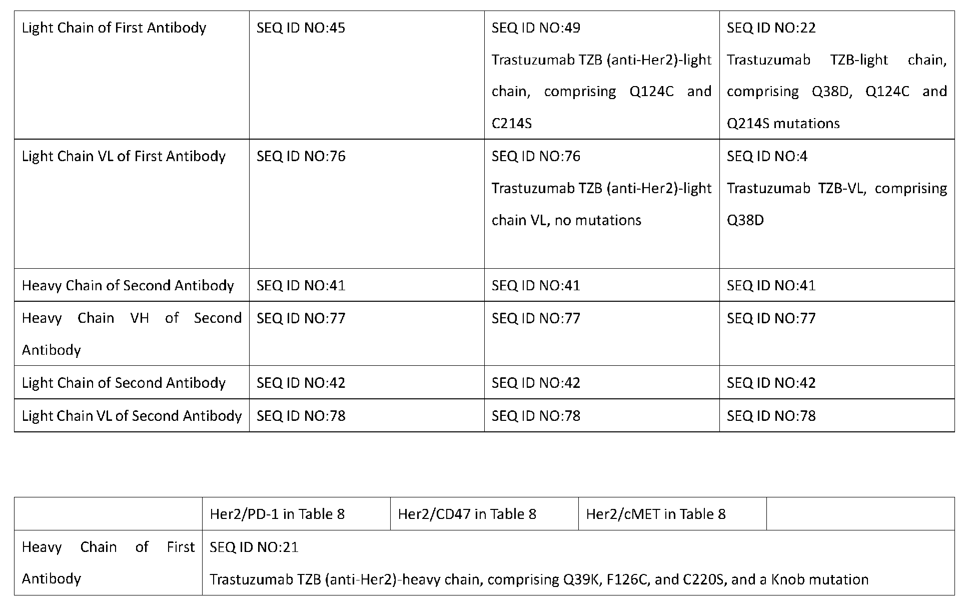

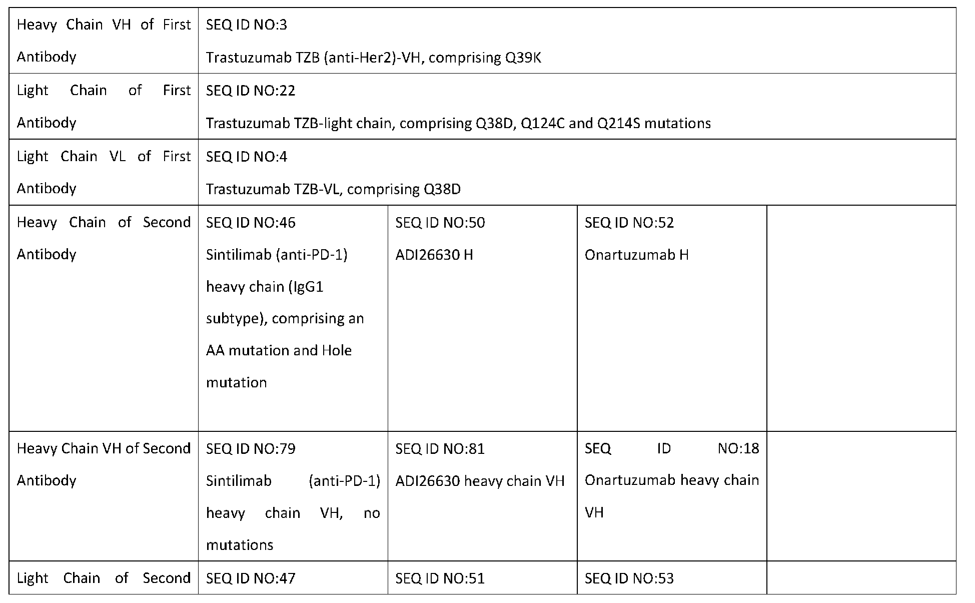

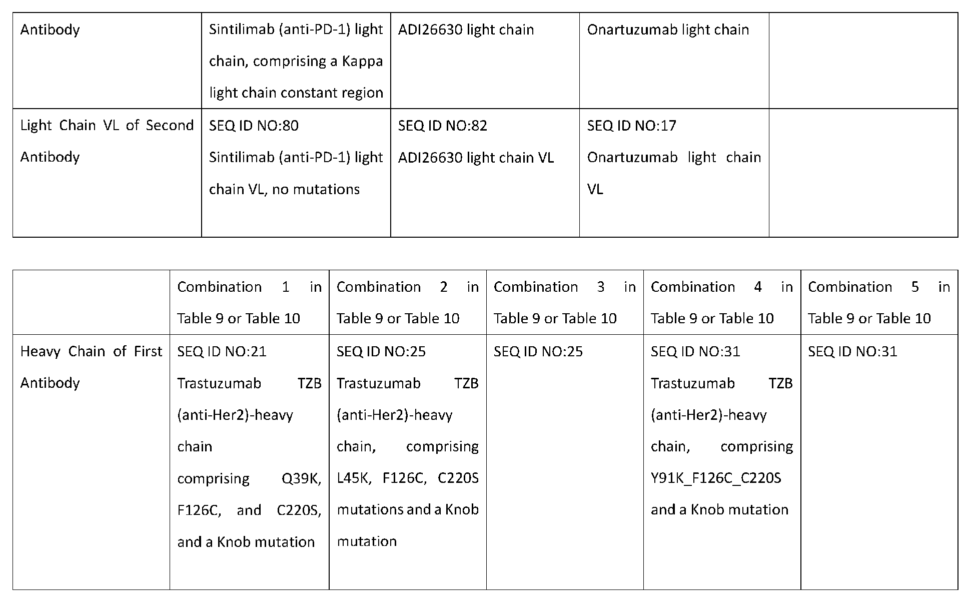

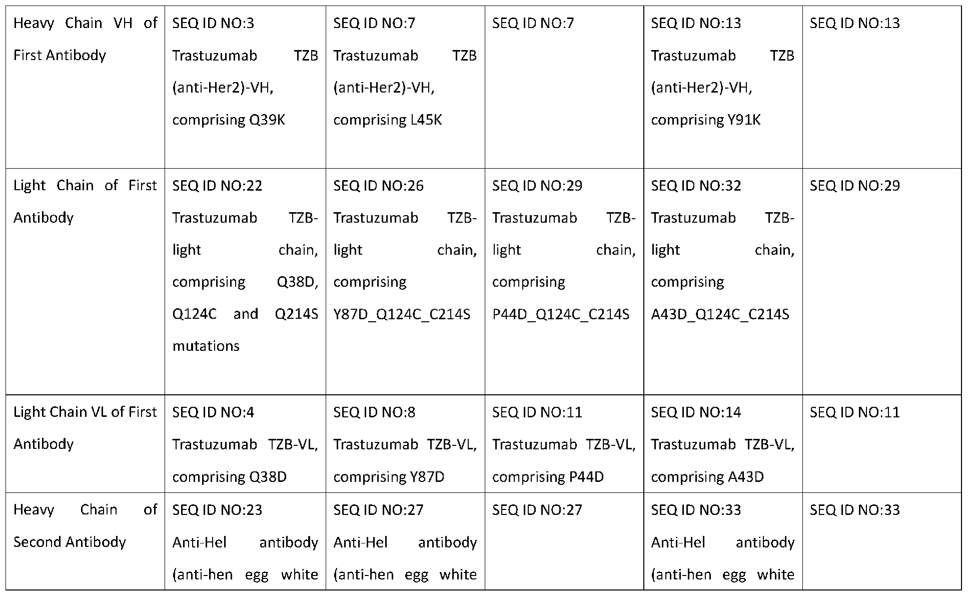

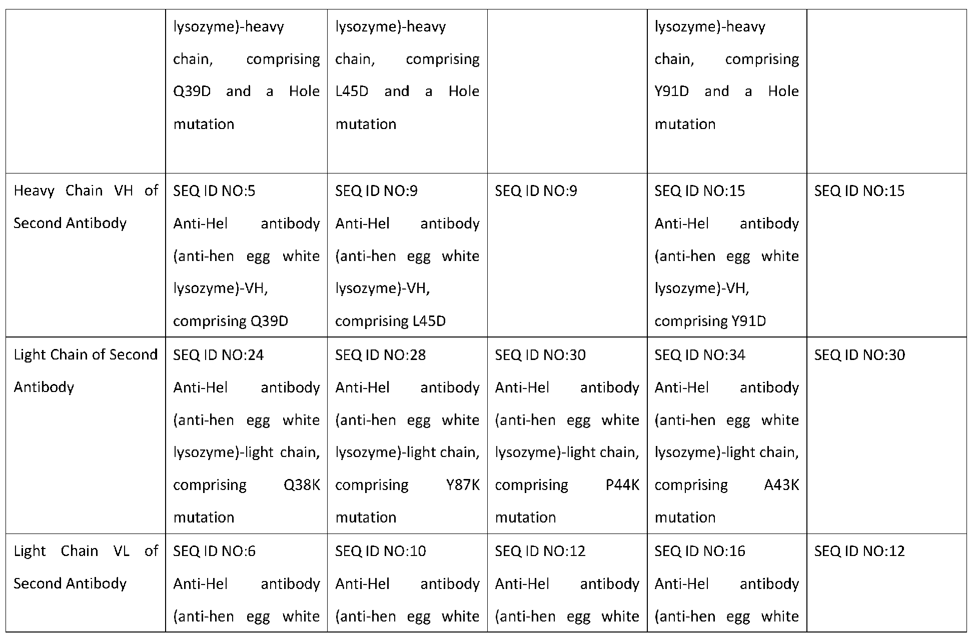

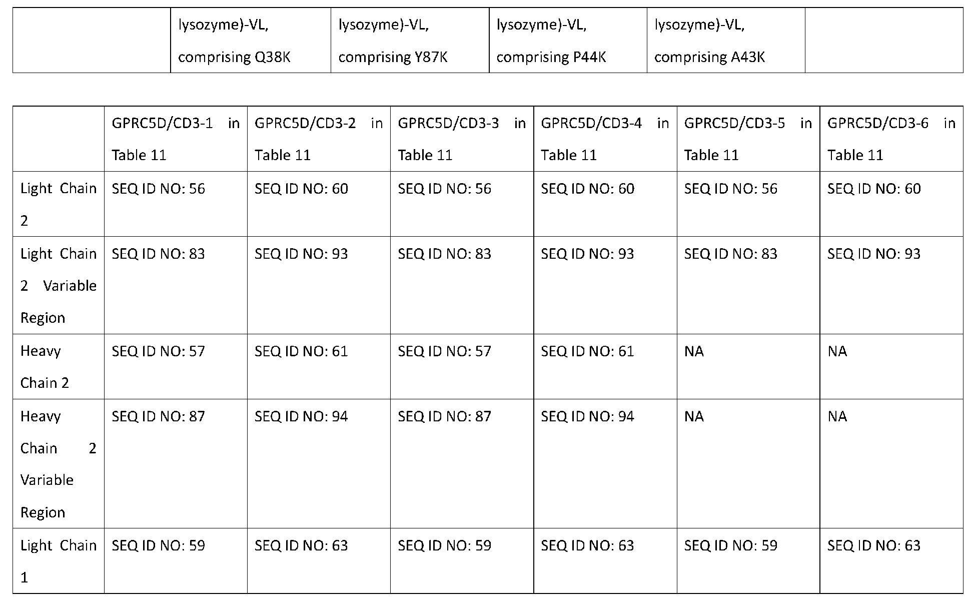

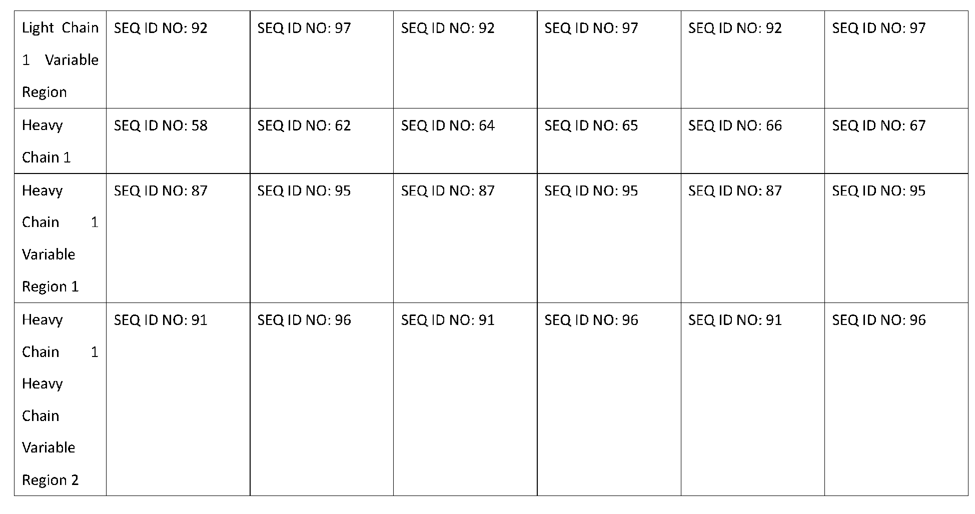

The plasmids were filtered using a 0.22 µm filter head for transient expression of cells. Expi293 cells (Invitrogen) were passaged based on the desired transfection volume and the cell density was adjusted to 3 × 10⁶ cells/mL the day before transfection. The cell density was adjusted to 3 × 10⁶ cells/mL again on the day of transfection. Opti-MEM culture medium (Gibco catalog no.: 31985-070) with a final volume of 1/10 (v/v) was used as the transfection buffer added into the expression plasmids at a ratio of antibody A heavy chain: antibody A light chain: antibody B heavy chain: antibody B light chain = 1: 1: 1: 1 or other suitable ratios, and mixed well. An appropriate amount of polyethyleneimine (PEI) (Polysciences, 23966) was added to the plasmids in the previous step (the mass ratio of plasmid to PEI was 1:3), mixed well and incubated at room temperature for 10 min to obtain a DNA/PEI mixture. The DNA/PEI mixture was gently poured into HEK293 cells and mixed well, and after culture at 37°C, 8% CO₂ conditions for 24 h, additional VPA (Sigma, catalog no.: P4543-100G) was added to a final concentration of 2 mM and 2% (v/v) Feed (1 g/L Phytone Peptone + 1 g/L Difco Select Phytone), and continued to be cultured for six days. The culture product was collected, centrifuged at 4,000 revolutions/minute for 30 minutes, the cell supernatant was collected and filtered through a 0.45 µM membrane, and purified through protein A affinity chromatography. Samples with special requirements were subject to further purification through ion-exchange chromatography, KappaSelect affinity chromatography and LambdaFabSelect affinity chromatography.

The specific operating steps of protein A affinity chromatography purification are: the supernatant was purified using a prepacked column Hitrap Mabselect Sure (GE, 11-0034-95). The operation is as follows: before purification, the packing column was equilibrated using five times the column volume of equilibration solution (20 mM Tris, 150 mM NaCl, pH 7.2); the collected supernatant was passed through the column, then the packing column was cleaned using 10 times the column volume of the equilibration solution to remove non-specific binding protein; the packing was flushed with five times the column volume of elution buffer (100 mM sodium citrate, pH 3.5) and the eluent was collected. 2 M Tris was added to neutralize pH to 6.5. The proportion of correctly paired bispecific antibody after purification was quantitatively measured using liquid chromatography-mass spectrometry (LC-MS).

The specific operating steps of ion exchange chromatography purification are: exchange was performed to a pH 6.0 low-salt PB buffer (10 mM phosphate, pH 6.0) using an ultrafiltration concentration tube (MILLIPORE, catalog no.: UFC901096), and fine purification was performed using Capto HiRes S 10/100 (GE, catalog no. 29275879). before purification, the packing column was equilibrated using five times the column volume of pH 6.0 low-salt PB; the exchanged sample was passed through the column, then the packing column was cleaned using 10 times the column volume of pH 6.0 low-salt PB buffer to remove non-specific binding protein; during elution, the concentration of pH 6.0 low-salt PB buffer (10 mM phosphate, pH 6.0, 1 M NaCl) was increased linearly (linear gradient: within 30 column volumes, the proportion of pH 6.0 high-salt PB buffer was increased from 30% to 100%), and the elution main peak was collected. exchange was performed to a PBS buffer (Gibco, catalog no.: 70011-044) using an ultrafiltration concentration tube (MILLIPORE, catalog no.: UFC901096). The proportion of correctly paired bispecific antibody after purification was quantitatively measured using liquid chromatography-mass spectrometry (LC-MS).

The specific operating steps of KappaSelect affinity chromatography purification are: the supernatant was purified using a prepacked column HiTrap KappaSelect (GE, 17-5458-11). The operation is as follows: before purification, the packing column was equilibrated using five times the column volume of equilibration solution (PBS pH 7.4); the collected supernatant was passed through the column, then the packing column was cleaned using 10 times the column volume of the equilibration solution to remove non-specific binding protein; the packing was flushed with five times the column volume of elution buffer (0.1 M glycine buffer, pH 2.5) and the eluent was collected. 2 M Tris was added to neutralize pH to 6.5. The proportion of correctly paired bispecific antibody after purification was quantitatively measured using liquid chromatography-mass spectrometry (LC-MS).

The specific operating steps of LambdaFabSelect affinity chromatography purification are: the supernatant was purified using a prepacked column Hitrap LambdaFabSelect (GE, 17-5482-11). The operation is as follows: before purification, the packing column was equilibrated using five times the column volume of equilibration solution (PBS pH 7.4); the collected supernatant was passed through the column, then the packing column was cleaned using 10 times the column volume of the equilibration solution to remove non-specific binding protein; the packing was flushed with five times the column volume of elution buffer (0.1 M acetate buffer, pH 3.5) and the eluent was collected. 2 M Tris was added to neutralize pH to 6.5. The proportion of correctly paired bispecific antibody after purification was quantitatively measured using liquid chromatography-mass spectrometry (LC-MS).

### Embodiment 3 Screening of mutated amino acid in the present disclosure

### Screening of mutation sites that are capable of directly forming disulfide bonds

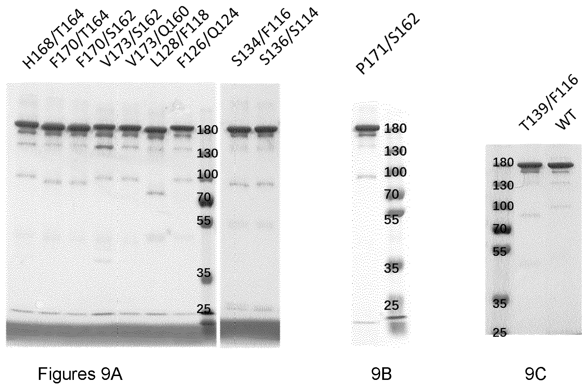

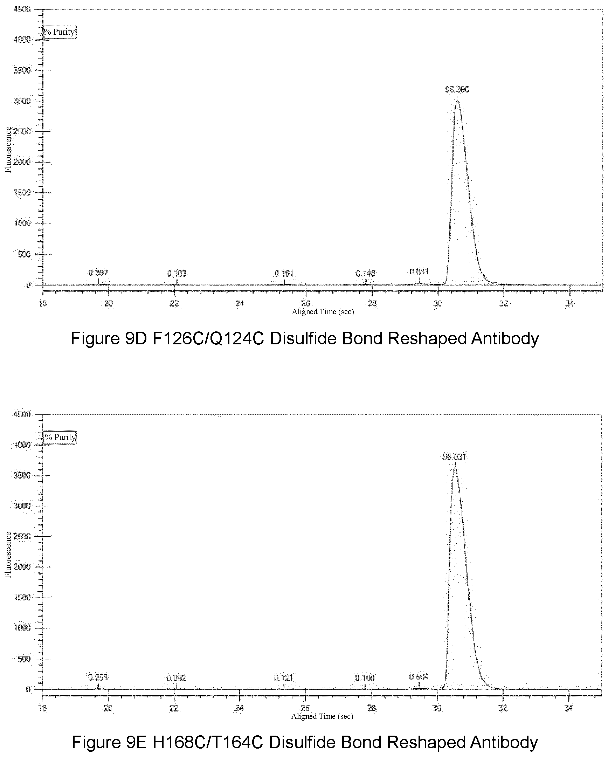

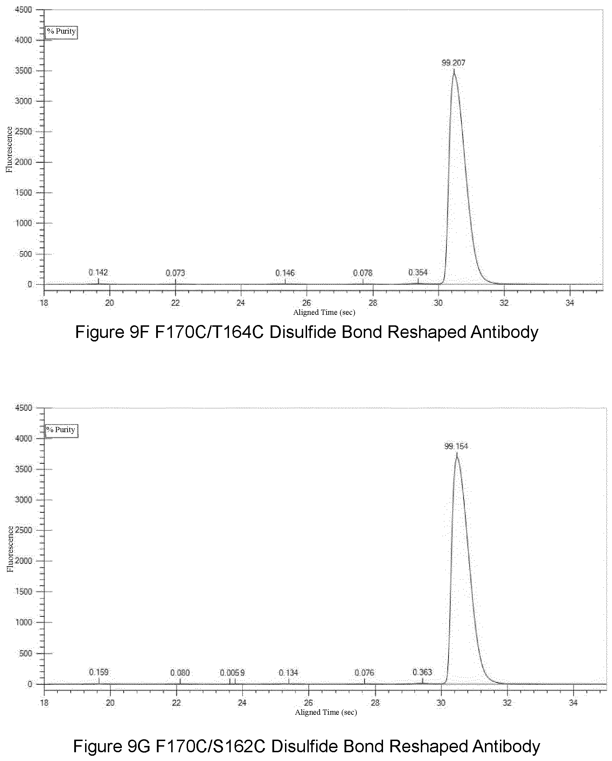

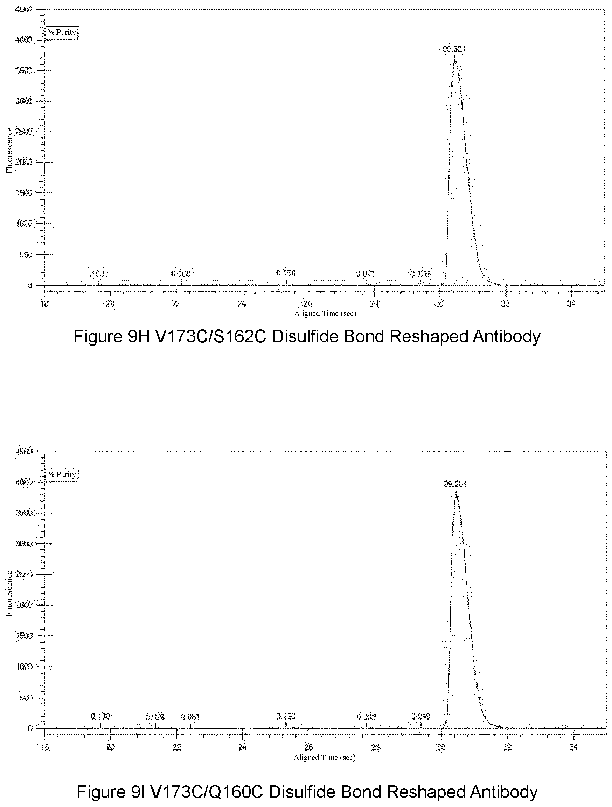

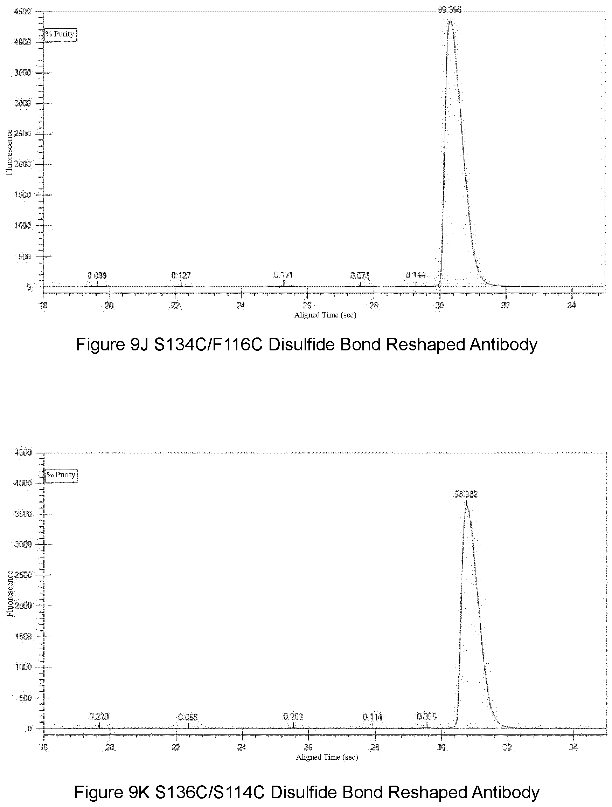

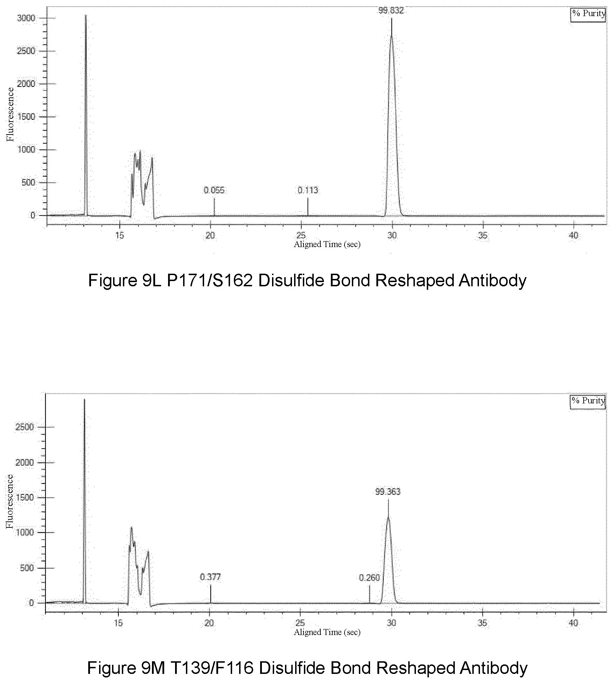

Trastuzumab, as the model antibody, was mutated to remove the natural disulfide bond between the heavy chain and light chain (H C220S, light chain C214S), then the selected amino acid pair according to Table 1 was mutated to Cys. The mutated mAb was expressed in the HEK293 system, purified using protein A, then non-reduced polyacrylamide gel electrophoresis was performed, as shown in Figure 9A - 9C. It can be seen that these mutated mAb may be maintained as complete molecules in non-reduced polyacrylamide gel electrophoresis and only minimal light chain components are dropped. The selected disulfide bond mutation sites basically formed disulfide bonds in a normal way.

Further, these antibodies that formed disulfide bonds were subject to denaturing nrCE-SDS (Figure 9D - Figure 9M), and quantitative measurement was performed on the integrity of the formed disulfide bonds. It can be seen that disulfide bond reshaped antibodies had over 98% complete molecules, which further prove that reshaped disulfide bonds can be formed correctly.

### Effect of disulfide bond reshaping mutation on the proportion of correct pairings

Using the F126/Q124C disulfide bond reshaping mutation in Table 1 as an example, the effect of the disulfide bond reshaping mutation on the proportion of correct pairings was tested, applying the mutated antibody of the present disclosure and using the wild-type antibody sequence as the control. After expression in HEK293 cells, only protein A affinity chromatography purification was used to prevent other purification methods from removing mismatched molecules. The proportion of correct anti-Her2/anti-Hel antibody pairs to anti-Her2/anti-PD-1 antibody pairs when the bispecific antibody was formed was tested using LC-MS. Heterologous pairing between heavy chain was promoted through Knob-into-hole.

**Table 5 Bispecific Antibody Subject to Disulfide Bond Reshaping and Proportion of Correct Pairing**

| Name of Antibody | Proportion of Correctly Paired Molecules |
|---|---|
| Her2/Hel_WT | 30.27% |
| Her2/Hel_126C_121C | 58.18% |
| Her2/Hel_126C_124C | 65.10% |
| Her2/PD-1_WT | 55.60% |
| Her2/PD-1_126C_121C | 70.78% |
| Her2/PD-1_126C_124C | 76.93% |

It can be seen from the above table that F126C/Q124C mutation is capable of significantly increasing the proportion of correct pairing of bispecific antibody, for example, the proportion of correct Her2/PD-1 antibody pairs to wild-type antibody pairs reached 55.60%. After adding F126C/Q124C mutation, the proportion of correct pairing increased up to 76.93%.

It can be seen that the proportion of correctly paired molecules formed by F126C/Q124C mutation was the highest, it was significantly higher than the wild-type antibody sequence, and was also higher than the mutation F126C/S121C disclosed in CN104011221B to a certain degree.

In addition, if no correct disulfide bonds were formed between mutated Cys pairs, Cys with complete thiols often react with free Cys in cells or solutions to form disulfide bonds, causing protein molecules to be modified by Cys. This modification may be detected by LC-MS.

**Table 6 Testing of Cys Modification in Bispecific Antibody Subjected to Disulfide Bond Reshaping**

| Name of Antibody | Proportion of Cys Modification |
|---|---|
| Her2/Hel_WT | N/A |
| Her2/Hel_126C_121C | N/A |
| Her2/Hel_126C_124C | N/A |
| Her2/PD-1_WT | N/A |
| Her2/PD-1_126C_121C | 10% |
| Her2/PD-1_126C_124C | N/A |

Regardless of whether in Her2/Hel bsAb or in Her2/PD-1 bsAb, the modified component of free Cys is almost undetectable after F126C/Q124C mutation, which further illustrates the extremely high proportion of correctly formed disulfide bonds. In contrast, Her2/PD-1 bispecific antibody that introduced mutation F126C/S121C as disclosed in CN104011221B still had approximately 10% of modified free Cys, meaning that part of the F126C/S121C disulfide bond could not be fully formed in a correct manner.

### Disulfide Bond Reshaping Mutation and V Region Charge Mutation

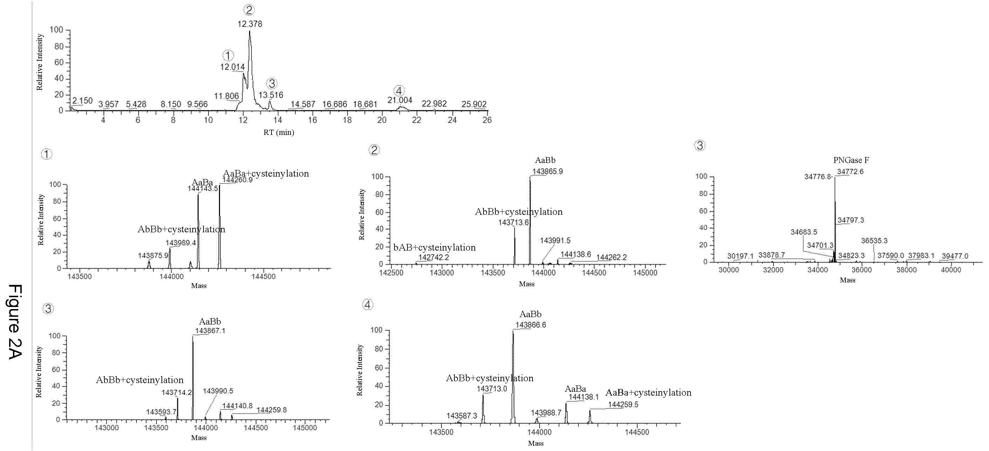

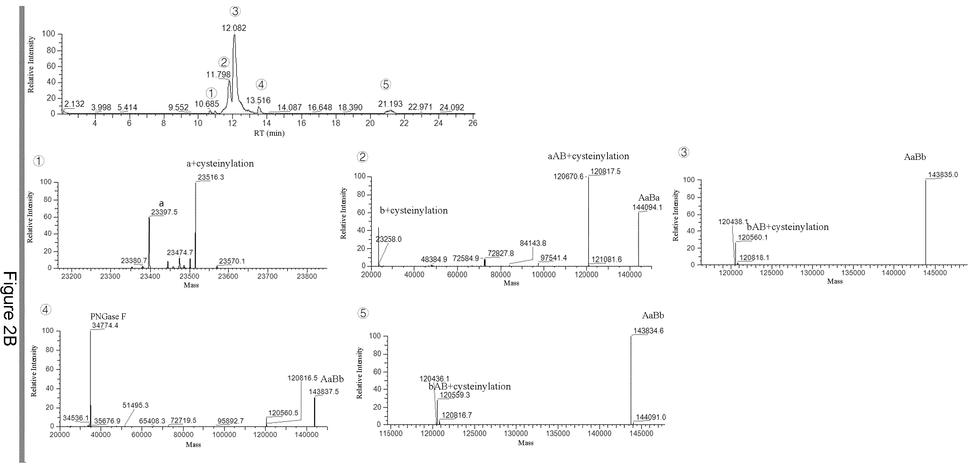

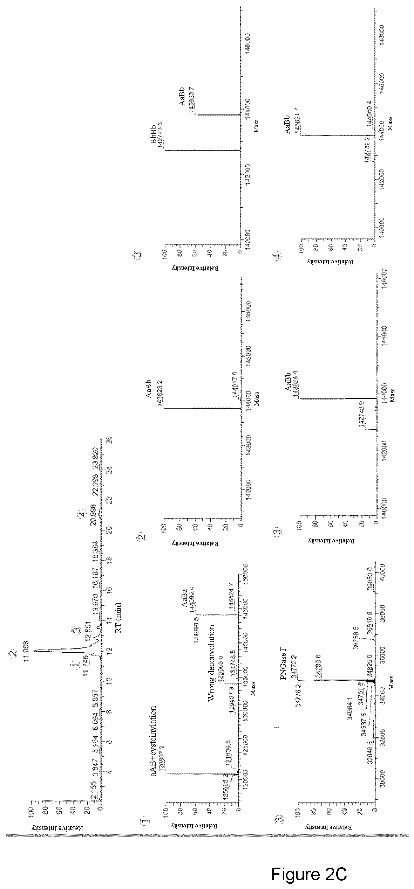

A charge mutation (Q39K/Q38D) was added to a disulfide bond reshaping mutation (F126C/Q124C), and after expression in HEK293 cells, only protein A affinity chromatography purification was used to prevent other purification methods from removing mismatched molecules. The proportion of correct anti-Her2/anti-Hel antibody pairs when the bispecific antibody was formed was tested using LC-MS (Figure 2). Heterologous pairing between heavy chain was promoted through Knob-into-hole.

**Table 7 Proportion of Correctly Paired Bispecific Antibody after Disulfide Bond Mutation and Charge Mutation**

| | Heavy Chain VH Mutation | Heavy Chain CH1 Mutation | Light Chain VL Mutation | Light Chain CL Mutation | Proportion of Correctly Paired Molecules |
|---|---|---|---|---|---|
| Combi nation 1 | N/A | F126C | N/A | Q124C | 47.4% |
| Combi nation 2 | N/A | F126C, C220S | N/A | Q124C, C214S | 65.6% |
| Combi nation 3 | Q39K | F126C, C220S | Q38D | Q124C, C214S | 96.3% |

It can be seen that the ratio of correctly paired bispecific antibody increased significantly after disulfide bond reshaping. After removing the natural disulfide bond of the antibody (Combination 2 in Table 7, Figure 2B), the correct pairing ratio increased more than when only disulfide bonds were added (Combination 1 in Table 7, Figure 2A). Further, when the charge mutation was added to the V region (Combination 3 in Table 7, Figure 2C), the correct pairing ratio increased further.

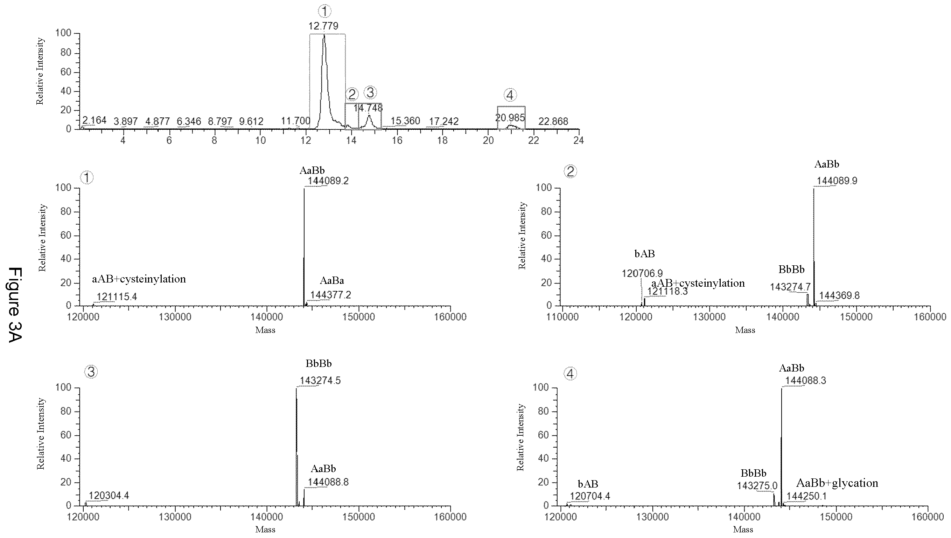

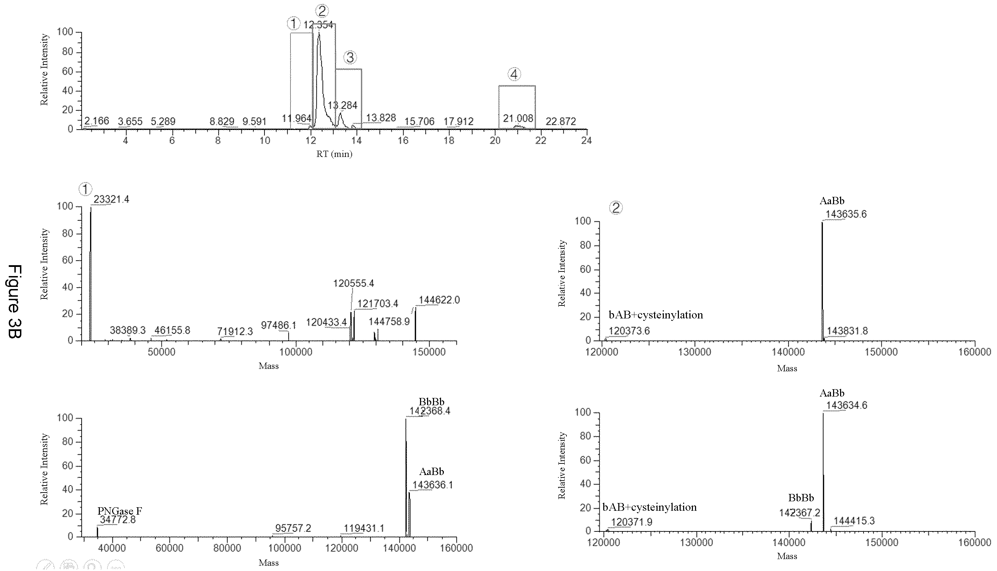

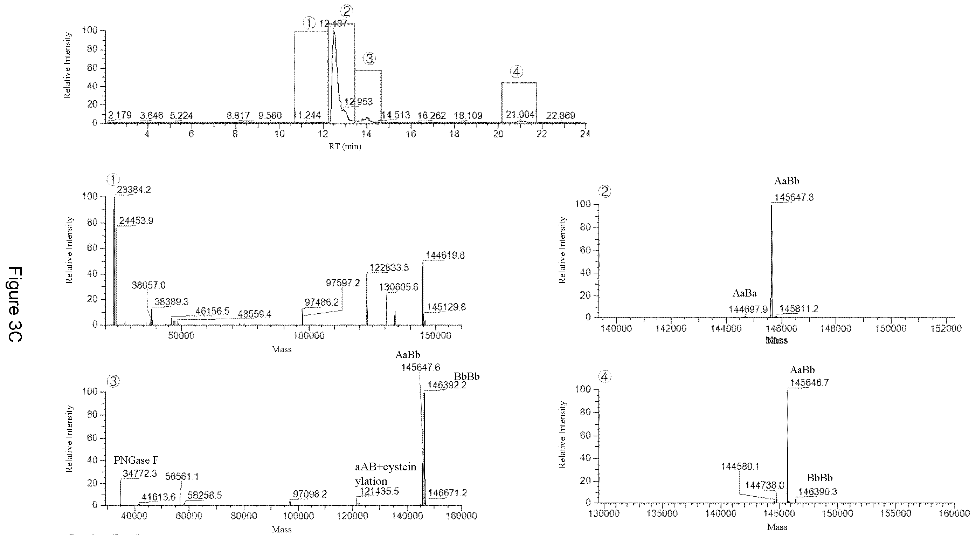

Using four pairs of different antibody pair combination of bsAb, after expression in HEk293 cells, only protein A affinity chromatography purification was used to prevent other purification methods from removing mismatched molecules. The proportion of correct pairing when the bispecific antibody was formed was tested using LC-MS (Figure 3). The universality of the disulfide bond reshaping and charge mutations on different antibodies was validated. At the same time, the expression level of the antibody was observed.

**Table 8-1 Antibody Sequence and Proportion of Correct Pairing after Introduction of Disulfide Bond Reshaping and Charge Mutations into Different Antibodies**

| Antibody Pair | Mutation | Proportion of Correctly Paired Molecules | Expressi on Level (mg/L) |
|---|---|---|---|
| Her2/PD-1 | Q39K,F126C,C220S/ Q38D,Q124C,C214S | 86.1% | 199.6 |
| Her2/CD47 | | 89.1% | 234.8 |
| Her2/cMET | | 94.6% | 222.9 |

The proportion of correct pairing was maintained at approximately 90% in four different antibody combinations, illustrating that disulfide bond reshaping and charge mutations have universality in different antibodies. The expression level of four different antibody compositions reached approximately 200 mg/L, which is a relatively high expression level, and is comparable to mAb.

### Effect of Disulfide Bond Reshaping and Charge Mutations at Different Positions on the Proportion of Correct Pairing

The natural assembly and secretory process of IgG in cells is: after heavy chain is expressed in cells, CH1 binds with molecular chaperone BiP and maintains an incomplete folded state before meeting the light chain. After meeting the light chain, the light chain first binds to the VH through the VL, then the CL substitutes BiP and binds to the CH1, and under the assistance of the CL, the CH1 is completely folded, and light chain and heavy chain assembly is complete. (Matthias J. Feige, An Unfolded CH1 Domain Controls the Assembly and Secretion of IgG Antibodies, Molecular Cell. 2009 Jun 12;34(5): 569 - 79). It can be seen from this biological process that the mutual recognition between the VH and VL is the first process that drives light chain and heavy chain assembly. As such, the charge mutation of the present disclosure is mainly designed in the VH and VL regions. In order to further prove the superiority of our design, we also introduced charges between CH1 and CL to compare the effect of charge mutations in the V region and C region on the proportion of correct pairing. The only difference in sequence between any mutated Her2/Hel bispecific antibody in Table 8-2 and the antibody of Combination 3 in Table 7 is a difference in the listed mutation combination while other sequences are unchanged.

**Table 8-2 Effect of Disulfide Bond Reshaping and Charge Mutations at Different Positions on the Proportion of Correct Pairing**

| Charge mutation | Disulfide Bond Reshaping Mutation | Proportion of Correctly Paired Molecules |
|---|---|---|
| Q39K/Q38D (V 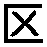 ) | F126/Q124 | 96.34% |
| H171K+T190K/N137D (C 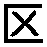 ) | F126/Q124 | 77.89% |
| K216E/E123R (C 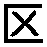 ) | F126/Q124 | 55.87% |

It can be seen that with the same disulfide bond reshaping mutation, [comparing] a combination of Q39K/Q38D charge mutations located in the V region, H171K+T190K/N137D and K216E/E123R located in the C region, the charge mutations located in the V region resulted in a high proportion of correctly paired molecules. This proves the design superiority of locating the charges in the V region.

### Combination of Other V Region Charge Mutations and Disulfide Bond Reshaping

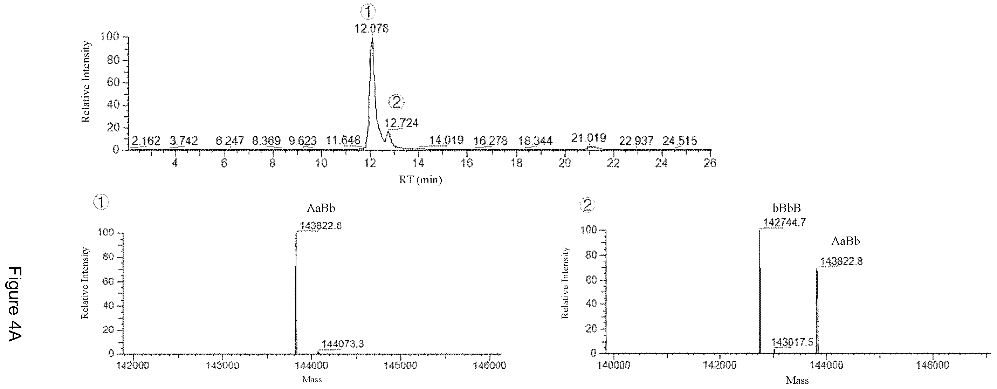

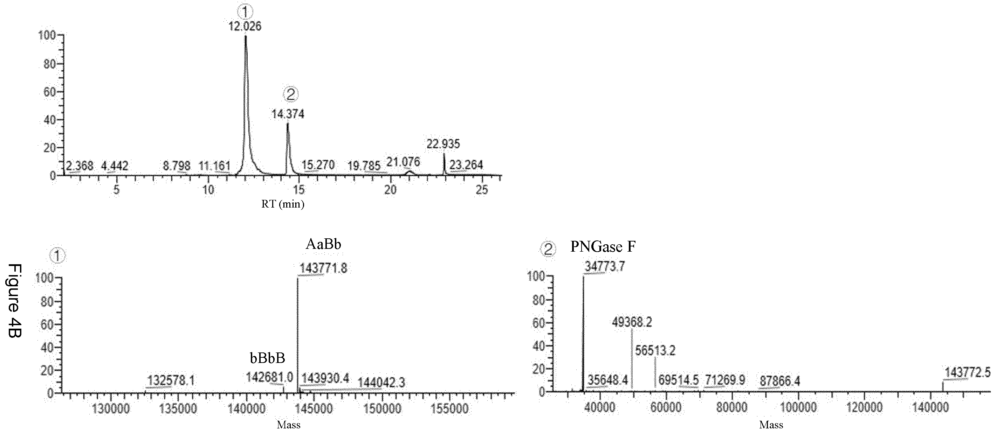

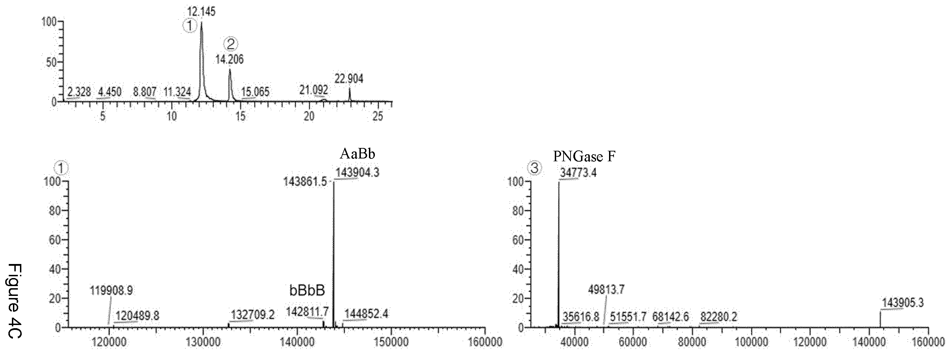

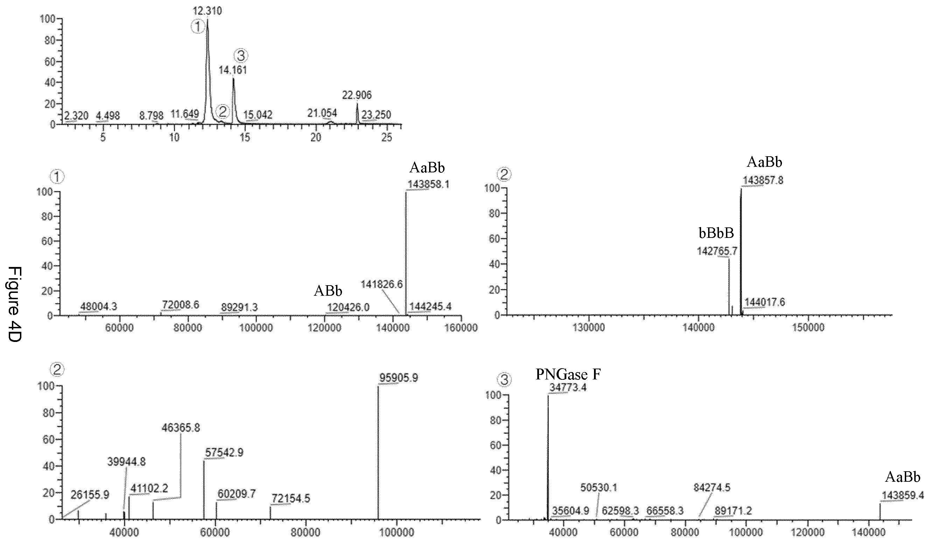

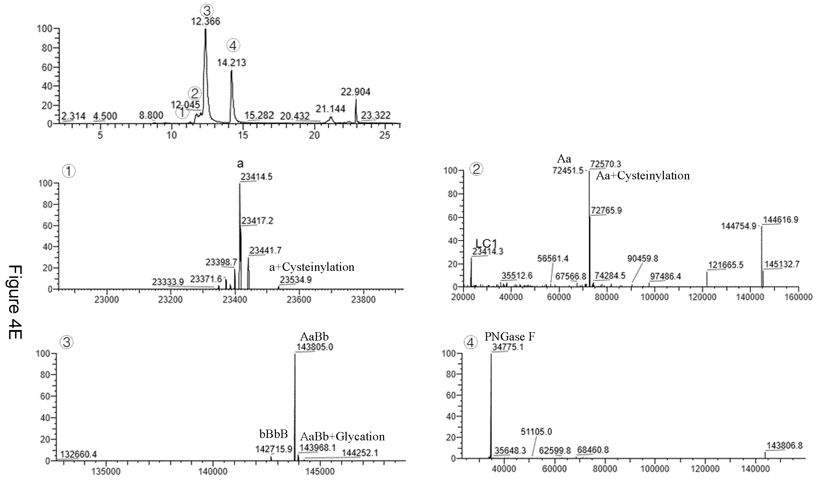

Apart from Q39K and Q38D, other charge mutations discovered in V region and disulfide bond reshaping mutation F126C/Q124C were combined to form Combination 1 - Combination 5 in Table 3. After expression in HEK293 cells, only protein A affinity chromatography purification was used to prevent other purification methods from removing mismatched molecules. The proportion of correct pairing when the bispecific antibody was formed was tested using LC-MS (Figure 4). Heterologous pairing between heavy chain was promoted through Knob-into-hole. At the same time, the expression level of the antibody was observed.

**Table 9-1 Proportion of Correctly Paired Molecules after V Region Mutation and Disulfide Bond Reshaping**

| Antibody Pair | Mutation | Proportion of Correctly Paired Molecules | Expression Level (mg/L) |
|---|---|---|---|
| Her2/Hel | Combination 1 | 92.17% | 305.6 |
| | Combination 2 | 98.88% | 221.0 |
| | Combination 3 | 99.11% | 183.5 |
| | Combination 4 | 98.47% | 83.8 |
| | Combination 5 | 99.47% | 104.6 |

A correct pairing ratio of over 90% was achieved by the mutation method in Combination 1 to Combination 5. An expression level (200 - 300 mg/L) close to mAb was achieved in Combination 1 to Combination 3. The expression level of Combination 4 and Combination 5 was slightly low, but was also up to approximately 100 mg/L. Therefore, the bispecific antibody comprising the mutation combination of the present disclosure is capable of achieving a relatively good expression level.

### Combination of V region charge mutation and other disulfide bond reshaping mutations

Apart from F126C/Q124C, other discovered disulfide bond reshaping mutations were combined with V region charge mutations Q39K and Q38D to form Combination 6 - Combination 14 in Table 3. The only difference in sequence between any mutated Her2/Hel bispecific antibody comprising Combination 6 - Combination 14 in Table 3 and any of Combination 1 - 5 in Table 3 is a difference in the listed mutation combination, while other sequences are unchanged.

After expression in HEK293 cells, only protein A affinity chromatography purification was used to prevent other purification methods from removing mismatched molecules. The proportion of correct pairing when the bispecific antibody was formed was tested using LC-MS. Heterologous pairing between heavy chain was promoted through Knob-into-hole. At the same time, the expression level of the antibody was observed.

**Table 9-2 Proportion of Correctly Paired Molecules after V Region Charge Mutation and Disulfide Bond Reshaping Mutation**

| Antibody Pair | Mutation | Proportion of Correctly Paired Molecules | Expression Level (mg/L) |
|---|---|---|---|
| Her2/Hel | Combination 6 | 83.90% | 199.6 |
| | Combination 7 | 97.50% | 229.5 |
| | Combination 8 | 98.90% | 243.2 |
| | Combination 9 | 91.90% | 252.9 |
| | Combination 10 | 80.52% | 253.2 |
| | Combination 11 | 79.46% | 264.1 |
| | Combination 12 | 86.86% | 262.2 |
| | Combination 13 | 93.16% | 224.0 |
| | Combination 14 | 83.82% | 246.1 |

The mutation method in Combination 6 to Combination 14 was capable of achieving a correct pairing ratio of up to 79.46% - 98.90% for the bsAb, and the expression level reached a level similar to mAb (200 - 300 mg/L). Therefore, the bispecific antibody comprising the mutation combination of the present disclosure is capable of achieving a relatively good proportion of correct pairing and expression level.

### Embodiment 4. Affinity testing experiment of antibody in the present disclosure

Biolayer Interferometry (BLI) technology was used to test the equilibration dissociation constant (KD) of bispecific antibody binding to the respective antigens in the present disclosure. The BLI method affinity test was performed using an existing method (Estep, P et al., High throughput solution Based measurement of antibody-antigen affinity and epitope binning. MAbs, 2013.5 (2): Pages 270 - 8).

Half an hour before the start of the experiment, a suitable number of ProA (ForteBio, 18-5010) sensors based on the sample size were soaked in SD buffer (PBS 1×, BSA 0.1%, Tween-20 0.05%). 100 µL of SD buffer and antigen fusion protein were taken and added to a 96-well black polystyrene half-area microplate (Greiner, 675076), respectively. The sensor position was selected based on the sample position and layout. The instrument setting parameters are as follows: Run steps: Baseline, Loading ^{~}1 nm, Baseline, Association and Dissociation; the run time of each step depended on the sample binding and dissociation rate, and the rotational speed was 400 rpm and temperature was 30 degrees. ForteBio analysis software was used to analysis the KD value.

Using anti-Her2 antibody as the model, after introducing the mutation of Combination 1 - Combination 5 in Table 3, the affinity data with antigen Her2 thereof is shown in the table below.

**Table 10. Affinity Test of Example Antibody and Her2**

| Name | Antigen | kon(1/Ms) | kdis(1/s) | KD (M) |
|---|---|---|---|---|
| WT | Human Her2 | 2.68E+05 | 2.00E-04 | 7.46E-10 |
| Combination 1 | | 2.49E+05 | 2.00E-04 | 8.05E-10 |
| Combination 2 | | 2.68E+05 | 2.00E-04 | 7.48E-10 |
| Combination 3 | | 2.64E+05 | 2.00E-04 | 7.58E-10 |
| Combination 4 | | 2.62E+05 | 2.00E-04 | 7.64E-10 |
| Combination 5 | | 2.78E+05 | 2.00E-04 | 7.20E-10 |

It can be seen from the results in the above table that there was no significant difference in affinity between the various mutants and wild-type antibodies. Hence, the introduction of mutation and disulfide bond at the binding surface of light chain and heavy chain in the V region according to the present disclosure does not affect antibody affinity.

### Embodiment 5. Expansion of multivalent configurations

Not only can the bispecific antibody of the present disclosure be applied in the monovalent 1+1 configuration (Figure 1), but it may also be expanded into multivalent configurations such as the 2+1 N-terminal configuration (Figure 5A), 2+1 C-terminal configuration (Figure 5B), 2+2 C-terminal configuration (Figure 5C), and other formats. Apart from tandem Fab configuration that does not require heavy chain heterologous pairing, heterologous pairing between heavy chain was promoted through Innobody mutation of the present company.

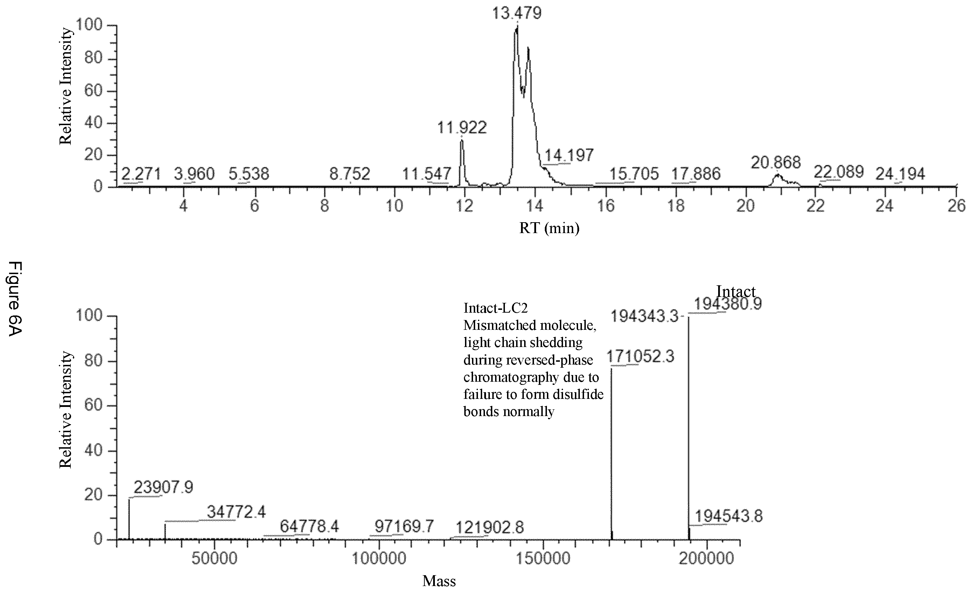

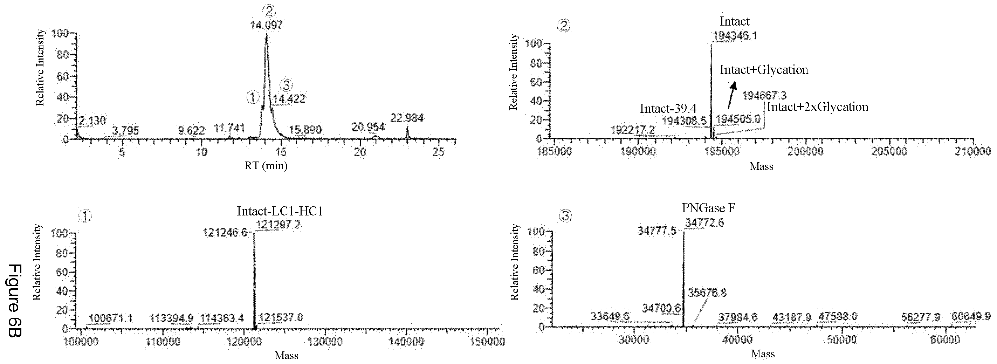

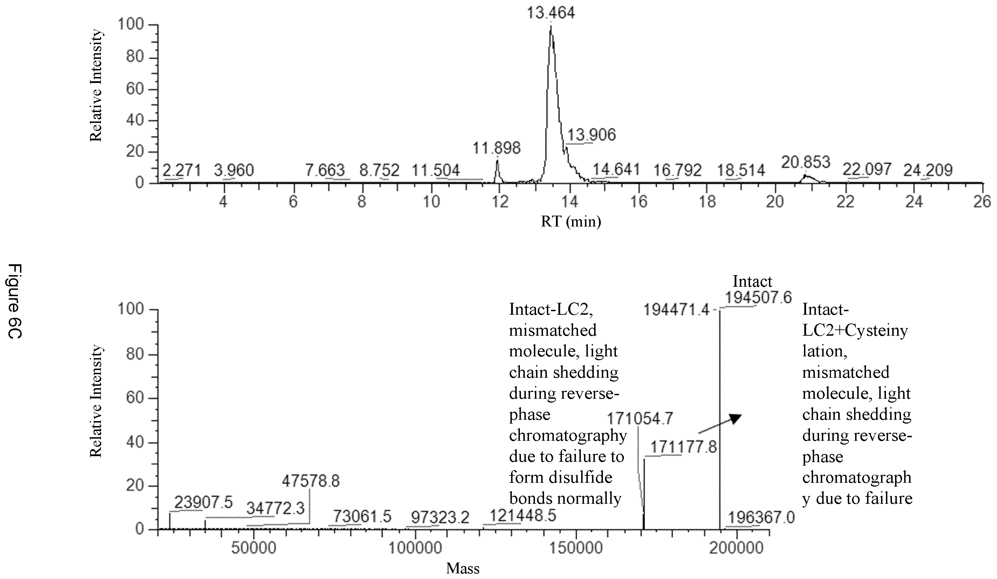

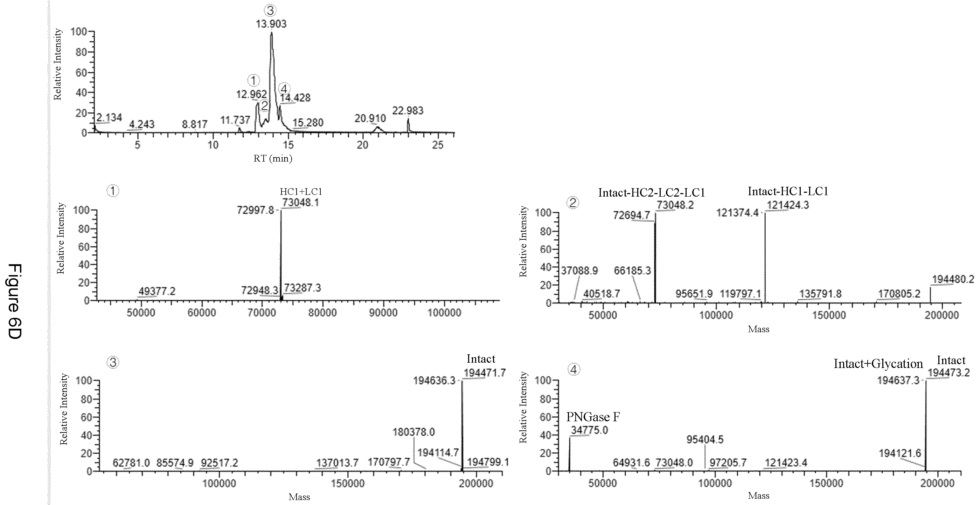

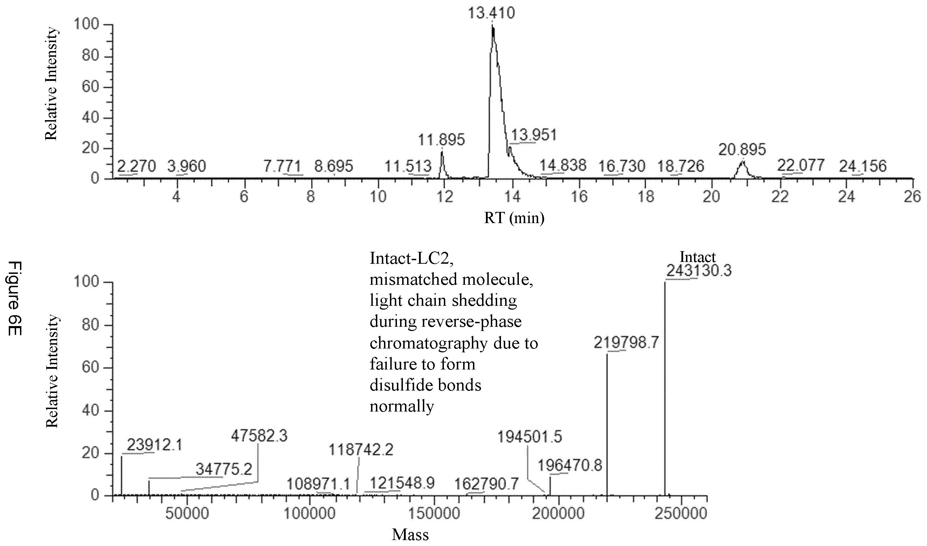

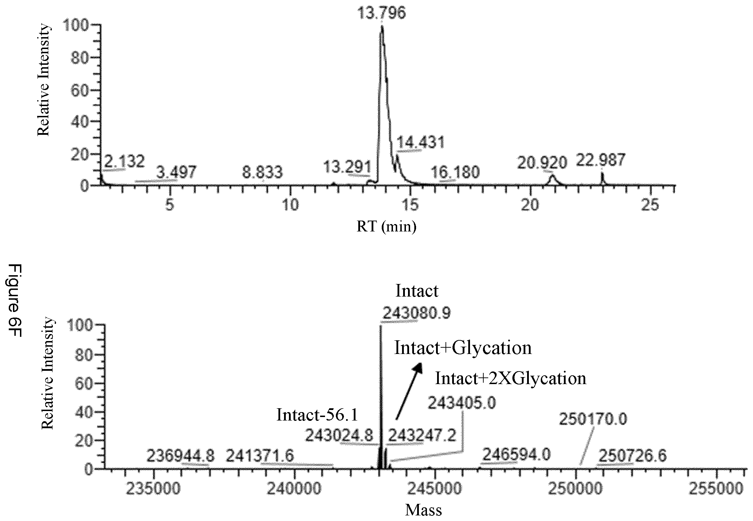

The multivalent 2+1N-terminal configuration, 2+1C-terminal configuration, 2+2C-terminal configuration were expressed and purified according to the method in Embodiment 2, and the proportion of correct pairing was tested using mass spectrometry (Figure 6). Refer to Table 11 for the summary of results.

**Table 11 Proportion of Correctly Paired Molecules with Multivalent Configuration**

| Antibody No. | Antibody Pair | Configuration | Mutation | Proportion of Correctly Paired Molecules |
|---|---|---|---|---|
| GPRC5D/CD3-1 | GPRC5D/CD3 | 2+1N-terminal configuration | Only disulfide bond reshaping | 37.80% |
| GPRC5D/CD3-2 | GPRC5D/CD3 | 2+1N-terminal configuration | Disulfide bond reshaping and charge mutations | 99.32% |
| GPRC5D/CD3-3 | GPRC5D/CD3 | 2+1C-terminal configuration | Only disulfide bond reshaping | 59.10% |
| GPRC5D/CD3-4 | GPRC5D/CD3 | 2+1C-terminal configuration | Disulfide bond | 98.58% |

| | | | | |
|---|---|---|---|---|
| | | | reshaping and charge mutations | |
| GPRC5D/CD3-5 | GPRC5D/CD3 | 2+2C-terminal configuration | Only disulfide bond reshaping | 42.60% |
| GPRC5D/CD3-6 | GPRC5D/CD3 | 2+2C-terminal configuration | Disulfide bond reshaping and charge mutations | 98.89% |

It can be seen from the above table that when the mutation declared in the present patent was applied to molecules with multivalent configurations, it could still maintain a very high proportion of correct pairings. Moreover, when it was applied to molecules with multivalent configurations, it was similarly seen that disulfide bond reshaping and charge mutations had a much higher proportion of correct pairings than disulfide bond reshaping mutation alone.

### Embodiment 6. Cell killing experiment

BCMA/GPRC5D/CD3 trispecific antibody (wild-type and introduced mutations declared in the present patent, see Figure 7 for the configuration, refer to the sequence table for the sequence) simultaneously bound to BCMA and GPRC5D on the surface of H929 cells and CD3 on the surface of T cells in primary PBMC, activating T cells, and mediating the killing of BCMA or GPRC5D-positive tumor cells. This study tested the LDH level released by dead cells in the supernatant collected 24 hours after the example antibody was added under co-culture conditions of PBMC and BCMA-positive H929 cells and GPRC5D-positive H929 cells using the lactate dehydrogenase (LDH) method, thereby assessing the killing capability of BCMA tumor cells and GPRC5D tumor cells by antibody-mediated T cells. The effect of the mutations declared in the present patent on the trispecific antibody was assessed based on the BCMA/GPRC5D/CD3 trispecific antibody in which charge mutation and disulfide bond reshaping mutation of the present disclosure were not introduced (Figure 7A) and the BCMA/GPRC5D/CD3 trispecific antibody in which the mutations declared in the present disclosure were introduced (Figure 7B).

PBMC (SallyBio, SLB-HP100A) was taken out from the liquid nitrogen tank and quickly thawed at 37°C, pre-heated 1640 medium (Gibco, 22400-071) (containing 0.1% DNase) was added dropwise to obtain 10 mL of a mixture. The mixture was centrifuged at 400 g for five minutes, re-suspended using 10 mL of 1640 medium, 10 µL of DNase was added and monolayer cultured at 37°C, 5% CO₂ overnight. Suspended cells were pipetted and centrifuged at 400 g for five minutes, re-suspended using 1640 medium, counted, the cell density was adjusted to 4 × 10⁶ cells/mL and used as effector cells. BCMA or GPRC5D-positive H929 cells (ATCC, CRL-9068) were used as target cells, centrifuged at 400g for five minutes, re-suspended using 1640 medium, counted, and the cell density was adjusted to 2 × 10⁵ cells/mL. The target cells with adjusted cell density were added into a 96-well plate, at 100 µL per well; the gradient diluted antibody was added into the 96-well plate, at 50 µL per well; then the effector cells PBMC were added into the 96-well plate, at 50 µL per well. The final effector-target ratio was 10:1. The above 96-well plate was placed in a 37°C, 5% CO₂ incubator and after 24 hours of culture, the supernatant was collected. The level of LDH released by dead cells into the supernatant was tested according to the LDH test kit (CytoTox96 non-radioactive cytotoxicity kit, Promega) instruction manual, then the proportion of MM cells killed by the example antibody was calculated.

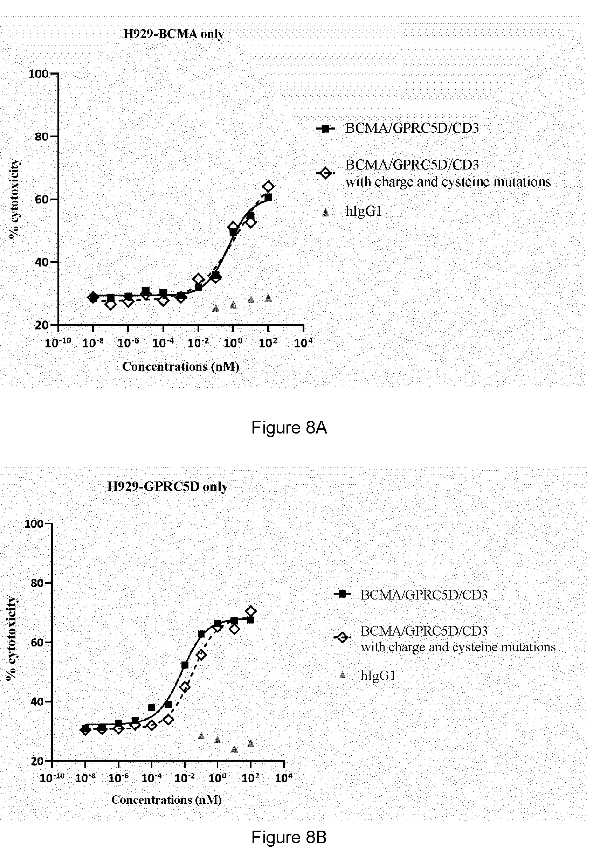

In the experiment carried out according to the above test method, the example antibody is capable of inducing the killing effect of human PBMC on H929 cells in a dose-dependent manner (see Figure 8).

In the experiment carried out according to the above test method, the killing effect of PBMC from the same donor on BCMA or GPRC5D-positive H929 cell line as induced by the example antibody was compared and whether mutations declared by the present patent were introduced had no effect on the killing effect of the example antibody. However, after introducing the mutations declared by the present patent, the example antibody is capable of being expressed and prepared in one cell line, saving the trouble of expression, preparation and in vitro recombination in two cell lines.

| In the Specifi cation | SEQ ID NO | Note | Sequence (Mutation Site Underlined) | Description |
|---|---|---|---|---|
| Table 1 | 1 | F126C | ASTKGPSVCPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSC | CH1 (CH1 of IgG1), contains a 126C mutation |
| | 2 | Q124C | RTVAAPSVFIFPPSDECLKSGTASVVCLLNNFYPREAKVQWKVDNALQSGNSQESVTEQ DSKDSTYSLSSTLTLSKADYEKHKVYACEVTHQGLSSPVTKSFNRGEC | CL (Kappa), contains a 124C mutation |
| Table 2: Combi nation 1 | 3 | Antibody 1, Heavy chain, Q39K mutation | EVQLVESGGGLVQPGGSLRLSCAASGFNIKDTYIHWVRKAPGKGLEWVARIYPTNGYTR YADSVKGRFTISADTSKNTAYLQMNSLRAEDTAVYYCSRWGGDGFYAMDYWGQGTLV TVSS | Trastuzumab TZB (anti-Her2)-VH, comprising Q39K |
| | 4 | Antibody 1, Light chain, Q38D mutation | DIQMTQSPSSLSASVGDRVTITCRASQDVNTAVAWYQDKPGKAPKLLIYSASFLYSGVPS RFSGSRSGTDFTLTISSLQPEDFATYYCQQHYTTPPTFGQGTKVEIK | Trastuzumab TZB-VL, comprising Q38D |
| | 5 | Antibody 2, Heavy chain, Q39D mutation | EVRLLESGGGLVQPGGSLRLSCAASGFTFSNYAMGWVRDAPGKGLEWVSAISGSGGST YYADSVKGRFTTSRDDSKNALYLQMNSLRAEDTAVYYCARGGPGWYAADVWGQGTT VTVSS | Anti-Hel antibody (anti-hen egg white lysozyme)-VH, comprising Q39D |
| | 6 | Antibody 2 light chain Q38K mutation | DIQMTQSPSSLSASVGDRVTITCRASQSISSYLNWYQKKPGKAPKLLIYAASSLQSGVPSR FSGSGSGTDFTLTISSLQPEDFATYYCQQADLPAFAFGGGTKVEIK | Anti-Hel antibody (anti-hen egg white lysozyme)-VL, comprising Q38K |
| Table 2: Combi nation 2 | 7 | Antibody 1, Heavy chain, L45K mutation | EVQLVESGGGLVQPGGSLRLSCAASGFNIKDTYIHWVRQAPGKGKEWVARIYPTNGYT RYADSVKGRFTISADTSKNTAYLQMNSLRAEDTAVYYCSRWGGDGFYAMDYWGQGTL VTVSS | Trastuzumab TZB (anti-Her2)-VH, comprising L45K |
| | 8 | Antibody 1, Light chain, Y87D mutation | DIQMTQSPSSLSASVGDRVTITCRASQDVNTAVAWYQQKPGKAPKLLIYSASFLYSGVPS RFSGSRSGTDFTLTISSLQPEDFATYDCQQHYTTPPTFGQGTKVEIK | Trastuzumab TZB-VL, comprising Y87D |
| | 9 | Antibody 2, Heavy chain, L45D mutation | EVRLLESGGGLVQPGGSLRLSCAASGFTFSNYAMGWVRQAPGKGDEWVSAISGSGGST YYADSVKGRFTTSRDDSKNALYLQMNSLRAEDTAVYYCARGGPGWYAADVWGQGTT VTVSS | Anti-Hel antibody (anti-hen egg white lysozyme)-VH, comprising L45D |
| | 10 | Antibody 2, Light chain, Y87K mutation | DIQMTQSPSSLSASVGDRVTITCRASQSISSYLNWYQQKPGKAPKLLIYAASSLQSGVPSR FSGSGSGTDFTLTISSLQPEDFATYKCQQADLPAFAFGGGTKVEIK | Anti-Hel antibody (anti-hen egg white lysozyme)-VL, comprising Y87K |
| Table 2: Combi | 7 | Antibody 1, Heavy chain, L45K mutation | SEQ ID NO:7 | Trastuzumab TZB (anti-Her2)-VH |
| nation 3 | 11 | Antibody 1, Light chain, P44D mutation | DIQMTQSPSSLSASVGDRVTITCRASQDVNTAVAWYQQKPGKADKLLIYSASFLYSGVPS RFSGSRSGTDFTLTISSLQPEDFATYYCQQHYTTPPTFGQGTKVEIK | Trastuzumab TZB-VL, comprising P44D |
| | 9 | Antibody 2, Heavy chain, L45D mutation | SEQ ID NO:9 | Anti-Hel antibody (anti-hen egg white lysozyme)-VH, comprising L45D |
| | 12 | Antibody 2, Light chain, P44K mutation | DIQMTQSPSSLSASVGDRVTITCRASQSISSYLNWYQQKPGKAKKLLIYAASSLQSGVPSR FSGSGSGTDFTLTISSLQPEDFATYYCQQADLPAFAFGGGTKVEIK | Anti-Hel antibody (anti-hen egg white lysozyme)-VL, comprising P44K |
| Table 2: Combi nation 4 | 13 | Antibody 1, Heavy chain, Y91K mutation | EVQLVESGGGLVQPGGSLRLSCAASGFNIKDTYIHWVRQAPGKGLEWVARIYPTNGYTR YADSVKGRFTISADTSKNTAYLQMNSLRAEDTAVYKCSRWGGDGFYAMDYWGQGTLV TVSS | Trastuzumab TZB (anti-Her2)-VH |
| | 14 | Antibody 1, Light chain, A43D mutation | DIQMTQSPSSLSASVGDRVTITCRASQDVNTAVAWYQQKPGKDPKLLIYSASFLYSGVPS RFSGSRSGTDFTLTISSLQPEDFATYYCQQHYTTPPTFGQGTKVEIK | Trastuzumab TZB-VL, comprising A43D |
| | 15 | Antibody 2, Heavy chain, Y91D mutation | EVRLLESGGGLVQPGGSLRLSCAASGFTFSNYAMGWVRQAPGKGLEWVSAISGSGGST YYADSVKGRFTTSRDDSKNALYLQMNSLRAEDTAVYDCARGGPGWYAADVWGQGTT VTVSS | Anti-Hel antibody (anti-hen egg white lysozyme)-VH, comprising Y91D |
| | 16 | Antibody 2, Light chain, A43K mutation | DIQMTQSPSSLSASVGDRVTITCRASQSISSYLNWYQQKPGKKPKLLIYAASSLQSGVPSR FSGSGSGTDFTLTISSLQPEDFATYYCQQADLPAFAFGGGTKVEIK | Anti-Hel antibody (anti-hen egg white lysozyme)-VL, comprising A43K |
| Table 2: Combi nation 5 | 13 | Antibody 1, Heavy chain, Y91K mutation | SEQ ID NO:13 | Trastuzumab TZB (anti-Her2)-VH, comprising Y91K |
| | 11 | Antibody 1, Light chain, P44D mutation | SEQ ID NO:11 | Trastuzumab TZB-VL |
| | 15 | Antibody 2, Heavy chain, Y91D mutation | SEQ ID NO:15 | Anti-Hel antibody (anti-hen egg white lysozyme)-VH |
| | 12 | Antibody 2, Light chain, P44K mutation | SEQ ID NO:12 | Anti-Hel antibody (anti-hen egg white lysozyme)-VL |
| Table 3: Combi | 21 | Antibody 1, Heavy chain, | EVQLVESGGGLVQPGGSLRLSCAASGFNIKDTYIHWVRKAPGKGLEWVARIYPTNGYTR YADSVKGRFTISADTSKNTAYLQMNSLRAEDTAVYYCSRWGGDGFYAMDYWGQGTLV TVSSASTKGPSVCPLAPSSKSTSGGTAALGCLVKDYFPEPVTVSWNSGALTSGVHTFPAV | Trastuzumab TZB (anti-Her2)-heavy chain |
| nation 1 | | Q39K_F126C_C2 20S_knob | LQSSGLYSLSSVVTVPSSSLGTQTYICNVNHKPSNTKVDKKVEPKSSDKTHTCPPCPAPEA AGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP CRDELTKNQVSLWCLVKGFYPSDIAVEWESNGQPENNYKTTPPVLDSDGSFFLYSKLTV DKSRWQQGNVFSCSVMHEALHNHYTQKSLSLSPGK | comprising Q39K, F126C, and C220S, and a Knob mutation |
| | 3 | Antibody 1, H VH | SEQ ID NO:3 | |
| | 22 | Antibody 1, Light chain, Q38D_Q124C_C 214S | DIQMTQSPSSLSASVGDRVTITCRASQDVNTAVAWYQDKPGKAPKLLIYSASFLYSGVPS RFSGSRSGTDFTLTISSLQPEDFATYYCQQHYTTPPTFGQGTKVEIKRTVAAPSVFIFPPSD ECLKSGTASVVCLLNNFYPREAKVQWKVDNALQSGNSQESVTEQDSKDSTYSLSSTLTLS KADYEKHKVYACEVTHQGLSSPVTKSFNRGES | Trastuzumab TZB-light chain, comprising Q38D, Q124C and Q214S mutations |
| | 4 | Antibody 1, Light chain, comprising Q38D | SEQ ID NO:4 | |
| | 23 | Antibody 2, Heavy chain, Q39D_hole | EVRLLESGGGLVQPGGSLRLSCAASGFTFSNYAMGWVRDAPGKGLEWVSAISGSGGST YYADSVKGRFTTSRDDSKNALYLQMNSLRAEDTAVYYCARGGPGWYAADVWGQGTT VTVSSASTKGPSVFPLAPSSKSTSGGTAALGCLVKDYFPEPVTVSWNSGALTSGVHTFPA | Anti-Hel antibody (anti-hen egg white lysozyme)-heavy |
| | | | VLQSSGLYSLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVCTL PPSRDELTKNQVSLSCAVKGFYPSDIAVEWESNGQPENNYKTTPPVLDSDGSFFLVSKLT VDKSRWQQGNVFSCSVMHEALHNHYTQKSLSLSPGK | chain, comprising Q39D and a Hole mutation |
| | 5 | Antibody 2, Heavy chain, Q39D_hole VH | SEQ ID NO:5 | Anti-Hel antibody (anti-hen egg white lysozyme)-VH, comprising Q39D |
| | 24 | Antibody 2, Light chain, Q38K | DIQMTQSPSSLSASVGDRVTITCRASQSISSYLNWYQKKPGKAPKLLIYAASSLQSGVPSR FSGSGSGTDFTLTISSLQPEDFATYYCQQADLPAFAFGGGTKVEIKRTVAAPSVFIFPPSDE QLKSGTASVVCLLNNFYPREAKVQWKVDNALQSGNSQESVTEQDSKDSTYSLSSTLTLSK ADYEKHKVYACEVTHQGLSSPVTKSFNRGEC | Anti-Hel antibody (anti-hen egg white lysozyme)-light chain, comprising Q38K mutation |
| | 6 | Antibody 2, Light chain, Q38K VL | SEQ ID NO:6 | Anti-Hel antibody (anti-hen egg white lysozyme)-VL, comprising Q38K |
| Table 3: Combi | 25 | Antibody 1, Heavy chain, | EVQLVESGGGLVQPGGSLRLSCAASGFNIKDTYIHWVRQAPGKGKEWVARIYPTNGYT RYADSVKGRFTISADTSKNTAYLQMNSLRAEDTAVYYCSRWGGDGFYAMDYWGQGTL VTVSSASTKGPSVCPLAPSSKSTSGGTAALGCLVKDYFPEPVTVSWNSGALTSGVHTFPA | Trastuzumab TZB (anti-Her2)-heavy chain, comprising L45K, |
| nation 2 | | L45K_F126C_C2 20S_knob | VLQSSGLYSLSSVVTVPSSSLGTQTYICNVNHKPSNTKVDKKVEPKSSDKTHTCPPCPAPE AAGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPCRDELTKNQVSLWCLVKGFYPSDIAVEWESNGQPENNYKTTPPVLDSDGSFFLYSKLT VDKSRWQQGNVFSCSVMHEALHNHYTQKSLSLSPGK | F126C, C220S mutations and a Knob mutation |
| | 7 | Antibody 1, H VH | SEQ ID NO:7 | |
| | 26 | Antibody 1, Light chain, Y87D_Q124C_C 214S | DIQMTQSPSSLSASVGDRVTITCRASQDVNTAVAWYQQKPGKAPKLLIYSASFLYSGVPS RFSGSRSGTDFTLTISSLQPEDFATYDCQQHYTTPPTFGQGTKVEIKRTVAAPSVFIFPPSD ECLKSGTASVVCLLNNFYPREAKVQWKVDNALQSGNSQESVTEQDSKDSTYSLSSTLTLS KADYEKHKVYACEVTHQGLSSPVTKSFNRGES | Trastuzumab TZB-light chain, comprising Y87D_Q124C_C214S |
| | 8 | Antibody 1, Light chain VL | SEQ ID NO:8 | |
| | 27 | Antibody 2, Heavy chain, L45D_hole | EVRLLESGGGLVQPGGSLRLSCAASGFTFSNYAMGWVRQAPGKGDEWVSAISGSGGST YYADSVKGRFTTSRDDSKNALYLQMNSLRAEDTAVYYCARGGPGWYAADVWGQGTT VTVSSASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHNAKTKP | Anti-Hel antibody (anti-hen egg white lysozyme)-heavy chain, comprising L45D and a Hole mutation |
| | | | REEEQYNSTYRVVSVLTVLHQDWLNGKEYKCKVSNKALPAPIEKTISKAKGQPREPQVCTL PPSRDELTKNQVSLSCAVKGFYPSDIAVEWESNGQPENNYKTTPPVLDSDGSFFLVSKLT VDKSRWQQGNVFSCSVMHEALHNHYTQKSLSLSPGK | |
| | 9 | | SEQ ID NO:9 | |
| | 28 | Antibody 2, Light chain, Y87K | DIQMTQSPSSLSASVGDRVTITCRASQSISSYLNWYQQKPGKAPKLLIYAASSLQSGVPSR FSGSGSGTDFTLTISSLQPEDFATYKCQQADLPAFAFGGGTKVEIKRTVAAPSVFIFPPSDE QLKSGTASVVCLLNNFYPREAKVQWKVDNALQSGNSQESVTEQDSKDSTYSLSSTLTLSK ADYEKHKVYACEVTHQGLSSPVTKSFNRGEC | Anti-Hel antibody (anti-hen egg white lysozyme)-light chain, comprising Y87K mutation |
| | 10 | Antibody 2, Light chain VL Y87K | SEQ ID NO:10 | |
| Table 3: Combi nation 3 | 25 | Antibody 1, Heavy chain, L45K_F126C_C2 20S_knob | SEQ ID NO:25 | Trastuzumab TZB (anti-Her2)-heavy chain, comprising L45K, F126C, C220S mutations and a Knob mutation |
| | 29 | Antibody 1, Light chain, | DIQMTQSPSSLSASVGDRVTITCRASQDVNTAVAWYQQKPGKADKLLIYSASFLYSGVPS RFSGSRSGTDFTLTISSLQPEDFATYYCQQHYTTPPTFGQGTKVEIKRTVAAPSVFIFPPSD | Trastuzumab TZB-light chain, comprising P44D_Q124C_C214S |
| | | P44D_Q124C_C 214S | ECLKSGTASVVCLLNNFYPREAKVQWKVDNALQSGNSQESVTEQDSKDSTYSLSSTLTLS KADYEKHKVYACEVTHQGLSSPVTKSFNRGES | |
| | 11 | | SEQ ID NO:11 | Trastuzumab TZB-VL, comprising P44D |
| | 27 | Antibody 2, Heavy chain, L45D_hole | SEQ ID NO:27 | Anti-Hel antibody (anti-hen egg white lysozyme)-heavy chain |
| | 9 | | SEQ ID NO:9 | Anti-Hel antibody (anti-hen egg white lysozyme)-VH, comprising L45D |
| | 30 | Antibody 2, Light chain, P44K | DIQMTQSPSSLSASVGDRVTITCRASQSISSYLNWYQQKPGKAKKLLIYAASSLQSGVPSR FSGSGSGTDFTLTISSLQPEDFATYYCQQADLPAFAFGGGTKVEIKRTVAAPSVFIFPPSDE QLKSGTASVVCLLNNFYPREAKVQWKVDNALQSGNSQESVTEQDSKDSTYSLSSTLTLSK ADYEKHKVYACEVTHQGLSSPVTKSFNRGEC | Anti-Hel antibody (anti-hen egg white lysozyme)-light chain, comprising P44K mutation |
| | 12 | | SEQ ID NO:12 | Anti-Hel antibody (anti-hen egg white lysozyme)-VL, comprising P44K |
| Table 3: Combi nation 4 | 31 | Antibody 1, Heavy chain, Y91K_F126C_C2 20S_knob | EVQLVESGGGLVQPGGSLRLSCAASGFNIKDTYIHWVRQAPGKGLEWVARIYPTNGYTR YADSVKGRFTISADTSKNTAYLQMNSLRAEDTAVYKCSRWGGDGFYAMDYWGQGTLV TVSSASTKGPSVCPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSSDKTHTCPPCPAPEA AGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP CRDELTKNQVSLWCLVKGFYPSDIAVEWESNGQPENNYKTTPPVLDSDGSFFLYSKLTV DKSRWQQGNVFSCSVMHEALHNHYTQKSLSLSPGK | Trastuzumab TZB (anti-Her2)-heavy chain, comprising Y91K_F126C_C220S and a Knob mutation |
| | | 13 | SEQ ID NO:13 | Trastuzumab TZB (anti-Her2)-VH, comprising Y91K |
| | 32 | Antibody 1, Light chain, A43D_Q124C_C 214S | DIQMTQSPSSLSASVGDRVTITCRASQDVNTAVAWYQQKPGKDPKLLIYSASFLYSGVPS RFSGSRSGTDFTLTISSLQPEDFATYYCQQHYTTPPTFGQGTKVEIKRTVAAPSVFIFPPSD ECLKSGTASVVCLLNNFYPREAKVQWKVDNALQSGNSQESVTEQDSKDSTYSLSSTLTLS KADYEKHKVYACEVTHQGLSSPVTKSFNRGES | Trastuzumab TZB-light chain, comprising A43D_Q124C_C214S |
| | 14 | | SEQ ID NO:14 | Trastuzumab TZB-VL, comprising A43D |
| | 33 | Antibody 2, Heavy chain, | EVRLLESGGGLVQPGGSLRLSCAASGFTFSNYAMGWVRQAPGKGLEWVSAISGSGGST YYADSVKGRFTTSRDDSKNALYLQMNSLRAEDTAVYDCARGGPGWYAADVWGQGTT VTVSSASTKGPSVFPLAPSSKSTSGGTAALGCLVKDYFPEPVTVSWNSGALTSGVHTFPA | Anti-Hel antibody (anti-hen egg white lysozyme)-heavy |
| | | Y91D_hole | VLQSSGLYSLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVCTL PPSRDELTKNQVSLSCAVKGFYPSDIAVEWESNGQPENNYKTTPPVLDSDGSFFLVSKLT VDKSRWQQGNVFSCSVMHEALHNHYTQKSLSLSPGK | chain, comprising Y91D and a Hole mutation |
| | | | SEQ ID NO:15 | |
| | 34 | Antibody 2, Light chain, A43K | DIQMTQSPSSLSASVGDRVTITCRASQSISSYLNWYQQKPGKKPKLLIYAASSLQSGVPSR FSGSGSGTDFTLTISSLQPEDFATYYCQQADLPAFAFGGGTKVEIKRTVAAPSVFIFPPSDE QLKSGTASVVCLLNNFYPREAKVQWKVDNALQSGNSQESVTEQDSKDSTYSLSSTLTLSK ADYEKHKVYACEVTHQGLSSPVTKSFNRGEC | Anti-Hel antibody (anti-hen egg white lysozyme)-light chain, comprising A43K mutation |
| | | | SEQ ID NO:16 | |
| Table 3: Combi nation 5 | 31 | Antibody 1, Heavy chain, Y91K_F126C_C2 20S_knob | SEQ ID NO:31 | Trastuzumab TZB (anti-Her2)-heavy chain, comprising Y91K_F126C_C220S and a Knob mutation |
| | 13 | | SEQ ID NO:13 | |
| | 29 | Antibody 1, Light chain, P44D_Q124C_C 214S | SEQ ID NO:29 | Trastuzumab TZB-light chain, comprising P44D, Q124C and C214S |
| | 33 | Antibody 2, Heavy chain, Y91D_hole | SEQ ID NO:33 | Anti-Hel antibody (anti-hen egg white lysozyme)-heavy chain, comprising Y91D and a Hole mutation |
| | 30 | Antibody 2, Light chain, P44K | SEQ ID NO:30 | Anti-Hel antibody (anti-hen egg white lysozyme)-light chain, comprising P44K mutation |
| | | | | |
| Table 4: Her2/ Hel_W T | 39 | TZB_HC_WT_kn ob | EVQLVESGGGLVQPGGSLRLSCAASGFNIKDTYIHWVRQAPGKGLEWVARIYPTNGYTR YADSVKGRFTISADTSKNTAYLQMNSLRAEDTAVYYCSRWGGDGFYAMDYWGQGTLV TVSSASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP | Trastuzumab TZB (anti-Her2)-heavy chain, comprising a Knob mutation |
| | | | CRDELTKNQVSLWCLVKGFYPSDIAVEWESNGQPENNYKTTPPVLDSDGSFFLYSKLTV DKSRWQQGNVFSCSVMHEALHNHYTQKSLSLSPGK | |
| | 75 | VH with TZB_HC_WT_kn ob | | Trastuzumab TZB (anti-Her2)-heavy chain VH |
| | 40 | TZB_LC_WT | DIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIKRTVAAPSVFIFPPSD EQLKSGTASVVCLLNNFYPREAKVQWKVDNALQSGNSQESVTEQDSKDSTYSLSSTLTLS KADYEKHKVYACEVTHQGLSSPVTKSFNRGEC | Trastuzumab TZB (anti-Her2)-light chain |
| | 76 | Variable region VL | | Trastuzumab TZB (anti-Her2)-light chain VL |
| | 41 | Hel_HC_hole | EVRLLESGGGLVQPGGSLRLSCAASGFTFSNYAMGWVRQAPGKGLEWVSAISGSGGST YYADSVKGRFTTSRDDSKNALYLQMNSLRAEDTAVYYCARGGPGWYAADVWGQGTT VTVSSASTKGPSVFPLAPSSKSTSGGTAALGCLVKDYFPEPVTVSWNSGALTSGVHTFPA VLQSSGLYSLSSVVTVPSSSLGTQTYICNVNHKPSNTKVDKKVEPKSCDKTHTCPPCPAPE AAGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVCTL PPSRDELTKNQVSLSCAVKGFYPSDIAVEWESNGQPENNYKTTPPVLDSDGSFFLVSKLT VDKSRWQQGNVFSCSVMHEALHNHYTQKSLSLSPGK | Anti-Hel antibody (anti-hen egg white lysozyme) heavy chain, comprising a Hole mutation |
| | | | | |
| | 77 | Hel_HC_hole VH | EVRLLESGGGLVQPGGSLRLSCAASGFTFSNYAMGWVRQAPGKGLEWVSAISGSGGST YYADSVKGRFTTSRDDSKNALYLQMNSLRAEDTAVYYCARGGPGWYAADVWGQGTT VTVSS | Anti-Hel antibody (anti-hen egg white lysozyme) heavy chain VH |
| | 42 | Hel_LC | DIQMTQSPSSLSASVGDRVTITCRASQSISSYLNWYQQKPGKAPKLLIYAASSLQSGVPSR FSGSGSGTDFTLTISSLQPEDFATYYCQQADLPAFAFGGGTKVEIKRTVAAPSVFIFPPSDE QLKSGTASVVCLLNNFYPREAKVQWKVDNALQSGNSQESVTEQDSKDSTYSLSSTLTLSK ADYEKHKVYACEVTHQGLSSPVTKSFNRGEC | Anti-Hel antibody (anti-hen egg white lysozyme) light chain |
| | 78 | Hel_LC VL | DIQMTQSPSSLSASVGDRVTITCRASQSISSYLNWYQQKPGKAPKLLIYAASSLQSGVPSR FSGSGSGTDFTLTISSLQPEDFATYYCQQADLPAFAFGGGTKVEIK | Anti-Hel antibody (anti-hen egg white lysozyme)-VL |
| Table 4: Her2/ Hel_12 6C_12 1C | 43 | TZB_HC_F126C_ knob | EVQLVESGGGLVQPGGSLRLSCAASGFNIKDTYIHWVRQAPGKGLEWVARIYPTNGYTR YADSVKGRFTISADTSKNTAYLQMNSLRAEDTAVYYCSRWGGDGFYAMDYWGQGTLV TVSSASTKGPSVCPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSCDKTHTCPPCPAPEA AGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP CRDELTKNQVSLWCLVKGFYPSDIAVEWESNGQPENNYKTTPPVLDSDGSFFLYSKLTV DKSRWQQGNVFSCSVMHEALHNHYTQKSLSLSPGK | Trastuzumab TZB (anti-Her2)-heavy chain, comprising F126C and a Knob mutation Those in italics are CH1 comprising F126C (SEQ ID NO:1) |
| | 75 | VH with TZB_HC_WT_kn ob | SEQ ID NO:75 | Trastuzumab TZB (anti-Her2)-heavy chain VH |
| | 44 | TZB_LC_S121C | DIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIKRTVAAPSVFIFPPCD EQLKSGTASVVCLLNNFYPREAKVQWKVDNALQSGNSQESVTEQDSKDSTYSLSSTLTLS KADYEKHKVYACEVTHQGLSSPVTKSFNRGEC | Trastuzumab TZB (anti-Her2)-light chain, comprising S121C |
| | 76 | Variable region VL | SEQ ID NO:76 | Trastuzumab TZB (anti-Her2)-light chain VL |
| | 41 | Hel_HC_hole | SEQ ID NO:41 | Anti-Hel antibody (anti-hen egg white lysozyme) heavy chain, comprising a Hole mutation |
| | 42 | Hel_LC | SEQ ID NO:42 | Anti-Hel antibody (anti-hen egg white lysozyme) light chain |
| Table 4: Her2/ | 43 | TZB_HC_F126C_ knob | SEQ ID NO:43 | Trastuzumab TZB (anti-Her2)-heavy chain, comprising F126C and a Knob mutation |
| Hel_12 6C_12 4C | 45 | TZB_LC_Q124C | DIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIKRTVAAPSVFIFPPSD ECLKSGTASVVCLLNNFYPREAKVQWKVDNALQSGNSQESVTEQDSKDSTYSLSSTLTLS KADYEKHKVYACEVTHQGLSSPVTKSFNRGEC | Trastuzumab TZB (anti-Her2)-light chain, comprising Q124C CL comprising Q124C is in italics (SEQ ID NO:2) |
| | 76 | Variable region VL | SEQ ID NO:76 | Trastuzumab TZB (anti-Her2)-light chain VL |
| | 41 | Hel_HC_hole | SEQ ID NO:41 | Anti-Hel antibody (anti-hen egg white lysozyme) heavy chain, comprising a Hole mutation |
| | 42 | Hel_LC | SEQ ID NO:42 | Anti-Hel antibody (anti-hen egg white lysozyme) light chain |
| Table 4: Her2/P | 39 | TZB_HC_WT_kn ob | SEQ ID NO:39 | Trastuzumab TZB (anti-Her2)-heavy chain, comprising a Knob mutation |
| | 40 | TZB_LC_WT | SEQ ID NO:40 | Trastuzumab TZB-light chain |
| D-1_WT | 46 | Sintilimab -VH-in IgG1AA-hole | QVQLVQSGAEVKKPGSSVKVSCKASGGTFSSYAISWVRQAPGQGLEWMGLIIPMFDTA GYAQKFQGRVAITVDESTSTAYMELSSLRSEDTAVYYCARAEHSSTGTFDYWGQGTLVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEAA GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFFLVSKLTVDK SRWQQGNVFSCSVMHEALHNHYTQKSLSLSPGK | Sintilimab (anti-PD-1) heavy chain, comprising an AA mutation and Hole mutation |
| | 79 | Sintilimab -VH | QVQLVQSGAEVKKPGSSVKVSCKASGGTFSSYAISWVRQAPGQGLEWMGLIIPMFDTA GYAQKFQGRVAITVDESTSTAYMELSSLRSEDTAVYYCARAEHSSTGTFDYWGQGTLVT VSS | Sintilimab (anti-PD-1) heavy chain VH |
| | 47 | Sintilimab -VL in pcDNA3.1-SP-IgKCL | DIQMTQSPSSVSASVGDRVTITCRASQGISSWLAWYQQKPGKAPKLLISAASSLQSGVPS RFSGSGSGTDFTLTISSLQPEDFATYYCQQANHLPFTFGGGTKVEIKRTVAAPSVFIFPPSD EQLKSGTASVVCLLNNFYPREAKVQWKVDNALQSGNSQESVTEQDSKDSTYSLSSTLTLS KADYEKHKVYACEVTHQGLSSPVTKSFNRGEC | Sintilimab (anti-PD-1) light chain, comprising a Kappa light chain constant region |
| | 80 | Sintilimab -VL | DIQMTQSPSSVSASVGDRVTITCRASQGISSWLAWYQQKPGKAPKLLISAASSLQSGVPS RFSGSGSGTDFTLTISSLQPEDFATYYCQQANHLPFTFGGGTKVEIK | Sintilimab (anti-PD-1) light chain VL |
| Table 4: Her2/P D-1_126 C_121 C | 43 | TZB_HC_F126C_ knob | SEQ ID NO:43 | Trastuzumab TZB (anti-Her2)-heavy chain, comprising F126C and a Knob mutation |
| | 44 | TZB_LC_S121C | SEQ ID NO:44 | Trastuzumab TZB (anti-Her2)-light chain, comprising S121C |
| | 46 | Sintilimab -VH-in IgG1AA-hole | SEQ ID NO:46 | Sintilimab (anti-PD-1) heavy chain, comprising an AA mutation and Hole mutation |
| | 47 | Sintilimab -VL in pcDNA3.1-SP-IgKCL | SEQ ID NO:47 | Sintilimab (anti-PD-1) light chain, comprising a Kappa light chain constant region |
| Table 4: Her2/P D-1_126 | 43 | TZB_HC_F126C_ knob | SEQ ID NO:43 | Trastuzumab TZB (anti-Her2)-heavy chain, comprising F126C and a Knob mutation |
| | 45 | TZB_LC_Q124C | SEQ ID NO:45 | Trastuzumab TZB (anti-Her2)-light chain, comprising Q124C |
| C_124 C | 46 | Sintilimab -VH-in IgG1AA-hole | SEQ ID NO:46 | Sintilimab (anti-PD-1) heavy chain, comprising an AA mutation and Hole mutation |
| | 47 | Sintilimab -VL in pcDNA3.1-SP-IgKCL | SEQ ID NO:47 | Sintilimab (anti-PD-1) light chain, comprising a Kappa light chain constant region |
| Table 7: Combi nation 1 | 43 | Antibody 1, Heavy chain, F126C_knob | SEQ ID NO:43 | |
| | 45 | Antibody 1, Light chain, Q124C | SEQ ID NO:45 | |
| | 41 | Antibody 2, Heavy chain, hole | SEQ ID NO:41 | |
| | 42 | Antibody 2, Light chain | SEQ ID NO:42 | |
| Table 7: Combi nation 2 | 48 | Antibody 1, Heavy chain, F126C_C220S_k nob | EVQLVESGGGLVQPGGSLRLSCAASGFNIKDTYIHWVRQAPGKGLEWVARIYPTNGYTR YADSVKGRFTISADTSKNTAYLQMNSLRAEDTAVYYCSRWGGDGFYAMDYWGQGTLV TVSSASTKGPSVCPLAPSSKSTSGGTAALGCLVKDYFPEPVTVSWNSGALTSGVHTFPAV LQSSGLYSLSSVVTVPSSSLGTQTYICNVNHKPSNTKVDKKVEPKSSDKTHTCPPCPAPEA AGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLPP CRDELTKNQVSLWCLVKGFYPSDIAVEWESNGQPENNYKTTPPVLDSDGSFFLYSKLTV DKSRWQQGNVFSCSVMHEALHNHYTQKSLSLSPGK | Trastuzumab TZB (anti-Her2)-heavy chain, comprising F126C and C220S |
| | 75 | VH with TZB_HC_WT_kn ob | SEQ ID NO:75 | Trastuzumab TZB (anti-Her2)-heavy chain VH |
| | 49 | Antibody 1, Light chain, Q124C_C214S | DIQMTQSPSSLSASVGDRVTITCRASQDVNTAVAWYQQKPGKAPKLLIYSASFLYSGVPS RFSGSRSGTDFTLTISSLQPEDFATYYCQQHYTTPPTFGQGTKVEIKRTVAAPSVFIFPPSD ECLKSGTASVVCLLNNFYPREAKVQWKVDNALQSGNSQESVTEQDSKDSTYSLSSTLTLS KADYEKHKVYACEVTHQGLSSPVTKSFNRGES | Trastuzumab TZB (anti-Her2)-light chain, comprising Q124C and C214S |
| | 76 | Variable region VL | SEQ ID NO:76 | Trastuzumab TZB (anti-Her2)-light chain VL |
| | 41 | Antibody 2 heavy chain hole | SEQ ID NO:41 | Anti-Hel antibody (anti-hen egg white lysozyme) heavy |
| | | | | chain, comprising a Hole mutation |
| | 42 | Antibody 2 light chain | SEQ ID NO:42 | Anti-Hel antibody (anti-hen egg white lysozyme) light chain |
| Table 7: Combi nation 3 | 21 | Antibody 1, Heavy chain, Q39K_F126C_C2 20S_knob | SEQ ID NO:21 | Trastuzumab TZB (anti-Her2)-heavy chain comprising Q39K, F126C, and C220S, and a Knob mutation |
| | 22 | Antibody 1 light chain Q38D_Q124C_C 214S | SEQ ID NO:22 | Trastuzumab TZB-light chain, comprising Q38D, Q124C and Q214S mutations |
| | 41 | Antibody 2 heavy chain hole | SEQ ID NO:41 | Anti-Hel antibody (anti-hen egg white lysozyme) heavy chain, comprising a Hole mutation |
| | 42 | Antibody 2 light chain | SEQ ID NO:42 | Anti-Hel antibody (anti-hen egg white lysozyme) light chain |
| Table 8: Her2/P D-1 | 21 | TZB_HC_Q39K_F 126C_C220S_kn ob | SEQ ID NO:21 | Trastuzumab TZB (anti-Her2)-heavy chain comprising Q39K, F126C, and C220S, and a Knob mutation |
| | 22 | TZB_LC_Q38D_ Q124C_C214S | SEQ ID NO:22 | Trastuzumab TZB-light chain, comprising Q38D, Q124C and Q214S mutations |
| | 46 | Sintilimab VH in IgG1AA-hole | SEQ ID NO:46 | Sintilimab H |
| | 47 | Sintilimab VL in pcDNA3.1-SP-IgKCL | SEQ ID NO:47 | Sintilimab light chain |
| Table 8: | 21 | TZB_HC_Q39K_F 126C_C220S_kn ob | SEQ ID NO:21 | Trastuzumab TZB (anti-Her2)-heavy chain |
| Her2/ CD47 | | | | comprising Q39K, F126C, and C220S, and a Knob mutation |
| | 22 | TZB_LC_Q38D_ Q124C_C214S | SEQ ID NO:22 | Trastuzumab TZB-light chain, comprising Q38D, Q124C and Q214S mutations |
| | 50 | CD47_HC_hole | QVQLQESGPGLVKPSETLSLTCTVSGGSISSYYWSWIRQPPGKGLEWIGYIYYSGSTNYN PSLKSRVTISVDTSKNQFSLKLSSVTAADTAVYYCARGKTGSAAWGQGTLVTVSSASTKG PSVFPLAPSSKSTSGGTAALGCLVKDYFPEPVTVSWNSGALTSGVHTFPAVLQSSGLYSLS SVVTVPSSSLGTQTYICNVNHKPSNTKVDKKVEPKSCDKTHTCPPCPAPEAAGGPSVFLF PPKPKDTLMISRTPEVTCVVVDVSHEDPEVKFNWYVDGVEVHNAKTKPREEQYNSTYR VVSVLTVLHQDWLNGKEYKCKVSNKALPAPIEKTISKAKGQPREPQVCTLPPSRDELTKN QVSLSCAVKGFYPSDIAVEWESNGQPENNYKTTPPVLDSDGSFFLVSKLTVDKSRWQQ GNVFSCSVMHEALHNHYTQKSLSLSPGK | ADI26630 H |
| | 81 | | QVQLQESGPGLVKPSETLSLTCTVSGGSISSYYWSWIRQPPGKGLEWIGYIYYSGSTNYN PSLKSRVTISVDTSKNQFSLKLSSVTAADTAVYYCARGKTGSAAWGQGTLVTVSS | ADI26630 heavy chain VH |
| | 51 | CD47_LC | DIQMTQSPSSVSASVGDRVTITCRASQGISRWLAWYQQKPGKAPKLLIYAASSLQSGVP SRFSGSGSGTDFTLTISSLQPEDFATYYCQQTVSFPITFGGGTKVEIKRTVAAPSVFIFPPSD EQLKSGTASVVCLLNNFYPREAKVQWKVDNALQSGNSQESVTEQDSKDSTYSLSSTLTLS KADYEKHKVYACEVTHQGLSSPVTKSFNRGEC | ADI26630 light chain |
| | 82 | | DIQMTQSPSSVSASVGDRVTITCRASQGISRWLAWYQQKPGKAPKLLIYAASSLQSGVP SRFSGSGSGTDFTLTISSLQPEDFATYYCQQTVSFPITFGGGTKVEIK | ADI26630 light chain VL |
| Table 8: Her2/c MET | 21 | TZB_HC_Q39K_F 126C_C220S_kn ob | SEQ ID NO:21 | Trastuzumab TZB (anti-Her2)-heavy chain comprising Q39K, F126C, and C220S, and a Knob mutation |
| | 22 | TZB_LC_Q38D_ Q124C_C214S | SEQ ID NO:22 | Trastuzumab TZB-light chain, comprising Q38D, Q124C and Q214S mutations |
| | 52 | cMet_HC_hole | EVQLVESGGGLVQPGGSLRLSCAASGYTFTSYWLHWVRQAPGKGLEWVGMIDPSNSD TRFNPNFKDRFTISADTSKNTAYLQMNSLRAEDTAVYYCATYRSYVTPLDYWGQGTLVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEAA GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVCTLPPS RDELTKNQVSLSCAVKGFYPSDIAVEWESNGQPENNYKTTPPVLDSDGSFFLVSKLTVDK SRWQQGNVFSCSVMHEALHNHYTQKSLSLSPGK | Onartuzumab H |
| | 18 | | | Onartuzumab heavy chain VH |
| | 53 | cMet_LC | DIQMTQSPSSLSASVGDRVTITCKSSQSLLYTSSQKNYLAWYQQKPGKAPKLLIYWASTR ESGVPSRFSGSGSGTDFTLTISSLQPEDFATYYCQQYYAYPWTFGQGTKVEIKRTVAAPSV FIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGEC | Onartuzumab light chain |
| | 17 | | | Onartuzumab light chain VL |
| | | | | |
| Table 10: WT | 39 | TZB_HC_WT_kn ob | SEQ ID NO:39 | Trastuzumab TZB-heavy chain |
| | 40 | TZB_LC_WT | SEQ ID NO:40 | Trastuzumab TZB-light chain |
| | 41 | Hel_HC_hole | SEQ ID NO:41 | Egg white lysozyme antibody Hel H |
| | 42 | Hel_LC | SEQ ID NO:42 | Anti-Hel antibody (anti-hen egg white lysozyme) light chain |
| Table 10: Combi nation 1-Combi nation 5 are exactly the same as Table 3 | | | | |
| Table 11: | 56 | 5E_LC | DIVMTQSPDSLAVSLGERATINCRASESVDSYGITFMNWYQQKPGQPPKLLIYAASNQG SGVPDRFSGSGSGTDFTLTISSLQAEDVAVYYCQQSKEVPWTFGQGTKLEIKRTVAAPSV | GPRC5D antibody light chain |
| 2+1N-termin al config uratio n: Only disulfi de bond reshap ing | | | FIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGEC | |
| | 83 | | DIVMTQSPDSLAVSLGERATINCRASESVDSYGITFMNWYQQKPGQPPKLLIYAASNQG SGVPDRFSGSGSGTDFTLTISSLQAEDVAVYYCQQSKEVPWTFGQGTKLEIK | GPRC5D antibody light chain, no mutations |
| | 84 | | RASESVDSYGITFMN | GPRC5D antibody light chain VL-CDR1 |
| | 85 | | AASNQGS | GPRC5D antibody light chain VL-CDR2 |
| | 86 | | QQSKEVPWT | GPRC5D antibody light chain VL-CDR3 |
| | 57 | 5E_HC_InnoA Innobody mutation (S364R+D399K) | QVQLVQSGAEVKKPGASVKVSCKASGYTFIGYVMHWVRQAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSREEMTKNQVRLTCLVKGFYPSDIAVEWESNGQPENNYKTTPPVLKSDGSFFLYSKLT VDKSRWQQGNVFSCSVMHEALHNHYTQKSLSLSPGK | GPRC5D antibody H |
| | 87 | | QVQLVQSGAEVKKPGASVKVSCKASGYTFIGYVMHWVRQAPGQGLEWMGYINPYND VSQHQGKFKGRVTMTSDTSTSTVYMELSSLRSEDTAVYYCAREGRLGLRVLAYWGQGT LVTVSS | GPRC5D antibody heavy chain VH, no mutations |
| | 88 | | GYVMH | GPRC5D antibody heavy chain VH-CDR1 |
| | 89 | | YINPYNDVSQHQGKFKG | GPRC5D antibody heavy chain VH-CDR2 |
| | 90 | | EGRLGLRVLAY | GPRC5D antibody heavy chain VH-CDR3 |
| | 58 | 5E_sp34.24_CS_ HC_InnoB(F126C +C220S+Y349T+ K370S+K409D) | QVQLVQSGAEVKKPGASVKVSCKASGYTFIGYVMHWVRQAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCGGGGSGGGGSE VQLVESGGGLVQPGGSLKLSCAASGFTFNTYAMNWVRQASGKGLEWVGRIRSKYNNY ATYYADSVKDRFTISRDDSKSTLYLQMNSLKTEDTAVYYCARHGNFGQSYVSWFAYWG QGTTVTVSSASTKGPSVCPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSSDKTHTCPPC PAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV | GPRC5D VH+sp34.24 VH+Fc |
| | | | TTLPPSREEMTKNQVSLTCLVSGFYPSDIAVEWESNGQPENNYKTTPPVLDSDGSFFLYS DLTVDKSRWQQGNVFSCSVMHEALHNHYTQKSLSLSPGK | |
| | 87 | | SEQ ID NO:87 | GPRC5D VH no mutations |
| | 91 | | EVQLVESGGGLVQPGGSLKLSCAASGFTFNTYAMNWVRQASGKGLEWVGRIRSKYNN YATYYADSVKDRFTISRDDSKSTLYLQMNSLKTEDTAVYYCARHGNFGQSYVSWFAYW GQGTTVTVSS | sp34.24 VH, no mutations |
| | 59 | SP34Vk_CS_LC (Q124C+C214S) | DIQMTQSPSSLSASVGDRVTITCRSSTGAVTTSNYANWVQQKPGKAPKGLIGGTNKRAP GVPSRFSGSLIGDKATLTISSLQPEDFATYYCALWYSNLWVFGQGTKVEIKRTVAAPSVFI FPPSDECLKSGTASVVCLLNNFYPREAKVQWKVDNALQSGNSQESVTEQDSKDSTYSLS STLTLSKADYEKHKVYACEVTHQGLSSPVTKSFNRGES | sp34.24 light chain |
| | 92 | | DIQMTQSPSSLSASVGDRVTITCRSSTGAVTTSNYANWVQQKPGKAPKGLIGGTNKRAP GVPSRFSGSLIGDKATLTISSLQPEDFATYYCALWYSNLWVFGQGTKVEIK | sp34.24 VL, no mutations |
| Table 11: 2+1N-termin | 60 | 5E_K_LC (Q38K) | DIVMTQSPDSLAVSLGERATINCRASESVDSYGITFMNWYQKKPGQPPKLLIYAASNQG SGVPDRFSGSGSGTDFTLTISSLQAEDVAVYYCQQSKEVPWTFGQGTKLEIKRTVAAPSV FIFPPSDEQLKSGTASVVCLLNNFYPREAKVQWKVDNALQSGNSQESVTEQDSKDSTYS LSSTLTLSKADYEKHKVYACEVTHQGLSSPVTKSFNRGEC | GPRC5D antibody light chain, comprising a Q38K mutation |
| al config uratio n: Disulfi de bond reshap ing and charge mutati ons | 93 | | DIVMTQSPDSLAVSLGERATINCRASESVDSYGITFMNWYQKKPGQPPKLLIYAASNQG SGVPDRFSGSGSGTDFTLTISSLQAEDVAVYYCQQSKEVPWTFGQGTKLEIK | GPRC5D antibody light chain-VL, comprising a Q38K mutation |
| | 61 | 5E_D_HC_InnoA | QVQLVQSGAEVKKPGASVKVSCKASGYTFIGYVMHWVRDAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSREEMTKNQVRLTCLVKGFYPSDIAVEWESNGQPENNYKTTPPVLKSDGSFFLYSKLT VDKSRWQQGNVFSCSVMHEALHNHYTQKSLSLSPGK | GPRC5D antibody heavy chain, comprising a Q39D |
| | | Innobody mutation (S364R+D399K) | | mutation |
| | 94 | | QVQLVQSGAEVKKPGASVKVSCKASGYTFIGYVMHWVRDAPGQGLEWMGYINPYND VSQHQGKFKGRVTMTSDTSTSTVYMELSSLRSEDTAVYYCAREGRLGLRVLAYWGQGT LVTVSS | GPRC5D antibody VH, comprising a Q39D mutation |
| | 62 | 5E_D_SP34.24_ KCS_HC_InnoB Inno mutation (GPRC5D:Q39D) | QVQLVQSGAEVKKPGASVKVSCKASGYTFIGYVMHWVRDAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCGGGGSGGGGSE VQLVESGGGLVQPGGSLKLSCAASGFTFNTYAMNWVRKASGKGLEWVGRIRSKYNNY | GPRC5D VH+sp34.24 VH+Fc, GPRC5D VH comprises a Q39D mutation sp34.24 VH comprises a Q39K mutation |
| 168 | | | | |
| | | (sp34.24: Q39K+F126C+C2 20S+Y349T+K37 0S+K409D) | ATYYADSVKDRFTISRDDSKSTLYLQMNSLKTEDTAVYYCARHGNFGQSYVSWFAYWG QGTTVTVSSASTKGPSVCPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSSDKTHTCPPC PAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQV TTLPPSREEMTKNQVSLTCLVSGFYPSDIAVEWESNGQPENNYKTTPPVLDSDGSFFLYS DLTVDKSRWQQGNVFSCSVMHEALHNHYTQKSLSLSPGK | |
| | 95 | | QVQLVQSGAEVKKPGASVKVSCKASGYTFIGYVMHWVRDAPGQGLEWMGYINPYND VSQHQGKFKGRVTMTSDTSTSTVYMELSSLRSEDTAVYYCAREGRLGLRVLAYWGQGT LVTVSS | GPRC5D antibody-VH, comprising a Q39D mutation |
| | 96 | | EVQLVESGGGLVQPGGSLKLSCAASGFTFNTYAMNWVRKASGKGLEWVGRIRSKYNN YATYYADSVKDRFTISRDDSKSTLYLQMNSLKTEDTAVYYCARHGNFGQSYVSWFAYW GQGTTVTVSS | sp34.24 VH, comprising a Q39K mutation |
| | 63 | SP34Vk_DCS_LC (Q38D+Q124C+C 2145) | DIQMTQSPSSLSASVGDRVTITCRSSTGAVTTSNYANWVQDKPGKAPKGLIGGTNKRAP GVPSRFSGSLIGDKATLTISSLQPEDFATYYCALWYSNLWVFGQGTKVEIKRTVAAPSVFI FPPSDECLKSGTASVVCLLNNFYPREAKVQWKVDNALQSGNSQESVTEQDSKDSTYSLS STLTLSKADYEKHKVYACEVTHQGLSSPVTKSFNRGES | sp34.24 light chain, wherein sp34.24 VL comprises a Q38D mutation |
| | 97 | | DIQMTQSPSSLSASVGDRVTITCRSSTGAVTTSNYANWVQDKPGKAPKGLIGGTNKRAP GVPSRFSGSLIGDKATLTISSLQPEDFATYYCALWYSNLWVFGQGTKVEIK 169 | sp34.24 VL, comprising a Q38D mutation |
| Table 11: 2+1C-termin al config uratio n: Only disulfi de bond reshap ing | 56 | 5E_LC | SEQ ID NO:56 | GPRC5D antibody light chain |
| | 57 | 5E_HC_InnoA Innobody mutation (S364R+D399K) | SEQ ID NO:57 | GPRC5D antibody H |
| | 64 | 5E_HC_InnoB_s p34.24_CS (Y349T+K370S+K 409D+126C+220 S) | QVQLVQSGAEVKKPGASVKVSCKASGYTFIGYVMHWVRQAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVTTL PPSREEMTKNQVSLTCLVSGFYPSDIAVEWESNGQPENNYKTTPPVLDSDGSFFLYSDLT VDKSRWQQGNVFSCSVMHEALHNHYTQKSLSLSPGKGGGGSGGGGSEVQLVESGGG LVQPGGSLKLSCAASGFTFNTYAMNWVRQASGKGLEWVGRIRSKYNNYATYYADSVKD RFTISRDDSKSTLYLQMNSLKTEDTAVYYCARHGNFGQSYVSWFAYWGQGTTVTVSSAS | GPRC5D VH+Fc+sp34.24 VH, no mutations in both VHs |
| | | | TKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS | |
| | 87 | | SEQ ID NO:87 | GPRC5D VH, no mutations |
| | 91 | | SEQ ID NO:91 | sp34.24 VH, no mutations |
| | 59 | SP34Vk_CS_LC( Q124C+C214S) | SEQ ID NO:59 | Modified sp34.24 light chain |
| Table 11: 2+1C-termin al config uratio n: Disulfi de bond reshap | 60 | 5E_K_LC(Q38K) | SEQ ID NO:60 | GPRC5D antibody light chain |
| | 61 | 5E_D_HC_InnoA Innobody mutation (Q39D+S364R+D 399K) | SEQ ID NO:61 | GPRC5D antibody H |
| | 65 | 5E_D_HC_InnoB _sp34.24_KCS Innobody mutation (Q39D+Y349T+K 370S+K409D)+( | QVQLVQSGAEVKKPGASVKVSCKASGYTFIGYVMHWVRDAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVTTL | GPRC5D VH+Fc+sp34.24 VH, GPRC5D VH comprises a Q39D mutation, sp34.24 VH comprises a Q39K mutation |
| ing and charge mutati ons | | Q39K+F126C+C2 20S) | PPSREEMTKNQVSLTCLVSGFYPSDIAVEWESNGQPENNYKTTPPVLDSDGSFFLYSDLT VDKSRWQQGNVFSCSVMHEALHNHYTQKSLSLSPGKGGGGSGGGGSEVQLVESGGG LVQPGGSLKLSCAASGFTFNTYAMNWVRKASGKGLEWVGRIRSKYNNYATYYADSVKD RFTISRDDSKSTLYLQMNSLKTEDTAVYYCARHGNFGQSYVSWFAYWGQGTTVTVSSAS TKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS | |
| | 95 | | SEQ ID NO:95 | GPRC5D antibody-VH, comprising a Q39D mutation |
| | 96 | | SEQ ID NO:96 | sp34.24 VH, comprising a Q39K mutation |
| | 63 | SP34Vk_DCS_LC (Q38D+Q124C+C 214S) | SEQ ID NO:63 | Modified sp34.24 light chain |
| Table 11: 2+2C-termin al config | 56 | 5E_LC | SEQ ID NO:56 | GPRC5D antibody light chain |
| | 66 | 5E_HC_sp34.24_ CS (F126C+C220S) | QVQLVQSGAEVKKPGASVKVSCKASGYTFIGYVMHWVRQAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAP | GPRC5D VH+Fc+sp34.24 VH, no mutations in GPRC5D VH and sp34.24 VH |
| uratio n: Only disulfi de bond reshap ing | | | ELLGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSREEMTKNQVSLTCLVKGFYPSDIAVEWESNGQPENNYKTTPPVLDSDGSFFLYSKLT VDKSRWQQGNVFSCSVMHEALHNHYTQKSLSLSPGKGGGGSGGGGSEVQLVESGGG LVQPGGSLKLSCAASGFTFNTYAMNWVRQASGKGLEWVGRIRSKYNNYATYYADSVKD RFTISRDDSKSTLYLQMNSLKTEDTAVYYCARHGNFGQSYVSWFAYWGQGTTVTVSSAS TKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS | |
| | 87 | | SEQ ID NO:87 | GPRC5D VH, no mutations |
| | 91 | | SEQ ID NO:91 | sp34.24 VH, no mutations |
| | 59 | SP34Vk_CS_LC | SEQ IDNO:59 | Modified sp34.24 light chain |
| Table 11: 2+2C-termin al config uratio n: | 60 | 5E_K_LC | SEQ ID NO:60 | GPRC5D antibody LC, wherein VL contains a Q38K mutation |
| | 67 | 5E_D_HC_sp34. 24_KCS (Q39D)+(Q39K+ F126C+C220S) | QVQLVQSGAEVKKPGASVKVSCKASGYTFIGYVMHWVRDAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAP ELLGGPSVFLFPPKPKDTLMISRTPEVTCVVVDVSHEDPEVKFNWYVDGVEVHNAKTKP | GPRC5D VH+Fc+sp34.24 VH, GPRC5D VH comprises a Q39D mutation, |
| Disulfi de bond reshap ing and charge mutati ons | | | REEQYNSTYRVVSVLTVLHQDWLNGKEYKCKVSNKALPAPIEKTISKAKGQPREPQVYTL PPSREEMTKNQVSLTCLVKGFYPSDIAVEWESNGQPENNYKTTPPVLDSDGSFFLYSKLT VDKSRWQQGNVFSCSVMHEALHNHYTQKSLSLSPGKGGGGSGGGGSEVQLVESGGG LVQPGGSLKLSCAASGFTFNTYAMNWVRKASGKGLEWVGRIRSKYNNYATYYADSVKD RFTISRDDSKSTLYLQMNSLKTEDTAVYYCARHGNFGQSYVSWFAYWGQGTTVTVSSAS TKGPSVCPLAPSSKSTSGGTAALGCLVKDYFPEPVTVSWNSGALTSGVHTFPAVLQSSGL YSLSSVVTVPSSSLGTQTYICNVNHKPSNTKVDKKVEPKSS | sp34.24 VH comprises a Q39K mutation |
| | 95 | | SEQ ID NO:95 | GPRC5D antibody H-VH, comprising a Q39D mutation |
| | 96 | | SEQ ID NO:96 | sp34.24VH, comprising a Q39K mutation |
| | 63 | SP34Vk_DCS_LC | SEQ ID NO:63 | Modified sp34.24 light chain |
| Figure 7A: BCMA /GPRC | 68 | GPRC5D_HC_kn ob (T366W) | QVQLVQSGAEVKKPGASVKVSCKASGYTFIGYVMHWVRQAPGQGLEWMGYINPYND VSQHQGKFKGRVTMTSDTSTSTVYMELSSLRSEDTAVYYCAREGRLGLRVLAYWGQGT LVTVSSASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSCDKTHTCPPCPAP EAAGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL | GPRC5D antibody H |
| 5D/CD 3 | | | PPSRDELTKNQVSLWCLVKGFYPSDIAVEWESNGQPENNYKTTPPVLDSDGSFFLYSKLT VDKSRWQQGNVFSCSVMHEALHNHYTQKSLSLSPGK | |
| | 87 | | SEQ ID NO:87 | GPRC5D VH, no mutations |
| | 56 | GPRC5D_LC | SEQ ID NO:56 | GPRC5D antibody light chain |
| | 69 | CD3_HC_hole_B CMAscFv (T366S+L368A+Y 407V)+(G100C+ G44C) | EVQLVESGGGLVQPGGSLKLSCAASGFTFNTYAMNWVRQASGKGLEWVGRIRSKYNN YATYYADSVKDRFTISRDDSKSTLYLQMNSLKTEDTAVYYCARHYNFGQSYVSWFAYWG QGTTVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCPPC PAPEAAGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQ VYTLPPSRDELTKNQVSLSCAVKGFYPSDIAVEWESNGQPENNYKTTPPVLDSDGSFFLV SKLTVDKSRWQQGNVFSCSVMHEALHNHYTQKSLSLSPGAGGGGSGGGGSDIQMTQ SPSSLSASVGDRVTITCRASQSISRYLNWYQQKPGKAPKLLIYAASSLQSGVPSRFSGSGS GTDFTLTISSLQPEDFATYYCQQKYFDITFGCGTKVEIKGGGGSGGGGSGGGGSQLQLQE SGPGLVKPSETLSLTCTVSGGSIVSSSYYWTWIRQPPGKCLEWIGSISIAGSTYYNPSLKSR VTISVDTSKNQFSLKLSSVTAADTAVYYCARDRGDQILDVWSQGTMVTVSS | sp34.87 VH + Fc + 38497 scFv |
| | 102 | | EVQLVESGGGLVQPGGSLKLSCAASGFTFNTYAMNWVRQASGKGLEWVGRIRSKYNNY ATYYADSVKDRFTISRDDSKSTLYLQMNSLKTEDTAVYYCARHYNFGQSYVSWFAYWGQ GTTVTVSS | sp34.87VH |
| | 103 | G44C | QLQLQESGPGLVKPSETLSLTCTVSGGSIVSSSYYWTWIRQPPGKCLEWIGSISIAGSTYYN PSLKSRVTISVDTSKNQFSLKLSSVTAADTAVYYCARDRGDQILDVWSQGTMVTVSS | BCMA-38497 VH, no mutations |
| | 104 | | SSSYYWT | BCMA-38497 VH-CDR1 |
| | 105 | | SISIAGSTYYNPSLKS | BCMA-38497 VH-CDR2 |
| | 106 | | DRGDQILDV | BCMA-38497 VH-CDR3 |
| | 107 | G100C | DIQMTQSPSSLSASVGDRVTITCRASQSISRYLNWYQQKPGKAPKLLIYAASSLQSGVPSR FSGSGSGTDFTLTISSLQPEDFATYYCQQKYFDITFGCGTKVEIK | BCMA-38497 VL |
| | 108 | | RASQSISRYLN | BCMA-38497 VL-CDR1 |
| | 109 | | AASSLQS | BCMA-38497 VL-CDR2 |
| | 110 | | QQKYFDIT | BCMA-38497 VL-CDR3 |
| | 111 | (G100C+G44C) | DIQMTQSPSSLSASVGDRVTITCRASQSISRYLNWYQQKPGKAPKLLIYAASSLQSGVPSR FSGSGSGTDFTLTISSLQPEDFATYYCQQKYFDITFGCGTKVEIKGGGGSGGGGSGGGGS QLQLQESGPGLVKPSETLSLTCTVSGGSIVSSSYYWTWIRQPPGKCLEWIGSISIAGSTYY NPSLKSRVTISVDTSKNQFSLKLSSVTAADTAVYYCARDRGDQILDVWSQGTMVTVSS | BCMA-38497VL-VH scFv |
| | 70 | CD3_LC | QAVVTQEPSLTVSPGGTVTLTCRSSTGAVTTSNYANWVQQKPGQAPRGLIGGTNKRAP GVPARFSGSLLGDKAALTLLGAQPEDEAEYYCALWYSNLWVFGQGTKLTVLGQPKAAPS VTLFPPSSEELQANKATLVCLISDFYPGAVTVAWKADSSPVKAGVETTTPSKQSNNKYAA SSYLSLTPEQWKSHRSYSCQVTHEGSTVEKTVAPTECS | sp34.87 light chain |
| | 112 | | QAVVTQEPSLTVSPGGTVTLTCRSSTGAVTTSNYANWVQQKPGQAPRGLIGGTNKRAP GVPARFSGSLLGDKAALTLLGAQPEDEAEYYCALWYSNLWVFGQGTKLTVL | sp34.87 light chain variable region, no mutations |
| Figure 7B: BCMA /GPRC 5D/CD 3 with Charge and cystein e mutati on | 71 | GPRC5D_KCS_H C_knob (Q39K+F126C+C 220S+T366W) | QVQLVQSGAEVKKPGASVKVSCKASGYTFIGYVMHWVRKAPGQGLEWMGYINPYND VSQHQGKFKGRVTMTSDTSTSTVYMELSSLRSEDTAVYYCAREGRLGLRVLAYWGQGT LVTVSSASTKGPSVCPLAPSSKSTSGGTAALGCLVKDYFPEPVTVSWNSGALTSGVHTFP AVLQSSGLYSLSSVVTVPSSSLGTQTYICNVNHKPSNTKVDKKVEPKSSDKTHTCPPCPAP EAAGGPSVFLFPPKPKDTLMISRTPEVTCVVVDVSHEDPEVKFNWYVDGVEVHNAKTKP REEQYNSTYRVVSVLTVLHQDWLNGKEYKCKVSNKALPAPIEKTISKAKGQPREPQVYTL PPSRDELTKNQVSLWCLVKGFYPSDIAVEWESNGQPENNYKTTPPVLDSDGSFFLYSKLT VDKSRWQQGNVFSCSVMHEALHNHYTQKSLSLSPGK | GPRC5D antibody heavy chain, comprising Q39K, F126C and C220S mutations, and a Knob mutation |
| | 113 | Q39K | QVQLVQSGAEVKKPGASVKVSCKASGYTFIGYVMHWVRKAPGQGLEWMGYINPYND VSQHQGKFKGRVTMTSDTSTSTVYMELSSLRSEDTAVYYCAREGRLGLRVLAYWGQGT LVTVSS | GPRC5D antibody heavy chain VH, comprising a Q39K mutation |
| | 72 | GPRC5D_DCS_L C Q39K+F126C+C2 20S | DIVMTQSPDSLAVSLGERATINCRASESVDSYGITFMNWYQDKPGQPPKLLIYAASNQG SGVPDRFSGSGSGTDFTLTISSLQAEDVAVYYCQQSKEVPWTFGQGTKLEIKRTVAAPSV FIFPPSDECLKSGTASVVCLLNNFYPREAKVQWKVDNALQSGNSQESVTEQDSKDSTYSL SSTLTLSKADYEKHKVYACEVTHQGLSSPVTKSFNRGES | GPRC5D antibody light chain, comprising Q38D, Q124C and C214S mutations |
| | 19 | Q38D | DIVMTQSPDSLAVSLGERATINCRASESVDSYGITFMNWYQDKPGQPPKLLIYAASNQG SGVPDRFSGSGSGTDFTLTISSLQAEDVAVYYCQQSKEVPWTFGQGTKLEIK | GPRC5D antibody light chain, comprising a Q38D mutation |
| | 73 | CD3_D_HC_hole _BCMAscFv (Q39D+T366S+L 368A+Y407V)+( G100C+G44C) | EVQLVESGGGLVQPGGSLKLSCAASGFTFNTYAMNWVRDASGKGLEWVGRIRSKYNN YATYYADSVKDRFTISRDDSKSTLYLQMNSLKTEDTAVYYCARHYNFGQSYVSWFAYWG QGTTVTVSSASTKGPSVFPLAPSSKSTSGGTAALGCLVKDYFPEPVTVSWNSGALTSGVH TFPAVLQSSGLYSLSSVVTVPSSSLGTQTYICNVNHKPSNTKVDKKVEPKSCDKTHTCPPC PAPEAAGGPSVFLFPPKPKDTLMISRTPEVTCVVVDVSHEDPEVKFNWYVDGVEVHNA KTKPREEQYNSTYRVVSVLTVLHQDWLNGKEYKCKVSNKALPAPIEKTISKAKGQPREPQ VYTLPPSRDELTKNQVSLSCAVKGFYPSDIAVEWESNGQPENNYKTTPPVLDSDGSFFLV SKLTVDKSRWQQGNVFSCSVMHEALHNHYTQKSLSLSPGAGGGGSGGGGSDIQMTQ SPSSLSASVGDRVTITCRASQSISRYLNWYQQKPGKAPKLLIYAASSLQSGVPSRFSGSGS GTDFTLTISSLQPEDFATYYCQQKYFDITFGCGTKVEIKGGGGSGGGGSGGGGSQLQLQE SGPGLVKPSETLSLTCTVSGGSIVSSSYYWTWIRQPPGKCLEWIGSISIAGSTYYNPSLKSR VTISVDTSKNQFSLKLSSVTAADTAVYYCARDRGDQILDVWSQGTMVTVSS | sp34.87 VH + Fc + 38497 scFv |
| | | | | 38497 is a BCMA antibody |
| | 103 | | SEQ ID NO:103 | BCMA-38497 VH, no mutations |
| | 107 | | SEQ ID NO:107 | BCMA-38497 VL |
| | 111 | G100C+G44C | SEQ ID NO:111 | BCMA-38497VL-VH scFv |
| | 114 | Q39D | EVQLVESGGGLVQPGGSLKLSCAASGFTFNTYAMNWVRDASGKGLEWVGRIRSKYNN YATYYADSVKDRFTISRDDSKSTLYLQMNSLKTEDTAVYYCARHYNFGQSYVSWFAYWG QGTTVTVSS | sp34.87VH, comprising a Q39D mutation |
| | 74 | CD3_K_LC Q39K | QAVVTQEPSLTVSPGGTVTLTCRSSTGAVTTSNYANWVQKKPGQAPRGLIGGTNKRAP GVPARFSGSLLGDKAALTLLGAQPEDEAEYYCALWYSNLWVFGQGTKLTVLGQPKAAPS VTLFPPSSEELQANKATLVCLISDFYPGAVTVAWKADSSPVKAGVETTTPSKQSNNKYAA SSYLSLTPEQWKSHRSYSCQVTHEGSTVEKTVAPTECS | sp34.87 light chain, comprising a Q38K mutation |
| | 115 | Q39K | QAVVTQEPSLTVSPGGTVTLTCRSSTGAVTTSNYANWVQKKPGQAPRGLIGGTNKRAP GVPARFSGSLLGDKAALTLLGAQPEDEAEYYCALWYSNLWVFGQGTKLTVL | sp34.87 light chain, comprising a Q38K mutation |
| | 116 | Wild-type IgG1 CH1 domain | ASTKGPSVFPLAPSSKSTSGGTAALGCLVKDYFPEPVTVSWNSGALTSGVHTFPAVLQSSG LYSLSSVVTVPSSSLGTQTYICNVNHKPSNTKVDKKV EPKSC | |
| | 117 | Wild-type IgG1 CH2 domain | APELLGGPSVFLFPPKPKDTLMISRTPEVTCVVVDVSHEDPEVKFNWYVDGVEVHNAKT KPREEQYNSTYRVVSVLTVLHQDWLNGKEYKCKVSNKALPAPIEKTISKAK | |
| | 118 | Wild-type IgG1 CH3 domain | GQPREPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK | |
| | 119 | | DKTHTCPXCP, wherein X is S or P | |
| | 120 | | HTCPXCP, wherein X is S or P | |
| | 121 | | CPXCP, wherein X is S or P | |
| | 122 | Human IgG1 Fc region polypeptide | DKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAK GQPREPQVYTLPPSRDELTKNQVSLTCLVKGFYPSDIAVEWESNGQPENNYKTTPPVLDS DGSFFLYSKLTVDKSRWQQGNVFSCSVMHEALHNHYTQKSLSLSPGK | |
| | 123 | Human IgG4 Fc region polypeptide | PPCPSCPAPEFLGGPSVFLFPPKPKDTLMISRTPEVTCVVVDVSQEDPEVQFNWYVDGVE VHNAKTKPREEQFNSTYRVVSVLTVLHQDWLNGKEYKCKVSNKGLPSSIEKTISKAKGQP REPQVYTLPPSQEEMTKNQVSLTCLVKGFYPSDIAVEWESNGQPENNYKTTPPVLDSDG SFFLYSRLTVDKSRWQEGNVFSCSVMHEAvLHNHYTQKSLSLSLGK | |
| | 124 | Human IgG1 Fc region, comprising Y349C, T366S, L368A, and Y407V | DKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKA KGQPREPQVCTLPPSRDELTKNQVSLSCAVKGFYPSDIAVEWESNGQPENNYKTTPPVL DSDGSFFLVSKLTVDKSRWQQGNVFSCSVMHEALHNHYTQKSLSLSPGK | |
| | 125 | Human IgG1 Fc region, comprising S354C and T366W | DKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKA KGQPREPQVYTLPPCRDELTKNQVSLWCLVKGFYPSDIAVEWESNGQPENNYKTTPPVL DSDGSFFLYSKLTVDKSRWQQGNVFSCSVMHEALHNHYTQKSLSLSPGK | |
| | 126 | Human IgG1 Fc region, comprising S364R and D399K mutations | DKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAK GQPREPQVYTLPPSREEMTKNQVRLTCLVKGFYPSDIAVEWESNGQPENNYKTTPPVLK SDGSFFLYSKLTVDKSRWQQGNVFSCSVMHEALHNHYTQKSLSLSPGK | |
| | 127 | Human IgG1 Fc region, comprising Y349T, K370S and K409D mutations | DKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAK GQPREPQVTTLPPSREEMTKNQVSLTCLVSGFYPSDIAVEWESNGQPENNYKTTPPVLD SDGSFFLYSDLTVDKSRWQQGNVFSCSVMHEALHNHYTQKSLSLSPGK | |
| | 128 | Human Kappa light chain CL region (comprising Q124C and C214S) | RTVAAPSVFIFPPSDECLKSGTASVVCLLNNFYPREAKVQWKVDNALQSGNSQESVTEQ DSKDSTYSLSSTLTLSKADYEKHKVYACEVTHQGLSSPVTKSFNRGES | |
| | 54 | Human Kappa light chain CL region | | |
| | 55 | Human Lambda light chain CL region | GQPKAAPSVTLFPPSSEELQANKATLVCLISDFYPGAVTVAWKADSSPVKAGVETTTPSK QSNNKYAASSYLSLTPEQWKSHRSYSCQVTHEGSTVEKTVAPTECS | |
| | 129 | Human IgG1 CH1 (comprising F126C and C220S) | ASTKGPSVCPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSS | |
| | 130 | Human IgG1 Fc region, comprising T366S, L368A, and Y407V and a LALA mutation | DKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKA KGQPREPQVYTLPPSRDELTKNQVSLSCAVKGFYPSDIAVEWESNGQPENNYKTTPPVL DSDGSFFLVSKLTVDKSRWQQGNVFSCSVMHEALHNHYTQKSLSLSPGK | |
| | 131 | Human IgG1 Fc region, | DKTHTCPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKA | |
| | | comprising T366W and a LALA mutation | KGQPREPQVYTLPPSRDELTKNQVSLWCLVKGFYPSDIAVEWESNGQPENNYKTTPPVL DSDGSFFLYSKLTVDKSRWQQGNVFSCSVMHEALHNHYTQKSLSLSPGK | |
| | 132 | Human IgG1 CH1 (comprising H168C) | ASTKGPSVFPLAPSSKSTSGGTAALGCLVKDYFPEPVTVSWNSGALTSGVCTFPAVLQSS GLYSLSSVVTVPSSSLGTQTYICNVNHKPSNTKVDKKVEPKSC | |
| | 133 | Human IgG1 CH1 (comprising H168C+C220S) | ASTKGPSVFPLAPSSKSTSGGTAALGCLVKDYFPEPVTVSWNSGALTSGVCTFPAVLQSS GLYSLSSVVTVPSSSLGTQTYICNVNHKPSNTKVDKKVEPKSS | |
| | 134 | Human IgG1 CH1 (comprising F170C) | ASTKGPSVFPLAPSSKSTSGGTAALGCLVKDYFPEPVTVSWNSGALTSGVHTCPAVLQSS GLYSLSSVVTVPSSSLGTQTYICNVNHKPSNTKVDKKVEPKSC | |
| | 135 | Human IgG1 CH1 | ASTKGPSVFPLAPSSKSTSGGTAALGCLVKDYFPEPVTVSWNSGALTSGVHTCPAVLQSS GLYSLSSVVTVPSSSLGTQTYICNVNHKPSNTKVDKKVEPKSS | |
| | | (comprising F170C+C220S) | | |
| | 136 | Human IgG1 CH1 (comprising V173C) | ASTKGPSVFPLAPSSKSTSGGTAALGCLVKDYFPEPVTVSWNSGALTSGVHTFPACLQSS GLYSLSSVVTVPSSSLGTQTYICNVNHKPSNTKVDKKVEPKSC | |
| | 137 | Human IgG1 CH1 (comprising V173C+C220S) | ASTKGPSVFPLAPSSKSTSGGTAALGCLVKDYFPEPVTVSWNSGALTSGVHTFPACLQSS GLYSLSSVVTVPSSSLGTQTYICNVNHKPSNTKVDKKVEPKSS | |
| | 138 | Human IgG1 CH1 (comprising L128C) | ASTKGPSVFPCAPSSKSTSGGTAALGCLVKDYFPEPVTVSWNSGALTSGVHTFPAVLQSS GLYSLSSVVTVPSSSLGTQTYICNVNHKPSNTKVDKKVEPKSC | |
| | 139 | Human IgG1 CH1 (comprising L128C+C220S) | ASTKGPSVFPCAPSSKSTSGGTAALGCLVKDYFPEPVTVSWNSGALTSGVHTFPAVLQSS GLYSLSSVVTVPSSSLGTQTYICNVNHKPSNTKVDKKVEPKSS | |
| | 140 | Human IgG1 CH1 (comprising S134C) | ASTKGPSVFPLAPSSKCTSGGTAALGCLVKDYFPEPVTVSWNSGALTSGVHTFPAVLQSS GLYSLSSVVTVPSSSLGTQTYICNVNHKPSNTKVDKKVEPKSC | |
| | 141 | Human IgG1 CH1 (comprising S134C+C220S) | ASTKGPSVFPLAPSSKCTSGGTAALGCLVKDYFPEPVTVSWNSGALTSGVHTFPAVLQSS GLYSLSSVVTVPSSSLGTQTYICNVNHKPSNTKVDKKVEPKSS | |
| | 142 | Human IgG1 CH1 (comprising S136C) | ASTKGPSVFPLAPSSKSTCGGTAALGCLVKDYFPEPVTVSWNSGALTSGVHTFPAVLQSS GLYSLSSVVTVPSSSLGTQTYICNVNHKPSNTKVDKKVEPKSC | |
| | 143 | Human IgG1 CH1 (comprising S136C+C220S) | ASTKGPSVFPLAPSSKSTCGGTAALGCLVKDYFPEPVTVSWNSGALTSGVHTFPAVLQSS GLYSLSSVVTVPSSSLGTQTYICNVNHKPSNTKVDKKVEPKSS | |
| | 144 | Human IgG1 CH1 | ASTKGPSVFPLAPSSKSTSGGTAALGCLVKDYFPEPVTVSWNSGALTSGVHTFCAVLQSS GLYSLSSVVTVPSSSLGTQTYICNVNHKPSNTKVDKKVEPKSC | |
| | | (comprising P171C) | | |
| | 145 | Human IgG1 CH1 (comprising P171C+C220S) | ASTKGPSVFPLAPSSKSTSGGTAALGCLVKDYFPEPVTVSWNSGALTSGVHTFCAVLQSS GLYSLSSVVTVPSSSLGTQTYICNVNHKPSNTKVDKKVEPKSS | |
| | 146 | Human IgG1 CH1 (comprising T139C) | ASTKGPSVFPLAPSSKSTSGGCAALGCLVKDYFPEPVTVSWNSGALTSGVHTFPAVLQSS GLYSLSSVVTVPSSSLGTQTYICNVNHKPSNTKVDKKVEPKSC | |
| | 147 | Human IgG1 CH1 (comprising T139C+C220S) | ASTKGPSVFPLAPSSKSTSGGCAALGCLVKDYFPEPVTVSWNSGALTSGVHTFPAVLQSS GLYSLSSVVTVPSSSLGTQTYICNVNHKPSNTKVDKKVEPKSS | |
| | 148 | Human Kappa light chain CL region | RTVAAPSVFIFPPSDEQLKSGTASVVCLLNNFYPREAKVQWKVDNALQSGNSQESVCEQ DSKDSTYSLSSTLTLSKADYEKHKVYACEVTHQGLSSPVTKSFNRGEC | |
| | | (comprising T164C) | | |
| | 149 | Human Kappa light chain CL region (comprising T164C and C214S) | RTVAAPSVFIFPPSDEQLKSGTASVVCLLNNFYPREAKVQWKVDNALQSGNSQESVCEQ DSKDSTYSLSSTLTLSKADYEKHKVYACEVTHQGLSSPVTKSFNRGES | |
| | 150 | Human Kappa light chain CL region (comprising S162C) | RTVAAPSVFIFPPSDEQLKSGTASVVCLLNNFYPREAKVQWKVDNALQSGNSQECVTEQ DSKDSTYSLSSTLTLSKADYEKHKVYACEVTHQGLSSPVTKSFNRGEC | |
| | 151 | Human Kappa light chain CL region (comprising S162C and C214S) | RTVAAPSVFIFPPSDEQLKSGTASVVCLLNNFYPREAKVQWKVDNALQSGNSQECVTEQ DSKDSTYSLSSTLTLSKADYEKHKVYACEVTHQGLSSPVTKSFNRGES | |
| | 152 | Human Kappa light chain CL region (comprising Q160C) | RTVAAPSVFIFPPSDEQLKSGTASVVCLLNNFYPREAKVQWKVDNALQSGNSCESVTEQ DSKDSTYSLSSTLTLSKADYEKHKVYACEVTHQGLSSPVTKSFNRGEC | |
| | 35 | Human Kappa light chain CL region (comprising Q160C and C214S) | RTVAAPSVFIFPPSDEQLKSGTASVVCLLNNFYPREAKVQWKVDNALQSGNSCESVTEQ DSKDSTYSLSSTLTLSKADYEKHKVYACEVTHQGLSSPVTKSFNRGES | |
| | 36 | Human Kappa light chain CL region (comprising F118C) | RTVAAPSVFICPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC | |
| | 37 | Human Kappa light chain CL region | RTVAAPSVFICPPSDEQLKSGTASVVCLLNNFYPREAKVQWKVDNALQSGNSQESVTEQ DSKDSTYSLSSTLTLSKADYEKHKVYACEVTHQGLSSPVTKSFNRGES | |
| | | (comprising F118C and C214S) | | |
| | 38 | Human Kappa light chain CL region (comprising F116C) | RTVAAPSVCIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC | |
| | 98 | Human Kappa light chain CL region (comprising F116C and C214S) | RTVAAPSVCIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGES | |
| | 99 | Human Kappa light chain CL region (comprising S114C) | RTVAAPCVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGEC | |
| | 100 | Human Kappa light chain CL region (comprising S114C and C214S) | RTVAAPCVFIFPPSDEQLKSGTASVVCLLNNFYPREAKVQWKVDNALQSGNSQESVTEQ DSKDSTYSLSSTLTLSKADYEKHKVYACEVTHQGLSSPVTKSFNRGES | |
| | 101 | Human IgG1 Fc region, comprising T366S, L368A and Y407V | DKTHTCPPCPAPELLGGPSVFLFPPKPKDTLMISRTPEVTCVVVDVSHEDPEVKFNWYVD GVEVHNAKTKPREEQYNSTYRVVSVLTVLHQDWLNGKEYKCKVSNKALPAPIEKTISKAK GQPREPQVYTLPPSRDELTKNQVSLSCAVKGFYPSDIAVEWESNGQPENNYKTTPPVLD SDGSFFLVSKLTVDKSRWQQGNVFSCSVMHEALHNHYTQKSLSLSPGK | |
| | 20 | Human IgG1 Fc region, comprising T366W | | |

## Claims

1. An antibody or antigen-binding fragment thereof, comprising one or a plurality of first antigen-binding regions that specifically bind to a first antigen and one or a plurality of second antigen-binding regions that specifically bind to a second antigen, wherein at least one or a plurality of the antigen-binding regions comprise a heavy chain CH1 and light chain CL, and comprises a disulfide bond reshaping mutation at the CH1-CL interface, and/or at least one or a plurality of the antigen-binding regions contains a heavy chain variable region and a light chain variable region wherein an amino acid pair at the heavy chain variable region and light chain variable region interface comprise a charge mutation that mutates the two amino acids on the amino acid pair into amino acids with opposite charges, respectively; wherein the first antigen and second antigen may be the same or different;
preferably, only one antigen-binding region comprises the disulfide bond reshaping mutation at the CH1-CL interface.

2. The antibody or antigen-binding fragment thereof according to Claim 1, wherein the disulfide bond reshaping mutation comprises
(x) substituting a non-Cys amino acid at position 126 of the heavy chain CH1 to a Cys amino acid (EU number), and substituting a non-Cys amino acid at position 124 of the light chain CL to a Cys amino acid (EU number);
(xi) substituting a non-Cys amino acid at position 168 of the heavy chain CH1 to a Cys amino acid (EU number), and substituting a non-Cys amino acid at position 164 of the light chain CL to a Cys amino acid (EU number);
(xii) substituting a non-Cys amino acid at position 170 of the heavy chain CH1 to a Cys amino acid (EU number), and substituting a non-Cys amino acid at position 162 or 164 of the light chain CL to a Cys amino acid (EU number);
(xiii) substituting a non-Cys amino acid at position 173 of the heavy chain CH1 to a Cys amino acid (EU number), and substituting a non-Cys amino acid at position 160 or 162 of the light chain CL to a Cys amino acid (EU number);
(xiv) substituting a non-Cys amino acid at position 128 of the heavy chain CH1 to a Cys amino acid (EU number), and substituting a non-Cys amino acid at position 118 of the light chain CL to a Cys amino acid (EU number);
(xv) substituting a non-Cys amino acid at position 134 of the heavy chain CH1 to a Cys amino acid (EU number), and substituting a non-Cys amino acid at position 116 of the light chain CL to a Cys amino acid (EU number);
(xvi) substituting a non-Cys amino acid at position 136 of the heavy chain CH1 to a Cys amino acid (EU number), and substituting a non-Cys amino acid at position 114 of the light chain CL to a Cys amino acid (EU number);
(xvii) substituting a non-Cys amino acid at position 171 of the heavy chain CH1 to a Cys amino acid (EU number), and substituting a non-Cys amino acid at position 162 of the light chain CL to a Cys amino acid (EU number);
(xviii) substituting a non-Cys amino acid at position 139 of the heavy chain CH1 to a Cys amino acid (EU number), and substituting a non-Cys amino acid at position 116 of the light chain CL to a Cys amino acid (EU number).

3. The antibody or antigen-binding fragment thereof according to Claim 2, wherein the disulfide bond reshaping mutation comprises
(xii) F126C in CH1 and Q124C in CL (EU number);
(xiii) H168C in CH1 and T164C in CL (EU number);
(xiv) F170C in CH1 and T164C in CL (EU number);
(xv) F170C in CH1 and S162C in CL (EU number);
(xvi) V173C in CH1 and S162C in CL (EU number);
(xvii) V173C in CH1 and Q160C in CL (EU number);
(xviii) L128C in CH1 and F118C in CL (EU number);
(xix) S134C in CH1 and F116C in CL (EU number);
(xx) S136C in CH1 and S114C in CL (EU number);
(xxi) P171C in CH1 and S162C in CL (EU number);
(xxii) T139C in CH1 and F116C in CL (EU number).

4. The antibody or antigen-binding fragment thereof, comprising one or a plurality of first antigen-binding regions that specifically bind to a first antigen and one or a plurality of second antigen-binding regions that specifically bind to a second antigen, wherein at least one of the antigen-binding regions comprise a heavy chain CH1 and light chain CL, and comprises a disulfide bond reshaping mutation at the CH1-CL interface, wherein the disulfide bond reshaping mutation comprises T139C in CH1 and F116C in CL, wherein the first antigen-binding region and the second antigen-binding region may the same or different.

5. The antibody or antigen-binding fragment thereof according to any one of Claims 2 - 4, wherein the disulfide bond reshaping mutation also comprises substituting an interchain Cys at the heavy chain constant region-light chain constant region interface with a non-Cys.

6. The antibody or antigen-binding fragment thereof according to Claim 5, wherein substituting an interchain Cys with a non-Cys comprises substituting the Cys at position 220 in the CH1 with a non-Cys, and substituting the Cys at position 214 of the CL with a non-Cys.

7. The antibody or antigen-binding fragment thereof according to Claim 6, wherein substituting an interchain Cys with a non-Cys comprises C220S/A/V in CH1 and C214S/A/V in CL (EU number).

8. The antibody or antigen-binding fragment thereof according to Claim 6, wherein the disulfide bond reshaping mutation comprises
(xii) F126C and C220S in CH1 and Q124C and C214S in CL (EU number);
(xiii) H168C and C220S in CH1 and T164C and C214S in CL (EU number);
(xiv) F170C and C220S in CH1 and T164C and C214S in CL (EU number);
(xv) F170C and C220S in CH1 and S162C and C214S in CL (EU number);
(xvi) V173C and C220S in CH1 and S162C and C214S in CL (EU number);
(xvii) V173C and C220S in CH1 and Q160C and C214S in CL (EU number);
(xviii) L128C and C220S in CH1 and F118C and C214S in CL (EU number);
(xix) S134C and C220S in CH1 and F116C and C214S in CL (EU number);
(xx) S136C and C220S in CH1 and S114C and C214S in CL (EU number);
(xxi) P171C and C220S in CH1 and S162C and C214S in CL (EU number);
(xxii) T139C and C220S in CH1 and F116C and C214S in CL (EU number).

9. The antibody or antigen-binding fragment thereof according to any one of Claims 1 - 8, wherein the CH1 region is a human IgG1 CH1, for example, human IgG1 CH1, human IgG2 CH1, human IgG3 CH1, or human IgG4 CH1, for example, the CH1 region comprises the amino acid sequence shown in SEQ ID NO:116;
and/or the CL region is a human Kappa light chain CL region or a human Lambda light chain CL region, for example, the CL region comprises the amino acid sequence of SEQ ID NO:54 or SEQ ID NO:55.

10. The antibody or antigen-binding fragment thereof according to Claim 8 or 9, wherein CH1
(i) comprises an amino acid sequence with at least 90%, 91%, 92%, 93%, 94%, 95%, 96%, 97%, 98%, or 99% identity with the amino acid sequence shown in SEQ ID NO:129, and comprises F126C and C220S; or comprises or is composed of the amino acid sequence shown in SEQ ID NO:129;
(ii) comprises an amino acid sequence with at least 90%, 91%, 92%, 93%, 94%, 95%, 96%, 97%, 98%, or 99% identity with the amino acid sequence shown in SEQ ID NO:133, and comprises H168C and C220S; or comprises or is composed of the amino acid sequence shown in SEQ ID NO:133;
(iii) comprises an amino acid sequence with at least 90%, 91%, 92%, 93%, 94%, 95%, 96%, 97%, 98%, or 99% identity with the amino acid sequence shown in SEQ ID NO:135, and comprises F170C and C220S; or comprises or is composed of the amino acid sequence shown in SEQ ID NO:135;
(iv) comprises an amino acid sequence with at least 90%, 91%, 92%, 93%, 94%, 95%, 96%, 97%, 98%, or 99% identity with the amino acid sequence shown in SEQ ID NO:137, and comprises V173C and C220S; or comprises or is composed of the amino acid sequence shown in SEQ ID NO:137;
(v) comprises an amino acid sequence with at least 90%, 91%, 92%, 93%, 94%, 95%, 96%, 97%, 98%, or 99% identity with the amino acid sequence shown in SEQ ID NO:139, and comprises L128C and C220S; or comprises or is composed of the amino acid sequence shown in SEQ ID NO:139;
(vi) comprises an amino acid sequence with at least 90%, 91%, 92%, 93%, 94%, 95%, 96%, 97%, 98%, or 99% identity with the amino acid sequence shown in SEQ ID NO:141, and comprises S134C and C220S; or comprises or is composed of the amino acid sequence shown in SEQ ID NO:141;
(vii) comprises an amino acid sequence with at least 90%, 91%, 92%, 93%, 94%, 95%, 96%, 97%, 98%, or 99% identity with the amino acid sequence shown in SEQ ID NO:143, and comprises S136C and C220S; or comprises or is composed of the amino acid sequence shown in SEQ ID NO:143;
(viii) comprises an amino acid sequence with at least 90%, 91%, 92%, 93%, 94%, 95%, 96%, 97%, 98%, or 99% identity with the amino acid sequence shown in SEQ ID NO:145, and comprises P171C and C220S; or comprises or is composed of the amino acid sequence shown in SEQ ID NO:145;
(ix) comprises an amino acid sequence with at least 90%, 91%, 92%, 93%, 94%, 95%, 96%, 97%, 98%, or 99% identity with the amino acid sequence shown in SEQ ID NO:147, and comprises T139C and C220S; or comprises or is composed of the amino acid sequence shown in SEQ ID NO:147.

11. The antibody or antigen-binding fragment thereof according to any one of Claims 8 - 10, wherein CL
(i) comprises an amino acid sequence with at least 90%, 91%, 92%, 93%, 94%, 95%, 96%, 97%, 98%, or 99% identity with the amino acid sequence shown in SEQ ID NO:128, and comprises Q124C and C1245; or comprises or is composed of the amino acid sequence shown in SEQ ID NO:128;
(ii) comprises an amino acid sequence with at least 90%, 91%, 92%, 93%, 94%, 95%, 96%, 97%, 98%, or 99% identity with the amino acid sequence shown in SEQ ID NO:149, and comprises T164C and C1245; or comprises or is composed of the amino acid sequence shown in SEQ ID NO:149;
(iii) comprises an amino acid sequence with at least 90%, 91%, 92%, 93%, 94%, 95%, 96%, 97%, 98%, or 99% identity with the amino acid sequence shown in SEQ ID NO:151, and comprises S162C and C1245; or comprises or is composed of the amino acid sequence shown in SEQ ID NO:151;
(iv) comprises an amino acid sequence with at least 90%, 91%, 92%, 93%, 94%, 95%, 96%, 97%, 98%, or 99% identity with the amino acid sequence shown in SEQ ID NO:35, and comprises Q160C and C1245; or comprises or is composed of the amino acid sequence shown in SEQ ID NO:35;
(v) comprises an amino acid sequence with at least 90%, 91%, 92%, 93%, 94%, 95%, 96%, 97%, 98%, or 99% identity with the amino acid sequence shown in SEQ ID NO:37, and comprises F118C and C124S; or comprises or is composed of the amino acid sequence shown in SEQ ID NO:37;
(vii) comprises an amino acid sequence with at least 90%, 91%, 92%, 93%, 94%, 95%, 96%, 97%, 98%, or 99% identity with the amino acid sequence shown in SEQ ID NO:98, and comprises F116C and C124S; or comprises or is composed of the amino acid sequence shown in SEQ ID NO:98; or
(viii) comprises an amino acid sequence with at least 90%, 91%, 92%, 93%, 94%, 95%, 96%, 97%, 98%, or 99% identity with the amino acid sequence shown in SEQ ID NO:100, and comprises S114C and C124S; or comprises or is composed of the amino acid sequence shown in SEQ ID NO:100.

12. The antibody or antigen-binding fragment thereof according to any one of Claims 1 - 11, wherein the position of the amino acid pair at the heavy chain variable region and light chain variable region interface is selected from: position 39 of the heavy chain variable region and position 38 of the light chain variable region, position 45 of the heavy chain variable region and position 87 of the light chain variable region, position 45 of the heavy chain variable region and position 44 of the light chain variable region, position 91 of the heavy chain variable region and position 43 of the light chain variable region, or position 91 of the heavy chain variable region and position 44 of the light chain variable region (Kabat number).

13. The antibody or antigen-binding fragment thereof according to any one of Claims 1- 12, wherein the position of the amino acid pair at the heavy chain variable region and light chain variable region interface in at least one first antigen-binding region is the same as the position in at least one second antigen-binding region.

14. The antibody or antigen-binding fragment thereof according to Claim 13, wherein the mutated amino acid at the position of the amino acid pair in the first antigen-binding region has the opposite charge to the mutated amino acid at the same position in the second antigen-binding region.

15. The antibody or antigen-binding fragment thereof according to Claim 14, wherein
(1) in the first antigen-binding region, the amino acid at position 39 of the heavy chain variable region is substituted with an amino acid carrying a positive charge, and the amino acid at position 38 of the light chain variable region is substituted with an amino acid carrying a negative charge; and in the second antigen-binding region, the amino acid at position 39 of the heavy chain variable region is substituted with an amino acid carrying a negative charge, and the amino acid at position 38 of the light chain variable region is substituted with an amino acid carrying a positive charge;
(2) in the first antigen-binding region, the amino acid at position 45 of the heavy chain variable region is substituted with an amino acid carrying a positive charge, and the amino acid at position 87 of the light chain variable region is substituted with an amino acid carrying a negative charge; and in the second antigen-binding region, the amino acid at position 45 of the heavy chain variable region is substituted with an amino acid carrying a negative charge, and the amino acid at position 87 of the light chain variable region is substituted with an amino acid carrying a positive charge;
(3) in the first antigen-binding region, the amino acid at position 45 of the heavy chain variable region is substituted with an amino acid carrying a positive charge, and the amino acid at position 44 of the light chain variable region is substituted with an amino acid carrying a negative charge; and in the second antigen-binding region, the amino acid at position 45 of the heavy chain variable region is substituted with an amino acid carrying a negative charge, and the amino acid at position 44 of the light chain variable region is substituted with an amino acid carrying a positive charge;
(4) in the first antigen-binding region, the amino acid at position 91 of the heavy chain variable region is substituted with an amino acid carrying a positive charge, and the amino acid at position 43 of the light chain variable region is substituted with an amino acid carrying a negative charge; and in the second antigen-binding region, the amino acid at position 91 of the heavy chain variable region is substituted with an amino acid carrying a negative charge, and the amino acid at position 43 of the light chain variable region is substituted with an amino acid carrying a positive charge; or
(5) in the first antigen-binding region, the amino acid at position 91 of the heavy chain variable region is substituted with an amino acid carrying a positive charge, and the amino acid at position 44 of the light chain variable region is substituted with an amino acid carrying a negative charge; and in the second antigen-binding region, the amino acid at position 91 of the heavy chain variable region is substituted with an amino acid carrying a negative charge, and the amino acid at position 44 of the light chain variable region is substituted with an amino acid carrying a positive charge.

16. The antibody or antigen-binding fragment thereof according to Claim 15, wherein the amino acid carrying a positive charge is selected from K, R, or H, and/or the amino acid carrying a negative charge is selected from E or D.

17. The antibody or antigen-binding fragment thereof according to Claim 16, wherein
(vi). in the first antigen-binding region, the amino acid at position 39 of the heavy chain variable region is substituted with K, and the amino acid at position 38 of the light chain variable region is substituted with D, while in the second antigen-binding region, the amino acid at position 39 of the heavy chain variable region is substituted with D, and the amino acid at position 38 of the light chain variable region is substituted with K;
(vii). in the first antigen-binding region, the amino acid at position 45 of the heavy chain variable region is substituted with K, and the amino acid at position 87 of the light chain variable region is substituted with D, while in the second antigen-binding region, the amino acid at position 45 of the heavy chain variable region is substituted with D, and the amino acid at position 87 of the light chain variable region is substituted with K;
(viii). in the first antigen-binding region, the amino acid at position 45 of the heavy chain variable region is substituted with K, and the amino acid at position 44 of the light chain variable region is substituted with D, while in the second antigen-binding region, the amino acid at position 45 of the heavy chain variable region is substituted with D, and the amino acid at position 44 of the light chain variable region is substituted with K;
(ix). in the first antigen-binding region, the amino acid at position 91 of the heavy chain variable region is substituted with K, and the amino acid at position 43 of the light chain variable region is substituted with D, while in the second antigen-binding region, the amino acid at position 91 of the heavy chain variable region is substituted with D, and the amino acid at position 43 of the light chain variable region is substituted with K;
(x). in the first antigen-binding region, the amino acid at position 91 of the heavy chain variable region is substituted with K, and the amino acid at position 44 of the light chain variable region is substituted with D, while in the second antigen-binding region, the amino acid at position 91 of the heavy chain variable region is substituted with D, and the amino acid at position 44 of the light chain variable region is substituted with K.

18. The antibody or antigen-binding fragment thereof according to any one of Claims 1- 17, wherein the multispecific antibody comprises a charge mutation and a disulfide bond reshaping mutation.

19. The antibody or antigen-binding fragment thereof according to any one of Claims 1- 18, wherein one or a plurality or all the antigen-binding regions that bind to the same antigen comprise the same mutation.

20. The antibody or antigen-binding fragment thereof according to any one of Claims 1- 19, wherein the first antigen-binding region comprises a heavy chain variable region, a heavy chain constant region CH1, a light chain variable region and a light chain constant region CL, and the second antigen-binding region comprises a heavy chain variable region VH and a light chain variable region VL.

21. The antibody or antigen-binding fragment thereof according to Claim 20, wherein
1) the first antigen-binding region comprises the disulfide bond reshaping mutation defined in any one of Claims 1 - 17; or
2) the first antigen-binding region comprises the charge mutation defined in any one of Claims 1 - 17;
3) the first antigen-binding region comprises the disulfide bond reshaping mutation and charge mutation defined in any one of Claims 1 - 17; or
4) the first antigen-binding region comprises the disulfide bond reshaping mutation and charge mutation defined in any one of Claims 1 - 17; and the second antigen-binding region comprises the charge mutation defined in any one of Claims 1 - 17.

22. The antibody or antigen-binding fragment thereof according to Claim 21, wherein
1) the first antigen-binding region comprises the disulfide bond reshaping mutation defined in any one of Claims 2 - 8;
2) the first antigen-binding region comprises the charge mutation defined in any one of Claims 12 - 17;
3) the first antigen-binding region comprises the disulfide bond reshaping mutation defined in any one of Claims 2 - 8 and comprises the charge mutation defined in any one of Claims 12 - 17; or
4) the first antigen-binding region and the second antigen-binding region comprise the charge mutation defined in any one of Claims 12 - 17, and the first antigen-binding region also comprises the disulfide bond reshaping mutation defined in any one of Claims 2 - 8.

23. The antibody or antigen-binding fragment thereof according to Claim 22, wherein the first antigen-binding region of the multispecific antibody comprises mutation combinations selected from the following:
| | | | | |
|---|---|---|---|---|
| | Heavy Chain VH Mutation | Heavy Chain CH1 Mutation | Light Chain VL Mutation | Light Chain CL Mutation |
| Combination 1 | Q39K | F126C, C220S | Q38D | Q124C, C214S |
| | Q39K | H168C, C220S | Q38D | T164C, C214S |
| | Q39K | F170C, C220S | Q38D | T164C, C214S |
| | Q39K | F170C, C220S | Q38D | S162C, C214S |
| | Q39K | V173C, C220S | Q38D | S162C, C214S |
| | Q39K | V173C, C220S | Q38D | Q160C, C214S |
| | Q39K | L128C, C220S | Q38D | F118C, C214S |
| | Q39K | S134C, C220S | Q38D | F116C, C214S |
| | Q39K | S136C, C2205 | Q38D | S114C, C214S |
| | Q39K | P171C, C220S | Q38D | S162C, C214S |
| | Q39K | T139C, C220S | Q38D | F116C, C214S |
| | | | | |
| Combination 2 | L45K | F126C, C220S | Y87D | Q124C, C214S |
| | L45K | H168C, C220S | Y87D | T164C, C214S |
| | L45K | F170C, C220S | Y87D | T164C, C214S |
| | L45K | F170C, C220S | Y87D | S162C, C214S |
| | L45K | V173C, C220S | Y87D | S162C, C214S |
| | L45K | V173C, C220S | Y87D | Q160C, C214S |
| | L45K | L128C, C220S | Y87D | F118C, C214S |
| | L45K | S134C, C220S | Y87D | F116C, C214S |
| | L45K | S136C, C2205 | Y87D | S114C, C214S |
| | L45K | P171C, C220S | Y87D | S162C, C214S |
| | L45K | T139C, C220S | Y87D | F116C, C214S |
| | | | | |
| Combination 3 | L45K | F126C, C220S | P44D | Q124C, C214S |
| | L45K | H168C, C220S | P44D | T164C, C214S |
| | L45K | F170C, C220S | P44D | T164C, C214S |
| | L45K | F170C, C220S | P44D | S162C, C214S |
| | L45K | V173C, C220S | P44D | S162C, C214S |
| | L45K | V173C, C220S | P44D | Q160C, C214S |
| | L45K | L128C, C220S | P44D | F118C, C214S |
| | L45K | S134C, C2205 | P44D | F116C, C214S |
| | L45K | S136C, C2205 | P44D | S114C, C214S |
| | L45K | P171C, C220S | P44D | S162C, C214S |
| | L45K | T139C, C220S | P44D | F116C, C214S |
| | | | | |
| Combination 4 | Y91K | F126C, C220S | A43D | Q124C, C214S |
| | Y91K | H168C, C220S | A43D | T164C, C214S |
| | Y91K | F170C, C220S | A43D | T164C, C214S |
| | Y91K | F170C, C220S | A43D | S162C, C214S |
| | Y91K | V173C, C220S | A43D | S162C, C214S |
| | Y91K | V173C, C220S | A43D | Q160C, C214S |
| | Y91K | L128C, C220S | A43D | F118C, C214S |
| | Y91K | S134C, C220S | A43D | F116C, C214S |
| | Y91K | S136C, C2205 | A43D | S114C, C214S |
| | Y91K | P171C, C220S | A43D | S162C, C214S |
| | Y91K | T139C, C220S | A43D | F116C, C214S |
| | | | | |
| Combination 5 | Y91K | F126C, C220S | P44D | Q124C, C214S |
| | Y91K | H168C, C220S | P44D | T164C, C214S |
| | Y91K | F170C, C220S | P44D | T164C, C214S |
| | Y91K | F170C, C220S | P44D | S162C, C214S |
| | Y91K | V173C, C220S | P44D | S162C, C214S |
| | Y91K | V173C, C220S | P44D | Q160C, C214S |
| | Y91K | L128C, C2205 | P44D | F118C, C214S |
| | Y91K | S134C, C2205 | P44D | F116C, C214S |
| | Y91K | S136C, C2205 | P44D | S114C, C214S |
| | Y91K | P171C, C220S | P44D | S162C, C214S |
| | Y91K | T139C, C220S | P44D | F116C, C214S |
optionally, the second antigen-binding region also comprises mutation combinations selected from the following:
| | Heavy Chain VH Mutation | Light Chain VL Mutation |
|---|---|---|
| Combination 1 | Q39D | Q38K |
| Combination 2 | L45D | Y87K |
| Combination 3 | L45D | P44K |
| Combination 4 | Y91D | A43K |
| Combination 5 | Y91D | P44K |
∘

24. The antibody or antigen-binding fragment thereof according to Claim 23, wherein the first antigen-binding region and the second antigen-binding region of the multispecific antibody comprise the following mutation combinations, respectively:
| | First Antigen-Binding Region | | | | Second Antigen-Binding Region | |
|---|---|---|---|---|---|---|
| | Heavy Chain VH Mutation | Heavy Chain CH1 Mutation | Light Chain VL Mutation | Light Chain CL Mutation | Heavy Chain VH Mutation | Light Chain VL Mutation |
| Combi | Q39K | F126C, | Q38D | Q124C, | Q39D | Q38K |
| nation 1 | | C2205 | | C2145 | | |
| | Q39K | H168C, C2205 | Q38D | T164C, C2145 | Q39D | Q38K |
| | Q39K | F170C, C2205 | Q38D | T164C, C2145 | Q39D | Q38K |
| | Q39K | F170C, C2205 | Q38D | S162C, C2145 | Q39D | Q38K |
| | Q39K | V173C, C2205 | Q38D | S162C, C2145 | Q39D | Q38K |
| | Q39K | V173C, C2205 | Q38D | Q160C, C2145 | Q39D | Q38K |
| | Q39K | L128C, C2205 | Q38D | F118C, C2145 | Q39D | Q38K |
| | Q39K | S134C, C2205 | Q38D | F116C, C2145 | Q39D | Q38K |
| | Q39K | S136C, C2205 | Q38D | S114C, C2145 | Q39D | Q38K |
| | Q39K | P171C, C2205 | Q38D | S162C, C2145 | Q39D | Q38K |
| | Q39K | T139C, C2205 | Q38D | F116C, C2145 | Q39D | Q38K |
| | | | | | | |
| Combi nation 2 | L45K | F126C, C2205 | Y87D | Q124C, C2145 | L45D | Y87K |
| | L45K | H168C, C2205 | Y87D | T164C, C2145 | L45D | Y87K |
| | L45K | F170C, | Y87D | T164C, | L45D | Y87K |
| | | C2205 | | C214S | | |
| | L45K | F170C, C2205 | Y87D | S162C, C2145 | L45D | Y87K |
| | L45K | V173C, C2205 | Y87D | S162C, C2145 | L45D | Y87K |
| | L45K | V173C, C2205 | Y87D | Q160C, C2145 | L45D | Y87K |
| | L45K | L128C, C2205 | Y87D | F118C, C2145 | L45D | Y87K |
| | L45K | S134C, C2205 | Y87D | F116C, C2145 | L45D | Y87K |
| | L45K | S136C, C2205 | Y87D | S114C, C2145 | L45D | Y87K |
| | L45K | P171C, C2205 | Y87D | S162C, C2145 | L45D | Y87K |
| | L45K | T139C, C2205 | Y87D | F116C, C2145 | L45D | Y87K |
| | | | | | | |
| Combi nation 3 | L45K | F126C, C2205 | P44D | Q124C, C2145 | L45D | P44K |
| | L45K | H168C, C2205 | P44D | T164C, C2145 | L45D | P44K |
| | L45K | F170C, C2205 | P44D | T164C, C2145 | L45D | P44K |
| | L45K | F170C, C2205 | P44D | S162C, C2145 | L45D | P44K |
| | L45K | V173C, C2205 | P44D | S162C, C2145 | L45D | P44K |
| | L45K | V173C, C2205 | P44D | Q160C, C2145 | L45D | P44K |
| | L45K | L128C, C2205 | P44D | F118C, C2145 | L45D | P44K |
| | L45K | S134C, C2205 | P44D | F116C, C2145 | L45D | P44K |
| | L45K | S136C, C2205 | P44D | S114C, C2145 | L45D | P44K |
| | L45K | P171C, C2205 | P44D | S162C, C2145 | L45D | P44K |
| | L45K | T139C, C2205 | P44D | F116C, C2145 | L45D | P44K |
| | | | | | | |
| Combi nation 4 | Y91K | F126C, C2205 | A43D | Q124C, C214S | Y91D | A43K |
| | Y91K | H168C, C2205 | A43D | T164C, C214S | Y91D | A43K |
| | Y91K | F170C, C2205 | A43D | T164C, C214S | Y91D | A43K |
| | Y91K | F170C, C2205 | A43D | S162C, C214S | Y91D | A43K |
| | Y91K | V173C, C2205 | A43D | S162C, C214S | Y91D | A43K |
| | Y91K | V173C, C2205 | A43D | Q160C, C214S | Y91D | A43K |
| | Y91K | L128C, C2205 | A43D | F118C, C2145 | Y91D | A43K |
| | Y91K | S134C, | A43D | F116C, | Y91D | A43K |
| | | C2205 | | C214S | | |
| | Y91K | S136C, C2205 | A43D | S114C, C214S | Y91D | A43K |
| | Y91K | P171C, C2205 | A43D | S162C, C214S | Y91D | A43K |
| | Y91K | T139C, C2205 | A43D | F116C, C214S | Y91D | A43K |
| | | | | | | |
| Combi nation 5 | Y91K | F126C, C2205 | P44D | Q124C, C214S | Y91D | P44K |
| | Y91K | H168C, C220S | P44D | T164C, C214S | Y91D | P44K |
| | Y91K | F170C, C220S | P44D | T164C, C214S | Y91D | P44K |
| | Y91K | F170C, C220S | P44D | S162C, C214S | Y91D | P44K |
| | Y91K | V173C, C220S | P44D | S162C, C214S | Y91D | P44K |
| | Y91K | V173C, C220S | P44D | Q160C, C214S | Y91D | P44K |
| | Y91K | L128C, C220S | P44D | F118C, C214S | Y91D | P44K |
| | Y91K | S134C, C2205 | P44D | F116C, C214S | Y91D | P44K |
| | Y91K | S136C, C2205 | P44D | S114C, C214S | Y91D | P44K |
| | Y91K | P171C, C220S | P44D | S162C, C214S | Y91D | P44K |
| | Y91K | T139C, C220S | P44D | F116C, C214S | Y91D | P44K |
∘

25. The antibody or antigen-binding fragment thereof, comprising one or a plurality of first antigen-binding regions that specifically bind to a first antigen and one or a plurality of second antigen-binding regions that specifically bind to a second antigen, wherein at least one of the antigen-binding regions comprise a heavy chain variable region and light chain variable region, an amino acid pair at the heavy chain variable and light chain variable region comprise a charge mutation that mutates the two amino acids on the amino acid pair into amino acids with opposite charges, respectively, wherein the position of the amino acid pair at the heavy chain variable region and light chain variable region interface is selected from: position 45 of the heavy chain variable region and position 87 of the light chain variable region, position 45 of the heavy chain variable region and position 44 of the light chain variable region, position 91 of the heavy chain variable region and position 43 of the light chain variable region, or position 91 of the heavy chain variable region and position 44 of the light chain variable region (Kabat number).

26. The antibody or antigen-binding fragment thereof according to Claim 25, wherein the position of the amino acid pair at the heavy chain variable region and light chain variable region interface in the at least one first antigen-binding region is the same as the position in the at least one second antigen-binding region, wherein the first antigen and second antigen are the same or different.

27. The antibody or antigen-binding fragment thereof according to Claim 26, wherein the mutated amino acid at the position of the amino acid pair in the first antigen-binding region has the opposite charge to the mutated amino acid at the same position in the second antigen-binding region.

28. The antibody or antigen-binding fragment thereof according to Claim 27, wherein
(1) in the first antigen-binding region, the amino acid at position 45 of the heavy chain variable region is substituted with an amino acid carrying a positive charge, and the amino acid at position 87 of the light chain variable region is substituted with an amino acid carrying a negative charge; and in the second antigen-binding region, the amino acid at position 45 of the heavy chain variable region is substituted with an amino acid carrying a negative charge, and the amino acid at position 87 of the light chain variable region is substituted with an amino acid carrying a positive charge;
(2) in the first antigen-binding region, the amino acid at position 45 of the heavy chain variable region is substituted with an amino acid carrying a positive charge, and the amino acid at position 44 of the light chain variable region is substituted with an amino acid carrying a negative charge; and in the second antigen-binding region, the amino acid at position 45 of the heavy chain variable region is substituted with an amino acid carrying a negative charge, and the amino acid at position 44 of the light chain variable region is substituted with an amino acid carrying a positive charge;
(3) in the first antigen-binding region, the amino acid at position 91 of the heavy chain variable region is substituted with an amino acid carrying a positive charge, and the amino acid at position 43 of the light chain variable region is substituted with an amino acid carrying a negative charge; and in the second antigen-binding region, the amino acid at position 91 of the heavy chain variable region is substituted with an amino acid carrying a negative charge, and the amino acid at position 43 of the light chain variable region is substituted with an amino acid carrying a positive charge; or
(4) in the first antigen-binding region, the amino acid at position 91 of the heavy chain variable region is substituted with an amino acid carrying a positive charge, and the amino acid at position 44 of the light chain variable region is substituted with an amino acid carrying a negative charge; and in the second antigen-binding region, the amino acid at position 91 of the heavy chain variable region is substituted with an amino acid carrying a negative charge, and the amino acid at position 44 of the light chain variable region is substituted with an amino acid carrying a positive charge.

29. The antibody or antigen-binding fragment thereof according to Claim 28, wherein the amino acid carrying a positive charge is selected from K, R, or H, and/or the amino acid carrying a negative charge is selected from E or D.

30. The antibody or antigen-binding fragment thereof according to Claim 29, wherein
(v). in the first antigen-binding region, the amino acid at position 45 of the heavy chain variable region is substituted with K, and the amino acid at position 87 of the light chain variable region is substituted with D, while in the second antigen-binding region, the amino acid at position 45 of the heavy chain variable region is substituted with D, and the amino acid at position 87 of the light chain variable region is substituted with K;
(vi). in the first antigen-binding region, the amino acid at position 45 of the heavy chain variable region is substituted with K, and the amino acid at position 44 of the light chain variable region is substituted with D, while in the second antigen-binding region, the amino acid at position 45 of the heavy chain variable region is substituted with D, and the amino acid at position 44 of the light chain variable region is substituted with K;
(vii). in the first antigen-binding region, the amino acid at position 91 of the heavy chain variable region is substituted with K, and the amino acid at position 43 of the light chain variable region is substituted with D, while in the second antigen-binding region, the amino acid at position 91 of the heavy chain variable region is substituted with D, and the amino acid at position 43 of the light chain variable region is substituted with K; or
(viii). in the first antigen-binding region, the amino acid at position 91 of the heavy chain variable region is substituted with K, and the amino acid at position 44 of the light chain variable region is substituted with D, while in the second antigen-binding region, the amino acid at position 91 of the heavy chain variable region is substituted with D, and the amino acid at position 44 of the light chain variable region is substituted with K.

31. The antibody or antigen-binding fragment thereof according to any one of Claims 1 - 30, wherein the first antigen-binding region comprises the same mutation, and the second antigen-binding region comprises the same mutation.

32. The antibody or antigen-binding fragment thereof according to any one of Claims 1 - 31, wherein the first antigen-binding region is a Fab fragment of the antibody, and/or the second antigen-binding region is the Fab fragment of the antibody.

33. The antibody or antigen-binding fragment thereof according to any one of Claims 1 - 32, wherein the light chain constant region that one or a plurality or all the first antigen-binding regions of the antibody comprise is a Kappa light chain constant region, and the light chain constant region that one or a plurality or all the second antigen-binding regions comprise is a Lambda light chain constant region, or wherein the light chain constant region that one or a plurality or all the first antigen-binding regions comprise is a Lambda light chain constant region, and the light chain constant region that one or a plurality or all the second antigen-binding regions comprise is a Kappa light chain constant region.

34. The antibody or antigen-binding fragment thereof according to any one of Claims 1 - 33, wherein the antibody also comprises a first Fc region and a second Fc region, wherein the first Fc region and the second Fc region are the same or different.

35. The antibody or antigen-binding fragment thereof according to Claim 34, wherein the first Fc region and the second Fc region are human IgG Fc, respectively, for example, human IgG1 Fc, human IgG2 Fc, human IgG3 Fc, or human IgG4 Fc, for example, it comprises or is composed of the amino acid sequence SEQ ID NO:122 or 123, or has at least 90% identity with it, for example, an amino acid sequence with 95%, 96%, 97%, 99%, or higher identity.

36. The multispecific antibody according to Claim 34 or 35, wherein based on the Knob-into-hole technology, a corresponding Knob mutation and Hole mutation are introduced into the first Fc region and the second Fc region.

37. The antibody or antigen-binding fragment thereof according to Claim 36, wherein
a) a polypeptide of one Fc region comprises mutation T366W, while a polypeptide of another Fc region comprises T366S, L368A, and Y407V (numbered based on the EU index), or
b) one Fc region comprises the amino acid substitutions S354C and T366W, and another Fc region comprises the amino acid substitutions Y349C, T366S, L368A, and Y407V (numbered based on the EU index).

38. The multispecific antibody according to Claim 36, wherein
a) a polypeptide of one Fc region comprises or is composed of the amino acid sequence shown in SEQ ID NO:125, while a polypeptide of another Fc region comprises or is composed of the amino acid sequence shown in SEQ ID NO:124;
b) a polypeptide of one Fc region comprises an amino acid sequence that has at least 85%, 90%, 95%, 96%, 97%, 98%, or 99% identity with the amino acid sequence shown in SEQ ID NO:125, while a polypeptide of another Fc region comprises an amino acid sequence that has at least 85%, 90%, 95%, 96%, 97%, 98%, or 99% identity with the amino acid sequence shown in SEQ ID NO:124;
c) a polypeptide of one Fc region comprises an amino acid sequence that has at least 85%, 90%, 95%, 96%, 97%, 98%, or 99% identity with the amino acid sequence shown in SEQ ID NO:125 and comprises mutations S354C and T366W, while a polypeptide of another Fc region comprises an amino acid sequence that has at least 85%, 90%, 95%, 96%, 97%, 98%, or 99% identity with the amino acid sequence shown in SEQ ID NO:124 and comprises mutations Y349C, T366S, L368A, and Y407V;
d) a polypeptide of one Fc region comprises or is composed of the amino acid sequence shown in SEQ ID NO:20 or 131, while a polypeptide of another Fc region comprises or is composed of the amino acid sequence shown in SEQ ID NO:101 or 130;
e) a polypeptide of one Fc region comprises an amino acid sequence that has at least 85%, 90%, 95%, 96%, 97%, 98%, or 99% identity with the amino acid sequence shown in SEQ ID NO:20 or 131, while a polypeptide of another Fc region comprises an amino acid sequence that has at least 85%, 90%, 95%, 96%, 97%, 98%, or 99% identity with the amino acid sequence shown in SEQ ID NO:101 or 130; or
f) a polypeptide of one Fc region comprises an amino acid sequence that has at least 85%, 90%, 95%, 96%, 97%, 98%, or 99% identity with the amino acid sequence shown in SEQ ID NO:20 or 131 and comprises mutation T366W, while a polypeptide of another Fc region comprises an amino acid sequence that has at least 85%, 90%, 95%, 96%, 97%, 98%, or 99% identity with the amino acid sequence shown in SEQ ID NO:101 or 130 and comprises T366S, L368A, and Y407V.

39. The antibody or antigen-binding fragment thereof according to Claim 34 or 35, wherein a mutation is introduced into the first Fc region and the second Fc region based on Innobody technology to promote heterodimerization of the first Fc region and the second Fc region.

40. The antibody or antigen-binding fragment thereof according to Claim 39, wherein the CH3 of one Fc region comprises S364R and D399K mutations, and the CH3 mutations of another Fc region comprise Y349T, K370S, and K409D mutations.

41. The antibody or antigen-binding fragment thereof according to Claim 40, wherein
a) a polypeptide of one Fc region comprises or is composed of the amino acid sequence shown in SEQ ID NO:126, while a polypeptide of another Fc region comprises or is composed of the amino acid sequence shown in SEQ ID NO:127;
b) a polypeptide of one Fc region comprises an amino acid sequence that has at least 85%, 90%, 95%, 96%, 97%, 98%, or 99% identity with the amino acid sequence shown in SEQ ID NO:126, while a polypeptide of another Fc region comprises an amino acid sequence that has at least 85%, 90%, 95%, 96%, 97%, 98%, or 99% identity with the amino acid sequence shown in SEQ ID NO:127; or
c) one Fc region comprises an amino acid sequence that has at least 85%, 90%, 95%, 96%, 97%, 98%, or 99% identity with the amino acid sequence shown in SEQ ID NO:126 and comprises mutations S364R and D399K, while a polypeptide of another Fc region comprises an amino acid sequence that has at least 85%, 90%, 95%, 96%, 97%, 98%, or 99% identity with the amino acid sequence shown in SEQ ID NO:127 and comprises mutations Y349T, K370S, and K409D.

42. The antibody or antigen-binding fragment thereof according to any one of Claims 34 - 41, wherein the first and/or second Fc region comprises a L234A/L235A mutation.

43. The antibody or antigen-binding fragment thereof according to any one of Claims 1 - 42, wherein the antigen bound to the antibody or antigen-binding fragment thereof is selected from one or a plurality of the following: CD3, BCMA, CD20, Her2, Her3, B7H3, EGFR, Hel, cMET, Axl, GPRC5D, immune checkpoint molecules such as PD-1, PD-L1, CD47, immune activation molecules such as 4-1BB, CD40, and OX40.

44. The antibody or antigen-binding fragment thereof according to any one of Claims 1 - 43, wherein the first antigen and second antigen are different and the antibody is a bispecific antibody.

45. The antibody or antigen-binding fragment thereof according to Claim 44, wherein the bispecific antibody binds to two antigens selected from the following: CD3/BCMA, CD3/CD20, CD3/Her2, Her2/Her3, B7H3/EGFR, PD-L1/CD47, PD-L1/CD40, PD-L1/4-1BB, EGFR/Her3, Her2/Hel, Her2/PD-1, Her2/PD-L1, Her2/CD47, Her2/cMet, or CD3/GPRC5D.

46. The antibody or antigen-binding fragment thereof according to Claim 44 or 45, wherein the bispecific antibody of the present disclosure comprises a first antigen-binding region that specifically binds to a first antigen and a second antigen-binding region that specifically binds to a second antigen, wherein the first antigen-binding region comprises a First Fab, the second antigen-binding region comprises a Second Fab, wherein the First Fab is linked to the N-terminus of the first Fc region at the C-terminus of the CH1 thereof (with or without a linker, for example, a hinge region), and the Second Fab is linked to the N-terminus of the second Fc region at the C-terminus of the CH1 thereof (with or without a linker, for example, a hinge region).

47. The antibody or antigen-binding fragment thereof according to Claim 46, wherein the bispecific antibody is an IgG-like antibody with the configuration shown in Figure 1.

48. The antibody or antigen-binding fragment thereof according to Claim 46 or 47, comprising Heavy chain 1: comprises or is composed of the following from the N-terminus to the C-terminus: the First Fab heavy chain variable region-heavy chain constant region CH1-first Fc region, wherein the heavy chain constant region CH1 is linked to the N-terminus of the first Fc region at the C-terminus thereof with or without a linker (for example, a hinge region);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-second Fc region, wherein the heavy chain constant region CH1 is linked to the N-terminus of the second Fc region at the C-terminus thereof with or without a linker (for example, a hinge region);
Light chain 2: comprises or is composed of the following from the N-terminus to the C-terminus: the Second Fab light chain variable region-light chain constant region.

49. The antibody or antigen-binding fragment thereof according to Claim 48; wherein
(1) the first antigen-binding region specifically binds to Her2, wherein heavy chain 1 and light chain 1 comprise the amino acid sequences shown in the following SEQ ID NO:, respectively,or comprise amino acid sequences that have at least 85%, 90%, 95%, 96%, 97%, 98%, or 99% identity with the amino acid sequences shown, or are composed of the sequences shown in the following SEQ ID NO:
i) SEQ ID NO:43 and SEQ ID NO:45;
ii) SEQ ID NO:48 and SEQ ID NO:49;
iii) SEQ ID NO:21 and SEQ ID NO:22;
iv) SEQ ID NO:25 and SEQ ID NO:26;
v) SEQ ID NO:25 and SEQ ID NO:29;
vi) SEQ ID NO:31 and SEQ ID NO:32; or
vii) SEQ ID NO:31 and SEQ ID NO:29;
and/or
(2) the second antigen-binding region specifically binds to Hel, and heavy chain 2 and light chain 2 comprise the following SEQ ID NO:, respectively:or comprise amino acid sequences that have at least 85%, 90%, 95%, 96%, 97%, 98%, or 99% identity with the amino acid sequences shown, or are composed of the sequences shown in the following SEQ ID NO:
i) SEQ ID NO:41 and SEQ ID NO:42;
ii) SEQ ID NO:23 and SEQ ID NO:24;
iii) SEQ ID NO:27 and SEQ ID NO:28;
iv) SEQ ID NO:27 and SEQ ID NO:30;
v) SEQ ID NO:33 and SEQ ID NO:34; or
vi) SEQ ID NO:33 and SEQ ID NO:30;
(2)-1 The second antigen-binding region specifically binds to PD-1, and heavy chain 2 and light chain 2 comprise the following SEQ ID NO:, respectively; or comprise amino acid sequences that have at least 85%, 90%, 95%, 96%, 97%, 98%, or 99% identity with the amino acid sequences shown, or are composed of the sequences shown in the following SEQ ID NO: SEQ ID NO:46 and SEQ ID NO:47;
(2)-2 The second antigen-binding region specifically binds to CD47, and heavy chain 2 and light chain 2 comprise the following SEQ ID NO:, respectively; or comprise amino acid sequences that have at least 85%, 90%, 95%, 96%, 97%, 98%, or 99% identity with the amino acid sequences shown, or are composed of the sequences shown in the following SEQ ID NO: SEQ ID NO:50 and SEQ ID NO:51;
(3)-2 The second antigen-binding region specifically binds to cMet, and heavy chain 2 and light chain 2 comprise the following SEQ ID NO:, respectively; or comprise amino acid sequences that have at least 85%, 90%, 95%, 96%, 97%, 98%, or 99% identity with the amino acid sequences shown, or are composed of the sequences shown in the following SEQ ID NO: SEQ ID NO:52 and SEQ ID NO:53.

50. The multispecific antibody according to Claim 44 or 45, wherein the bispecific antibody comprises one first antigen-binding region that specifically binds to a first antigen and two second antigen-binding regions that specifically bind to a second antigen, wherein the first antigen-binding region comprises a First Fab, the second antigen-binding region comprises a Second Fab, wherein one second Fab is linked to the N-terminus of the first Fc region at the C-terminus of the CH1 thereof (with or without a linker, for example, a hinge region), one first Fab is linked to the N-terminus of the second Fc region at the C-terminus of the CH1 thereof (with or without a linker, for example, a hinge region), and the other second Fab is linked to the N-terminus of the First Fab heavy chain variable region at the C-terminus of the CH1 thereof (with or without a linker).

51. The antibody or antigen-binding fragment thereof according to Claim 50, wherein the bispecific antibody has a 2+1N-terminal configuration with the configuration shown in Figure 5A.

52. The antibody or antigen-binding fragment thereof according to Claim 51, wherein the bispecific antibody comprises
Heavy chain 1: comprises or is composed of the following from the N-terminus to the C-terminus: the Second Fab heavy chain variable region-heavy chain constant region CH1-First Fab heavy chain variable region-heavy chain constant region CH1-second Fc region, wherein the First Fab heavy chain constant region CH1 is linked to the N-terminus of the second Fc region at the C-terminus thereof with or without a linker (for example, a hinge region), and the Second Fab is linked to the N-terminus of the First Fab heavy chain variable region of at the C-terminus of the CH1 thereof (with or without a linker);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-first Fc region, wherein the heavy chain constant region CH1 is linked to the N-terminus of the first Fc region at the C-terminus thereof with or without a linker (for example, a hinge region);
Light chain 2: comprises or is composed of the following from the N-terminus to the C-terminus: the Second Fab light chain variable region-light chain constant region,
wherein the bispecific antibody comprises two light chains 2.

53. The antibody or antigen-binding fragment thereof according to Claim 52, wherein the first antigen-binding region binds to CD3, and the second antigen-binding region binds to GPRC5D, for example, wherein
1) heavy chain 1 comprises the amino acid sequence shown in SEQ ID NO:58, or comprises or is composed of an amino acid sequence with at least 85%, 90%, 95%, 96%, 97%, 98%, or 99% identity with the amino acid sequence shown in SEQ ID NO:58;
light chain 1 comprises the amino acid sequence shown in SEQ ID NO:59, or comprises or is composed of an amino acid sequence with at least 85%, 90%, 95%, 96%, 97%, 98%, or 99% identity with the amino acid sequence shown in SEQ ID NO:59;
heavy chain 2 comprises the amino acid sequence shown in SEQ ID NO:57, or comprises or is composed of an amino acid sequence with at least 85%, 90%, 95%, 96%, 97%, 98%, or 99% identity with the amino acid sequence shown in SEQ ID NO:57; and/or
light chain 2 comprises the amino acid sequence shown in SEQ ID NO:56, or comprises or is composed of an amino acid sequence with at least 85%, 90%, 95%, 96%, 97%, 98%, or 99% identity with the amino acid sequence shown in SEQ ID NO:56; and/or
2) heavy chain 1 comprises the amino acid sequence shown in SEQ ID NO:62, or comprises or is composed of an amino acid sequence with at least 85%, 90%, 95%, 96%, 97%, 98%, or 99% identity with the amino acid sequence shown in SEQ ID NO:62;
light chain 1 comprises the amino acid sequence shown in SEQ ID NO:63, or comprises or is composed of an amino acid sequence with at least 85%, 90%, 95%, 96%, 97%, 98%, or 99% identity with the amino acid sequence shown in SEQ ID NO:63;
heavy chain 2 comprises the amino acid sequence shown in SEQ ID NO:61, or comprises or is composed of an amino acid sequence with at least 85%, 90%, 95%, 96%, 97%, 98%, or 99% identity with the amino acid sequence shown in SEQ ID NO:61; and/or
light chain 2 comprises the amino acid sequence shown in SEQ ID NO:60, or comprises or is composed of an amino acid sequence with at least 85%, 90%, 95%, 96%, 97%, 98%, or 99% identity with the amino acid sequence shown in SEQ ID NO:60.

54. The antibody or antigen-binding fragment thereof according to Claim 44 or 45, wherein the bispecific antibody of the present disclosure comprises one first antigen-binding region that specifically binds to a first antigen and two second antigen-binding regions that specifically bind to a second antigen, wherein the first antigen-binding region comprises one First Fab, the second antigen-binding region comprises one Second Fab, wherein the two Second Fabs are linked to the N-terminus of the first Fc region and the N-terminus of the second Fc region, respectively, at the C-terminus of the CH1 thereof (with or without a linker, for example, a hinge region), and the N-terminus of the First Fab heavy chain variable region is linked to the C-terminus of the second Fc region (with or without a linker).

55. The antibody or antigen-binding fragment thereof according to Claim 54, wherein the bispecific antibody has a 2+1C-terminal configuration with the configuration shown in Figure 5B.

56. The antibody or antigen-binding fragment thereof according to Claim 55, wherein the bispecific antibody comprises
Heavy chain 1: comprises or is composed of the following from the N-terminus to the C-terminus: the Second Fab heavy chain variable region-heavy chain constant region CH1-second Fc region-First Fab heavy chain variable region-heavy chain constant region CH1, wherein the Second Fab is linked to the N-terminus of the second Fc region at the C-terminus of the CH1 thereof (with or without a linker, for example, a hinge region), and the N-terminus of the First Fab heavy chain variable region is linked to the C-terminus of the second Fc region (with or without a linker);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-first Fc region, wherein the heavy chain constant region CH1 is linked to the N-terminus of the first Fc region at the C-terminus thereof with or without a linker (for example, a hinge region);
Light chain 2: comprises or is composed of the following from the N-terminus to the C-terminus: the Second Fab light chain variable region-light chain constant region,
wherein the bispecific antibody comprises two light chains 2.

57. The antibody or antigen-binding fragment thereof according to Claim 56, wherein the first antigen-binding region binds to CD3, and the second antigen-binding region binds to GPRC5D, for example, wherein
1) heavy chain 1 comprises the amino acid sequence shown in SEQ ID NO:64, or comprises or is composed of an amino acid sequence with at least 85%, 90%, 95%, 96%, 97%, 98%, or 99% identity with the amino acid sequence shown in SEQ ID NO:64;
light chain 1 comprises the amino acid sequence shown in SEQ ID NO:59, or comprises or is composed of an amino acid sequence with at least 85%, 90%, 95%, 96%, 97%, 98%, or 99% identity with the amino acid sequence shown in SEQ ID NO:59;
heavy chain 2 comprises the amino acid sequence shown in SEQ ID NO:57, or comprises or is composed of an amino acid sequence with at least 85%, 90%, 95%, 96%, 97%, 98%, or 99% identity with the amino acid sequence shown in SEQ ID NO:57; and/or
light chain 2 comprises the amino acid sequence shown in SEQ ID NO:56, or comprises or is composed of an amino acid sequence with at least 85%, 90%, 95%, 96%, 97%, 98%, or 99% identity with the amino acid sequence shown in SEQ ID NO:56; and/or
2) heavy chain 1 comprises the amino acid sequence shown in SEQ ID NO:65, or comprises or is composed of an amino acid sequence with at least 85%, 90%, 95%, 96%, 97%, 98%, or 99% identity with the amino acid sequence shown in SEQ ID NO:65;
light chain 1 comprises the amino acid sequence shown in SEQ ID NO:63, or comprises or is composed of an amino acid sequence with at least 85%, 90%, 95%, 96%, 97%, 98%, or 99% identity with the amino acid sequence shown in SEQ ID NO:63;
heavy chain 2 comprises the amino acid sequence shown in SEQ ID NO:61, or comprises or is composed of an amino acid sequence with at least 85%, 90%, 95%, 96%, 97%, 98%, or 99% identity with the amino acid sequence shown in SEQ ID NO:61; and/or
light chain 2 comprises the amino acid sequence shown in SEQ ID NO:60, or comprises or is composed of an amino acid sequence with at least 85%, 90%, 95%, 96%, 97%, 98%, or 99% identity with the amino acid sequence shown in SEQ ID NO:60.

58. The antibody or antigen-binding fragment thereof according to Claim 44 or 45, wherein the bispecific antibody of the present disclosure comprises two first antigen-binding regions that specifically bind to a first antigen and two second antigen-binding regions that specifically bind to a second antigen, wherein the first antigen-binding region comprises a First Fab, the second antigen-binding region comprises a Second Fab, wherein the two Second Fabs are linked to the N-terminus of the 2 Fc regions at the C-terminus of the CH1 thereof (with or without a linker, for example, a hinge region) (the Fc region may be the same or different), and the N-terminus of the heavy chain variable region of the two First Fabs is linked to the C-terminus of the two Fc regions (with or without a linker).

59. The antibody or antigen-binding fragment thereof according to Claim 58, wherein the bispecific antibody has a 2+2C-terminal configuration with the configuration shown in Figure 5C.

60. The antibody or antigen-binding fragment thereof according to Claim 59, wherein the bispecific antibody comprises
Heavy chain: comprises or is composed of the following from the N-terminus to the C-terminus: the Second Fab heavy chain variable region-heavy chain constant region CH1 -Fc region- First Fab heavy chain variable region- heavy chain constant region CH1, wherein the Second Fab is linked to the N-terminus of the Fc region at the C-terminus of the CH1 thereof (with or without a linker, for example, a hinge region), and the N-terminus of the First Fab heavy chain variable region is linked to the C-terminus of the Fc region (with or without a linker).
Light chain 1: comprises or is composed of the following from the N-terminus to the C-terminus: the First Fab light chain variable region-light chain constant region;
Light chain 2: comprises or is composed of the following from the N-terminus to the C-terminus: the Second Fab light chain variable region-light chain constant region,
wherein the bispecific antibody comprises two heavy chains.

61. The antibody or antigen-binding fragment thereof according to Claim 60, wherein the first antigen-binding region binds to BD3, and the second antigen-binding region binds to GPRC5D, for example, wherein
1) the heavy chain comprises the amino acid sequence shown in SEQ ID NO:66, or comprises or is composed of an amino acid sequence with at least 85%, 90%, 95%, 96%, 97%, 98%, or 99% identity with the amino acid sequence shown in SEQ ID NO:66;
light chain 1 comprises the amino acid sequence shown in SEQ ID NO:59, or comprises or is composed of an amino acid sequence with at least 85%, 90%, 95%, 96%, 97%, 98%, or 99% identity with the amino acid sequence shown in SEQ ID NO:59; and/or
light chain 2 comprises the amino acid sequence shown in SEQ ID NO:56, or comprises or is composed of an amino acid sequence with at least 85%, 90%, 95%, 96%, 97%, 98%, or 99% identity with the amino acid sequence shown in SEQ ID NO:56; and/or
2) the heavy chain comprises the amino acid sequence shown in SEQ ID NO:67, or comprises or is composed of an amino acid sequence with at least 85%, 90%, 95%, 96%, 97%, 98%, or 99% identity with the amino acid sequence shown in SEQ ID NO:67;
light chain 1 comprises the amino acid sequence shown in SEQ ID NO:63, or comprises or is composed of an amino acid sequence with at least 85%, 90%, 95%, 96%, 97%, 98%, or 99% identity with the amino acid sequence shown in SEQ ID NO:63; and/or
light chain 2 comprises the amino acid sequence shown in SEQ ID NO:60, or comprises or is composed of an amino acid sequence with at least 85%, 90%, 95%, 96%, 97%, 98%, or 99% identity with the amino acid sequence shown in SEQ ID NO:60.

62. The antibody or antigen-binding fragment thereof according to any one of Claims 1 - 43, wherein the first antigen and second antigen are different, and the antibody also comprises an antigen-binding region that specifically binds to one or a plurality of other antigens.

63. The antibody or antigen-binding fragment thereof according to Claim 62, wherein the antibody is a trispecific antibody.

64. The antibody or antigen-binding fragment thereof according to Claim 63, wherein the trispecific antibody of the present disclosure comprises a first antigen-binding region that specifically binds to a first antigen, a second antigen-binding region that specifically binds to a second antigen, and a third antigen-binding region that specifically binds to a third antigen, wherein the first antigen-binding region comprises a First Fab, the second antigen-binding region comprises a Second Fab, wherein the First Fab is linked to the N-terminus of the first Fc region at the C-terminus of the CH1 thereof (with or without a linker, for example, a hinge region), the Second Fab is linked to the N-terminus of the second Fc region at the C-terminus of the CH1 thereof (with or without a linker, for example, a hinge region), the third antigen-binding region comprises a scFv (for example, VH-VL, or VL-VH) that is linked to the C-terminus of the second Fc region at the N-terminus of the VH or VL thereof (for example, scFv is VL-VH, for example, the N-terminus of VL) with or without a linker.

65. The antibody or antigen-binding fragment thereof according to Claim 64, wherein the configuration of the trispecific antibody is a 2+1C-terminal configuration, as shown in Figure 7A or B.

66. The antibody or antigen-binding fragment thereof according to Claim 65, wherein the multispecific antibody comprises
Heavy chain 1: comprises or is composed of the following from the N-terminus to the C-terminus: the First Fab heavy chain variable region-heavy chain constant region CH1-first Fc region, wherein the First Fab is linked to the N-terminus of the first Fc region at the C-terminus of the CH1 thereof (with or without a linker, for example, a hinge region);
Light chain 1: comprises or is composed of the following from the N-terminus to the C-terminus: the First Fab light chain variable region-light chain constant region;
Heavy chain 2: comprises or is composed of the following from the N-terminus to the C-terminus: the Second Fab heavy chain variable region-heavy chain constant region CH1-second Fc region -scFv, wherein the heavy chain constant region CH1 is linked to the N-terminus of the second Fc region at the C-terminus of the heavy chain constant region CH1 with or without a linker (for example, a hinge region), and scFv is linked to the C-terminus of the second Fc region at the N-terminus or C-terminus of the VH or VL thereof (for example, scFv is VL-VH, for example, the N-terminus of VL) with or without a linker.
Light chain 2: comprises or is composed of the following from the N-terminus to the C-terminus: the Second Fab light chain variable region-light chain constant region.

67. The antibody or antigen-binding fragment thereof according to Claim 66, wherein the first antigen-binding region specifically binds to GPRC5D, the second antigen-binding region specifically binds to CD3, and/or the third antigen-binding region specifically binds to BCMA, for example, wherein
heavy chain 1 comprises the amino acid sequence shown in SEQ ID NO:71, or comprises or is composed of an amino acid sequence with at least 85%, 90%, 95%, 96%, 97%, 98%, or 99% identity with the amino acid sequence shown in SEQ ID NO:71;
light chain 1 comprises the amino acid sequence shown in SEQ ID NO:72, or comprises or is composed of an amino acid sequence with at least 85%, 90%, 95%, 96%, 97%, 98%, or 99% identity with the amino acid sequence shown in SEQ ID NO:72;
heavy chain 2 comprises the amino acid sequence shown in SEQ ID NO:73, or comprises or is composed of an amino acid sequence with at least 85%, 90%, 95%, 96%, 97%, 98%, or 99% identity with the amino acid sequence shown in SEQ ID NO:73; and/or
light chain 2 comprises the amino acid sequence shown in SEQ ID NO:74, or comprises or is composed of an amino acid sequence with at least 85%, 90%, 95%, 96%, 97%, 98%, or 99% identity with the amino acid sequence shown in SEQ ID NO:74.

68. A nucleic acid, coding the antibody or antigen-binding fragment thereof according to any one of Claims 1 - 67, or any or a plurality of chains thereof.

69. An expression vector, comprising the nucleic acid according to Claim 68.

70. A host cell, comprising the nucleic acid according to Claim 68 or the expression vector according to Claim 69.

71. A method for preparing the antibody or antigen-binding fragment thereof according to any one of Claims 1 - 67, comprising
a) introducing the nucleic acid coding the various chains of the antibody or antigen-binding fragment thereof into the host cell;
b) expressing in the host cell and assembling the antibody or antigen-binding fragment thereof;
optionally, the antibody or antigen-binding fragment thereof is purified, for example, purified by Protein A.

72. An immunoconjugate, comprising the antibody or antigen-binding fragment thereof according to any one of Claims 1 - 67.

73. A fusion protein, comprising the antibody or antigen-binding fragment thereof according to any one of Claims 1 - 67.

74. A T cell receptor, comprising the antibody or antigen-binding fragment thereof according to any one of Claims 1 - 67.
